# EUROPÄISCHE PATENTSCHRIFT

(11) **EP 2 852 573 B1**
(45) Veröffentlichungstag und Bekanntmachung des Hinweises auf die Patenterteilung: **01.02.2017**
(21) Anmeldenummer: 13722773.2
(22) Anmeldetag: 17.05.2013
(51) Int. Cl.: C07D 211/06, C07D 211/14, C07D 211/18, C07D 211/46, C07D 217/04, C07D 221/20, C07D 295/15, A61K 31/438, A61K 31/445, A61K 31/4453, A61K 31/451, A61K 31/472, A61P 9/00

(54) **N-[3-(2-CARBOXYETHYL)PHENYL]-PIPERIDIN-1-YLACETAMID- DERIVATE UND IHRE VERWENDUNG ALS AKTIVATOREN DER LÖSLICHEN GUANYLATCYCLASE**
N-[3-(2-CARBOXYETHYL)PHENYL]-PIPERIDIN-1-YL ACETAMIDE DERIVATIVES AND THEIR USE AS ACTIVATORS OF SOLUBLE GUANYLATE CYCLASE
DÉRIVÉS DE N-[3-(2-CARBOXYETHYL)PHÉNYL]-PIPÉRIDIN-1-YL ACÉTAMIDE ET LEUR UTILISATION COMME ACTIVATEURS DE GUANYLATE CYCLASE SOLUBLE

(30) Priorität: 22.05.2012 DE 102012208530
(43) Veröffentlichungstag der Anmeldung: 01.04.2015
(73) Patentinhaber: Bayer Pharma Aktiengesellschaft, 13353 Berlin (DE)
(72) Erfinder: LAMPE, Thomas, 40545 Düsseldorf (DE); HAHN, Michael, 40764 Langenfeld (DE); STASCH, Johannes-Peter, 42651 Solingen (DE); SCHLEMMER, Karl-Heinz, 42113 Wuppertal (DE); WUNDER, Frank, 42117 Wuppertal (DE); EL SHEIKH, Sherif, 51107 Köln (DE); LI, Volkhart, Min-Jian, 42553 Velbert (DE); BECKER-PELSTER, Eva Maria, 42327 Wuppertal (DE); STOLL, Friederike, 40215 Düsseldorf (DE); KNORR, Andreas, 40699 Erkrath (DE)
(74) Vertreter: BIP Patents
(86) Internationale Anmeldenummer: PCT/EP2013/060222
(87) Internationale Veröffentlichungsnummer: WO 2013/174736

(56) Entgegenhaltungen:
- WO-A1-2011/051165

## Beschreibung

Die vorliegende Anmeldung betrifft neue, substituierte 2-(Piperidin-1-yl)acetamid-Derivate, Verfahren zu ihrer Herstellung, ihre Verwendung zur Behandlung und/oder Prävention von Krankheiten sowie ihre Verwendung zur Herstellung von Arzneimitteln zur Behandlung und/oder Prävention von Krankheiten, insbesondere zur Behandlung und/oder Prävention kardiovaskulärer Erkrankungen.

Eines der wichtigsten zellulären Übertragungssysteme in Säugerzellen ist das cyclische Guanosinmonophosphat (cGMP). Zusammen mit Stickstoffmonoxid (NO), das aus dem Endothel freigesetzt wird und hormonelle und mechanische Signale überträgt, bildet es das NO/cGMP-System. Die Guanylatcyclasen katalysieren die Biosynthese von cGMP aus Guanosintriphosphat (GTP). Die bisher bekannten Vertreter dieser Familie lassen sich sowohl nach strukturellen Merkmalen als auch nach der Art der Liganden in zwei Gruppen aufteilen: Die partikulären, durch natriuretische Peptide stimulierbaren Guanylatcyclasen und die löslichen, durch NO stimulierbaren Guanylatcyclasen. Die löslichen Guanylatcyclasen bestehen aus zwei Untereinheiten und enthalten ein Häm pro Heterodimer, das ein Teil des regulatorischen Zentrums ist. Dieses hat eine zentrale Bedeutung für den Aktivierungsmechanismus. NO kann an das Eisenatom des Häms binden und so die Aktivität des Enzyms deutlich erhöhen. Hämfreie Präparationen lassen sich hingegen nicht durch NO stimulieren. Auch Kohlenmonoxid (CO) ist in der Lage, am Eisen-Zentralatom des Häms anzugreifen, wobei die Stimulierung durch CO deutlich geringer ist als die durch NO.

Durch die Bildung von cGMP und der daraus resultierenden Regulation von Phosphodiesterasen, Ionenkanälen und Proteinkinasen spielt die Guanylatcyclase eine entscheidende Rolle bei unterschiedlichen physiologischen Prozessen, insbesondere bei der Relaxation und Proliferation glatter Muskelzellen, der Plättchenaggregation und -adhäsion und der neuronalen Signalübertragung sowie bei Erkrankungen, welche auf einer Störung der vorstehend genannten Vorgänge beruhen. Unter pathophysiologischen Bedingungen kann das NO/cGMP-System supprimiert sein, was zum Beispiel zu Bluthochdruck, einer Plättchenaktivierung, einer vermehrten Zellproliferation, endothelialer Dysfunktion, Atherosklerose, Angina pectoris, Herzinsuffizienz, Thrombosen, Schlaganfall und Myokardinfarkt führen kann.

Eine auf die Beeinflussung des cGMP-Signalweges in Organismen abzielende NO-unabhängige Behandlungsmöglichkeit für derartige Erkrankungen ist aufgrund der zu erwartenden hohen Effizienz und geringen Nebenwirkungen ein vielversprechender Ansatz.

Zur therapeutischen Stimulation der löslichen Guanylatcyclase wurden bisher ausschließlich Verbindungen wie organische Nitrate verwendet, deren Wirkung auf NO beruht. Dieses wird durch Biokonversion gebildet und aktiviert die lösliche Guanylatcyclase durch Angriffe am Eisen-Zentralatom des Häms. Neben den Nebenwirkungen gehört die Toleranzentwicklung zu den entscheidenden Nachteilen dieser Behandlungsweise [O.V. Evgenov et al., Nature Rev. Drug Disc. 5 (2006), 755].

In den letzten Jahren wurden Substanzen identifiziert, die die lösliche Guanylatcyclase direkt, d.h. ohne vorherige Freisetzung von NO stimulieren. Mit dem Indazolderivat YC-1 wurde erstmals ein NO-unabhängiger, jedoch Häm-abhängiger Stimulator der sGC beschrieben [Evgenov et al., *ibid*.]. Ausgehend von YC-1 wurden weitere Substanzen gefunden, welche eine höhere Potenz als YC-1 besitzen und keine relevante Hemmung von Phosphodiesterasen (PDE) aufweisen. Dies führte zur Identifizierung der Pyrazolopyridin-Derivate BAY 41-2272, BAY 41-8543 und BAY 63-2521. Diese Verbindungen bilden gemeinsam mit den kürzlich publizierten, strukturell diversen Substanzen CMF-1571 und A-350619 die neue Klasse der sGC-Stimulatoren [Evgenov et al., *ibid*.]. Gemeinsames Charakteristikum dieser Substanzklasse ist eine NO-unabhängige und selektive Aktivierung der hämhaltigen sGC. Darüber hinaus zeigen die sGC-Stimulatoren in Kombination mit NO einen synergistischen Effekt auf die sGC-Aktivierung, welcher auf einer Stabilisierung des Nitrosyl-Häm-Komplexes basiert. Die genaue Bindungsstelle der sGC-Stimulatoren an der sGC ist bis heute Gegenstand der Diskussion. Entfernt man von der löslichen Guanylatcyclase die HämGruppe, zeigt das Enzym immer noch eine nachweisbare katalytische Basalaktivität, d.h. es wird nach wie vor cGMP gebildet. Die verbleibende katalytische Basalaktivität des Häm-freien Enzyms ist durch keinen der vorstehend genannten Stimulatoren stimulierbar [Evgenov et al., *ibid*.].

Darüber hinaus wurden NO- und Häm-unabhängige sGC-Aktivatoren, mit BAY 58-2667 als Prototyp dieser Klasse, identifiziert. Gemeinsame Charakteristika dieser Substanzen sind, dass sie in Kombination mit NO nur einen additiven Effekt auf die Enzymaktivierung ausüben, und dass die Aktivierung des oxidierten oder hämfreien Enzyms im Vergleich zum hämhaltigen Enzym deutlich stärker ist [Evgenov et al., *ibid*.; J.P. Stasch et al., Br. J. Pharmacol. 136 (2002), 773; J.P. Stasch et al., J. Clin. Invest. 116 (2006), 2552]. Spektroskopische Untersuchungen lassen erkennen, dass BAY 58-2667 die oxidierte Hämgruppe verdrängt, die durch Schwächung der Eisen-Histidin-Bindung nur schwach an der sGC gebunden ist. Auch wurde gezeigt, dass das charakteristische sGC-Hämbindungsmotiv Tyr-x-Ser-x-Arg sowohl für die Interaktion der negativ geladenen Propionsäuren der Hämgruppe als auch für die Wirkung von BAY 58-2667 zwingend erforderlich ist. Vor diesem Hintergrund wird angenommen, dass die Bindungsstelle von BAY 58-2667 an der sGC identisch zur Bindungsstelle der Hämgruppe ist [J.P. Stasch et al., J. Clin. Invest. 116 (2006), 2552].

Die in der vorliegenden Erfindung beschriebenen Verbindungen sind nun ebenfalls in der Lage, die Häm-freie Form der löslichen Guanylatcyclase zu aktivieren. Dies wird auch dadurch belegt, dass diese neuartigen Aktivatoren einerseits am Häm-haltigen Enzym keine synergistische Wirkung mit NO zeigen und andererseits sich ihre Wirkung nicht durch den Häm-abhängigen Inhibitor der löslichen Guanylatcyclase, 1*H*-1,2,4-Oxadiazolo[4,3-a]chinoxalin-1-on (ODQ), blockieren lässt, sondern durch diesen sogar potenziert wird [vgl. O.V. Evgenov et al., Nature Rev. Drug Disc. 5 (2006), 755; J.P. Stasch et al., J. Clin. Invest. 116 (2006), 2552].

Aufgabe der vorliegenden Erfindung war somit die Bereitstellung neuer Verbindungen, welche in der oben beschriebenen Weise als Aktivatoren der löslichen Guanylatcyclase wirken und als solche insbesondere zur Behandlung und zur Prävention kardiovaskulärer Erkrankungen eingesetzt werden können.

In WO 94/12181-A1 werden substituierte Arylverbindungen als Fibrinogen-Rezeptorantagonisten zur Prävention von Thrombosen und Embolien offenbart, und in EP 0 638 553-A1 werden Carbonsäureamide mit terminaler Carboxylgruppe als antithrombotische Wirkstoffe beschrieben. Aus WO 02/36553-A2 sind substituierte Phenylalkancarbonsäuren als Inhibitoren der Zelladhäsion bekannt. In WO 2006/066948-A1 werden Piperidin-Derivate mit anti-inflammatorischer Wirkung beschrieben. In EP 1 939 189-A1 werden multicyclische Verbindungen mit PPAR-agonistischer Aktivität zur Behandlung von Erkrankungen des Lipidstoffwechsels beansprucht. In WO 2011/ 051165-A1 werden Phenylacetamido-substituierte 3-Phenylpropionsäuren offenbart, die als Aktivatoren der löslichen Guanylatcyclase wirken.

Gegenstand der vorliegenden Erfindung sind Verbindungen der allgemeinen Formel (I) 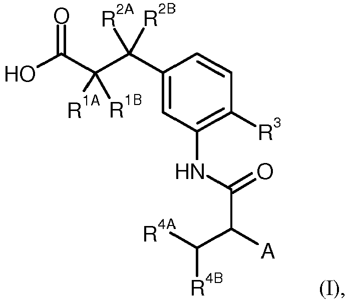 in welcher
- R^{1A} und R^{1B}: unabhängig voneinander für Wasserstoff, Methyl, Ethyl oder *n*-Propyl stehen oder
miteinander verknüpft sind und gemeinsam mit dem Kohlenstoffatom, an das sie gebunden sind, einen Cyclopropyl- oder Cyclobutyl-Ring bilden,
- R^{2A}: für Wasserstoff, Methyl, Ethyl, *n*-Propyl, Isopropyl, Cyclopropyl, Methoxy, Ethoxy oder Cyclopropyloxy steht,
- R^{2B}: für Wasserstoff oder Methyl steht,

oder
- R^{2A} und R^{2B}: miteinander verknüpft sind und gemeinsam mit dem Kohlenstoffatom, an das sie gebunden sind, einen Cyclopropyl- oder Cyclobutyl-Ring bilden,
- R³: für Wasserstoff, Fluor, Chlor, Methyl, Trifluormethyl oder Ethyl steht,
- R^{4A} und R^{4B}: unabhängig voneinander für Methyl, Trifluormethyl oder Ethyl stehen oder
miteinander verknüpft sind und gemeinsam mit dem Kohlenstoffatom, an das sie gebunden sind, einen Cyclopropyl-, Cyclobutyl- oder Cyclopentyl-Ring bilden, der bis zu zweifach mit Fluor substituiert sein kann,
und
- A: für einen optional substituierten oder anellierten Piperidin-Ring der Formel 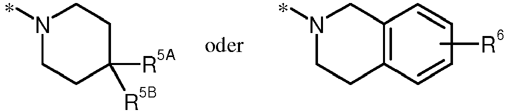 steht, worin
* die Verknüpfungsstelle mit dem Rest des Moleküls bezeichnet,
R^{5A} Wasserstoff, (C₁-C₄)-Alkyl, Cyclopropyl, Cyclobutyl, Methoxy, Ethoxy oder Phenyl bedeutet, wobei (C₁-C₄)-Alkyl seinerseits bis zu dreifach mit Fluor substituiert sein kann,
R^{5B} Wasserstoff oder Methyl bedeutet, oder
- R^{5A} und R^{5B}: miteinander verknüpft sind und gemeinsam mit dem Kohlenstoffatom, an das sie gebunden sind, einen Cyclopropyl- oder Cyclobutyl-Ring bilden,
und
- R⁶: Wasserstoff, Fluor, Chlor, Methyl oder Trifluormethyl bedeutet,
sowie ihre Salze, Solvate und Solvate der Salze.

Erfindungsgemäße Verbindungen sind die Verbindungen der Formel (I) und deren Salze, Solvate und Solvate der Salze, die von Formel (I) umfassten Verbindungen der nachfolgend genannten Formeln und deren Salze, Solvate und Solvate der Salze sowie die von Formel (I) umfassten, nachfolgend als Ausführungsbeispiele genannten Verbindungen und deren Salze, Solvate und Solvate der Salze, soweit es sich bei den von Formel (I) umfassten, nachfolgend genannten Verbindungen nicht bereits um Salze, Solvate und Solvate der Salze handelt.

Als Salze sind im Rahmen der vorliegenden Erfindung physiologisch unbedenkliche Salze der erfindungsgemäßen Verbindungen bevorzugt. Umfasst sind auch Salze, die für pharmazeutische Anwendungen selbst nicht geeignet sind, jedoch beispielsweise für die Isolierung, Reinigung oder Lagerung der erfindungsgemäßen Verbindungen verwendet werden können.

Physiologisch unbedenkliche Salze der erfindungsgemäßen Verbindungen umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Benzolsulfonsäure, Toluolsulfonsäure, Naphthalindisulfonsäure, Ameisensäure, Essigsäure, Trifluoressigsäure, Propionsäure, Milchsäure, Weinsäure, Äpfelsäure, Zitronensäure, Fumarsäure, Maleinsäure und Benzoesäure.

Physiologisch unbedenkliche Salze der erfindungsgemäßen Verbindungen umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, *N*,*N*-Diisopropylethylamin, Monoethanolamin, Diethanolamin, Triethanolamin, Dimethylaminoethanol, Diethylaminoethanol, Procain, Dicyclohexylamin, Dibenzylamin, *N*-Methylmorpholin, *N*-Methylpiperidin, Arginin, Lysin und 1,2-Ethylendiamin.

Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt. Als Solvate sind im Rahmen der vorliegenden Erfindung Hydrate bevorzugt.

Die erfindungsgemäßen Verbindungen können in Abhängigkeit von ihrer Struktur in unterschiedlichen stereoisomeren Formen existieren, d.h. in Gestalt von Konfigurationsisomeren oder gegebenenfalls auch als Konformationsisomere (Enantiomere und/oder Diastereomere, einschließlich solcher bei Atropisomeren). Die vorliegende Erfindung umfasst deshalb die Enantiomere und Diastereomere und ihre jeweiligen Mischungen. Aus solchen Mischungen von Enantiomeren und/ oder Diastereomeren lassen sich die stereoisomer einheitlichen Bestandteile in bekannter Weise isolieren; vorzugsweise werden hierfür chromatographische Verfahren verwendet, insbesondere die HPLC-Chromatographie an achiraler bzw. chiraler Phase.

Sofern die erfindungsgemäßen Verbindungen in tautomeren Formen vorkommen können, umfasst die vorliegende Erfindung sämtliche tautomere Formen.

Die vorliegende Erfindung umfasst auch alle geeigneten isotopischen Varianten der erfindungsgemäßen Verbindungen. Unter einer isotopischen Variante einer erfindungsgemäßen Verbindung wird hierbei eine Verbindung verstanden, in welcher mindestens ein Atom innerhalb der erfindungsgemäßen Verbindung gegen ein anderes Atom der gleichen Ordnungszahl, jedoch mit einer anderen Atommasse als der gewöhnlich oder überwiegend in der Natur vorkommenden Atommasse ausgetauscht ist. Beispiele für Isotope, die in eine erfindungsgemäße Verbindung inkorporiert werden können, sind solche von Wasserstoff, Kohlenstoff, Stickstoff, Sauerstoff, Phosphor, Schwefel, Fluor, Chlor, Brom und Iod, wie ²H (Deuterium), ³H (Tritium), ¹³C, ¹⁴C, ¹⁵N, ¹⁷O, ¹⁸O, ³²P, ³³P, ³³S, ³⁴S, ³⁵S, ³⁶S, ¹⁸F, ³⁶Cl, ⁸²Br, ¹²³I, ¹²⁴I, ¹²⁹I und ¹³¹I. Bestimmte isotopische Varianten einer erfindungsgemäßen Verbindung, wie insbesondere solche, bei denen ein oder mehrere radioaktive Isotope inkorporiert sind, können von Nutzen sein beispielsweise für die Untersuchung des Wirkmechanismus oder der Wirkstoff-Verteilung im Körper; aufgrund der vergleichsweise leichten Herstell- und Detektierbarkeit sind hierfür insbesondere mit ³H- oder ¹⁴C-Isotopen markierte Verbindungen geeignet. Darüber hinaus kann der Einbau von Isotopen, wie beispielsweise von Deuterium, zu bestimmten therapeutischen Vorteilen als Folge einer größeren metabolischen Stabilität der Verbindung führen, wie beispielsweise zu einer Verlängerung der Halbwertszeit im Körper oder zu einer Reduktion der erforderlichen Wirkdosis; solche Modifikationen der erfindungsgemäßen Verbindungen können daher gegebenenfalls auch eine bevorzugte Ausführungsform der vorliegenden Erfindung darstellen. Isotopische Varianten der erfindungsgemäßen Verbindungen können nach allgemein gebräuchlichen, dem Fachmann bekannten Verfahren hergestellt werden, so beispielsweise nach den weiter unten beschriebenen Methoden und den bei den Ausführungsbeispielen wiedergegebenen Vorschriften, indem hierbei entsprechende isotopische Modifikationen der jeweiligen Reagentien und/oder Ausgangsverbindungen eingesetzt werden.

Außerdem soffenbart sind Prodrugs der erfindungsgemäßen Verbindungen. Der Begriff "Prodrugs" bezeichnet hierbei Verbindungen, welche selbst biologisch aktiv oder inaktiv sein können, jedoch während ihrer Verweilzeit im Körper auf beispielsweise metabolischem oder hydrolytischem Wege zu erfindungsgemäßen Verbindungen umgesetzt werden.

Insbesondere offenbart sind als Prodrugs hydrolysierbare Ester-Derivate der erfindungsgemäßen Carbonsäuren der Formel (I). Hierunter werden Ester verstanden, die in physiologischen Medien, unter den Bedingungen der im weiteren beschriebenen biologischen Tests und insbesondere *in vivo* auf enzymatischem oder chemischem Wege zu den freien Carbonsäuren, als den biologisch hauptsächlich aktiven Verbindungen, hydrolysiert werden können. Als solche Ester werden (C₁-C₄)-Alkylester, in welchen die Alkylgruppe geradkettig oder verzweigt sein kann, bevorzugt. Besonders bevorzugt sind Methyl-, Ethyl- oder *tert*.-Butylester.

Im Rahmen der vorliegenden Erfindung haben die Substituenten, soweit nicht anders spezifiziert, die folgende Bedeutung:
(C₁-C₄)-Alkyl steht im Rahmen der Erfindung für einen geradkettigen oder verzweigten Alkylrest mit 1 bis 4 Kohlenstoffatomen. Beispielhaft und vorzugsweise seien genannt: Methyl, Ethyl, *n*-Propyl, Isopropyl, *n*-Butyl, *iso*-Butyl, sec.-Butyl und *tert*.-Butyl.

Im Rahmen der vorliegenden Erfindung gilt, dass für alle Reste, die mehrfach auftreten, deren Bedeutung unabhängig voneinander ist. Wenn Reste in den erfindungsgemäßen Verbindungen substituiert sind, können die Reste, soweit nicht anders spezifiziert, ein- oder mehrfach substituiert sein. Eine Substitution mit einem oder mit zwei oder drei gleichen oder verschiedenen Substituenten ist bevorzugt. Besonders bevorzugt ist die Substitution mit einem oder mit zwei gleichen oder verschiedenen Substituenten.

Bevorzugt im Rahmen der vorliegenden Erfindung sind Verbindungen der Formel (I), in welcher
- R^{1A}: für Wasserstoff oder Methyl steht,
- R^{1B}: für Wasserstoff steht,
oder
- R^{1A} und R^{1B}: miteinander verknüpft sind und gemeinsam mit dem Kohlenstoffatom, an das sie gebunden sind, einen Cyclopropyl- oder Cyclobutyl-Ring bilden,
- R^{2A}: für Wasserstoff, Methyl, Ethyl, Isopropyl oder Cyclopropyl steht,
- R^{2B}: für Wasserstoff steht,
oder
- R^{2A} und R^{2B}: miteinander verknüpft sind und gemeinsam mit dem Kohlenstoffatom, an das sie gebunden sind, einen Cyclopropyl-Ring bilden,
- R³: für Fluor, Chlor oder Methyl steht,
- R^{4A}: für Methyl steht,
- R^{4B}: für Trifluormethyl steht,
oder
- R^{4A} und R^{4B}: miteinander verknüpft sind und gemeinsam mit dem Kohlenstoffatom, an das sie gebunden sind, einen Cyclopentyl-Ring bilden, der bis zu zweifach mit Fluor substituiert sein kann,
und
- A: für einen optional substituierten oder anellierten Piperidin-Ring der Formel 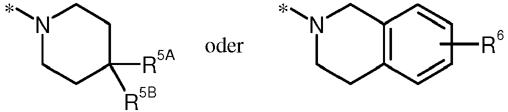 steht, worin
* die Verknüpfungsstelle mit dem Rest des Moleküls bezeichnet,
R^{5A} Wasserstoff, Methyl, Trifluormethyl, Ethyl, Isopropyl oder Cyclopropyl bedeutet,
R^{5B} Wasserstoff bedeutet,
oder
- R^{5A} und R^{5B}: miteinander verknüpft sind und gemeinsam mit dem Kohlenstoffatom, an das sie gebunden sind, einen Cyclopropyl-Ring bilden,
und
- R⁶: Wasserstoff oder Fluor bedeutet,
sowie ihre Salze, Solvate und Solvate der Salze.

Eine besondere Ausführungsform der vorliegenden Erfindung umfasst Verbindungen der Formel (I), in welcher
- R^{1A}, R^{1B}, R^{2A} und R^{2B}: jeweils für Wasserstoff stehen,
sowie ihre Salze, Solvate und Solvate der Salze.

Eine weitere besondere Ausführungsform der vorliegenden Erfindung umfasst Verbindungen der Formel (I), in welcher
- R^{1A}: für Methyl steht,
- R^{1B}: für Wasserstoff steht,
oder
- R^{1A} und R^{1B}: miteinander verknüpft sind und gemeinsam mit dem Kohlenstoffatom, an das sie gebunden sind, einen Cyclopropyl-Ring bilden,
und
- R^{2A} und R^{2B}: jeweils für Wasserstoff stehen,
sowie ihre Salze, Solvate und Solvate der Salze.

Eine weitere besondere Ausführungsform der vorliegenden Erfindung umfasst Verbindungen der Formel (I), in welcher
- R^{2A}: für Methyl steht,
und
- R^{1A}, R^{1B} und R^{2B}: jeweils für Wasserstoff stehen,
sowie ihre Salze, Solvate und Solvate der Salze.

Eine weitere besondere Ausführungsform der vorliegenden Erfindung umfasst Verbindungen der Formel (I), in welcher
- R^{4A}: für Methyl steht,
und
- R^{4B}: für Trifluormethyl steht,
sowie ihre Salze, Solvate und Solvate der Salze.

Eine weitere besondere Ausführungsform der vorliegenden Erfindung umfasst Verbindungen der Formel (I), in welcher
- R^{4A} und R^{4B}: miteinander verknüpft sind und gemeinsam mit dem Kohlenstoffatom, an das sie gebunden sind, einen Cyclopentyl-Ring bilden,
sowie ihre Salze, Solvate und Solvate der Salze.

Eine weitere besondere Ausführungsform der vorliegenden Erfindung umfasst Verbindungen der Formel (I), in welcher
- A: für einen optional substituierten Piperidin-Ring der Formel 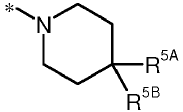 steht, worin
* die Verknüpfungsstelle mit dem Rest des Moleküls bezeichnet,
R^{5A} Wasserstoff, Methyl, Trifluormethyl, Ethyl oder Cyclopropyl bedeutet,
R^{5B} Wasserstoff bedeutet, oder
R^{5A} und R^{5B} miteinander verknüpft sind und gemeinsam mit dem Kohlenstoffatom, an das sie gebunden sind, einen Cyclopropyl-Ring bilden,
sowie ihre Salze, Solvate und Solvate der Salze.

Besonders bevorzugt im Rahmen der vorliegenden Erfindung sind Verbindungen der Formel (I), in welcher
- R^{1A}: für Wasserstoff oder Methyl steht,
- R^{1B}: für Wasserstoff steht,
oder
- R^{1A} und R^{1B}: miteinander verknüpft sind und gemeinsam mit dem Kohlenstoffatom, an das sie gebunden sind, einen Cyclopropyl-Ring bilden,
- R^{2A}: für Wasserstoff oder Methyl steht,
- R^{2B}: für Wasserstoff steht,
- R³: für Fluor oder Chlor steht,
- R^{4A}: für Methyl steht,
- R^{4B}: für Trifluormethyl steht,
und
- A: für einen optional substituierten Piperidin-Ring der Formel 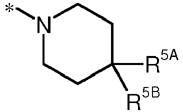 steht, worin
* die Verknüpfungsstelle mit dem Rest des Moleküls bezeichnet,
R^{5A} Wasserstoff, Methyl, Trifluormethyl oder Ethyl bedeutet,
R^{5B} Wasserstoff bedeutet, oder
R^{5A} und R^{5B} miteinander verknüpft sind und gemeinsam mit dem Kohlenstoffatom, an das sie gebunden sind, einen Cyclopropyl-Ring bilden,
sowie ihre Salze, Solvate und Solvate der Salze.

Die in den jeweiligen Kombinationen bzw. bevorzugten Kombinationen von Resten im einzelnen angegebenen Reste-Definitionen werden unabhängig von den jeweiligen angegebenen Kombinationen der Reste beliebig auch durch Reste-Definitionen anderer Kombinationen ersetzt. Ganz besonders bevorzugt sind Kombinationen von zwei oder mehreren der oben genannten Vorzugsbereiche.

Weiterer Gegenstand der Erfindung ist ein Verfahren zur Herstellung der erfindungsgemäßen Verbindungen der Formel (I), dadurch gekennzeichnet, dass man eine geschützte α-Aminocarbonsäure der Formel (II) 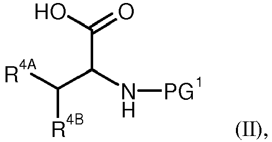 in welcher R^{4A} und R^{4B} die oben angegebenen Bedeutungen haben
und
- PG¹: für eine geeignete Amino-Schutzgruppe wie beispielsweise Allyloxycarbonyl (Alloc), Benzyloxycarbonyl (Z), *tert*.-Butoxycarbonyl (Boc) oder 9-Fluorenylmethoxycarbonyl (Fmoc) steht,
in einem inerten Lösungsmittel mit Hilfe eines Kondensationsmittels in Gegenwart einer Base mit einem Amin der Formel (III) 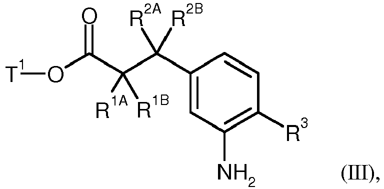 in welcher R^{1A}, R^{1B}, R^{2A}, R^{2B} und R³ die oben angegebenen Bedeutungen haben
und
- T¹: für (C₁-C₄)-Alkyl oder Benzyl steht,
zu einem Carbonsäureamid der Formel (IV) 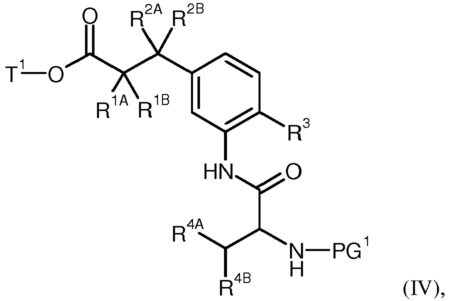 in welcher PG¹, R^{1A}, R^{1B}, R^{2A}, R^{2B}, R³, R^{4A}, R^{4B} und T¹ die oben angegebenen Bedeutungen haben, kuppelt, anschließend durch Abspaltung der Schutzgruppe PG¹ die Amin-Verbindung (V) 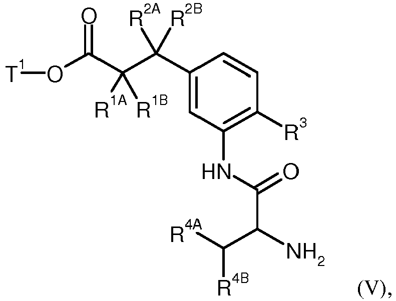 in welcher R^{1A}, R^{1B}, R^{2A}, R^{2B}, R³, R^{4A}, R^{4B} und T¹ die oben angegebenen Bedeutungen haben,
freisetzt, diese dann in Gegenwart eines geeigneten Reduktionsmittels mit einem Dialdehyd der Formel (VI) 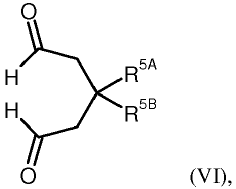 in welcher R^{5A} und R^{5B} die oben angegebenen Bedeutungen haben,
zu einem Gemisch (mit variierenden Anteilen) der beiden Cyclisierungsprodukte (VII) und (VIII) 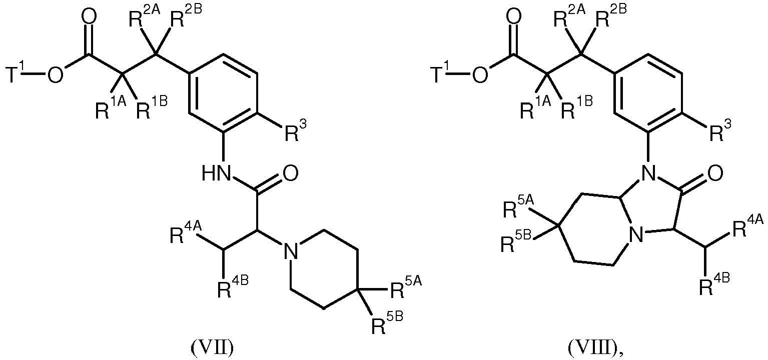 in welchen R^{1A}, R^{1B}, R^{2A}, R^{2B}, R³, R^{4A}, R^{4B}, R^{5A}, R^{5B} und T¹ die oben angegebenen Bedeutungen haben,
umsetzt, dieses Gemisch danach mit einem Überschuss an Triethylsilan in Trifluoressigsäure behandelt, um so auch das "falsche" Cyclisierungsprodukt (VIII) in die gewünschte Zielverbindung (VII) umzuwandeln, und schließlich den Ester-Rest T¹ durch basische oder saure Solvolyse oder im Fall, dass T¹ für Benzyl steht, auch durch Hydrogenolyse unter Erhalt der Carbonsäure der Formel (I-A) 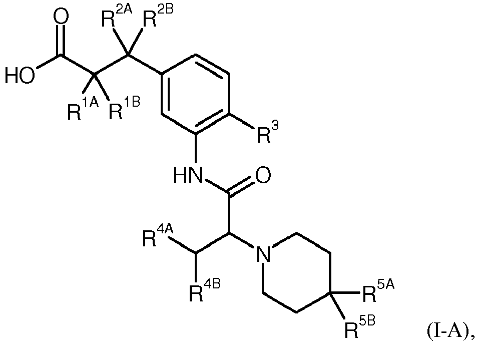 in welcher R^{1A}, R^{1B}, R^{2A}, R^{2B}, R³, R^{4A}, R^{4B}, R^{5A} und R^{5B} die oben angegebenen Bedeutungen haben, abspaltet
und gegebenenfalls die so hergestellten erfindungsgemäßen Verbindungen der Formel (I-A) nach üblichen Methoden in ihre Enantiomere und/oder Diastereomere trennt und/oder mit den entsprechenden (*i*) Lösungsmitteln und/oder (*ii*) Säuren oder Basen in ihre Solvate, Salze und/oder Solvate der Salze überführt.

Inerte Lösungsmittel für den Verfahrensschritt (II) + (III) → (IV) [Amid-Kupplung] sind beispielsweise Ether wie Diethylether, Diisopropylether, Methyl-*tert*.-butylether, 1,2-Dimethoxyethan, Bis-(2-methoxyethyl)-ether, Tetrahydrofuran oder 1,4-Dioxan, Kohlenwasserstoffe wie Benzol, Toluol, Xylol, Pentan, Hexan, Heptan, Cyclohexan oder Erdölfraktionen, Halogenkohlenwasserstoffe wie Dichlormethan, Trichlormethan, Tetrachlormethan, 1,2-Dichlorethan, Trichlorethylen oder Chlorbenzol, oder dipolar-aprotische Lösungsmittel wie Aceton, Acetonitril, Ethylacetat, Pyridin, *N*,*N*-Dimethylformamid (DMF), *N*,*N*-Dimethylacetamid (DMA), Dimethylsulfoxid (DMSO), *N*-Methylpyrrolidinon (NMP) oder *N*,*N*'-Dimethylpropylenharnstoff (DMPU). Ebenso ist es möglich, Gemische solcher Lösungsmittel einzusetzen. Bevorzugt wird Dimethylformamid oder ein Gemisch von Dimethylformamid und Pyridin verwendet.

Als Kondensationsmittel für diese Kupplungsreaktion eignen sich beispielsweise Carbodiimide wie *N*,*N*'-Diethyl-, *N*,*N*'-Dipropyl-, *N*,*N*'-Diisopropyl-, *N*,*N*'-Dicyclohexylcarbodiimid (DCC) oder *N*-(3-Dimethylaminopropyl)-*N*'-ethylcarbodiimid-Hydrochlorid (EDC), Phosgen-Derivate wie *N*,*N*'-Carbonyldiimidazol (CDI) oder Isobutylchlorformiat, 1,2-Oxazolium-Verbindungen wie 2-Ethyl-5-phenyl-1,2-oxazolium-3-sulfat oder 2-*tert*.-Butyl-5-methylisoxazolium-perchlorat, Acylamino-Verbindungen wie 2-Ethoxy-1-ethoxycarbonyl-1,2-dihydrochinolin, α-Chlorenamine wie 1-Chlor-2-methyl-1-dimethylamino-1-propen, Phosphor-Verbindungen wie Propanphosphonsäureanhydrid, Cyanophosphonsäurediethylester, Bis-(2-oxo-3-oxazolidinyl)-phosphorylchlorid, Benzotriazol-1-yloxy-tris(dimethylamino)phosphonium-hexafluorophosphat oder Benzotriazol-1-yloxy-tris(pyrrolidino)phosphonium-hexafluorophosphat (PyBOP), oder Uronium-Verbindungen wie *O*-(Benzotriazol-1-yl)-*N*,*N*,*N'*,*N'*-tetramethyluronium-tetrafluoroborat (TBTU), *O*-(Benzotriazol-1-yl)-*N*,*N*,*N'*,*N*'-tetramethyluronium-hexafluorophosphat (HBTU), 2-(2-Oxo-1-(2*H*)-pyridyl)-1,1,3,3-tetramethyluronium-tetrafluoroborat (TPTU), *O*-(7-Azabenzotriazol-1-yl)-*N*,*N*,*N'*,*N'*-tetramethyl-uronium-hexafluorophosphat (HATU) oder *O*-(1*H*-6-Chlorbenzotriazol-1-yl)-1,1,3,3-tetramethyluronium-tetrafluoroborat (TCTU), gegebenenfalls in Kombination mit weiteren Hilfsstoffen wie 1-Hydroxybenzotriazol (HOBt) oder *N*-Hydroxysuccinimid (HOSu), sowie als Basen Alkalicarbonate, z.B. Natrium- oder Kaliumcarbonat, oder tertiäre Aminbasen wie Triethylamin, N-Methylmorpholin, N-Methylpiperidin, *N*,*N*-Diisopropylethylamin, Pyridin oder 4-*N*,*N*-Dimethylaminopyridin (DMAP). Bevorzugt wird *O*-(7-Azabenzotriazol-1-yl)-*N*,*N*,*N'*,*N'*-tetramethyluronium-hexafluorophosphat (HATU) in Kombination mit Pyridin eingesetzt.

Die Reaktion (II) + (III) → (IV) wird in der Regel in einem Temperaturbereich von 0°C bis +60°C, bevorzugt bei +10°C bis +40°C durchgeführt.

Als Schutzgruppe PG¹ in Verbindung (II) eignen sich übliche Amino-Schutzgruppen wie beispielsweise Allyloxycarbonyl (Alloc), Benzyloxycarbonyl (Z), *tert*.-Butoxycarbonyl (Boc) oder 9-Fluorenylmethoxycarbonyl (Fmoc), wobei die Schutzgruppe PG¹ so ausgewählt wird, dass die Bedingungen ihrer Abspaltung im Verfahrensschritt (IV) → (V) kompatibel mit dem jeweils eingesetzten Ester-Rest T¹ aus Verbindung (III) sind [siehe z.B. T.W. Greene und P.G.M. Wuts, Protective Groups in Organic Synthesis, Wiley, New York, 1999]. Bevorzugt wird die Allyloxycarbonyl- oder die Benzyloxycarbonyl-Gruppe verwendet. Die Abspaltung der Allyloxycarbonyl-Gruppe erfolgt vorzugsweise mit Hilfe von Dimedon (5,5-Dimethylcyclohexan-1,3-dion) in Gegenwart des Tetrakis(triphenylphosphin)palladium(0)-Katalysators, während die Benzyloxycarbonyl-Gruppe im Allgemeinen durch Hydrierung (Hydrogenolyse) in Gegenwart von 5-10%-igem Palladium auf Aktivkohle entfernt wird.

Als Reduktionsmittel im Verfahrensschritt (V) + (VI) → (VII) [+ (VIII)] sind übliche Alkaliborhydride, wie Lithium-, Natrium- oder Kaliumborhydrid, Natriumcyanoborhydrid oder Natriumtriacetoxyborhydrid, geeignet. Bevorzugt wird Natriumcyanoborhydrid verwendet. Die Umsetzung wird in der Regel in Gegenwart eines Säure/Base-Gemisches, wie beispielsweise Salzsäure und Triethylamin, in einem alkoholischen Lösungsmittel wie Methanol, Ethanol oder Isopropanol durchgeführt. Die Reaktion erfolgt in Abhängigkeit von der Reaktivität der jeweils eingesetzten Komponenten im Allgemeinen in einem Temperaturbereich von -20°C bis +60°C.

Das bei der Borhydrid-vermittelten Umsetzung von Amin (V) und Dialdehyd (VI) in variierenden Anteilen entstehende "falsche" Cyclisierungsprodukt der Formel (VIII) kann durch nachfolgende Reduktion mit Triethylsilan in Trifluoressigsäure als Lösungsmittel ebenfalls in das gewünschte Cyclisierungsprodukt der Formel (VII) umgewandelt werden [vgl. z.B. D.N. Kursanov et al., Synthesis 1974, 633-651; U. Rosentreter, Synthesis 1985, 210-212], wodurch sich im Ergebnis eine höhere Ausbeute an (VII) erzielen lässt. Diese Transformation kann gegebenenfalls nach vorheriger Trennung der beiden Cyclisierungsprodukte (VII) und (VIII) erfolgen oder sie kann vorteilhafterweise, unter Verwendung eines Überschusses an Triethylsilan, direkt mit dem aus der Cyclisierungsreaktion erhaltenen, aufgereinigten Produktgemisch durchgeführt werden.

Die Abspaltung der Ester-Gruppe T¹ im Verfahrensschritt (VII) → (I-A) wird nach üblichen Methoden durchgeführt, indem man den Ester in einem inerten Lösungsmittel mit einer Säure oder einer Base behandelt, wobei bei letzterer Variante das zunächst entstehende Salz durch nachfolgendes Einwirken einer Säure in die freie Carbonsäure überführt wird. Im Falle der *tert*.-Butylester erfolgt die Esterspaltung vorzugsweise mit Säuren. Benzylester werden bevorzugt durch Hydrierung (Hydrogenolyse) in Gegenwart eines geeigneten Katalysators, wie beispielsweise Palladium auf Aktivkohle, abgespalten.

Als inerte Lösungsmittel eignen sich für diese Reaktionen Wasser oder die für eine Esterspaltung üblichen organischen Lösungsmittel. Hierzu gehören bevorzugt Alkohole wie Methanol, Ethanol, *n*-Propanol, Isopropanol, *n*-Butanol oder *tert*.-Butanol, oder Ether wie Diethylether, Tetrahydrofuran, 1,4-Dioxan oder 1,2-Dimethoxyethan, oder andere Lösungsmittel wie Aceton, Essigsäure, Dichlormethan, *N*,*N*-Dimethylformamid oder Dimethylsulfoxid. Ebenso ist es möglich, Gemische dieser Lösungsmittel einzusetzen. Im Falle einer basischen Ester-Hydrolyse werden bevorzugt Gemische von Wasser mit Dioxan, Tetrahydrofuran, Methanol und/oder Ethanol eingesetzt. Im Falle der Umsetzung mit Trifluoressigsäure wird bevorzugt Dichlormethan und im Falle der Umsetzung mit Chlorwasserstoff bevorzugt Tetrahydrofuran, Diethylether, Dioxan, Essigsäure und/ oder Wasser verwendet.

Als Basen sind die üblichen anorganischen Basen geeignet. Hierzu gehören insbesondere Alkali- oder Erdalkalihydroxide wie beispielsweise Lithium-, Natrium-, Kalium- oder Bariumhydroxid, oder Alkali- oder Erdalkalicarbonate wie Natrium-, Kalium- oder Calciumcarbonat. Bevorzugt sind Lithium-, Natrium- oder Kaliumhydroxid.

Als Säuren eignen sich für die Esterspaltung im Allgemeinen Schwefelsäure, Chlorwasserstoff/ Salzsäure, Bromwasserstoff/Bromwasserstoffsäure, Phosphorsäure, Essigsäure, Trifluoressigsäure, Toluolsulfonsäure, Methansulfonsäure oder Trifluormethansulfonsäure oder deren Gemische gegebenenfalls unter Zusatz von Wasser. Bevorzugt sind Chlorwasserstoff oder Trifluoressigsäure im Falle der *tert*.-Butylester und Salzsäure oder Schwefelsäure im Falle der Methyl- oder Ethylester.

Die Esterspaltung wird im Allgemeinen in einem Temperaturbereich von -20°C bis +100°C, bevorzugt bei 0°C bis +60°C durchgeführt.

Im Falle, dass die Ester-Gruppe T¹ in (VII) bzw. (VIII) für *tert*.-Butyl steht, erfolgt unter den Bedingungen der oben beschriebenen Behandlung von (VIII) oder des Gemisches von (VII) und (VIII) mit Triethylsilan und Trifluoressigsäure gleichzeitig auch eine Abspaltung dieser Ester-Gruppe, so dass hier die erfindungsgemäße Verbindung der Formel (I-A) direkt erhalten wird.
Erfindungsgemäße Verbindungen der Formel (I) können gegebenenfalls auch dadurch hergestellt werden, dass man eine α-Aminocarbonsäure der Formel (IX) 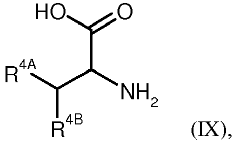 in welcher R^{4A} und R^{4B} die oben angegebenen Bedeutungen haben,
in einem inerten Lösungsmittel in Gegenwart einer Base mit einer Verbindung der Formel (X) 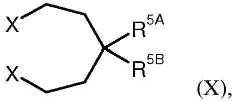 in welcher R^{5A} und R^{5B} die oben angegebenen Bedeutungen haben
und
- X: für eine geeignete Abgangsgruppe wie beispielsweise Chlor, Brom, Iod, Mesylat, Tosylat oder Triflat steht,
zu einer Verbindung der Formel (XI) 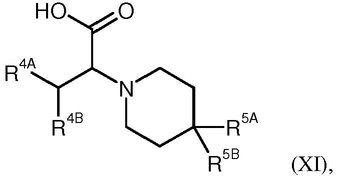 in welcher R^{4A}, R^{4B}, R^{5A} und R^{5B} die oben angegebenen Bedeutungen haben,
alkyliert, diese anschließend in einem inerten Lösungsmittel mit Hilfe eines Kondensationsmittels in Gegenwart einer Base mit einem Amin der Formel (III) 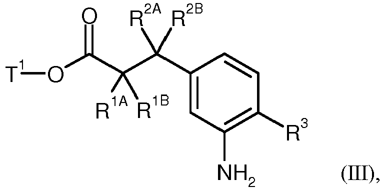 in welcher R^{1A}, R^{1B}, R^{2A}, R^{2B} und R³ die oben angegebenen Bedeutungen haben
und
- T¹: für (C₁-C₄)-Alkyl oder Benzyl steht,
zu einem Carbonsäureamid der Formel (VII) 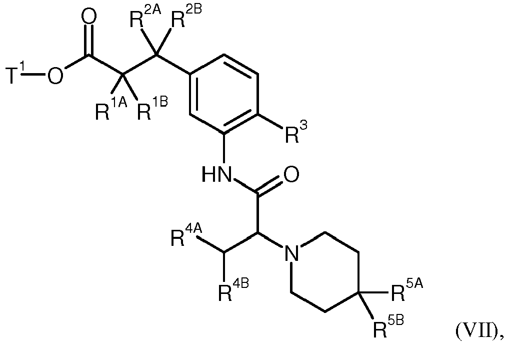 in welcher R^{1A}, R^{1B}, R^{2A}, R^{2B}, R³, R^{4A}, R^{4B}, R^{5A}, R^{5B} und T¹ die oben angegebenen Bedeutungen haben,
kuppelt und den Ester-Rest T¹ dann, wie zuvor beschrieben, unter Erhalt der Carbonsäure der Formel (I-A) 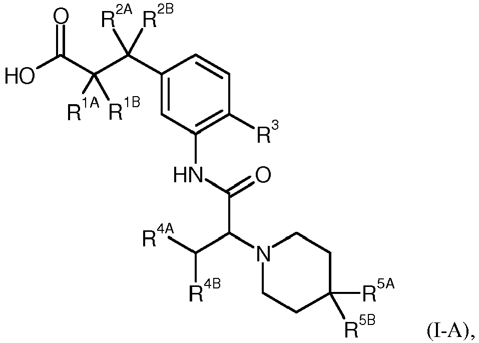 in welcher R^{1A}, R^{1B}, R^{2A}, R^{2B}, R³, R^{4A}, R^{4B}, R^{5A} und R^{5B} die oben angegebenen Bedeutungen haben, abspaltet.

Die Alkylierungsreaktion (IX) + (X) → (XI) wird vorzugsweise in Wasser oder in Gemischen von Wasser mit wassermischbaren organischen Lösungsmitteln, wie Methanol, Ethanol, Tetrahydrofuran, 1,4-Dioxan, 1,2-Dimethoxyethan, Acetonitril, *N*,*N*-Dimethylformamid oder Dimethylsulfoxid, durchgeführt. Als Base eignen sich insbesondere Alkalihydroxide, wie beispielsweise Lithium-, Natrium- oder Kaliumhydroxid, oder Alkali- oder Erdalkalicarbonate, wie Lithium-, Natrium-, Kalium-, Calcium- oder Cäsiumcarbonat. Bevorzugt wird Natrium- oder Kaliumhydroxid verwendet. Die Umsetzung erfolgt im Allgemeinen in einem Temperaturbereich von +50°C bis +150°C, bevorzugt bei +80°C bis +120°C.

Die Kupplungsreaktion (XI) + (III) → (VII) wird unter analogen Bedingungen durchgeführt wie zuvor für die Umsetzung (II) + (III) → (IV) beschrieben.

Erfindungsgemäße Verbindungen der Formel (I), in welcher R^{4A} und R^{4B} miteinander verknüpft sind und gemeinsam mit dem Kohlenstoffatom, an das sie gebunden sind, einen Cyclobutyl- oder Cyclopentyl-Ring bilden, können auch dadurch hergestellt werden, dass man eine Cycloalkenylboronsäure oder -boronsäureester der Formel (XII) 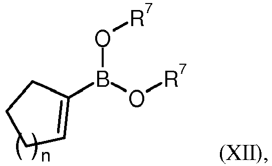 in welcher
- n: für die Zahl 0 oder 1 steht
und
- R⁷: für Wasserstoff oder (C₁-C₄)-Alkyl steht oder beide Reste R⁷ miteinander verknüpft sind und zusammen eine -(CH₂)₂-, -C(CH₃)₂-C(CH₃)₂-, -(CH₂)₃- oder -CH₂-C(CH₃)₂-CH₂-Brücke bilden,
in Anwesenheit einer Base mit Glyoxalsäure [OHC-COOH] und einem Piperidin-Derivat der Formel (XIII) 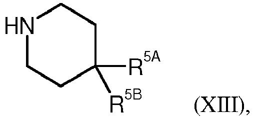 in welcher R^{5A} und R^{5B} die oben angegebenen Bedeutungen haben,
zu einer α-Piperidinocarbonsäure der Formel (XIV) 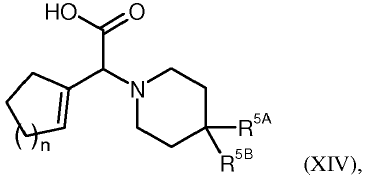 in welcher n, R^{5A} und R^{5B} die oben angegebenen Bedeutungen haben,
umsetzt, diese anschließend in einem inerten Lösungsmittel mit Hilfe eines Kondensationsmittels in Gegenwart einer Base mit einem Amin der Formel (III) 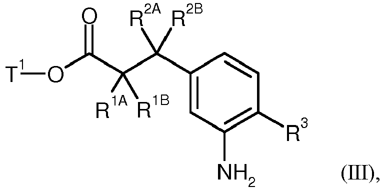 in welcher R^{1A}, R^{1B}, R^{2A}, R^{2B} und R³ die oben angegebenen Bedeutungen haben
und
- T¹: für (C₁-C₄)-Alkyl oder Benzyl steht,
zu einem Carbonsäureamid der Formel (XV) 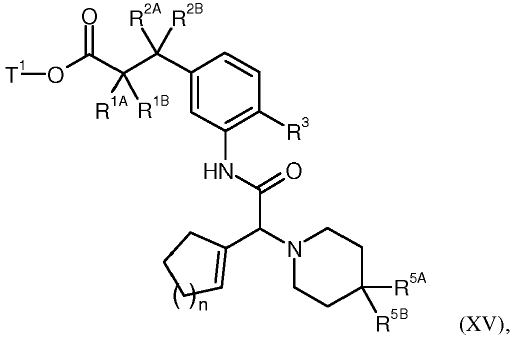 in welcher n, R^{1A}, R^{1B}, R^{2A}, R^{2B}, R³, R^{5A}, R^{5B} und T¹ die oben angegebenen Bedeutungen haben,
kuppelt, danach in Gegenwart eines geeigneten Palladium- oder Platin-Katalysators zu einer Verbindung der Formel (XVI) 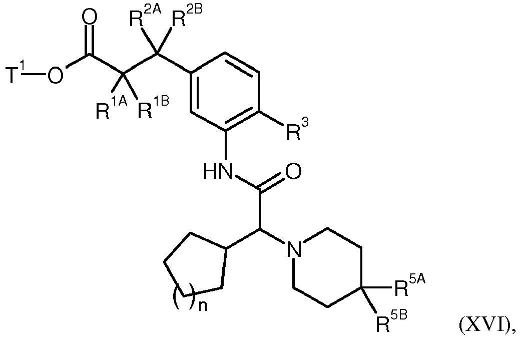 in welcher n, R^{1A}, R^{1B}, R^{2A}, R^{2B}, R³, R^{5A}, R^{5B} und T¹ die oben angegebenen Bedeutungen haben,
hydriert und schließlich den Ester-Rest T¹ durch basische oder saure Solvolyse unter Erhalt der Carbonsäure der Formel (I-B) 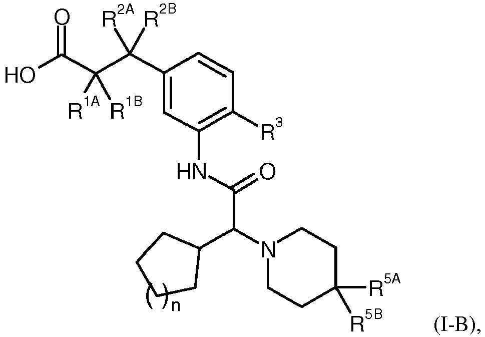 in welcher n, R^{1A}, R^{1B}, R^{2A}, R^{2B}, R³, R^{5A} und R^{5B} die oben angegebenen Bedeutungen haben,
abspaltet.

Die Herstellung der α-Piperidinocarbonsäure (XIV) ausgehend vom Cycloalkenylboronat (XII), Glyoxalsäure und dem Piperidin-Derivat (XIII) erfolgt in Anlehnung an eine in der Literatur beschriebene Methode [siehe N.A. Petasis und I.A. Zavialov, J. Am. Chem. Soc. 119, 445-446 (1997)]. Von Vorteil ist hierbei der Zusatz einer tertiären Amin-Base wie beispielsweise Triethylamin oder *N*,*N*-Diisopropylethylamin.

Die Hydrierung (XV) → (XVI) wird in der Regel unter einer stationären Wasserstoffatmosphäre bei normalem oder leicht erhöhtem Druck durchgeführt. Als Katalysator eignen sich insbesondere Palladium oder Platin auf Aktivkohle (als Trägermaterial) oder Platin(IV)oxid. Die Reaktion erfolgt in einem für solche Zwecke üblichen inerten Lösungsmittel wie beispielsweise Methanol, Ethanol, Tetrahydrofuran oder Ethylacetat.

Im Falle, dass die Ester-Gruppe T¹ in (XV) für Benzyl steht, erfolgt unter den Hydrierungsbedingungen gleichzeitig auch eine Abspaltung dieser Ester-Gruppe, so dass hier die erfindungsgemäße Verbindung der Formel (I-B) direkt erhalten wird.

Die Kupplungsreaktion (XIV) + (III) → (XV) und die Esterspaltung (XVI) → (I-B) werden unter analogen Bedingungen durchgeführt wie zuvor für die Umsetzungen (II) + (III) → (IV) bzw. (VII) → (I-A) beschrieben.

Erfindungsgemäße Verbindungen der Formel (I-C) 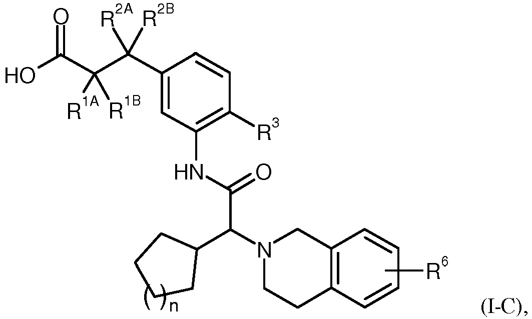 in welcher n, R^{1A}, R^{1B}, R^{2A}, R^{2B}, R³ und R⁶ die oben angegebenen Bedeutungen haben,
können auf analoge Weise hergestellt werden, indem in der zuvor beschriebenen Reaktionssequenz anstelle des Piperidin-Derivats (XIII) ein 1,2,3,4-Tetrahydroisochinolin der Formel (XVII) 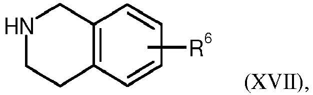 in welcher R⁶ die oben angegebene Bedeutung hat,
eingesetzt wird.

Die Verbindungen der Formeln (I-A), (I-B) und (I-C), welche nach den oben beschriebenen Verfahren hergestellt werden können, stellen jeweils Teilmengen der erfindungsgemäßen Verbindungen der allgemeinen Formel (I) dar.

Die α-Aminocarbonsäure-Intermediate der Formel (IX) 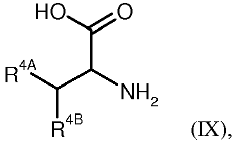 in welcher R^{4A} und R^{4B} die oben angegebenen Bedeutungen haben,
sind zum Teil kommerziell erhältlich oder als solche in der Literatur beschrieben, oder sie können nach bekannten Methoden beispielsweise dadurch hergestellt werden, dass man einen β,β-disubstituierten Carbonsäureester der Formel (XVIII) 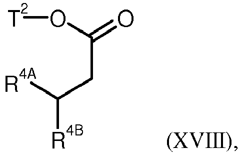 in welcher R^{4A} und R^{4B} die oben angegebenen Bedeutungen haben
und
- T²: für (C₁-C₄)-Alkyl oder Benzyl steht,
in einem inerten Lösungsmittel mit Hilfe einer Base in α-Position deprotoniert, in den entsprechenden Silylenolester (Ketenacetal) überführt und dann mit *N*-Bromsuccinimid zum α-Bromderivat der Formel (XIX) 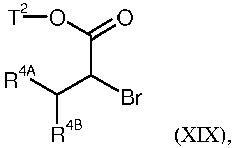 in welcher R^{4A}, R^{4B} und T² die oben angegebenen Bedeutungen haben,
bromiert, anschließend mit Natriumazid zur Verbindung der Formel (XX) 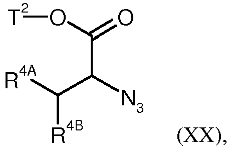 in welcher R^{4A}, R^{4B} und T² die oben angegebenen Bedeutungen haben,
umsetzt, diese durch katalytische Hydrierung zum α-Aminosäureester der Formel (XXI) 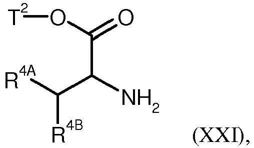 in welcher R^{4A}, R^{4B} und T² die oben angegebenen Bedeutungen haben,
reduziert und schließlich den Ester-Rest T² durch basische oder saure Solvolyse oder im Fall, dass T² für Benzyl steht, auch durch Hydrogenolyse unter Erhalt der α-Aminocarbonsäure (IX) abspaltet.

Die geschützten α-Aminocarbonsäure-Intermediate der Formel (II) 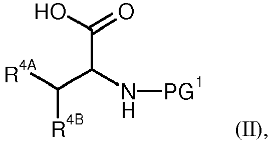 in welcher R^{4A} und R^{4B} die oben angegebenen Bedeutungen haben
und
- PG¹: für eine Amino-Schutzgruppe wie beispielsweise Allyloxycarbonyl (Alloc), Benzyloxycarbonyl (Z), *tert*.-Butoxycarbonyl (Boc) oder 9-Fluorenylmethoxycarbonyl (Fmoc) steht,
sind auf einfache Weise durch Einführung der betreffenden Schutzgruppe PG¹ auf der Stufe der α-Aminocarbonsäure (IX) oder des entsprechenden Esters (XXI) (mit nachfolgender Abspaltung des T²-Restes) zugänglich.

Zur α-Deprotonierung des Carbonsäureesters (XVIII) eignen sich insbesondere nicht-nukleophile, starke Basen wie beispielsweise Natrium- oder Kalium-*tert*.-butylat, Natrium- oder Kaliumhydrid, Lithiumdiisopropylamid oder Lithium-, Natrium- oder Kalium-bis(trimethylsilyl)amid; bevorzugt wird Lithiumdiisopropylamid verwendet. Als inertes Lösungsmittel werden üblicherweise Ether wie Diethylether, Diisopropylether, Methyl-*tert*.-butylether, Tetrahydrofuran, 1,2-Dimethoxyethan oder Bis-(2-methoxyethyl)-ether eingesetzt.

Nach *in situ*-Überführung des deprotonierten Esters in den Silylenolester durch Zugabe von Trimethylsilylchlorid erfolgt die Bromierung zu (XIX) bevorzugt mittels *N*-Bromsuccinimid (NBS). Die Reaktionssequenz wird vorteilhafterweise als Eintopf-Verfahren durchgeführt und findet im Allgemeinen in einem Temperaturbereich von -80°C bis +25°C statt.

Die Substitutionsreaktion zur Azid-Verbindung (XX) wird nach Standardmethoden durchgeführt, beispielsweise durch Behandlung mit Natriumazid in einem dipolar-aprotischen Lösungsmittel wie *N*,*N*-Dimethylformamid. Die anschließende Reduktion zum α-Aminosäureester (XXI) erfolgt vorzugsweise durch Hydrierung in Gegenwart eines Palladium auf Aktivkohle-Katalysators in einem alkoholischen Lösungsmittel wie Methanol oder Ethanol.

Die Abspaltung der Ester-Gruppe T² im Verfahrensschritt (XXI) → (IX) wird auf analoge Weise, wie zuvor für den Ester-Rest T¹ beschrieben, durchgeführt. Im Falle, dass die Ester-Gruppe T² in (XX) für Benzyl steht, erfolgt unter den Bedingungen der Hydrierung (XX) → (XXI) auch hier eine simultane Abspaltung dieser Ester-Gruppe, so dass die Zielverbindung (IX) direkt erhalten wird.

Die Einführung der Schutzgruppe PG¹ auf der Stufe der α-Aminocarbonsäure (IX) oder des entsprechenden Esters (XXI) erfolgt nach allgemein üblichen Methoden [siehe z.B. T.W. Greene und P.G.M. Wuts, Protective Groups in Organic Synthesis, Wiley, New York, 1999].

Die Intermediate der Formel (III) können in Abhängigkeit von ihrem Substitutionsmuster beispielsweise dadurch hergestellt werden, dass man entweder

### [A-1] einen Phosphonoessigsäureester der Formel (XXII)

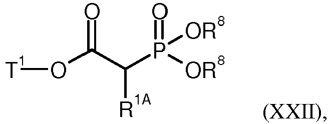

in welcher R^{1A} und T¹ die oben angegebenen Bedeutungen haben
und
- R⁸: für (C₁-C₄)-Alkyl steht,

in einem inerten Lösungsmittel in einer Basen-induzierten Olefinierungsreaktion mit einer 3-Nitrobenzoyl-Verbindung der Formel (XXIII) 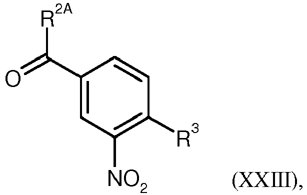 in welcher R^{2A} und R³ die oben angegebenen Bedeutungen haben,
zu einer Verbindung der Formel (XXIV) 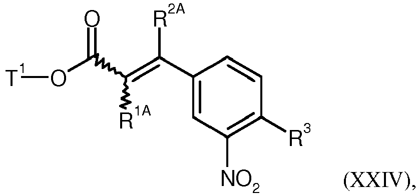 in welcher R^{1A}, R^{2A}, R³ und T¹ die oben angegebenen Bedeutungen haben,
umsetzt und diese dann in Gegenwart eines geeigneten Palladium- oder Platin-Katalysators zu einem 3-(3-Aminophenyl)propionsäureester der Formel (III-A) 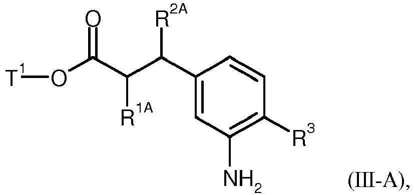 in welcher R^{1A}, R^{2A}, R³ und T¹ die oben angegebenen Bedeutungen haben,
hydriert,
oder

### [A-2] einen Phosphonoessigsäureester der Formel (XXII)

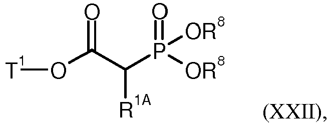

in welcher R^{1A} und T¹ die oben angegebenen Bedeutungen haben
und
- R⁸: für (C₁-C₄)-Alkyl steht,
in einem inerten Lösungsmittel in einer Basen-induzierten Olefinierungsreaktion mit einer geschützten 3-Aminobenzoyl-Verbindung der Formel (XXV) 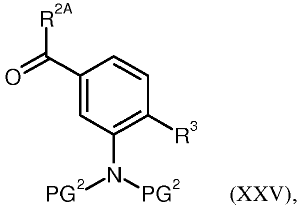 in welcher R^{2A} und R³ die oben angegebenen Bedeutungen haben
und
- PG²: für Benzyl oder 4-Methoxybenzyl als inerte Amino-Schutzgruppe steht,
zu einer Verbindung der Formel (XXVI) 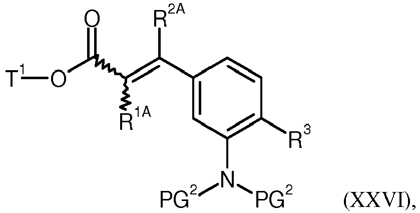 in welcher PG², R^{1A}, R^{2A}, R³ und T¹ die oben angegebenen Bedeutungen haben,
umsetzt, diese dann entweder (*i*) mit Magnesium in Methanol zu einer Verbindung der Formel (XXVII) 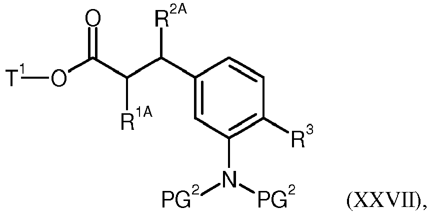 in welcher PG², R^{1A}, R^{2A}, R³ und T¹ die oben angegebenen Bedeutungen haben,
reduziert und anschließend die Amino-Schutzgruppen PG² nach üblichen Methoden durch Hydrogenolyse oder auf oxidativem Wege unter Erhalt des 3-(3-Aminophenyl)propionsäureesters der Formel (III-A) 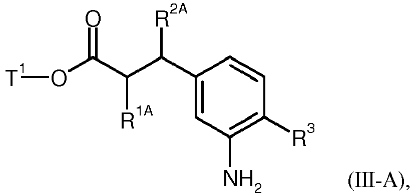 in welcher R^{1A}, R^{2A}, R³ und T¹ die oben angegebenen Bedeutungen haben,
entfernt oder (*ii*) die Verbindung der Formel (XXVI) in einem einstufigen Verfahren durch Hydrierung in Gegenwart eines geeigneten Palladium- oder Platin-Katalysators in den 3-(3-Aminophenyl)propionsäureester der Formel (III-A) überführt,
oder

### [B] ein Acrylsäureester-Derivat der Formel (XXVIII)

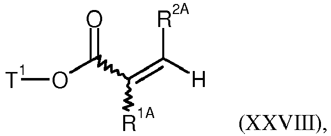

in welcher R^{1A}, R^{2A} und T¹ die oben angegebenen Bedeutungen haben,
in einem inerten Lösungsmittel unter Palladium-Katalyse mit einem 3-Amino- oder 3-Nitrophenylbromid der Formel (XXIX) 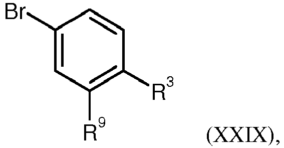 in welcher R³ die oben angegebene Bedeutung hat
und
- R⁹: für Amino oder Nitro steht,
zu einer Verbindung der Formel (XXX) 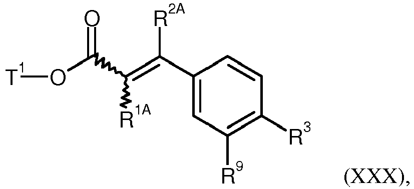 in welcher R^{1A}, R^{2A}, R³, R⁹ und T¹ die oben angegebenen Bedeutungen haben,
kuppelt und diese dann mit Wasserstoff in Gegenwart eines geeigneten Palladium- oder Platin-Katalysators oder im Fall, dass R⁹ für Amino steht, alternativ mit Magnesium in Methanol zum 3-(3-Aminophenyl)propionsäureester der Formel (III-A) 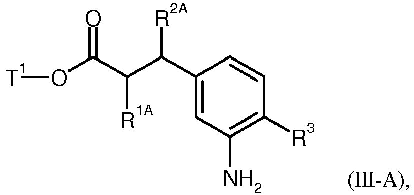 in welcher R^{1A}, R^{2A}, R³ und T¹ die oben angegebenen Bedeutungen haben,
reduziert,
oder

### [C] einen Carbonsäureester der Formel (XXXI)

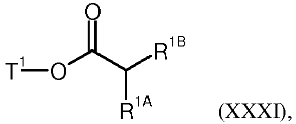

in welcher R^{1A}, R^{1B} und T¹ die oben angegebenen Bedeutungen haben,
in einem inerten Lösungsmittel nach α-Deprotonierung mit einer 3-Brombenzyl-Verbindung der Formel (XXXII) 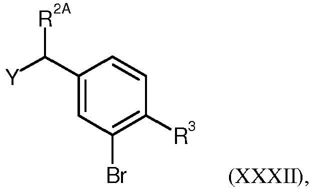 in welcher R^{2A} und R³ die oben angegebenen Bedeutungen haben
und
- Y: für eine geeignete Abgangsgruppe wie Chlor, Brom, Iod, Mesylat, Triflat oder Tosylat steht,
zu einer Verbindung der Formel (XXXIII) 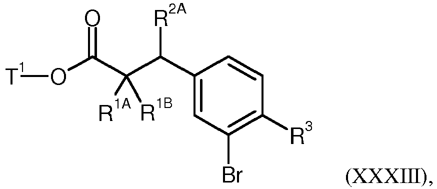 in welcher R^{1A}, R^{1B}, R^{2A}, R³ und T¹ die oben angegebenen Bedeutungen haben,
alkyliert, anschließend mit Benzylamin in Gegenwart einer Base und eines PalladiumKatalysators zu einer Verbindung der Formel (XXXIV) 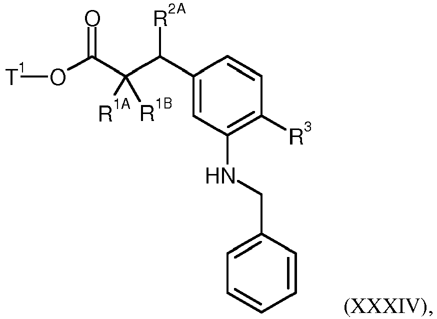 in welcher R^{1A}, R^{1B}, R^{2A}, R³ und T¹ die oben angegebenen Bedeutungen haben,
umsetzt und die *N*-Benzylgruppe dann durch Hydrogenolyse unter Erhalt eines 3-(3-Aminophenyl)propionsäureesters der Formel (III-B) 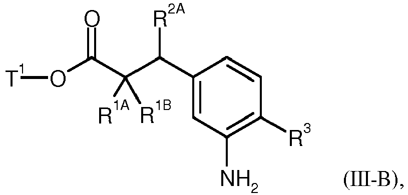 in welcher R^{1A}, R^{1B}, R^{2A}, R³ und T¹ die oben angegebenen Bedeutungen haben,
entfernt,
oder

### [D] einen Acrylsäureester der Formel (XXXV)

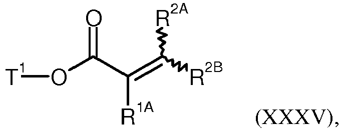

in welcher R^{1A}, R^{2A}, R^{2B} und T¹ die oben angegebenen Bedeutungen haben,
in einem inerten Lösungsmittel entweder (i) unter Rhodium(I)-Katalyse mit einer Phenylboronsäure der Formel (XXXVI) 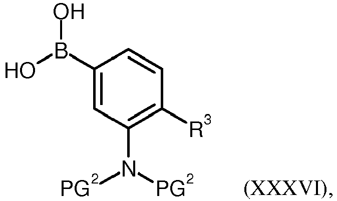 in welcher R³ die oben angegebene Bedeutung hat
und
- PG²: für Benzyl oder 4-Methoxybenzyl als inerte Amino-Schutzgruppe steht,
oder (*ii*) unter Kupfer(I)-Katalyse mit einer Phenylmagnesium-Verbindung der Formel (XXXVII) 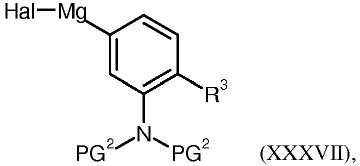 in welcher PG² und R³ die oben angegebenen Bedeutungen haben
und
- Hal: für Chlor oder Brom steht,
zu einer Verbindung der Formel (XXXVIII) 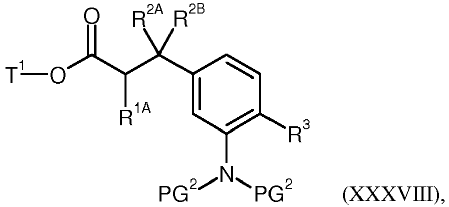 in welcher PG², R^{1A}, R^{2A}, R^{2B}, R³ und T¹ die oben angegebenen Bedeutungen haben, umsetzt und nachfolgend die Amino-Schutzgruppen PG² nach üblichen Methoden durch Hydrogenolyse oder auf oxidativem Wege unter Erhalt eines 3-(3-Aminophenyl)propionsäureesters der Formel (III-C) 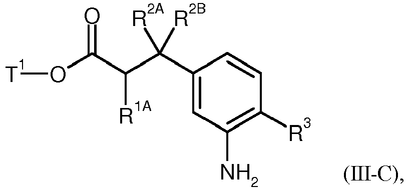 in welcher R^{1A}, R^{2A}, R^{2B}, R³ und T¹ die oben angegebenen Bedeutungen haben,
entfernt.

Zur Deprotonierung des Phosphonoesters (XXII) bei den Olefinierungsreaktionen (XXII) + (XXIII) → (XXIV) und (XXII) + (XXV) → (XXVI) [Horner-Wittig-Reaktion] eignen sich insbesondere nicht-nukleophile, starke Basen wie beispielsweise Natrium- oder Kaliumhydrid, Lithium-, Natrium- oder Kalium-bis(trimethylsilyl)amid oder Lithiumdiisopropylamid; bevorzugt wird Natriumhydrid verwendet. Als inertes Lösungsmittel werden bei dieser Reaktion vorzugsweise Ether wie Diethylether, Diisopropylether, Methyl-*tert*.-butylether, Tetrahydrofuran, 1,2-Dimethoxyethan oder Bis-(2-methoxyethyl)-ether eingesetzt, gegebenenfalls im Gemisch mit *N*,*N*-Dimethylformamid, Dimethylsulfoxid oder Kohlenwasserstoffen wie Pentan, Hexan oder Heptan. Die Umsetzung erfolgt üblicherweise in einem Temperaturbereich von -20°C bis +40°C.

Die Hydrierung in den Verfahrensschritten (XXIV) → (III-A), (XXVI) → (III-A) und (XXX) → (III-A) wird in der Regel unter einer stationären Wasserstoffatmosphäre bei normalem oder erhöhtem Druck durchgeführt. Als Katalysator wird hierbei bevorzugt Palladium oder Platin auf Aktivkohle (als Trägermaterial) eingesetzt. Die Entfernung der Amino-Schutzgruppe(n) in den Transformationen (XXVII) → (III-A), (XXXIV) → (III-B) und (XXXVIII) → (III-C) erfolgt üblicherweise durch Hydrogenolyse nach der gleichen Prozedur; im Falle, dass PG² in (XXVII) bzw. (XXXVIII) für 4-Methoxybenzyl steht, kann dies alternativ auch auf oxidativem Wege geschehen, beispielsweise mit Hilfe von 2,3-Dichlor-5,6-dicyano-1,4-benzochinon (DDQ) oder Ammoniumcer(IV)-nitrat.

Als Palladium-Katalysator für die Umsetzung (XXVIII) + (XXIX) → (XXX) [Heck-Reaktion] wird vorzugsweise Palladium(II)acetat oder Tris(dibenzylidenaceton)dipalladium(0), jeweils in Kombination mit einem Phosphin-Liganden wie beispielsweise Tri-*tert*.-butylphosphin, Triphenylphosphin oder Tri-2-tolylphosphin, eingesetzt.

Zur α-Deprotonierung des Carbonsäureesters (XXXI) bei der Alkylierungsreaktion (XXXI) + (XXXII) → (XXXIII) eignen sich insbesondere nicht-nukleophile, starke Basen wie beispielsweise Natrium- oder Kalium-*tert*.-butylat, Natrium- oder Kaliumhydrid, Lithiumdiisopropylamid, Lithium-, Natrium- oder Kalium-bis(trimethylsilyl)amid, oder *n*-, *sec.-* oder *tert*.-Butyllithium; bevorzugt wird Lithiumdiisopropylamid oder *n*-Butyllithium verwendet. Als inertes Lösungsmittel werden bei dieser Reaktion vorzugsweise Ether wie Diethylether, Diisopropylether, Methyl-*tert*.-butylether, Tetrahydrofuran, 1,2-Dimethoxyethan oder Bis-(2-methoxyethyl)-ether eingesetzt, gegebenenfalls im Gemisch mit *N*,*N*-Dimethylformamid oder Kohlenwasserstoffen wie Pentan, Hexan oder Heptan. Die Umsetzung erfolgt üblicherweise in einem Temperaturbereich von -80°C bis +25°C.

Für die Transformation (XXXIII) → (XXXIV) [Buchwald-Hartwig-Kupplung mit Benzylamin] wird bevorzugt Tris(dibenzylidenaceton)dipalladium(0) als Katalysator in Verbindung mit (±)-2,2'-Bis(diphenylphosphino)-1,1'-binaphthyl als Phosphin-Liganden und Natrium- oder Kalium-*tert*.-butylat als Base verwendet [vgl. z.B. J. P. Wolfe und S. L. Buchwald, Organic Syntheses, Coll. Vol. 10, 423 (2004), Vol. 78, 23 (2002)].

Die Kupplungsreaktionen (XXXV) + (XXXVI) → (XXXVIII) und (XXXV) + (XXXVII) → (XXXVIII) werden in Anlehnung an literaturbeschriebene Methoden durchgeführt [siehe z.B. N. Miyaura et al., Organometallics 16, 4229 (1997); T. Hayashi, Synlett, Special Issue 2001, 879-887; P. Knochel et al., Tetrahedron 56, 2727-2731 (2000), Angew. Chem. 120, 6907-6911 (2008)].

Die zuvor beschriebenen Reaktionen können bei normalem, bei erhöhtem oder bei erniedrigtem Druck durchgeführt werden (z.B. im Bereich von 0.5 bis 5 bar); im Allgemeinen arbeitet man jeweils bei Normaldruck.

Eine Trennung der erfindungsgemäßen Verbindungen in die entsprechenden Enantiomere und/ oder Diastereomere kann gegebenenfalls, je nach Zweckmäßigkeit, auch bereits auf der Stufe der Verbindungen (II), (III), (IV), (V), (VII), (IX), (XI), (XIV), (XV), (XVI), (XXI), (XXVII), (XXXIII), (XXXIV) und/oder (XXXVIII) erfolgen, welche dann in separierter Form entsprechend den zuvor beschriebenen Verfahrenssequenzen weiter umgesetzt werden. Eine solche Auftrennung der Stereoisomeren läßt sich nach üblichen, dem Fachmann bekannten Methoden durchführen. Im Rahmen der vorliegenden Erfindung werden vorzugsweise chromatographische Verfahren an achiralen bzw. chiralen Trennphasen angewandt; im Falle von Carbonsäuren als Zwischen- oder Endprodukten kann alternativ auch eine Trennung über diastereomere Salze erfolgen.

Die Verbindungen der Formeln (VI), (X), (XII), (XIII), (XVII), (XVIII), (XXII), (XXIII), (XXV), (XXVIII), (XXIX), (XXXI), (XXXII), (XXXV), (XXXVI) und (XXXVII) sind entweder kommerziell erhältlich oder als solche in der Literatur beschrieben, oder sie können auf für den Fachmann offenkundigem Wege in Anlehnung an in der Literatur publizierte Methoden hergestellt werden. Zahlreiche detaillierte Vorschriften sowie Literaturangaben zur Herstellung der Ausgangsmaterialien befinden sich auch im Experimentellen Teil im Abschnitt zur Herstellung der Ausgangsverbindungen und Intermediate.

Die Herstellung der erfindungsgemäßen Verbindungen kann durch die folgenden Reaktionsschemata beispielhaft veranschaulicht werden:

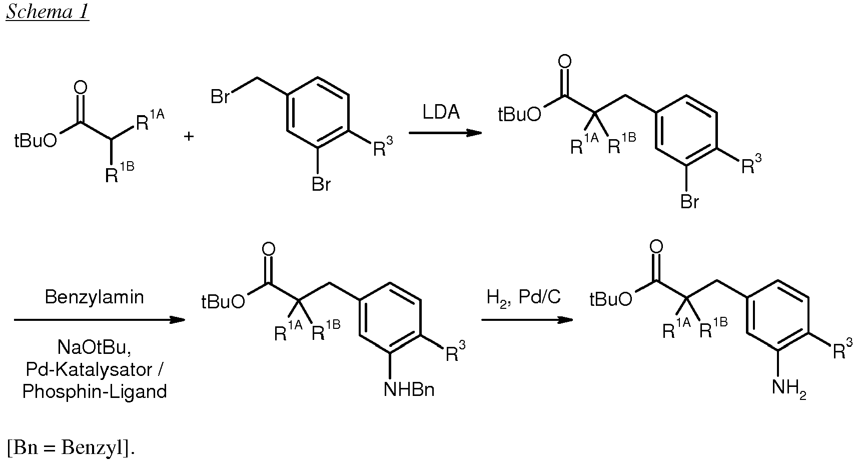

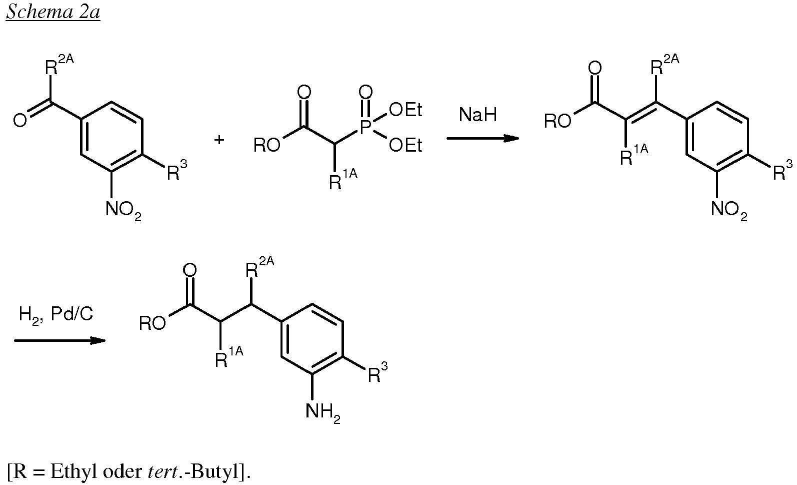

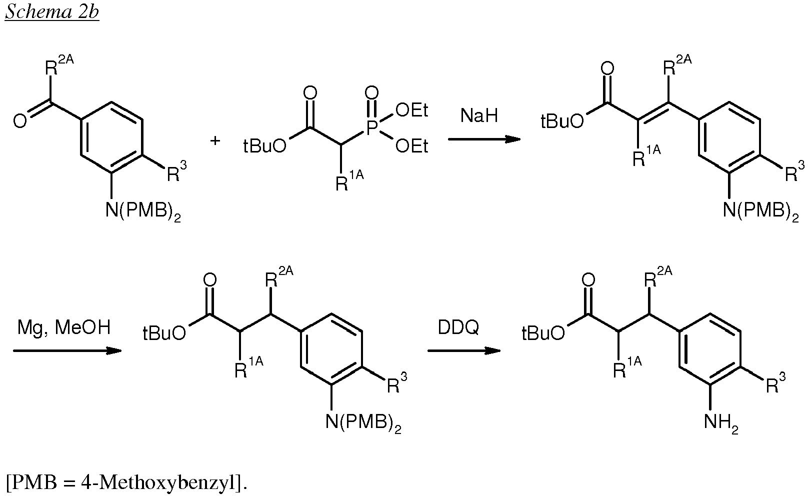

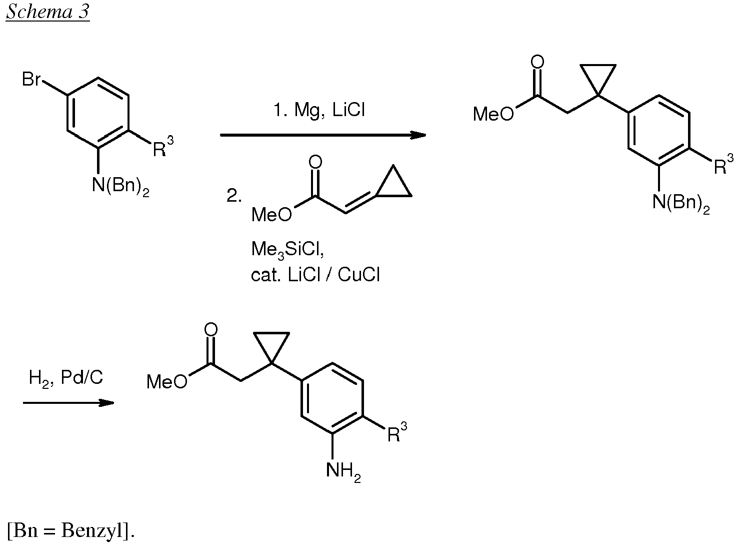

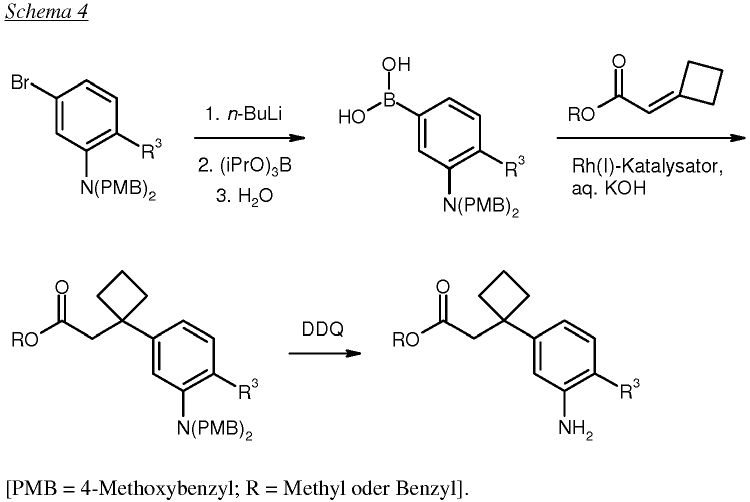

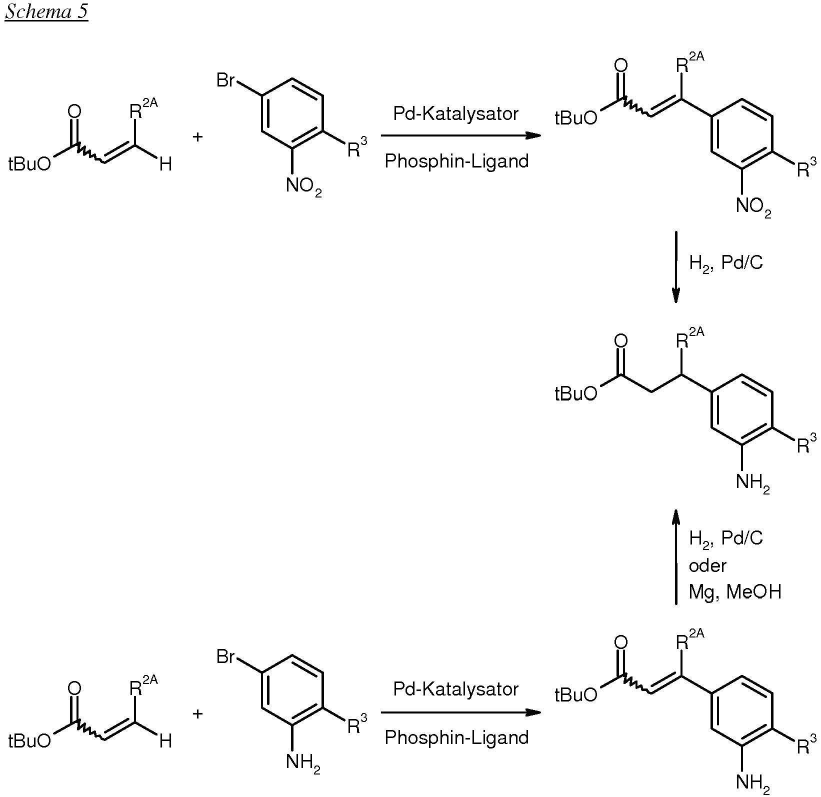

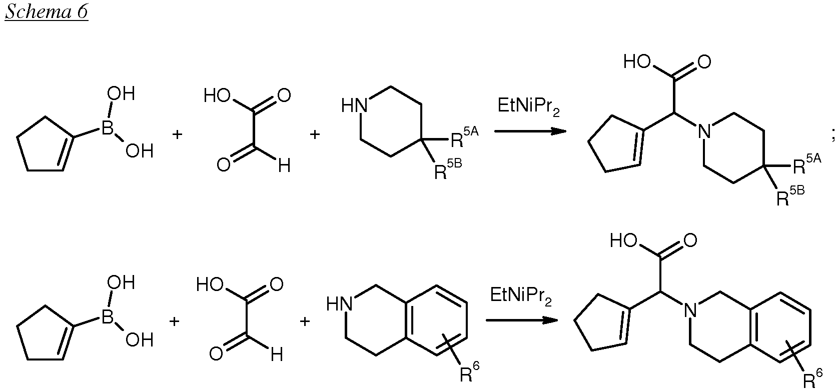

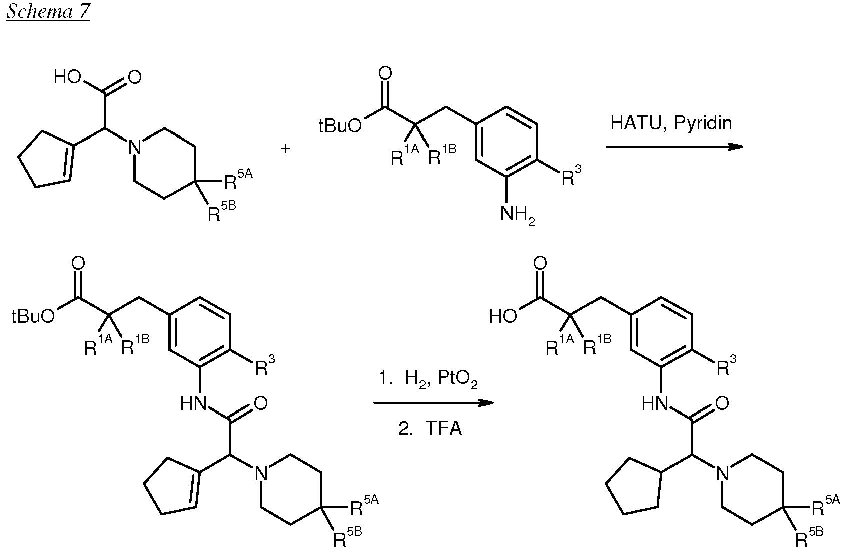

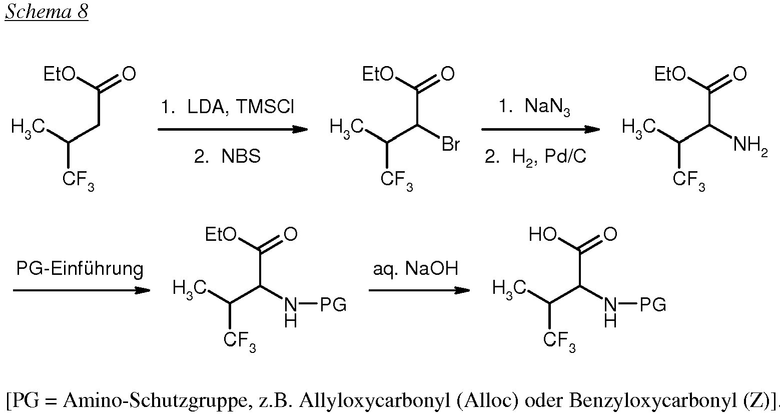

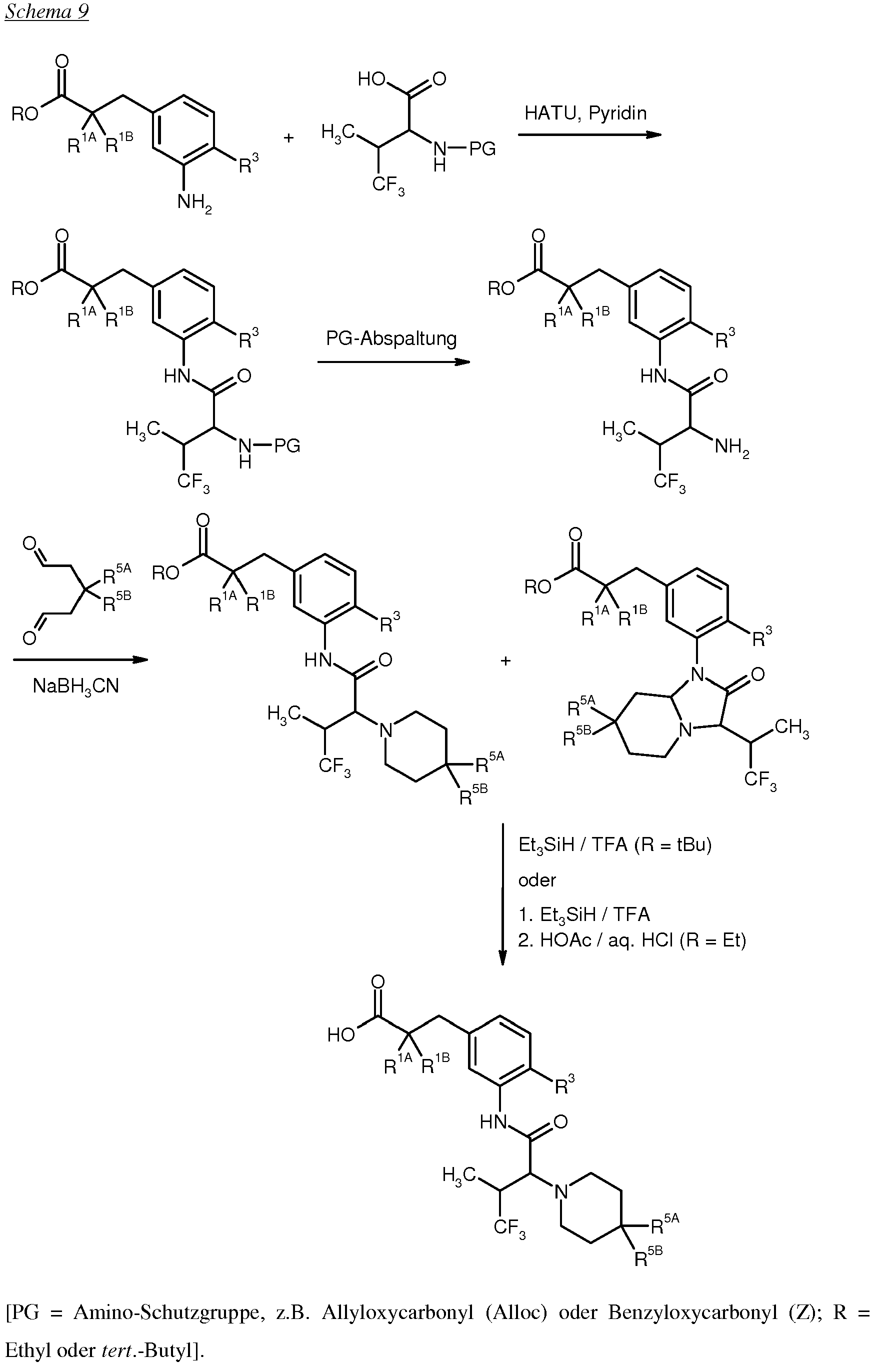

Die erfindungsgemäßen Verbindungen besitzen wertvolle pharmakologische Eigenschaften und können zur Vorbeugung und Behandlung von Erkrankungen bei Menschen und Tieren verwendet werden.

Die erfindungsgemäßen Verbindungen stellen potente Aktivatoren der löslichen Guanylatcyclase dar. Sie führen zu einer Gefäßrelaxation, zu einer Thrombozytenaggregationshemmung und zu einer Blutdrucksenkung sowie zu einer Steigerung des koronaren Blutflusses. Diese Wirkungen sind über eine direkte, Häm-unabhängige Aktivierung der löslichen Guanylatcyclase und einen intrazellulären cGMP-Anstieg vermittelt.

Darüber hinaus verfügen die erfindungsgemäßen Verbindungen über vorteilhafte pharmakokinetische Eigenschaften, insbesondere bezüglich ihrer Bioverfügbarkeit und/oder Wirkdauer nach intravenöser oder oraler Gabe.

Die erfindungsgemäßen Verbindungen eignen sich in besonderem Maße zur Behandlung und/oder Prävention von kardiovaskulären, pulmonalen, thromboembolischen und fibrotischen Erkrankungen.

Die erfindungsgemäßen Verbindungen können daher in Arzneimitteln eingesetzt werden zur Behandlung und/oder Prävention von kardiovaskulären Erkrankungen, wie beispielsweise Bluthochdruck (Hypertonie), Herzinsuffizienz, koronare Herzerkrankung, stabile und instabile Angina pectoris, pulmonale arterielle Hypertonie (PAH) und andere Formen der pulmonalen Hypertonie (PH), renale Hypertonie, periphere und kardiale Gefäßerkrankungen, Arrhythmien, Rhythmusstörungen der Vorhöfe und der Kammern sowie Überleitungsstörungen wie beispielsweise atrioventrikuläre Blockaden des Grades I-III, supraventrikuläre Tachyarrhythmie, Vorhofflimmern, Vorhofflattern, Kammerflimmern, Kammerflattern, ventrikuläre Tachyarrhythmie, Torsade de pointes-Tachykardie, Extrasystolen des Vorhofs und des Ventrikels, AV-junktionale Extrasystolen, Sick-Sinus-Syndrom, Synkopen, AV-Knoten-Reentry-Tachykardie, Wolff-Parkinson-White-Syndrom, akutes Koronarsyndrom (ACS), autoimmune Herzerkrankungen (Perikarditis, Endokarditis, Valvolitis, Aortitis, Kardiomyopathien), Boxerkardiomyopathie, Aneurysmen, Schock wie kardiogener Schock, septischer Schock und anaphylaktischer Schock, ferner zur Behandlung und/oder Prävention von thromboembolischen Erkrankungen und Ischämien, wie myokardiale Ischämie, Myokardinfarkt, Hirnschlag, Herzhypertrophie, transistorische und ischämische Attacken, Präeklampsie, entzündliche kardiovaskuläre Erkrankungen, Spasmen der Koronararterien und peripherer Arterien, Ödembildung wie beispielsweise pulmonales Ödem, Hirnödem, renales Ödem oder Herzinsuffizienz-bedingtes Ödem, periphere Durchblutungsstörungen, Reperfusionsschäden, arterielle und venöse Thrombosen, Mikroalbuminurie, Herzmuskelschwäche, endotheliale Dysfunktion, mikro- und makrovaskuläre Schädigungen (Vaskulitis), sowie zur Verhinderung von Restenosen beispielsweise nach Thrombolyse-Therapien, percutan-transluminalen Angioplastien (PTA), percutan-transluminalen Koronarangioplastien (PTCA), Herztransplantationen und Bypass-Operationen.

Im Sinne der vorliegenden Erfindung umfasst der Begriff Herzinsuffizienz sowohl akute als auch chronische Erscheinungsformen der Herzinsuffizienz wie auch spezifische oder verwandte Krankheitsformen hiervon, wie akute dekompensierte Herzinsuffizienz, Rechtsherzinsuffizienz, Linksherzinsuffizienz, Globalinsuffizienz, ischämische Kardiomyopathie, dilatative Kardiomyopathie, hypertrophe Kardiomyopathie, idiopathische Kardiomyopathie, angeborene Herzfehler, Herzklappenfehler, Herzinsuffizienz bei Herzklappenfehlern, Mitralklappenstenose, Mitralklappeninsuffizienz, Aortenklappenstenose, Aortenklappeninsuffizienz, Trikuspidalstenose, Trikuspidalinsuffizienz, Pulmonalklappenstenose, Pulmonalklappeninsuffizienz, kombinierte Herzklappenfehler, Herzmuskelentzündung (Myokarditis), chronische Myokarditis, akute Myokarditis, virale Myokarditis, diabetische Herzinsuffizienz, alkoholtoxische Kardiomyopathie, kardiale Speichererkrankungen sowie diastolische und systolische Herzinsuffizienz.

Darüber hinaus können die erfindungsgemäßen Verbindungen auch zur Behandlung und/oder Prävention von Arteriosklerose, Lipidstoffwechselstörungen, Hypolipoproteinämien, Dyslipidämien, Hypertriglyceridämien, Hyperlipidämien, kombinierten Hyperlipidämien, Hypercholesterolämien, Abetalipoproteinämie, Sitosterolämie, Xanthomatose, Tangier-Krankheit, Fettsucht (Adipositas), Fettleibigkeit (Obesitas) sowie des Metabolischen Syndroms eingesetzt werden.

Weiterhin können die erfindungsgemäßen Verbindungen zur Behandlung und/oder Prävention von primärem und sekundärem Raynaud-Phänomen, Mikrozirkulationsstörungen, Claudicatio, Tinnitus, peripheren und autonomen Neuropathien, diabetischen Mikroangiopathien, diabetischer Retinopathie, diabetischen Geschwüren an den Extremitäten, Gangrän, CREST-Syndrom, Erythematose, Onychomykose sowie von rheumatischen Erkrankungen verwendet werden.

Die erfindungsgemäßen Verbindungen können darüber hinaus zur Verhinderung von ischämie- und/oder reperfusionsbedingten Schädigungen von Organen oder Geweben sowie als Zusatzstoffe für Perfusions- und Konservierungslösungen von Organen, Organteilen, Geweben oder Gewebeteilen menschlichen oder tierischen Ursprungs, insbesondere bei chirurgischen Eingriffen oder im Bereich der Transplantationsmedizin, Verwendung finden.

Die erfindungsgemäßen Verbindungen eignen sich außerdem zur Behandlung und/oder Prävention von Nierenerkrankungen, insbesondere von Niereninsuffizienz und Nierenversagen. Im Sinne der vorliegenden Erfindung umfassen die Begriffe Niereninsuffizienz und Nierenversagen sowohl akute als auch chronische Erscheinungsformen hiervon wie auch diesen zugrundeliegende oder verwandte Nierenerkrankungen, wie renale Hypoperfusion, intradialytische Hypotonie, obstruktive Uropathie, Glomerulopathien, Glomerulonephritis, akute Glomerulonephritis, Glomerulosklerose, tubulointerstitielle Erkrankungen, nephropathische Erkrankungen wie primäre und angeborene Nierenerkrankung, Nierenentzündung, immunologische Nierenerkrankungen wie Nierentransplantat-Abstoßung und Immunkomplex-induzierte Nierenerkrankungen, durch toxische Substanzen induzierte Nephropathie, Kontrastmittel-induzierte Nephropathie, diabetische und nicht-diabetische Nephropathie, Pyelonephritis, Nierenzysten, Nephrosklerose, hypertensive Nephrosklerose und nephrotisches Syndrom, welche diagnostisch beispielsweise durch abnorm verminderte Kreatinin- und/oder Wasser-Ausscheidung, abnorm erhöhte Blutkonzentrationen von Harnstoff, Stickstoff, Kalium und/oder Kreatinin, veränderte Aktivität von Nierenenzymen wie z.B. Glutamylsynthetase, veränderte Urinosmolarität oder Urinmenge, erhöhte Mikroalbuminurie, Makroalbuminurie, Läsionen an Glomerula und Arteriolen, tubuläre Dilatation, Hyperphosphatämie und/oder die Notwendigkeit zur Dialyse charakterisiert werden können. Die vorliegende Erfindung umfasst auch die Verwendung der erfindungsgemäßen Verbindungen zur Behandlung und/oder Prävention von Folgeerscheinungen einer Niereninsuffizienz, wie beispielsweise Hypertonie, Lungenödem, Herzinsuffizienz, Urämie, Anämie, Elektrolytstörungen (z.B. Hyperkalämie, Hyponaträmie) und Störungen im Knochen- und Kohlenhydrat-Metabolismus.

Darüber hinaus sind die erfindungsgemäßen Verbindungen zur Behandlung und/oder Prävention von Erkrankungen des Urogenitalsystems geeignet, wie beispielsweise benignes Prostata-Syndrom (BPS), benigne Prostatahyperplasie (BPH), benigne Prostatavergrößerung (BPE), Blasenentleerungsstörungen (BOO), untere Harnwegssyndrome (LUTS), neurogene überaktive Blase (OAB), Inkontinenz wie beispielsweise Misch-, Drang-, Stress- oder Überlauf-Inkontinenz (MUI, UUI, SUI, OUI), Beckenschmerzen sowie erektile Dysfunktion und weibliche sexuelle Dysfunktion.

Die erfindungsgemäßen Verbindungen eignen sich auch zur Behandlung und/oder Prävention von asthmatischen Erkrankungen, chronisch-obstruktiven Atemwegserkrankungen (COPD), des akuten Atemwegssyndroms (ARDS) und der akuten Lungenschädigung (ALI), alpha-1-Antitrypsin-Defizienz (AATD), Lungenfibrose, Lungenemphysem (z.B. durch Zigarettenrauch induziertes Lungenemphysem) und zystischer Fibrose (CF) sowie von pulmonaler arterieller Hypertonie (PAH) und anderen Formen der pulmonalen Hypertonie (PH), einschließlich der mit Linksherzerkrankung, HIV, Sichelzellanämie, Thromboembolien, Sarkoidose, COPD oder Lungenfibrose assoziierten pulmonalen Hypertonie.

Die in der vorliegenden Erfindung beschriebenen Verbindungen stellen auch Wirkstoffe zur Bekämpfung von Krankheiten im Zentralnervensystem dar, die durch Störungen des NO/cGMP-Systems gekennzeichnet sind. Insbesondere sind sie geeignet zur Verbesserung der Wahrnehmung, Konzentrationsleistung, Lernleistung oder Gedächtnisleistung nach kognitiven Störungen, wie sie insbesondere bei Situationen/Krankheiten/Syndromen auftreten wie "Mild cognitive impairment", altersassoziierte Lern- und Gedächtnisstörungen, altersassoziierte Gedächtnisverluste, vaskuläre Demenz, Schädel-Hirn-Trauma, Schlaganfall, Demenz, die nach Schlaganfällen auftritt ("post stroke dementia"), post-traumatisches Schädel-Hirn-Trauma, allgemeine Konzentrationsstörungen, Konzentrationsstörungen bei Kindern mit Lern- und Gedächtnisproblemen, Alzheimer'sche Krankheit, Demenz mit Lewy-Körperchen, Demenz mit Degeneration der Frontallappen einschließlich des Pick's-Syndroms, Parkinson'sche Krankheit, progressiver nuclear palsy, Demenz mit corticobasaler Degeneration, Amyolateralsklerose (ALS), Huntington'sche Krankheit, Demyelinisation, Multiple Sklerose, thalamische Degeneration, Creutzfeld-Jacob-Demenz, HIV-Demenz, Schizophrenie mit Demenz oder Korsakoff-Psychose. Sie eignen sich auch zur Behandlung und/oder Prävention von Erkrankungen des Zentralnervensystems wie Angst-, Spannungs- und Depressionszuständen, zentral-nervös bedingten Sexualdysfunktionen und Schlafstörungen sowie zur Regulierung krankhafter Störungen der Nahrungs-, Genuss- und Suchtmittelaufnahme.

Weiterhin eignen sich die erfindungsgemäßen Verbindungen auch zur Regulation der cerebralen Durchblutung und stellen wirkungsvolle Mittel zur Bekämpfung von Migräne dar. Auch eignen sie sich zur Prophylaxe und Bekämpfung der Folgen cerebraler Infarktgeschehen (Apoplexia cerebri) wie Schlaganfall, cerebraler Ischämien und des Schädel-Hirn-Traumas. Ebenso können die erfindungsgemäßen Verbindungen zur Bekämpfung von Schmerzzuständen eingesetzt werden.

Zudem besitzen die erfindungsgemäßen Verbindungen anti-inflammatorische Wirkung und können daher als entzündungshemmende Mittel zur Behandlung und/oder Prävention von Sepsis (SIRS), multiplem Organversagen (MODS, MOF), entzündlichen Erkrankungen der Niere, chronischen Darmentzündungen (IBD, Morbus Crohn, Colitis ulcerosa), Pankreatitis, Peritonitis, rheumatoiden Erkrankungen, entzündlichen Hauterkrankungen und entzündlichen Augenerkrankungen eingesetzt werden.

Die erfindungsgemäßen Verbindungen sind ferner zur Behandlung und/oder Prävention von fibrotischen Erkrankungen der inneren Organe, wie beispielsweise der Lunge, des Herzens, der Niere, des Knochenmarks und insbesondere der Leber, sowie von dermatologischen Fibrosen und fibrotischen Erkrankungen des Auges geeignet. Im Sinne der vorliegenden Erfindung umfasst der Begriff fibrotische Erkrankungen insbesondere solche Erkrankungen wie Leberfibrose, Leberzirrhose, Lungenfibrose, Endomyokardfibrose, Nephropathie, Glomerulonephritis, interstitielle Nierenfibrose, fibrotische Schäden in Folge von Diabetes, Knochenmarksfibrose und ähnliche fibrotische Erkrankungen, Sklerodermie, Morphaea, Keloide, hypertrophe Narbenbildung, Naevi, diabetische Retinopathie, proliferative Vitroretinopathie und Erkrankungen des Bindegewebes (z.B. Sarkoidose). Die erfindungsgemäßen Verbindungen können ebenso verwendet werden zur Förderung der Wundheilung, zur Bekämpfung postoperativer Narbenbildung, z.B. nach Glaukom-Operationen, und zu kosmetischen Zwecken bei alternder oder verhornender Haut.

Aufgrund ihres Wirkprofils eignen sich die erfindungsgemäßen Verbindungen insbesondere zur Behandlung und/oder Prävention von kardiovaskulären Erkrankungen, wie Herzinsuffizienz, Angina pectoris, Hypertonie und pulmonale Hypertonie, sowie von thromboembolischen Erkrankungen, Ischämien, Gefäßerkrankungen, Mikrozirkulationsstörungen, Niereninsuffizienz, fibrotischen Erkrankungen und Arteriosklerose.

Weiter offenbart ist die Verwendung der erfindungsgemäßen Verbindungen zur Behandlung und/oder Prävention von Erkrankungen, insbesondere der zuvor genannten Erkrankungen.

Weiterer Gegenstand der vorliegenden Erfindung ist die Verwendung der erfindungsgemäßen Verbindungen zur Herstellung eines Arzneimittels zur Behandlung und/oder Prävention von Erkrankungen, insbesondere der zuvor genannten Erkrankungen.

Weiter offenbart ist die Verwendung der erfindungsgemäßen Verbindungen in einem Verfahren zur Behandlung und/oder Prävention von Erkrankungen, insbesondere der zuvor genannten Erkrankungen.

Weiter offenbart ist ein Verfahren zur Behandlung und/oder Prävention von Erkrankungen, insbesondere der zuvor genannten Erkrankungen, unter Verwendung einer wirksamen Menge von mindestens einer der erfindungsgemäßen Verbindungen.

Die erfindungsgemäßen Verbindungen können allein oder bei Bedarf in Kombination mit anderen Wirkstoffen eingesetzt werden. Weiterer Gegenstand der vorliegenden Erfindung sind Arzneimittel, enthaltend mindestens eine der erfindungsgemäßen Verbindungen und einen oder mehrere weitere Wirkstoffe, insbesondere zur Behandlung und/oder Prävention der zuvor genannten Erkrankungen. Als geeignete Kombinationswirkstoffe seien beispielhaft und vorzugsweise genannt:
- organische Nitrate und NO-Donatoren, wie beispielsweise Natriumnitroprussid, Nitroglycerin, Isosorbidmononitrat, Isosorbiddinitrat, Molsidomin oder SIN-1, sowie inhalatives NO;
- Verbindungen, die den Abbau von cyclischem Guanosinmonophosphat (cGMP) inhibieren, wie beispielsweise Inhibitoren der Phosphodiesterasen (PDE) 1, 2 und/oder 5, insbesondere PDE 5-Inhibitoren wie Sildenafil, Vardenafil und Tadalafil;
- NO-unabhängige, jedoch Häm-abhängige Stimulatoren der Guanylatcyclase, wie insbesondere Riociguat sowie die in WO 00/06568, WO 00/06569, WO 02/42301 und WO 03/095451 beschriebenen Verbindungen;
- antithrombotisch wirkende Mittel, beispielhaft und vorzugsweise aus der Gruppe der Thrombozytenaggregationshemmer, der Antikoagulantien oder der profibrinolytischen Substanzen;
- den Blutdruck senkende Wirkstoffe, beispielhaft und vorzugsweise aus der Gruppe der Calcium-Antagonisten, Angiotensin AII-Antagonisten, ACE-Hemmer, Endothelin-Antagonisten, Renin-Inhibitoren, alpha-Rezeptoren-Blocker, beta-Rezeptoren-Blocker, Mineralocorticoid-Rezeptor-Antagonisten sowie der Diuretika; und/oder
- den Fettstoffwechsel verändernde Wirkstoffe, beispielhaft und vorzugsweise aus der Gruppe der Thyroidrezeptor-Agonisten, Cholesterinsynthese-Inhibitoren wie beispielhaft und vorzugsweise HMG-CoA-Reduktase- oder Squalensynthese-Inhibitoren, der ACAT-Inhibitoren, CETP-Inhibitoren, MTP-Inhibitoren, PPAR-alpha-, PPAR-gamma- und/oder PPAR-delta-Agonisten, Cholesterin-Absorptionshemmer, Lipase-Inhibitoren, polymeren Gallensäureadsorber, Gallensäure-Reabsorptionshemmer und Lipoprotein(a)-Antagonisten.

Unter antithrombotisch wirkenden Mittel werden vorzugsweise Verbindungen aus der Gruppe der Thrombozytenaggregationshemmer, der Antikoagulantien oder der profibrinolytischen Substanzen verstanden.

Bei einer bevorzugten Ausführungsform der Erfindung werden die erfindungsgemäßen Verbindungen in Kombination mit einem Thrombozytenaggregationshemmer, wie beispielhaft und vorzugsweise Aspirin, Clopidogrel, Ticlopidin oder Dipyridamol, verabreicht.

Bei einer bevorzugten Ausführungsform der Erfindung werden die erfindungsgemäßen Verbindungen in Kombination mit einem Thrombin-Inhibitor, wie beispielhaft und vorzugsweise Ximelagatran, Melagatran, Dabigatran, Bivalirudin oder Clexane, verabreicht.

Bei einer bevorzugten Ausführungsform der Erfindung werden die erfindungsgemäßen Verbindungen in Kombination mit einem GPIIb/IIIa-Antagonisten, wie beispielhaft und vorzugsweise Tirofiban oder Abciximab, verabreicht.

Bei einer bevorzugten Ausführungsform der Erfindung werden die erfindungsgemäßen Verbindungen in Kombination mit einem Faktor Xa-Inhibitor, wie beispielhaft und vorzugsweise Rivaroxaban, Apixaban, Fidexaban, Razaxaban, Fondaparinux, Idraparinux, DU-176b, PMD-3112, YM-150, KFA-1982, EMD-503982, MCM-17, MLN-1021, DX 9065a, DPC 906, JTV 803, SSR-126512 oder SSR-128428, verabreicht.

Bei einer bevorzugten Ausführungsform der Erfindung werden die erfindungsgemäßen Verbindungen in Kombination mit Heparin oder einem low molecular weight (LMW)-Heparin-Derivat verabreicht.

Bei einer bevorzugten Ausführungsform der Erfindung werden die erfindungsgemäßen Verbindungen in Kombination mit einem Vitamin K-Antagonisten, wie beispielhaft und vorzugsweise Coumarin, verabreicht.

Unter den Blutdruck senkenden Mitteln werden vorzugsweise Verbindungen aus der Gruppe der Calcium-Antagonisten, Angiotensin AII-Antagonisten, ACE-Hemmer, Endothelin-Antagonisten, Renin-Inhibitoren, alpha-Rezeptoren-Blocker, beta-Rezeptoren-Blocker, Mineralocorticoid-Rezeptor-Antagonisten sowie der Diuretika verstanden.

Bei einer bevorzugten Ausführungsform der Erfindung werden die erfindungsgemäßen Verbindungen in Kombination mit einem Calcium-Antagonisten, wie beispielhaft und vorzugsweise Nifedipin, Amlodipin, Verapamil oder Diltiazem, verabreicht.

Bei einer bevorzugten Ausführungsform der Erfindung werden die erfindungsgemäßen Verbindungen in Kombination mit einem alpha-1-Rezeptoren-Blocker, wie beispielhaft und vorzugsweise Prazosin, verabreicht.

Bei einer bevorzugten Ausführungsform der Erfindung werden die erfindungsgemäßen Verbindungen in Kombination mit einem beta-Rezeptoren-Blocker, wie beispielhaft und vorzugsweise Propranolol, Atenolol, Timolol, Pindolol, Alprenolol, Oxprenolol, Penbutolol, Bupranolol, Metipranolol, Nadolol, Mepindolol, Carazalol, Sotalol, Metoprolol, Betaxolol, Celiprolol, Bisoprolol, Carteolol, Esmolol, Labetalol, Carvedilol, Adaprolol, Landiolol, Nebivolol, Epanolol oder Bucindolol, verabreicht.

Bei einer bevorzugten Ausführungsform der Erfindung werden die erfindungsgemäßen Verbindungen in Kombination mit einem Angiotensin AII-Antagonisten, wie beispielhaft und vorzugsweise Losartan, Candesartan, Valsartan, Telmisartan oder Embursatan, verabreicht.

Bei einer bevorzugten Ausführungsform der Erfindung werden die erfindungsgemäßen Verbindungen in Kombination mit einem ACE-Hemmer, wie beispielhaft und vorzugsweise Enalapril, Captopril, Lisinopril, Ramipril, Delapril, Fosinopril, Quinopril, Perindopril oder Trandopril, verabreicht.

Bei einer bevorzugten Ausführungsform der Erfindung werden die erfindungsgemäßen Verbindungen in Kombination mit einem Endothelin-Antagonisten, wie beispielhaft und vorzugsweise Bosentan, Darusentan, Ambrisentan oder Sitaxsentan, verabreicht.

Bei einer bevorzugten Ausführungsform der Erfindung werden die erfindungsgemäßen Verbindungen in Kombination mit einem Renin-Inhibitor, wie beispielhaft und vorzugsweise Aliskiren, SPP-600 oder SPP-800, verabreicht.

Bei einer bevorzugten Ausführungsform der Erfindung werden die erfindungsgemäßen Verbindungen in Kombination mit einem Mineralocorticoid-Rezeptor-Antagonisten, wie beispielhaft und vorzugsweise Spironolacton oder Eplerenon, verabreicht.

Bei einer bevorzugten Ausführungsform der Erfindung werden die erfindungsgemäßen Verbindungen in Kombination mit einem Diuretikum, wie beispielhaft und vorzugsweise Furosemid, Bumetanid, Torsemid, Bendroflumethiazid, Chlorthiazid, Hydrochlorthiazid, Hydroflumethiazid, Methyclothiazid, Polythiazid, Trichlormethiazid, Chlorthalidon, Indapamid, Metolazon, Quinethazon, Acetazolamid, Dichlorphenamid, Methazolamid, Glycerin, Isosorbid, Mannitol, Amilorid oder Triamteren, verabreicht.

Unter den Fettstoffwechsel verändernden Mitteln werden vorzugsweise Verbindungen aus der Gruppe der CETP-Inhibitoren, Thyroidrezeptor-Agonisten, Cholesterinsynthese-Inhibitoren wie HMG-CoA-Reduktase- oder Squalensynthese-Inhibitoren, der ACAT-Inhibitoren, MTP-Inhibitoren, PPAR-alpha-, PPAR-gamma- und/oder PPAR-delta-Agonisten, Cholesterin-Absorptionshemmer, polymeren Gallensäureadsorber, Gallensäure-Reabsorptionshemmer, Lipase-Inhibitoren sowie der Lipoprotein(a)-Antagonisten verstanden.

Bei einer bevorzugten Ausführungsform der Erfindung werden die erfindungsgemäßen Verbindungen in Kombination mit einem CETP-Inhibitor, wie beispielhaft und vorzugsweise Torcetrapib (CP-529 414), JJT-705 oder CETP-vaccine (Avant), verabreicht.

Bei einer bevorzugten Ausführungsform der Erfindung werden die erfindungsgemäßen Verbindungen in Kombination mit einem Thyroidrezeptor-Agonisten, wie beispielhaft und vorzugsweise D-Thyroxin, 3,5,3'-Triiodothyronin (T3), CGS 23425 oder Axitirome (CGS 26214), verabreicht. Bei einer bevorzugten Ausführungsform der Erfindung werden die erfindungsgemäßen Verbindungen in Kombination mit einem HMG-CoA-Reduktase-Inhibitor aus der Klasse der Statine, wie beispielhaft und vorzugsweise Lovastatin, Simvastatin, Pravastatin, Fluvastatin, Atorvastatin, Rosuvastatin oder Pitavastatin, verabreicht.

Bei einer bevorzugten Ausführungsform der Erfindung werden die erfindungsgemäßen Verbindungen in Kombination mit einem Squalensynthese-Inhibitor, wie beispielhaft und vorzugsweise BMS-188494 oder TAK-475, verabreicht.

Bei einer bevorzugten Ausführungsform der Erfindung werden die erfindungsgemäßen Verbindungen in Kombination mit einem ACAT-Inhibitor, wie beispielhaft und vorzugsweise Avasimibe, Melinamide, Pactimibe, Eflucimibe oder SMP-797, verabreicht.

Bei einer bevorzugten Ausführungsform der Erfindung werden die erfindungsgemäßen Verbindungen in Kombination mit einem MTP-Inhibitor, wie beispielhaft und vorzugsweise Implitapide, BMS-201038, R-103757 oder JTT-130, verabreicht.

Bei einer bevorzugten Ausführungsform der Erfindung werden die erfindungsgemäßen Verbindungen in Kombination mit einem PPAR-gamma-Agonisten, wie beispielhaft und vorzugsweise Pioglitazone oder Rosiglitazone, verabreicht.

Bei einer bevorzugten Ausführungsform der Erfindung werden die erfindungsgemäßen Verbindungen in Kombination mit einem PPAR-delta-Agonisten, wie beispielhaft und vorzugsweise GW 501516 oder BAY 68-5042, verabreicht.

Bei einer bevorzugten Ausführungsform der Erfindung werden die erfindungsgemäßen Verbindungen in Kombination mit einem Cholesterin-Absorptionshemmer, wie beispielhaft und vorzugsweise Ezetimibe, Tiqueside oder Pamaqueside, verabreicht.

Bei einer bevorzugten Ausführungsform der Erfindung werden die erfindungsgemäßen Verbindungen in Kombination mit einem Lipase-Inhibitor, wie beispielhaft und vorzugsweise Orlistat, verabreicht.

Bei einer bevorzugten Ausführungsform der Erfindung werden die erfindungsgemäßen Verbindungen in Kombination mit einem polymeren Gallensäureadsorber, wie beispielhaft und vorzugsweise Cholestyramin, Colestipol, Colesolvam, CholestaGel oder Colestimid, verabreicht.

Bei einer bevorzugten Ausführungsform der Erfindung werden die erfindungsgemäßen Verbindungen in Kombination mit einem Gallensäure-Reabsorptionshemmer, wie beispielhaft und vorzugsweise ASBT (= IBAT)-Inhibitoren wie z.B. AZD-7806, S-8921, AK-105, BARI-1741, SC-435 oder SC-635, verabreicht.

Bei einer bevorzugten Ausführungsform der Erfindung werden die erfindungsgemäßen Verbindungen in Kombination mit einem Lipoprotein(a)-Antagonisten, wie beispielhaft und vorzugsweise Gemcabene calcium (CI-1027) oder Nicotinsäure, verabreicht.

Weiterer Gegenstand der vorliegenden Erfindung sind Arzneimittel, die mindestens eine erfindungsgemäße Verbindung, üblicherweise zusammen mit einem oder mehreren inerten, nichttoxischen, pharmazeutisch geeigneten Hilfsstoffen enthalten, sowie deren Verwendung zu den zuvor genannten Zwecken.

Die erfindungsgemäßen Verbindungen können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch oder als Implantat bzw. Stent.

Für diese Applikationswege können die erfindungsgemäßen Verbindungen in geeigneten Applikationsformen verabreicht werden.

Für die orale Applikation eignen sich nach dem Stand der Technik funktionierende, die erfindungsgemäßen Verbindungen schnell und/oder modifiziert abgebende Applikationsformen, die die erfindungsgemäßen Verbindungen in kristalliner und/oder amorphisierter und/oder gelöster Form enthalten, wie z.B. Tabletten (nicht-überzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer), Nasentropfen, -lösungen oder -sprays, lingual, sublingual oder buccal zu applizierende Tabletten, Filme/Oblaten oder Kapseln, Suppositorien, Ohren- oder Augenpräparationen, Vaginalkapseln, wäßrige Suspensionen (Lotionen, Schüttelmixturen), lipophile Suspensionen, Salben, Cremes, transdermale therapeutische Systeme (z.B. Pflaster), Milch, Pasten, Schäume, Streupuder, Implantate oder Stents.

Bevorzugt sind die orale oder parenterale Applikation, insbesondere die orale und die intravenöse Applikation.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Lactose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und/oder Geruchskorrigentien.

Im Allgemeinen hat es sich als vorteilhaft erwiesen, bei parenteraler Applikation Mengen von etwa 0.001 bis 1 mg/kg, vorzugsweise etwa 0.01 bis 0.5 mg/kg Körpergewicht zur Erzielung wirksamer Ergebnisse zu verabreichen. Bei oraler Applikation beträgt die Dosierung etwa 0.01 bis 100 mg/kg, vorzugsweise etwa 0.01 bis 20 mg/kg und ganz besonders bevorzugt 0.1 bis 10 mg/kg Körpergewicht.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die nachfolgenden Ausführungsbeispiele erläutern die Erfindung. Die Erfindung ist nicht auf die Beispiele beschränkt.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

### A. Beispiele

### Abkürzungen und Akronyme:

- abs.: absolut
- Ac: Acetyl
- Alloc: Allyloxycarbonyl
- aq.: wässrig, wässrige Lösung
- ATP: Adenosin-5'-triphosphat
- Bn: Benzyl
- Boc: *tert*.-Butoxycarbonyl
- Brij^{®}: Polyethylenglycoldodecylether
- BSA: bovines Serumalbumin
- Bsp.: Beispiel
- Bu: Butyl
- c: Konzentration
- ca.: *circa,* ungefähr
- cat.: katalytisch
- CI: chemische Ionisation (bei MS)
- d: Tag(e)
- DC: Dünnschichtchromatographie
- DCI: direkte chemische Ionisation (bei MS)
- DDQ: 2,3-Dichlor-5,6-dicyano-1,4-benzochinon
- de: Diastereomerenüberschuss
- DMF: Dimethylformamid
- DMSO: Dimethylsulfoxid
- d. Th.: der Theorie (bei chemischer Ausbeute)
- DTT: Dithiothreitol
- ee: Enantiomerenüberschuss
- EI: Elektronenstoß-Ionisation (bei MS)
- ent: enantiomerenrein, Enantiomer
- eq.: Äquivalent(e)
- ESI: Elektrospray-Ionisation (bei MS)
- Et: Ethyl
- Fmoc: 9-Fluorenylmethoxycarbonyl
- GC: Gaschromatographie
- ges.: gesättigt
- GTP: Guanosin-5'-triphosphat
- h: Stunde(n)
- HATU: *O*-(7-Azabenzotriazol-1-yl)-*N*,*N*,*N*',*N*'-tetramethyluronium-Hexafluorophosphat
- HOAc: Essigsäure
- HOSu: *N*-Hydroxysuccinimid
- HPLC: Hochdruck-, Hochleistungsflüssigchromatographie
- iPr: Isopropyl
- konz.: konzentriert
- LC-MS: Flüssigchromatographie-gekoppelte Massenspektroskopie
- LDA: Lithiumdiisopropylamid
- Me: Methyl
- min: Minute(n)
- MS: Massenspektroskopie
- NBS: *N*-Bromsuccinimid
- NMR: Kernresonanzspektroskopie
- *p*: para
- Pd/C: Palladium auf Aktivkohle
- Ph: Phenyl
- PMB: *p*-Methoxybenzyl (4-Methoxybenzyl)
- Pr: Propyl
- rac: racemisch, Racemat
- R_{f}: Retentionsindex (bei DC)
- RP: reverse phase (Umkehrphase, bei HPLC)
- RT: Raumtemperatur
- Rₜ: Retentionszeit (bei HPLC oder GC)
- s.o.: siehe oben
- tBu: *tert*.-Butyl
- TEA: Triethanolamin
- TFA: Trifluoressigsäure
- THF: Tetrahydrofuran
- TMS: Trimethylsilyl
- UV: Ultraviolett-Spektroskopie
- v/v: Volumen zu Volumen-Verhältnis (einer Lösung)
- Z: Benzyloxycarbonyl
- zus.: zusammen

### GC-MS- und LC-MS-Methoden:

### Methode 1 (GC-MS):

Instrument: Micromass GCT, GC 6890; Säule: Restek RTX-35, 15 m x 200 µm x 0.33 µm; konstanter Fluss mit Helium: 0.88 ml/min; Ofen: 70°C; Inlet: 250°C; Gradient: 70°C, 30°C/min → 310°C (3 min halten).

### Methode 2 (LC-MS):

Instrument: Waters Acquity SQD UPLC System; Säule: Waters Acquity UPLC HSS T3 1.8 µ, 50 mm x 1 mm; Eluent A: 11 Wasser + 0.25 ml 99%-ige Ameisensäure, Eluent B: 11 Acetonitril + 0.25 ml 99%-ige Ameisensäure; Gradient: 0.0 min 90% A → 1.2 min 5% A → 2.0 min 5% A; Fluss: 0.40 ml/min; Ofen: 50°C; UV-Detektion: 210-400 nm.

### Methode 3 (LC-MS):

Instrument: Micromass Quattro Premier mit Waters UPLC Acquity; Säule: Thermo Hypersil GOLD 1.9 µ, 50 mm x 1 mm; Eluent A: 11 Wasser + 0.5 ml 50%-ige Ameisensäure, Eluent B: 11 Acetonitril + 0.5 ml 50%-ige Ameisensäure; Gradient: 0.0 min 90% A → 0.1 min 90% A → 1.5 min 10% A → 2.2 min 10% A; Fluss: 0.33 ml/min; Ofen: 50°C; UV-Detektion: 210 nm.

### Methode 4 (LC-MS):

Gerätetyp MS: Waters ZQ; Gerätetyp HPLC: Agilent 1100 Series; UV DAD; Säule: Thermo Hypersil GOLD 3 µ, 20 mm x 4 mm; Eluent A: 11 Wasser + 0.5 ml 50%-ige Ameisensäure, Eluent B: 1 1 Acetonitril + 0.5 ml 50%-ige Ameisensäure; Gradient: 0.0 min 100% A → 3.0 min 10% A → 4.0 min 10% A → 4.1 min 100% A (Fluss 2.5 ml/min); Ofen: 55°C; Fluss: 2 ml/min; UV-Detektion: 210 nm.

### Methode 5 (LC-MS):

Gerätetyp MS: Waters Micromass Quattro Micro; Gerätetyp HPLC: Agilent 1100 Series; Säule: Thermo Hypersil GOLD 3 µ, 20 mm x 4 mm; Eluent A: 1 1 Wasser + 0.5 ml 50%-ige Ameisensäure, Eluent B: 1 1 Acetonitril + 0.5 ml 50%-ige Ameisensäure; Gradient: 0.0 min 100% A → 3.0 min 10% A → 4.0 min 10% A → 4.01 min 100% A (Fluss 2.5 ml/min) → 5.00 min 100% A; Ofen: 50°C; Fluss: 2 ml/min; UV-Detektion: 210 nm.

### Ausgangsverbindungen und Intermediate:

### Beispiel 1A

### tert.-Butyl-1-(3-brom-4-fluorbenzyl)cyclopropancarboxylat

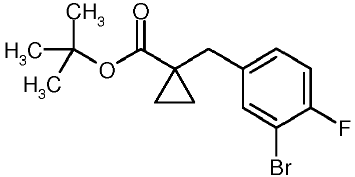

199.5 ml (1.42 mol) Diisopropylamin wurden unter Argon in 1300 ml trockenem THF vorgelegt und auf -50°C abgekühlt. Es wurden langsam 569.1 ml (1.42 mol) *n*-Butyllithium-Lösung (2.5 M in Hexan) zugetropft. Das resultierende Gemisch wurde auf 0°C erwärmt und dann auf -70°C abgekühlt. Die Reaktionslösung wurde mit einer Lösung von 161.9 g (1.14 mol) Cyclopropancarbonsäure-*tert*.-butylester in 380 ml THF versetzt, wobei die Temperatur unterhalb von -60°C gehalten wurde. Nach 4 h Rühren bei -78°C wurde eine Lösung von 262 g (0.95 mol) 2-Brom-4-(brommethyl)-1-fluorbenzol in 480 ml THF zugegeben, wobei die Temperatur wiederum unterhalb von -60°C gehalten wurde. Das Reaktionsgemisch wurde dann langsam über Nacht auf RT erwärmt, bevor vorsichtig mit 1.5 Liter gesättigter wässriger Ammoniumchlorid-Lösung und 3.0 Liter Ethylacetat versetzt wurde. Nach Phasentrennung wurde die wässrige Phase mit Ethylacetat extrahiert. Die vereinigten organischen Phasen wurden mit gesättigter Natriumchlorid-Lösung gewaschen, über Magnesiumsulfat getrocknet und im Vakuum eingeengt. Das Rohprodukt wurde durch Chromatographie an 3 kg Silicagel gereinigt (Laufmittel Cyclohexan/Dichlormethan 9:1, dann 5:1). Es wurden so 189.9 g (50.4% d. Th.) der Titelverbindung erhalten.

¹H-NMR (400 MHz, DMSO-*d*₆): δ [ppm] = 0.86-0.92 (m, 2H), 1.06-1.12 (m, 2H), 1.30 (s, 9H), 2.81 (s, 2H), 7.27-7.33 (m, 2H), 7.55-7.60 (m, 1H).

### Beispiel 2A

### tert.-Butyl-1-[3-(benzylamino)-4-fluorbenzyl]cyclopropancarboxylat

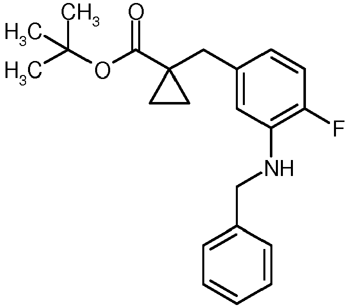

Unter Argon und trockenen Bedingungen wurden 174.0 g (528.5 mmol) *tert*.-Butyl-1-(3-brom-4-fluorbenzyl)cyclopropancarboxylat, 69.2 ml (634.2 mmol) Benzylamin, 4.84 g (5.29 mmol) Tris-(dibenzylidenaceton)dipalladium, 60.95 g (634.2 mmol) Natrium-*tert*.-butylat sowie 3.29 g (5.29 mmol) (+/-)-2,2'-Bis(diphenylphosphino)-1,1'-binaphthyl in 1218 ml Toluol suspendiert. Die Reaktionsmischung wurde anschließend für 2.0 h bei 110°C gerührt. Nach Abkühlen wurde das Reaktionsgemisch mit 2.1 Liter Ethylacetat und 1.7 Liter halbgesättigter wässriger Ammoniumchlorid-Lösung versetzt. Nach Phasentrennung wurde die organische Phase mit gesättigter Ammoniumchlorid-Lösung und gesättigter Natriumchlorid-Lösung gewaschen, über Magnesiumsulfat getrocknet und im Vakuum eingeengt. Das Rohprodukt wurde durch Chromatographie an 3.7 kg Silicagel gereinigt (Laufmittel Petrolether/Ethylacetat 20:1). Es wurden so 145.0 g (68.7% d. Th.) der Titelverbindung erhalten.

¹H-NMR (400 MHz, DMSO-*d*₆): δ [ppm] = 0.51-0.66 (m, 2H), 0.86-0.99 (m, 2H), 1.25 (m, 9H), 2.65 (s, 2H), 4.30 (d, 2H), 6.07 (t, 1H), 6.29-6.54 (m, 2H), 6.88 (dd, 1H), 7.15-7.25 (m, 1H), 7.25-7.42 (m, 4H).

### Beispiel 3A

### tert.-Butyl-1-(3-amino-4-fluorbenzyl)cyclopropancarboxylat

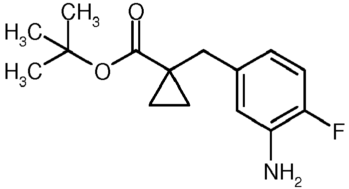

145.0 g (407.9 mmol) *tert*.-Butyl-1-[3-(benzylamino)-4-fluorbenzyl]cyclopropancarboxylat wurden in 1450 ml Ethanol gelöst und mit 9.67 g Palladiumhydroxid (20% auf Kohle) versetzt. Die Suspension wurde bei RT 18 h lang unter einer Wasserstoffatmosphäre bei Normaldruck gerührt. Das Reaktionsgemisch wurde danach über Kieselgur abgesaugt und das Filtrat eingeengt. Nach Trocknen im Hochvakuum wurde der Rückstand mit 500 ml Pentan versetzt, worauf das Produkt als Feststoff ausfiel. Die Suspension wurde 1 h im Eisbad gerührt. Der Feststoff wurde dann abgesaugt, zweimal mit wenig Pentan gewaschen und im Hochvakuum getrocknet. Es wurden so 88.5 g (73.6% d. Th.) der Titelverbindung erhalten.

¹H-NMR (400 MHz, DMSO-*d*₆): δ [ppm] = 0.70-0.80 (m, 2H), 1.00-1.10 (m, 2H), 1.30 (s, 9H), 2.68 (s, 2H), 4.98 (s, 2H), 6.28-6.45 (m, 1H), 6.63 (dd, 1H), 6.84 (dd, 1H).

### Beispiel 4A

### tert.-Butyl-(2E)-3-(4-fluor-3-nitrophenyl)acrylat

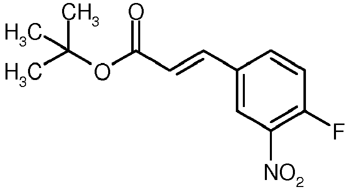

Zu einer Suspension von 15.74 g (394 mmol) Natriumhydrid (60%-ig in Mineralöl) in 540 ml THF wurden bei RT unter leichter Kühlung 99.23 g (394 mmol) Diethylphosphonoessigsäure-*tert*.-butylester getropft. Nach Ende der Zugabe wurde 30 min bei RT nachgerührt und nach Abkühlen auf 0°C eine Lösung von 60.5 g (358 mmol) 4-Fluor-3-nitrobenzaldehyd in 324 ml THF zugegeben. Die Reaktionsmischung wurde über Nacht auf RT erwärmt und dann auf 10%-ige wässrige Natriumchlorid-Lösung gegeben. Es wurde zweimal mit Ethylacetat extrahiert. Die vereinigten organischen Phasen wurden mit ges. Natriumchlorid-Lösung gewaschen, über Magnesiumsulfat getrocknet und im Vakuum eingeengt. Der Rückstand wurde durch zweimalige Chromatographie an Silicagel gereinigt (Laufmittel Petrolether/Ethylacetat 9:1). Es wurden so 82.0 g (84.9% d. Th.) des Zielprodukts erhalten.

GC-MS (Methode 1): Rₜ = 6.47 min; m/z = 211 (M-C₄H₈)⁺.

¹H-NMR (400 MHz, DMSO-*d*₆): δ [ppm] = 1.49 (s, 9H), 6.70 (d, 1H), 7.60-7.75 (m, 2H), 8.20 (ddd, 1H), 8.51 (dd, 1H).

### Beispiel 5A

### tert.-Butyl-3-(3-amino-4-fluorphenyl)propanoat

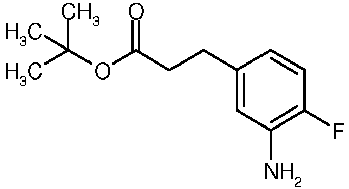

Ein Lösung von 81.0 g (303 mmol) *tert*.-Butyl-(2*E*)-3-(4-fluor-3-nitrophenyl)acrylat in einer Mischung aus 810 ml THF und 810 ml Ethanol wurde mit Argon inertisiert und mit 8.1 g 10%-igem Palladium auf Kohle versetzt. Die Suspension wurde bei RT 9 h lang unter einer Wasserstoffatmosphäre bei Normaldruck gerührt. Das Reaktionsgemisch wurde danach über Kieselgur abgesaugt und das Filtrat eingeengt. Das Rohprodukt wurde durch Chromatographie an Silicagel gereinigt (Laufmittel Petrolether/Ethylacetat 4:1). Es wurden 66.0 g (91% d. Th.) der Titelverbindung erhalten.

¹H-NMR (400 MHz, DMSO-*d*₆): δ [ppm] = 1.36 (s, 9H), 2.42 (t, 2H), 2.64 (t, 2H), 5.00 (s, 2H), 6.33 (ddd, 1H), 6.59 (dd, 1H), 6.84 (dd, 1H).

### Beispiel 6A

### tert.-Butyl-(2E)-3-(4-chlor-3-nitrophenyl)acrylat

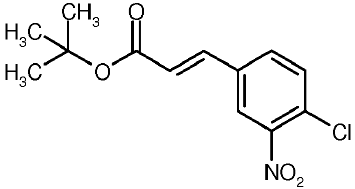

Zu einer Suspension von 23.71 g (593 mmol) Natriumhydrid (60%-ig in Mineralöl) in 1.0 Liter THF wurden bei 0°C unter Kühlung 156.32 g (620 mmol) Diethylphosphonoessigsäure-*tert*.-butylester getropft. Nach Ende der Zugabe wurde 30 min bei 0°C nachgerührt und anschließend eine Lösung von 100.0 g (539 mmol) 4-Chlor-3-nitrobenzaldehyd in 200 ml THF zugegeben. Die Reaktionsmischung wurde über Nacht auf RT erwärmt und dann mit Wasser und Ethylacetat versetzt. Die organische Phase wurde abgetrennt, mit Magnesiumsulfat getrocknet und im Vakuum eingeengt. Der Rückstand wurde aus Isopropanol umkristallisiert. Nach Trocknen im Hochvakuum wurden 144.0 g (94.2% d. Th.) des Zielprodukts erhalten.

LC-MS (Methode 2): Rₜ = 1.28 min; m/z = 269 (M-14)⁺.

### Beispiel 7A

### tert.-Butyl-3-(3-amino-4-chlorphenyl)propanoat

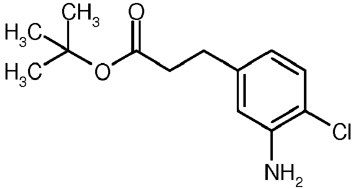

Zu einer Lösung von 72.0 g (254 mmol) *tert*.-Butyl-(2*E*)-3-(4-chlor-3-nitrophenyl)acrylat in 1.0 Liter Ethylacetat wurden unter Argon 5.4 g 10%-iges Palladium auf Kohle gegeben. Die Suspension wurde bei RT 7 h lang unter einer Wasserstoffatmosphäre bei Normaldruck gerührt. Das Reaktionsgemisch wurde danach über Kieselgur abgesaugt und das Filtrat eingeengt. Das Rohprodukt wurde durch Chromatographie an Silicagel gereinigt (Laufmittel Cyclohexan/Ethylacetat 9:1). Es wurden 38 g (58% d. Th.) der Titelverbindung erhalten.

LC-MS (Methode 2): Rₜ = 1.14 min; m/z = 256 (M+H)⁺.

¹H-NMR (400 MHz, DMSO-*d*₆): δ [ppm] = 1.36 (s, 9H), 2.43 (t, 2H), 2.65 (t, 2H), 5.21 (br. s, 2H), 6.39 (dd, 1H), 6.62 (d, 1H), 7.05 (d, 1H).

### Beispiel 8A

### (E/Z)-3-(4-Fluor-3-nitrophenyl)-2-methylprop-2-ensäureethylester

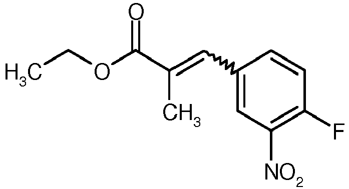

3.17 g Natriumhydrid (60%-ige Suspension in Mineralöl, 79.36 mmol) wurden in 90 ml eines THF/DMF-Gemisches (2:1) suspendiert. Die Mischung wurde auf 0°C gekühlt und tropfenweise mit einer Lösung von 19.76 g (82.96 mmol) 2-Phosphonopropionsäuretriethylester in 60 ml THF/ DMF (2:1) versetzt. Nach 30 min wurde bei 0°C eine Lösung von 12.2 g (72.14 mmol) 4-Fluor-3-nitrobenzaldehyd in 60 ml THF/DMF (2:1) hinzugetropft. Nach Ende der Zugabe wurde die Reaktionsmischung langsam auf RT erwärmt und 2 h bei dieser Temperatur gerührt. Danach wurde die Reaktionsmischung auf Wasser gegeben. Es wurde dreimal mit Ethylacetat extrahiert, und die vereinigten organischen Phasen wurden im Vakuum eingeengt. Der Rückstand wurde durch Chromatographie an Silicagel gereinigt (Laufmittel Cyclohexan/Ethylacetat 20:1). Es wurden 15.2 g (83.2% d. Th.) des Zielprodukts als *E*/*Z*-Isomerengemisch (*E*/*Z* 91:9) erhalten.

LC-MS (Methode 2): Z-Isomer: Rₜ = 1.11 min; m/z = 254 (M+H)⁺; E-Isomer: Rₜ = 1.14 min; m/z = 254 (M+H)⁺.

¹H-NMR (400 MHz, DMSO-d₆): E-Isomer: δ [ppm] = 1.28 (t, 3H), 4.22 (q, 2H), 7.59-7.73 (m, 2H), 7.92 (ddd, 1H), 8.24 (dd, 1H).

### Beispiel 9A

### (+/-)-3-(3-Amino-4-fluorphenyl)-2-methylpropansäureethylester

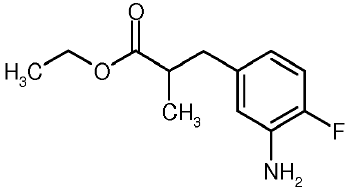

15.2 g (60.02 mmol) (*E*/*Z*)-3-(4-Fluor-3-nitrophenyl)-2-methylprop-2-ensäureethylester (*E*/*Z* 91:9) wurden in einem Gemisch aus 100 ml Ethanol und 100 ml THF mit Palladium auf Kohle (10%) versetzt und über Nacht unter einer Wasserstoffatmosphäre bei Normaldruck kräftig gerührt. Die Reaktionsmischung wurde dann über Celite filtriert, der Rückstand mit Ethanol/Dichlormethan gewaschen und die vereinigten Filtrate im Vakuum eingeengt. Das Produkt wurde im Hochvakuum getrocknet. Erhalten wurden 13.34 g des Zielprodukts (98.7% d. Th).

LC-MS (Methode 2): Rₜ = 0.98 min; m/z = 226 (M+H)⁺.

¹H-NMR (400 MHz, DMSO-d₆): δ [ppm] = 1.04 (d, 3H), 1.12 (t, 3H), 2.46-2.50 (m, 1H), 2.55-2.66 (m, 1H), 2.66-2.78 (m, 1H), 4.01 (q, 2H), 5.00 (s, 2H), 6.18-6.35 (m, 1H), 6.55 (dd, 1H), 6.84 (dd, 1H).

Das oben erhaltene Racemat wurde durch präparative HPLC an chiraler Phase in die Enantiomere aufgetrennt [Säule: Daicel Chiralpak AD-H, 5 µm, 250 mm x 20 mm; Injektionsvolumen: 0.15 ml; Temperatur: 30°C; Eluent: 90% Isohexan / 10% Ethanol; Fluss: 15 ml/min; Detektion: 220 nm]. Ausgehend von 7.25 g Racemat wurden 3.43 g Enantiomer 1 (*Beispiel 10A*) und 3.35 g Enantiomer *2* (*Beispiel 11A*) erhalten:

### Beispiel 10A

### (+)-(2S)-3-(3-Amino-4-fluorphenyl)-2-methylpropansäureethylester

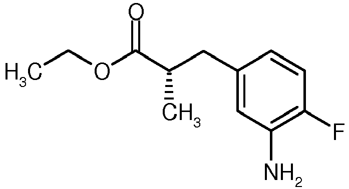

### Ausbeute: 3.43 g

LC-MS (Methode 2): Rₜ = 0.97 min; m/z = 226 (M+H)⁺.

¹H-NMR (400 MHz, DMSO-*d*₆): δ [ppm] = 1.04 (d, 3H), 1.12 (t, 3H), 2.46-2.50 (m, 1H), 2.55-2.66 (m, 1H), 2.66-2.78 (m, 1H), 4.01 (q, 2H), 5.00 (s, 2H), 6.18-6.35 (m, 1H), 6.55 (dd, 1H), 6.84 (dd, 1H).

[α]_{D}²⁰ = +18.3°, c = 0.465, Chloroform.

### Beispiel 11A

### (-)-(2R)-3-(3-Amino-4-fluorphenyl)-2-methylpropansäureethylester

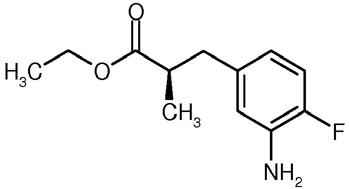

### Ausbeute: 3.35 g

LC-MS (Methode 2): Rₜ = 0.97 min; m/z = 226 (M+H)⁺.

¹H-NMR (400 MHz, DMSO-*d*₆): δ [ppm] = 1.04 (d, 3H), 1.12 (t, 3H), 2.46-2.50 (m, 1H), 2.55-2.66 (m, 1H), 2.68-2.79 (m, 1H), 4.01 (q, 2H), 5.00 (br. s, 2H), 6.30 (dd, 1H), 6.55 (dd, 1H), 6.84 (dd, 1H).

[α]_{D}²⁰ = -31.4°, c = 0.520, Chloroform.

### Beispiel 12A

### (E/Z)-3-(4-Chlor-3-nitrophenyl)-2-methylprop-2-ensäureethylester

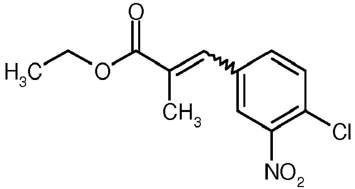

4.74 g Natriumhydrid (60%-ige Suspension in Mineralöl, 118.56 mmol) wurden in 93 ml eines THF/DMF-Gemisches (1:1) suspendiert. Die Mischung wurde auf 0°C gekühlt und tropfenweise mit 26.6 ml (123.95 mmol) 2-Phosphonopropionsäuretriethylester versetzt. Nach 30 min wurden bei 0°C 20.0 g (107.78 mmol) 4-Chlor-3-nitrobenzaldehyd hinzugefügt. Nach Ende der Zugabe wurde die Reaktionsmischung langsam auf RT erwärmt und noch 3 h bei dieser Temperatur gerührt. Dann wurde die Reaktionsmischung auf Wasser gegeben. Es wurde dreimal mit Ethylacetat extrahiert, und die vereinigten organischen Phasen wurden im Vakuum eingeengt. Der Rückstand wurde durch Chromatographie an Silicagel gereinigt (Laufmittel Cyclohexan/Ethylacetat 70:1 → 50:1). Es wurden 26.7 g (91.9% d. Th.) des Zielprodukts als *E*/*Z*-Isomerengemisch (*E*/*Z* 91:9) erhalten.

LC-MS (Methode 3): Z-Isomer: Rₜ = 1.32 min; m/z = 255; E-Isomer: Rₜ = 1.36 min; m/z = 270 (M+H)⁺.

¹H-NMR (400 MHz, DMSO-d₆): E-Isomer: δ [ppm] = 1.28 (t, 3H), 2.06 (d, 3H), 4.22 (q, 2H), 7.56-7.67 (m, 1H), 7.75-7.87 (m, 2H), 8.17 (d, 1H).

### Beispiel 13A

### (+/-)-3-(3-Amino-4-chlorphenyl)-2-methylpropansäureethylester

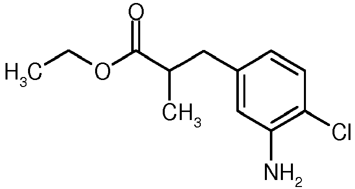

10.0 g (37.08 mmol) (*E*/*Z*)-3-(4-Chlor-3-nitrophenyl)-2-methylprop-2-ensäureethylester (*E*/*Z* 91:9) wurden in 25 ml Ethylacetat und 25 ml Essigsäure gelöst und mit Palladium auf Kohle (10%) versetzt. Die Reaktionsmischung wurde insgesamt 6 h unter einer Wasserstoffatmosphäre bei Normaldruck kräftig gerührt, wobei nach 2 h nochmals 25 ml Essigsäure und weitere Portionen an 10% Palladium auf Kohle hinzugefügt wurden. Es wurde dann über Celite filtriert und der Rückstand mit Ethanol/Dichlormethan gewaschen. Die vereinigten Filtrate wurden mit gesättigter Natriumhydrogencarbonat-Lösung gewaschen, über Natriumsulfat getrocknet und im Vakuum eingeengt. Das Rohprodukt wurde durch Chromatographie an Silicagel gereinigt (Laufmittel Cyclohexan/ Ethylacetat 30:1 → 10:1). Erhalten wurden 4.01 g des Zielprodukts (44.7% d. Th).

LC-MS (Methode 2): Rₜ = 1.06 min; m/z = 242 (M+H)⁺.

¹H-NMR (400 MHz, DMSO-d₆): δ [ppm] = 1.05 (d, 3H), 1.12 (t, 3H), 2.47-2.50 (m, 1H), 2.56-2.67 (m, 1H), 2.67-2.78 (m, 1H), 4.02 (q, 2H), 5.23 (s, 2H), 6.35 (dd, 1H), 6.58 (d, 1H), 7.05 (d, 1H).

Das oben erhaltene Racemat wurde durch präparative HPLC an chiraler Phase in die Enantiomere aufgetrennt [Säule: Daicel Chiralpak OJ-H, 5 µm, 250 mm x 20 mm; Injektionsvolumen: 0.15 ml; Temperatur: 35°C; Eluent: 50% Isohexan / 50% Isopropanol; Fluss: 15 ml/min; Detektion: 220 nm]. Ausgehend von 10.3 g Racemat wurden 4.0 g Enantiomer 1 (*Beispiel 14A*) und 3.7 g Enantiomer 2 (*Beispiel 15A*) erhalten:

### Beispiel 14A

### (-)-(2R)-3-(3-Amino-4-chlorphenyl)-2-methylpropansäureethylester

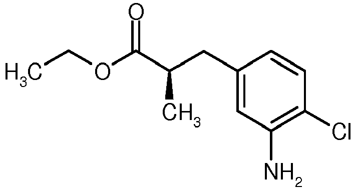

### Ausbeute: 4.0 g

LC-MS (Methode 4): Rₜ = 2.27 min; m/z = 196/198.

¹H-NMR (400 MHz, DMSO-*d*₆): δ [ppm] = 1.05 (d, 3H), 1.12 (t, 3H), 2.47-2.50 (m, 1H), 2.54-2.66 (m, 2H), 2.68-2.80 (m, 1H), 4.02 (q, 2H), 5.23 (s, 2H), 6.35 (dd, 1H), 6.58 (d, 1H), 7.05 (d, 1H).

[α]_{D}²⁰ = -35.8°, c = 0.560, Chloroform.

### Beispiel 15A

### (+)-(2S)-3-(3-Amino-4-chlorphenyl)-2-methylpropansäureethylester

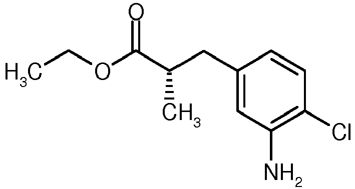

### Ausbeute: 3.7 g

¹H-NMR (400 MHz, DMSO-*d*₆): δ [ppm] = 1.05 (d, 3H), 1.12 (t, 3H), 2.47-2.50 (m, 1H), 2.56-2.67 (m, 1H), 2.67-2.81 (m, 1H), 4.02 (q, 2H), 5.23 (br. s, 2H), 6.35 (dd, 1H), 6.58 (d, 1H), 7.05 (d, 1H).

[α]_{D}²⁰ = +35.1°, c = 0.525, Chloroform.

### Beispiel 16A

### tert.-Butyl-(2E/Z)-3-(4-chlor-3-nitrophenyl)but-2-enoat

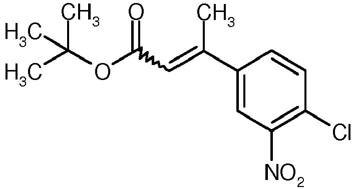

2.87 g Natriumhydrid (60%-ige Suspension in Mineralöl, 71.65 mmol) wurden in 80 ml THF suspendiert. Die Mischung wurde auf 0°C gekühlt und tropfenweise mit 17.6 ml (74.9 mmol) *tert.-*Butyl-(diethoxyphosphoryl)acetat versetzt. Nach 30 min wurden bei 0°C 13.0 g (65.1 mmol) 4-Chlor-3-nitroacetophenon hinzugefügt. Nach Ende der Zugabe wurde die Reaktionsmischung langsam auf RT erwärmt und noch 1.5 h bei RT gerührt, bevor das Gemisch dann auf Wasser gegeben wurde. Es wurde dreimal mit Ethylacetat extrahiert, und die vereinigten organischen Phasen wurden im Vakuum eingeengt. Der Rückstand wurde durch Chromatographie an Silicagel gereinigt (Laufmittel Cyclohexan/Ethylacetat 20:1 → 10:1). Es wurden 17.03 g (87.8% d. Th.) des Zielprodukts als *E*/*Z*-Isomerengemisch erhalten (*E*/*Z* ca. 1:1).

LC-MS (Methode 5): Isomer 1: Rₜ = 2.61 min; m/z = 255; Isomer 2: Rₜ = 2.77 min; m/z = 224.

### Beispiel 17A

### (+/-)-3-(3-Amino-4-chlorphenyl)butansäure-tert.-butylester

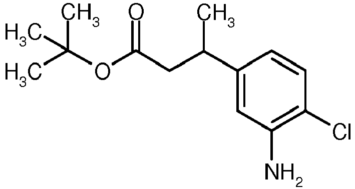

11.5 g (38.62 mmol) *tert*.-Butyl-(2*E*/*Z*)-3-(4-chlor-3-nitrophenyl)but-2-enoat (*E*/*Z* ca. 1:1) wurden in 60 ml Ethylacetat und 60 ml Essigsäure gelöst und mit Palladium auf Kohle (10%) versetzt. Die Reaktionsmischung wurde 6 h bei Normaldruck unter einer Wasserstoffatmosphäre kräftig gerührt. Es wurde dann über Celite filtriert und der Rückstand mit Ethylacetat gewaschen. Die vereinigten Filtrate wurden mit gesättigter Natriumhydrogencarbonat-Lösung gewaschen, über Natriumsulfat getrocknet und im Vakuum eingeengt. Das Rohprodukt wurde durch Chromatographie an Silicagel gereinigt (Laufmittel Cyclohexan/Ethylacetat 30:1). Es wurden 3.90 g (37.4% d. Th.) des Zielprodukts erhalten.

¹H-NMR (400 MHz, DMSO-d₆): δ [ppm] = 1.14 (d, 3H), 1.31 (s, 9H), 2.38 (dd, 2H), 2.95 (q, 1H), 5.21 (br. s, 2H), 6.42 (dd, 1H), 6.65 (d, 1H), 7.06 (d, 1H).

Das oben erhaltene Racemat wurde durch präparative HPLC an chiraler Phase in die Enantiomere aufgetrennt [Säule: Daicel Chiralpak AD-H, 5 µm, 250 mm x 20 mm; Injektionsvolumen: 0.15 ml; Temperatur: 30°C; Eluent: 90% Isohexan / 10% Ethanol; Fluss: 15 ml/min; Detektion: 220 nm]. Ausgehend von 5.0 g Racemat wurden 2.1 g Enantiomer 1 (*Beispiel 18A*) und 1.8 g Enantiomer 2 (*Beispiel 19A*) erhalten:

### Beispiel 18A

### (+)-tert.-Butyl-(3R)-3-(3-amino-4-chlorphenyl)butanoat

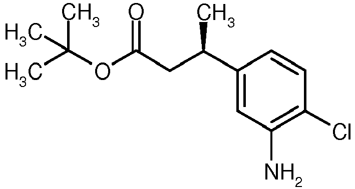

LC-MS (Methode 3): Rₜ = 1.34 min; m/z = 270 (M+H)⁺.

¹H-NMR (400 MHz, DMSO-*d*₆): δ [ppm] = 1.14 (d, 3H), 1.31 (s, 9H), 2.19-2.45 (m, 2H), 2.95 (q, 1H), 5.20 (s, 2H), 6.42 (dd, 1H), 6.65 (d, 1H), 7.06 (d, 1H).

[α]_{D}²⁰ = +20.9°, c = 0.670, Chloroform.

### Beispiel 19A

(-)-*tert*.-Butyl-(3*S*)-3-(3-amino-4-chlorphenyl)butanoat

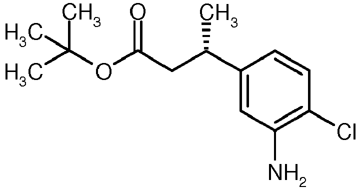

LC-MS (Methode 3): Rₜ = 1.34 min; m/z = 214 (M+H-C₄H₈)⁺.

¹H-NMR (400 MHz, DMSO-*d*₆): δ [ppm] = 1.14 (d, 3H), 1.31 (s, 9H), 2.38 (dd, 2H), 2.95 (q, 1H), 5.20 (br. s, 2H), 6.42 (dd, 1H), 6.65 (d, 1H), 7.06 (d, 1H).

[α]_{D}²⁰ = -24.1°, c = 0.570, Chloroform.

### Beispiel 20A

### 2-Brom-4-(brommethyl)-1-chlorbenzol

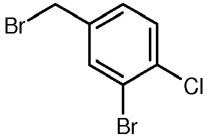

### Stufe 1:

199.0 g (0.845 mol) 3-Brom-4-chlorbenzoesäure wurden in 2.5 Liter THF gelöst, auf -10°C gekühlt und bei dieser Temperatur mit 1.69 Liter (1.69 mol) einer 1 M Boran-Lösung in THF versetzt. Die Reaktionsmischung wurde über Nacht auf RT erwärmt, bevor mit gesättigter wässriger Ammoniumchlorid-Lösung versetzt wurde. Nach Zugabe von Wasser wurde zweimal mit Ethylacetat extrahiert, die vereinigten organischen Phasen über Magnesiumsulfat getrocknet und im Vakuum eingeengt. Erhalten wurden als Rohprodukt 206 g (3-Brom-4-chlorphenyl)methanol, die ohne weitere Reinigung in der Folgestufe eingesetzt wurden.

### Stufe 2:

260 g (ca. 1.05 mol) rohes (3-Brom-4-chlorphenyl)methanol wurden in 2.86 Liter Dichlormethan gelöst, auf -5°C gekühlt und langsam mit 127.1 g (44.6 ml, 460 mmol) Phosphortribromid versetzt. Nach Ende der Zugabe wurde noch 1 h bei -5°C gerührt, bevor mit Dichlormethan und Wasser verdünnt wurde. Die organische Phase wurde abgetrennt, über Magnesiumsulfat getrocknet und im Vakuum eingeengt. Erhalten wurden als Rohprodukt 280.5 g (ca. 84% d. Th.) 2-Brom-4-(brommethyl)-1-chlorbenzol.

GC-MS (Methode 1): Rₜ = 5.36 min; m/z = 281/283/285 (M+H)⁺.

¹H-NMR (400 MHz, DMSO-*d*₆): δ [ppm] = 4.71 (s, 2H), 7.49 (dd, 1H), 7.63 (d, 1H), 7.89 (d, 1H).

### Beispiel 21A

### tert.-Butyl-1-(3-brom-4-chlorbenzyl)cyclopropancarboxylat

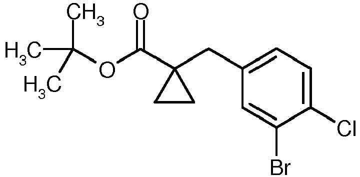

140.2 ml (1.0 mol) Diisopropylamin wurden unter Argon in 1200 ml trockenem THF gelöst und auf -30°C abgekühlt. Es wurden 400 ml (1.0 mol) *n*-Butyllithium-Lösung (2.5 M in Hexan) zugetropft. Das resultierende Gemisch wurde auf 0°C erwärmt und dann auf -70°C abgekühlt. Die Reaktionslösung wurde mit einer Lösung von 94.8 g (0.667 mol) Cyclopropancarbonsäure-*tert*.-butylester in 750 ml THF versetzt, wobei die Temperatur unterhalb von -60°C gehalten wurde. Nach 4 h Rühren bei -60°C wurde eine Lösung von 208.6 g (0.733 mol) 2-Brom-4-(brommethyl)-1-chlorbenzol in 550 ml THF zugegeben, wobei die Temperatur wiederum unterhalb von -60°C gehalten wurde. Das Reaktionsgemisch wurde langsam über Nacht auf RT erwärmt, bevor vorsichtig mit gesättigter wässriger Ammoniumchlorid-Lösung versetzt wurde. Nach Phasentrennung wurde die wässrige Phase mit Ethylacetat extrahiert. Die vereinigten organischen Phasen wurden über Magnesiumsulfat getrocknet und im Vakuum eingeengt. Das Rohprodukt wurde durch Chromatographie an Silicagel gereinigt (Laufmittel Cyclohexan/Dichlormethan 4:1). Es wurden 95.5 g (41.4% d. Th.) der Titelverbindung erhalten.

GC-MS (Methode 1): Rₜ = 6.54 min; m/z = 288/290 (M-C₄H₈)⁺.

LC-MS (Methode 3): Rₜ = 1.65 min; m/z = 288/290 (M-C₄H₈)⁺.

### Beispiel 22A

### tert.-Butyl-1-[3-(benzylamino)-4-chlorbenzyl]cyclopropancarboxylat

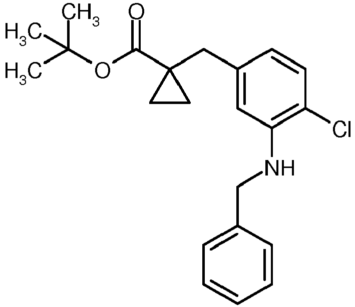

Unter Argon wurden zu 633 ml trockenem Toluol nacheinander 95.0 g (274.8 mmol) *tert*.-Butyl-1-(3-brom-4-chlorbenzyl)cyclopropancarboxylat, 36.0 ml (330.0 mmol) Benzylamin, 12.58 g (13.7 mmol) Tris(dibenzylidenaceton)dipalladium, 31.69 g (329.8 mmol) Natrium-*tert*.-butylat sowie 6.85 g (5.29 mmol) (+/-)-2,2'-Bis(diphenylphosphino)-1,1'-binaphthyl gegeben. Die Reaktionsmischung wurde 3.0 h bei 110°C und anschließend über Nacht bei RT gerührt. Das Reaktionsgemisch wurde dann über Kieselgur abgesaugt und der Rückstand gründlich mit Toluol und Ethylacetat gewaschen. Die vereinigten Filtrate wurden im Vakuum eingeengt. Das Rohprodukt wurde durch Chromatographie an Silicagel gereinigt (Laufmittel Petrolether/Ethylacetat 10:1). Es wurden 50.0 g der Titelverbindung erhalten (48.9% d. Th.).

LC-MS (Methode 2): Rₜ = 1.48 min; m/z = 372 (M+H)⁺.

### Beispiel 23A

### tert.-Butyl-1-(3-amino-4-chlorbenzyl)cyclopropancarboxylat

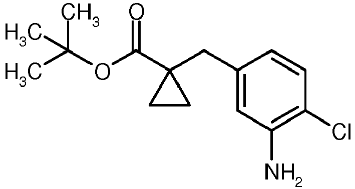

50.0 g (134.4 mmol) *tert*.-Butyl-1-[3-(benzylamino)-4-chlorbenzyl]cyclopropancarboxylat wurden in 1.5 Liter Ethylacetat gelöst und mit 1.43 g (1.34 mmol) Palladium (10% auf Kohle) versetzt. Die Reaktionsmischung wurde bei RT über Nacht unter einer Wasserstoffatmosphäre bei Normaldruck gerührt. Das Reaktionsgemisch wurde danach über Kieselgur abgesaugt und das Filtrat eingeengt. Das Rohprodukt wurde durch Chromatographie an Silicagel gereinigt (Laufmittel Petrolether/ Ethylacetat 10:1). Das erhaltene Produkt wurde in einem Methanol/Wasser-Gemisch (70:30) verrührt und der Feststoff isoliert. Es wurden 24.3 g (64.1% d. Th.) der Zielverbindung erhalten.

LC-MS (Methode 2): Rₜ = 1.22 min; m/z = 282 (M+H)⁺.

¹H-NMR (400 MHz, DMSO-*d*₆): δ [ppm] = 0.74-0.82 (m, 2H), 1.02-1.09 (m, 2H), 1.30 (s, 9H), 2.69 (s, 2H), 5.21 (br. s, 2H), 6.42 (dd, 1H), 6.67 (d, 1H), 7.05 (d, 1H).

### Beispiel 24A

### N-[(Benzyloxy)carbonyl]-4,4,4-trifluorvalin (racemisches Diastereomerengemisch)

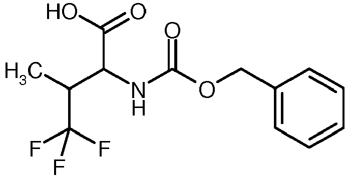

15.0 g (72 mmol) D,L-4,4,4-Trifluorvalin-Hydrochlorid wurden portionsweise in 160 ml gesättigte wässrige Natriumhydrogencarbonat-Lösung eingetragen. Die Mischung wurde bei RT mit einer Lösung von 19.8 g (79 mmol) N-(Benzyloxycarbonyloxy)succinimid in 150 ml Dioxan versetzt. Das resultierende Reaktionsgemisch wurde 2 h kräftig gerührt. Danach wurde das Dioxan weitgehend im Vakuum entfernt. Nach Ansäuern mit 1 N Salzsäure auf pH ca. 2 wurde dreimal mit Dichlormethan extrahiert, und die vereinigten organischen Phasen wurden über Magnesiumsulfat getrocknet und im Vakuum eingeengt. Das Rohprodukt wurde im Hochvakuum getrocknet und ohne weitere Reinigung in Folgereaktionen eingesetzt. Es wurden 26.0 g des Zielprodukts (ca. 80% Reinheit, ca. 95% d. Th.) erhalten.

LC-MS (Methode 5): Rₜ = 1.96 min; m/z = 306 (M+H)⁺.

¹H-NMR (400 MHz, DMSO-*d*₆): beide Diastereomere δ [ppm] = 1.09/1.12 (je d, zus. 3H), 2.82-3.05 (m, 1H), 4.30/4.59 (je dd, zus. 1H), 5.06/5.07 (je s, zus. 2H), 7.31-7.39 (m, 5H), 7.77/7.83 (je d, zus. 1H).

### Beispiel 25A

### N-[(Allyloxy)carbonyl]-4,4,4-trifluorvalin (racemisches Diastereomerengemisch)

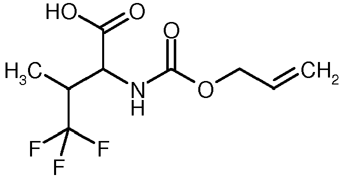

6.63 g (57.8 mmol) Succinimid wurden in 100 ml THF vorgelegt und bei RT mit 10.1 ml (72.6 mmol) Triethylamin versetzt. Nach Abkühlen auf 0°C wurde die Mischung tropfenweise mit einer Lösung von 6.98 g (57.8 mmol) Chlorameisensäureallylester in 10 ml THF versetzt. Die Mischung wurde 1 h bei 0°C gerührt, bevor 10.1 ml (72.6 mmol) Triethylamin sowie 10.0 g (48.2 mmol) D,L-4,4,4-Trifluorvalin-Hydrochlorid hinzugefügt wurden. Nach beendeter Zugabe wurde die Reaktionsmischung unter kräftigem Rühren langsam auf RT erwärmt und nach 2 h mit Wasser versetzt. Nach Ansäuern mit 1 N Salzsäure auf pH ca. 2 wurde die wässrige Phase dreimal mit Ethylacetat extrahiert. Die vereinigten organischen Phasen wurden über Magnesiumsulfat getrocknet und im Vakuum eingeengt. Das Rohprodukt wurde durch Chromatographie an Silicagel gereinigt (Laufmittel Cyclohexan/Ethylacetat 4:1 + 1% Essigsäure). Es wurden 9.15 g des Zielprodukts erhalten (74.4% d. Th.).

¹H-NMR (400 MHz, DMSO-*d*₆): beide Diastereomere δ [ppm] = 1.09/1.12 (je d, zus. 3H), 2.83-3.06 (m, 1H), 4.28/4.57 (je dd, zus. 1H), 4.45-4.53 (m, 2H), 5.14-5.23 (m, 1H), 5.25-5.34 (m, 1H), 5.79-5.96 (m, 1H), 7.70/7.77 (je d, zus. 1H), 13.16 (br. s, 1H).

### Beispiel 26A

### Ethyl-(3R)-4,4,4-trifluor-3-methylbutanoat

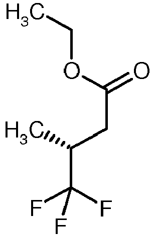

287 g (1.65 mol) (3*R*)-4,4,4-Trifluor-3-methylbutansäure [A. Gerlach und U. Schulz, Speciality Chemicals Magazine 24 (4), 37-38 (2004); CAS Acc.-Nr. 142:179196] in 580 ml Ethanol wurden bei Raumtemperatur langsam mit 133 ml (1.82 mol) Thionylchlorid versetzt. Die Reaktionslösung wurde anschließend auf 80°C erwärmt und 2 h bei dieser Temperatur gerührt. Danach wurde auf Raumtemperatur abgekühlt, langsam mit 250 ml Wasser versetzt und dreimal mit je 150 ml *tert.-*Butylmethylether extrahiert. Die vereinigten organischen Phasen wurden über Natriumsulfat getrocknet. Nach Filtration wurde das Lösungsmittel im Vakuum bei 30°C und einem Druck von 300 mbar entfernt. Das Rohprodukt wurde anschließend bei 100 mbar und einer Kopftemperatur von 65°C destilliert. Es wurden 225.8 g (113 mol, 74% d. Th.) der Titelverbindung als farblose Flüssigkeit isoliert.

¹H-NMR (400 MHz, DMSO-*d*₆): δ [ppm] = 4.10 (2H, q), 2.88-2.72 (1H, m), 2.66-2.57 (1H, m), 2.46-2.36 (1H, m), 1.19 (3H, t), 1.11 (3H, d).

GC-MS (Methode 1): Rₜ = 1.19 min; m/z = 184 (M)⁺.

[α]_{D}²⁰ = +16.1°, c = 0.41, Methanol.

### Beispiel 27A

### Ethyl-(3R)-4,4,4-trifluorvalinat (Diastereomerengemisch)

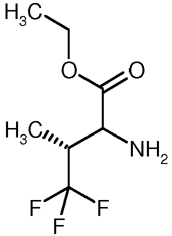

### Stufe 1: Ethyl-(3S)-2-brom-4,4,4-trifluor-3-methylbutanoat (Diastereomerengemisch)

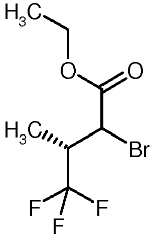

Eine auf -40°C gekühlte Lösung von 32.0 ml (228 mmol) Diisopropylamin in 160 ml abs. THF wurde mit 91.2 ml (228 mmol) 2.5 M n-Butyllithium-Lösung in Hexan versetzt. Die resultierende LDA-Lösung wurde noch 30 min bei -40°C gerührt und dann auf -78°C gekühlt, bevor 52.4 ml (413 mmol) Chlortrimethylsilan zugetropft wurden. Nach 5 min wurden 40.0 g (217.2 mmol) (+)-Ethyl-(3R)-4,4,4-trifluor-3-methylbutanoat, gelöst in 40 ml abs. THF, über 1 h tropfenweise zugegeben. Die resultierende Suspension wurde bei -78°C in drei Portionen mit insgesamt 40.59 g (228 mmol) *N*-Bromsuccinimid versetzt und dann langsam über Nacht auf RT erwärmt. Die Reaktionsmischung wurde vorsichtig mit Wasser versetzt und mit Ethylacetat extrahiert. Die organische Phase wurde nacheinander mit 1 N Salzsäure, ges. Natriumhydrogencarbonat-Lösung und ges. Natriumchlorid-Lösung gewaschen, über Natriumsulfat getrocknet und in der Kälte im Vakuum eingeengt. Die resultierende Suspension wurde mit 130 ml Diethylether versetzt und über Nacht bei 4°C gelagert. Der ungelöste Feststoff wurde danach abfiltriert und verworfen. Das Filtrat wurde in der Kälte im Vakuum eingeengt und der Rückstand kurz im Hochvakuum getrocknet. Es wurden so 48.5 g Ethyl-(3*S*)-2-brom-4,4,4-trifluor-3-methylbutanoat (Diastereomerengemisch) als Rohprodukt isoliert.

¹H-NMR (400 MHz, DMSO-*d*₆): beide Diastereomere δ [ppm] = 1.18-1.29 (m, 6H), 3.15-3.29 (m, 1H), 4.15-4.25 (m, 2H), 4.84-4.90 (m, 1H).

### Stufe 2: Ethyl-(3R)-2-azido-4,4,4-trifluor-3-methylbutanoat (Diastereomerengemisch)

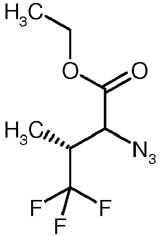

48.5 g rohes Ethyl-(3*S*)-2-brom-4,4,4-trifluor-3-methylbutanoat (Diastereomerengemisch) wurden in 130 ml DMF gelöst und bei RT mit 17.98 g (276.55 mmol) Natriumazid versetzt. Die Reaktionsmischung wurde über Nacht bei RT kräftig gerührt. Dann wurde mit 600 ml Ethylacetat verdünnt und mit einer auf pH 8 eingestellten 1:1-Mischung aus ges. Natriumchlorid-Lösung und Wasser gewaschen. Nach Phasentrennung wurde die wässrige Phase mit 200 ml Ethylacetat rückextrahiert. Die vereinigten organischen Phasen wurden nacheinander zweimal mit je 100 ml und zweimal mit je 50 ml einer 1:1-Mischung aus ges. Natriumchlorid-Lösung und Wasser gewaschen, über Natriumsulfat getrocknet und in der Kälte im Vakuum eingeengt. Der Rückstand wurde kurz im Hochvakuum getrocknet (Vorsicht beim Belüften). Es wurden 32.8 g Ethyl-(3R)-2-azido-4,4,4-trifluor-3-methylbutanoat (Diastereomerengemisch) als Rohprodukt erhalten, das direkt weiter umgesetzt wurde.

### Stufe 3: Ethyl-(3R)-4,4,4-trifluorvalinat (Diastereomerengemisch)

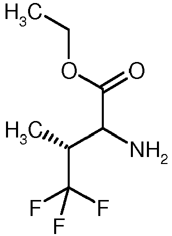

32.8 g rohes Ethyl-(3*R*)-2-azido-4,4,4-trifluor-3-methylbutanoat (Diastereomerengemisch) wurden in 110 ml Ethanol gelöst und nach Inertisierung mit Argon mit 6.98 g Palladium auf Kohle (5%-ig) versetzt. Das Gemisch wurde über Nacht bei Normaldruck unter einer Wasserstoffatmosphäre gerührt, wobei das Reaktionsgefäß mehrfach entlüftet und wieder frisch mit Wasserstoff gefüllt wurde. Die Reaktionsmischung wurde dann über Celite abfiltriert, der Filter-Rückstand mit 50 ml Ethanol gewaschen und das Filtrat im Vakuum eingeengt (bis ca. 20 mm Hg). Die verbliebene leicht gelbliche Flüssigkeit wurde kurz im Hochvakuum von Lösungsmittelresten befreit. Erhalten wurden 23.5 g der Zielverbindung als ca. 70%-iges Rohprodukt, das nicht weiter gereinigt wurde (Ausbeute über drei Stufen ca. 38% d. Th.).

GC-MS (Methode 1): Rₜ = 1.92 min und 2.01 min; jeweils m/z = 126 (M-C₃H₅O₂)⁺.

¹H-NMR (400 MHz, DMSO-*d*₆): beide Diastereomere δ [ppm] = 0.99-1.10 (m, 3H), 1.10-1.24 (m, 3H), 2.75-2.82 (m, 1H), 3.61/3.66 (je d, zus. 1H), 4.04-4.18 (m, 2H).

### Beispiel 28A

### Ethyl-(3R)-N-[(benzyloxy)carbonyl]-4,4,4-trifluorvalinat (Diastereomerengemisch)

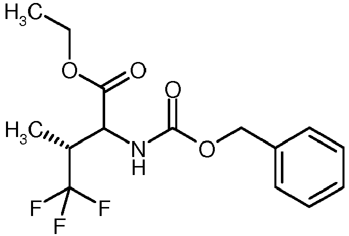

23.5 g Ethyl-(3*R*)-4,4,4-trifluorvalinat (Diastereomerengemisch, Rohprodukt ca. 70%) und 18.1 ml (130 mmol) Triethylamin wurden in 235 ml THF gelöst und mit 32.3 g (130 mmol) N-(Benzyloxy-carbonyloxy)succinimid versetzt. Die Reaktionsmischung wurde über Nacht bei RT gerührt und dann mit Ethylacetat verdünnt. Das Gemisch wurde nacheinander mit ges. Natriumhydrogencarbonat-Lösung, 1 N Salzsäure und ges. Natriumchlorid-Lösung gewaschen. Die organische Phase wurde über Magnesiumsulfat getrocknet und im Vakuum eingeengt. Das Rohprodukt wurde durch Chromatographie an Silicagel gereinigt (Laufmittel Cyclohexan/Ethylacetat 6:1). Es wurden 22.1 g des Zielprodukts erhalten (ca. 80% d. Th., Diastereomerenverhältnis ca. 2:1).

GC-MS (Methode 1): Rₜ = 6.02 min und 6.10 min; jeweils m/z = 333 (M)⁺.

LC-MS (Methode 2): Rₜ = 1.13 min; m/z = 334 (M+H)⁺.

¹H-NMR (400 MHz, DMSO-*d*₆): beide Diastereomere δ [ppm] = 1.10 (2d, zus. 3H), 1.15-1.23 (m, 3H), 2.80-3.07 (m, 1H), 4.08-4.21 (m, 2H), 4.38/4.62 (je dd, zus. 1H), 5.03-5.10 (m, 2H), 7.21-7.44 (m, 5H), 7.99 (2d, zus. 1H).

### Beispiel 29A

### (3R)-N-[(Benzyloxy)carbonyl]-4,4,4-trifluorvalin (Diastereomerengemisch)

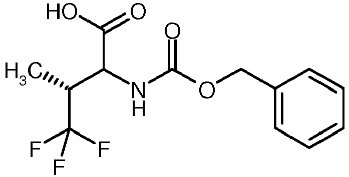

2.15 g (6.45 mmol) Ethyl-(3*R*)-*N*-[(benzyloxy)carbonyl]-4,4,4-trifluorvalinat (Diastereomerengemisch) wurden in einer Mischung aus je 8 ml THF, Methanol und Wasser gelöst und bei 0°C mit 3.87 g (96.8 mmol) Natriumhydroxid versetzt. Die Eiskühlung wurde entfernt, und die Reaktionsmischung wurde 1 h bei RT gerührt. Danach wurde der Ansatz auf Wasser gegeben, mit halbkonzentrierter Salzsäure sauer gestellt und mit Ethylacetat extrahiert. Die organische Phase wurde mit ges. Natriumchlorid-Lösung gewaschen, über Natriumsulfat getrocknet und im Vakuum eingeengt. Es wurden 1.95 g des Zielprodukts erhalten, das nicht weiter aufgereinigt wurde (99% d. Th., Diastereomerenverhältnis ca. 2.8:1).

LC-MS (Methode 5): Rₜ = 2.01 min; m/z = 306 (M+H)⁺.

¹H-NMR (400 MHz, DMSO-*d*₆): beide Diastereomere δ [ppm] = 1.09/1.11 (je d, zus. 3H), 2.79-3.06 (m, 1H), 4.31/4.61 (je dd, zus. 1H), 5.05-5.09 (m, 2H), 7.26-7.40 (m, ca. 5H), 7.76/7.83 (je d, zus. 1H), 12.63 (br. s, 1H).

### Beispiel 30A

### Ethyl-(3R)-N-[(allyloxy)carbonyl]-4,4,4-trifluorvalinat (Diastereomerengemisch)

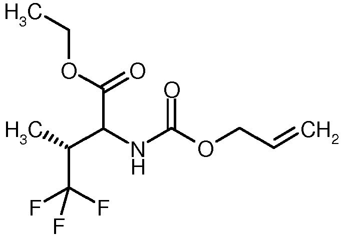

Zu einer Lösung von 6.38 g (55.4 mmol) Succinimid in 90 ml abs. THF wurden 9.7 ml (69.3 mmol) Triethylamin getropft. Die resultierende Suspension wurde auf 0°C gekühlt und tropfenweise mit einer Lösung von 6.68 g (55.4 mmol) Chlorameisensäureallylester in 10 ml abs. THF versetzt. Die Mischung wurde 1 h bei 0°C gerührt, bevor in drei Portionen insgesamt 9.2 g (ca. 46 mmol) Ethyl-(3*R*)-4,4,4-trifluorvalinat (Diastereomerengemisch, Rohprodukt) hinzugefügt wurden. Die Reaktionsmischung wurde über Nacht auf RT erwärmt, dann mit Ethylacetat verdünnt, mit ges. Natriumhydrogencarbonat-Lösung und ges. Natriumchlorid-Lösung gewaschen, über Natriumsulfat getrocknet und im Vakuum eingeengt. Das Rohprodukt wurde durch Chromatographie an Silicagel gereinigt (Laufmittel Cyclohexan/Ethylacetat 10:1 → 4:1). Es wurden 6.50 g des Zielprodukts erhalten (ca. 50% d. Th., Diastereomerenverhältnis ca. 2:1).

GC-MS (Methode 1): Rₜ = 3.96 min und 4.06 min; jeweils m/z = 210 (M-C₃H₅O₂)⁺.

¹H-NMR (400 MHz, DMSO-*d*₆): beide Diastereomere δ [ppm] = 1.07-1.13 (m, 3H), 1.17-1.23 (m, 3H), 2.82-3.05 (m, 1H), 4.10-4.18 (m, 2H), 4.36/4.60 (je dd, zus. 1H), 4.48-4.54 (m, 2H), 5.19 (d, 1H), 5.30 (d, 1H), 5.82-5.98 (m, 1H), 7.84-7.96 (m, 1H).

### Beispiel 31A

### (3R)-N-[(Allyloxy)carbonyl]-4,4,4-trifluorvalin (Diastereomerengemisch)

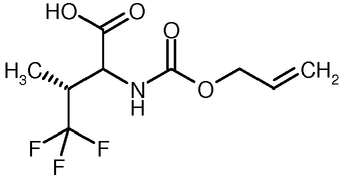

6.50 g (22.9 mmol) Ethyl-(3*R*)-*N*-[(allyloxy)carbonyl]-4,4,4-trifluorvalinat (Diastereomerengemisch) wurden in einer Mischung aus je 28 ml THF, Ethanol und Wasser gelöst und bei 0°C mit 13.77 g (344 mmol) Natriumhydroxid versetzt. Die Eiskühlung wurde entfernt, und die Reaktionsmischung wurde über Nacht bei RT gerührt. Der Ansatz wurde danach auf Wasser gegeben, mit halbkonzentrierter Salzsäure sauer gestellt und mit Ethylacetat extrahiert. Die organische Phase wurde mit ges. Natriumchlorid-Lösung gewaschen, über Natriumsulfat getrocknet und im Vakuum eingeengt. Es wurden 4.39 g des Zielprodukts erhalten, das nicht weiter aufgereinigt wurde (75% d. Th., Diastereomerenverhältnis ca. 2.5:1).

MS (DCI): m/z = 186 (M-CF₃)⁺.

¹H-NMR (400 MHz, DMSO-*d*₆): beide Diastereomere δ [ppm] = 1.07-1.11 (m, 3H), 2.83-3.03 (m, 1H), 4.28/4.57 (je dd, zus. 1H), 4.47-4.53 (m, 2H), 5.19 (d, 1H), 5.30 (d, 1H), 5.83-5.98 (m, 1H), 7.70/7.78 (je d, zus. 1H).

### Beispiel 32A

### tert.-Butyl-1-[3-({N-[(benzyloxy)carbonyl]-4,4,4-trifluorvalyl}amino)-4-fluorbenzyl]cyclopropancarboxylat (racemisches Diastereomerengemisch)

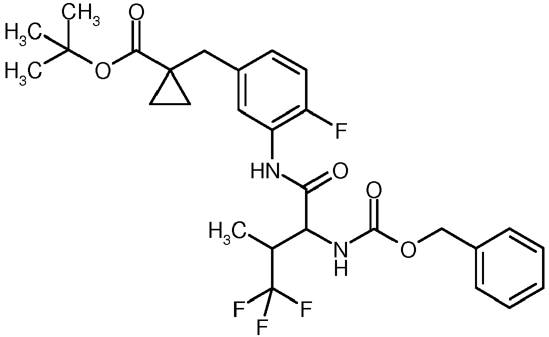

4.80 g (15.7 mmol) *N*-[(Benzyloxy)carbonyl]-4,4,4-trifluorvalin (racemisches Diastereomerengemisch) und 5.01 g (18.9 mmol) *tert*.-Butyl-1-(3-amino-4-fluorbenzyl)cyclopropancarboxylat wurden in einem Gemisch aus 40 ml DMF und 10 ml Pyridin gelöst und bei RT mit 6.58 g (17.3 mmol) HATU versetzt. Die Reaktionsmischung wurde über Nacht bei RT gerührt und dann auf Wasser gegeben. Es wurde dreimal mit Ethylacetat extrahiert, und die vereinigten organischen Phasen wurden nacheinander mit ges. Natriumhydrogencarbonat-Lösung, 1 N Salzsäure und ges. Natriumchlorid-Lösung gewaschen, über Magnesiumsulfat getrocknet und im Vakuum eingeengt. Das Rohprodukt wurde durch Chromatographie an Silicagel gereinigt (Laufmittel Cyclohexan/ Ethylacetat 20:1 → 10:1). Es wurden 5.34 g des Zielprodukts erhalten (61.5% d. Th., Diastereomerenverhältnis ca. 2.5:1).

LC-MS (Methode 5): Rₜ = 2.84 min; m/z = 497 (M-C₄H₇)⁺.

¹H-NMR (400 MHz, DMSO-*d*₆): beide Diastereomere δ [ppm] = 0.80-0.86 (m, 2H), 1.03-1.09 (m, 2H), 1.12 (d, 3H), 1.28/1.29 (je s, zus. 9H), 2.78-2.83 (m, 2H), 2.97-3.07 (m, 1H), 4.67/4.84 (t und dd, zus. 1H), 5.07/5.08 (je s, zus. 2H), 7.02-7.10 (m, 1H), 7.17 (dd, 1H), 7.28-7.40 (m, 5H), 7.62/ 7.67 (je dd, zus. 1H), 7.80-7.89 (m, 1H), 9.98/10.12 (je s, zus. 1H).

### Beispiel 33A

### tert.-Butyl-3-[3-({N-[(benzyloxy)carbonyl]-4,4,4-trifluorvalyl}amino)-4-fluorphenyl]propanoat (racemisches Diastereomerengemisch)

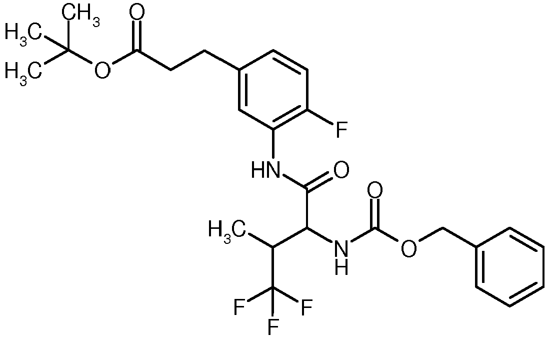

10.30 g (33.7 mmol) *N*-[(Benzyloxy)carbonyl]-4,4,4-trifluorvalin (racemisches Diastereomerengemisch) und 9.69 g (40.5 mmol) *tert*.-Butyl-3-(3-amino-4-fluorphenyl)propanoat wurden in einem Gemisch aus 40 ml DMF und 10 ml Pyridin gelöst und bei RT mit 16.68 g (43.9 mmol) HATU versetzt. Die Reaktionsmischung wurde über Nacht bei RT gerührt und dann mit Ethylacetat verdünnt. Das Gemisch wurde nacheinander mit ges. Natriumhydrogencarbonat-Lösung, 1 N Salzsäure und ges. Natriumchlorid-Lösung gewaschen, über Magnesiumsulfat getrocknet und im Vakuum eingeengt. Das Rohprodukt wurde durch Chromatographie an Silicagel vorgereinigt (Laufmittel Cyclohexan/Ethylacetat 10:1). Produkthaltige Fraktionen wurden im Vakuum eingeengt und der Rückstand mit Cyclohexan/Diethylether 5:1 verrührt. Der erhaltene Feststoff wurde abgesaugt und im Hochvakuum getrocknet. Es wurden 12.29 g des Zielprodukts erhalten (69% d. Th., Diastereomerenverhältnis ca. 2.5:1).

LC-MS (Methode 2): Rₜ = 1.29 min; m/z = 471 (M-C₄H₇)⁺.

¹H-NMR (400 MHz, DMSO-*d*₆): beide Diastereomere δ [ppm] = 1.12 (d, 3H), 1.30-1.37 (m, 9H), 2.48 (t, ca. 2H), 2.78 (t, 2H), 2.95-3.08 (m, 1H), 4.67/4.84 (t und dd, zus. 1H), 5.07/5.08 (je s, zus. 2H), 7.00-7.08 (m, 1H), 7.16 (dd, 1H), 7.26-7.44 (m, 5H), 7.55-7.63 (m, 1H), 7.77-7.88 (m, 1H), 9.96/10.10 (je s, zus. 1H).

### Allgemeine Vorschrift 1: HATU-vermittelte Amidkupplung von N-geschützten 4,4,4-Trifluorvalin-Derivaten mit Anilinen

Zu einer Lösung des betreffenden *N*-geschützten 4,4,4-Trifluorvalin-Derivats (ca. 0.8 bis 1.5 eq., 0.15 bis 1.5 mol/l) und eines Anilins (ca. 0.8 bis 1.5 eq., 0.15 bis 1.5 mol/l) in einem Gemisch aus DMF und Pyridin (Mischungsverhältnis ca. 3:1 bis 1.5:1) wird bei RT HATU (1.0 bis 2.0 eq.) gegeben. Alternativ kann statt Pyridin auch *N,N*-Diisopropylethylamin (2.0 bis 5.0 eq.) verwendet werden. Die resultierende Mischung wird für 4 h bis 48 h bei einer Temperatur von RT bis 60°C gerührt. Gegebenenfalls wird nach 24 h ein weiterer Anteil an Anilin-Komponente oder an Trifluorvalin-Derivat und HATU zugesetzt. Nach Ende der Reaktion kann das Rohprodukt nach Entfernen des Lösungsmittels im Vakuum durch präparative RP-HPLC (Eluent: Acetonitril/Wasser-Gradient) oder alternativ, nach wässriger Aufarbeitung der Reaktionsmischung, durch Chromatographie an Silicagel (Eluenten: Cyclohexan/Diethylether-, Petrolether/Ethylacetat-, Cyclohexan/ Ethylacetat- oder Dichlormethan/Methanol-Gemische) gereinigt werden.

Die folgenden Beispiele wurden gemäß der Allgemeinen Vorschrift 1 hergestellt:

| **Beispiel** | **Name / Struktur / Edukte** | **Analytische Daten** |
|---|---|---|
| **34A** | *tert*.-Butyl-3-[3-({N-[(benzyloxy)carbonyl]-4,4,4-trifluorvalyl}amino)-4-chlorphenyl]propanoat *(racemisches Diastereomerengemisch)* | LC-MS (Methode 3): Rₜ = 1.52 min; m/z = 487 (M-C₄H₇)⁺. |
| | 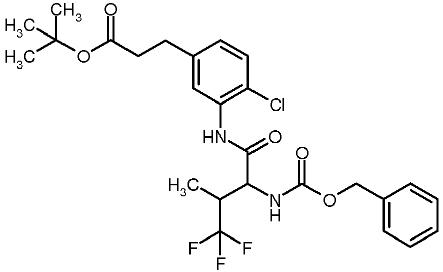 | ¹H-NMR (400 MHz, DMSO-*d₆*): beide Diastereomere δ [ppm] = 1.15 (d, 3H), 1.35 (s, 9H), 2.79 (t, 2H), 3.02-3.18 (m, 1H), 4.65/4.85 (t und dd, zus. 1H), 5.03-5.16 (m, 2H), 7.10 (dd, 1H), 7.27-7.43 (m, 6H), 7.49 (s, 1H), 7.89 (d, 1H), 9.75/9.89 (je s, zus. 1H). Diastereomerenverhältnis ca. 7:1 |
| | aus *N*-[(Benzyloxy)carbonyl]-4,4,4-trifluorvalin *(racemisches Diastereomerengemisch)* und *tert*.-Butyl-3-(3-amino-4-chlorphenyl)propanoat | |
| **35A** | *tert.* -Butyl-3-[3-({(3*R*)-*N*-[(benzyloxy)carbonyl]-4,4,4-trifluorvalyl}amino)-4-chlorphenyl]propanoat (*Diastereomerengemisch*) | LC-MS (Methode 2): Rₜ = 1.27 min; m/z = 487 (M-C₄H₇)⁺. |
| | 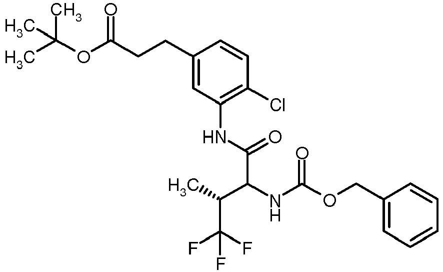 | ¹H-NMR (400 MHz, DMSO-*d₆*): Hauptdiastereomer δ [ppm] = 1.15 (d, 3H), 1.35 (s, 9H), 2.49 (t, verdeckt, ca. 2H), 2.79 (t, 2H), 4.85 (dd, 1H), 5.10 (d, 2H), 7.10 (dd, 1H), 7.27-7.44 (m, 7H), 7.49 (s, 1H), 7.89 (d, 1H), 9.75 (s, 1H). |
| | aus (3R)-N-[(Benzyloxy)carbonyl]-4,4,4-trifluorvalin *(Diastereomerengemisch)* und *tert*.-Butyl-3-(3-amino-4-chlorphenyl)propanoat | |
| **36A** | Ethyl-(2*S*)-3-[3-({*N*-[(benzyloxy)carbonyl]-4,4,4-trifluorvalyl}amino)-4-fluorphenyl]-2-methyl-propanoat (*Isomerengemisch*) | LC-MS (Methode 3): Rₜ = 1.40 min; m/z = 469 (M-C₂H₃O)⁺. |
| | 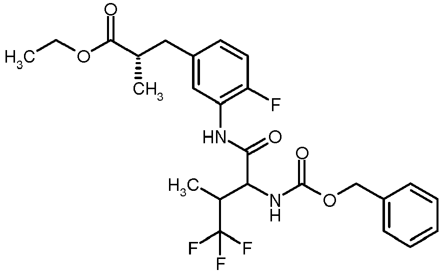 | ¹H-NMR (400 MHz, DMSO-*d₆*): beide Diastereomere δ [ppm] = 1.00-1.16 (m, 9H), 2.61-2.72 (m, 2H), 2.75-2.87 (m, 1H), 2.97-3.08 (m, 1H), 4.00 (q, 2H), 4.67/4.84 (t und dd, zus. 1H), 5.04-5.11 (m, 2H), 6.94-7.04 (m, 1H), 7.16 (dd, 1H), 7.27-7.41 (m, 5H), 7.49-7.59 (m, 1H), 7.82 (d, 1H), 9.97/10.11 (je s, zus. 1H). Diastereomerenverhältnis ca. 3.8:1 |
| | aus *N*-[(Benzyloxy)carbonyl]-4,4,4-trifluorvalin *(racemisches Diastereomerengemisch)* und (+)-Ethyl-(2*S*)-3-(3-amino-4-fluorphenyl)-2-methyl-propanoat | |
| **37A** | Ethyl-(2*R*)-3-[3-({*N*-[(benzyloxy)carbonyl]-4,4,4-trifluorvalyl}amino)-4-fluorphenyl]-2-methyl-propanoat (*Isomerengemisch*) | LC-MS (Methode 2): Rₜ = 1.24 min; m/z = 511 (M-H)⁻. ¹H-NMR (400 MHz, DMSO-*d₆*): beide Diastereomere δ [ppm] = 1.03-1.15 (m, 9H), 2.61-2.72 (m, 2H), 2.78-2.87 (m, 1H), 2.97-3.08 (m, 1H), 4.00 (q, 2H), 4.67/4.84 (t und dd, zus. 1H), 5.04-5.11 (m, 2H), 6.95-7.04 (m, 1H), 7.16 (dd, 1H), 7.27-7.41 (m, 5H), 7.49-7.59 (m, 1H), 7.82 (d, 1H), 9.97/10.11 (je s, zus. 1H). Diastereomerenverhältnis ca. 4.5:1 |
| | 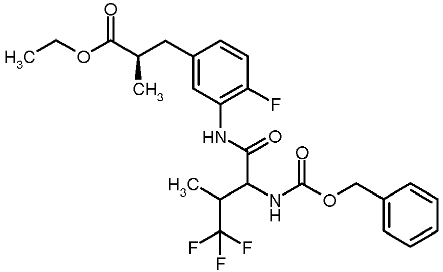 | |
| | aus *N*-[(Benzyloxy)carbonyl]-4,4,4-trifluorvalin *(racemisches Diastereomerengemisch)* und (-)-Ethyl-(2*R*)-3-(3-amino-4-fluorphenyl)-2-methyl-propanoat | |
| **38A** | Ethyl-(2*R*)-3-[3-({*N*-[(allyloxy)carbonyl]-4,4,4-trifluorvalyl}amino)-4-chlorphenyl]-2-methyl-propanoat (*Isomerengemisch*) | LC-MS (Methode 2): Rₜ = 1.24 min; m/z = 479 (M+H)⁺. ¹H-NMR (400 MHz, DMSO-*d₆*): beide Diastereomere δ [ppm] = 1.05-1.17 (m, 9H), 2.63-2.74 (m, 2H), 2.78-2.90 (m, 1H), 3.03-3.12 (m, 1H), 4.01 (q, 2H), 4.51-4.56 (m, 2H), 4.63/4.83 (t und dd, zus. 1H), 5.15-5.23 (m, 1H), 5.32 (dd, 1H), 5.92 (ddd, 1H), 7.02-7.09 (m, 1H), 7.37-7.49 (m, 2H), 7.75-7.82 (m, 1H), 9.76/ 9.89 (je s, zus. 1H). Diastereomerenverhältnis ca. 8:1 |
| | 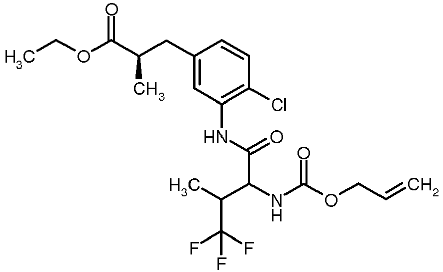 | |
| | aus N-[(Allyloxy)carbonyl]-4,4,4-trifluorvalin *(racemisches Diastereomerengemisch)* und (-)-Ethyl-(2*R*)-3-(3-amino-4-chlorphenyl)-2-methyl-propanoat | |
| **39A** | Ethyl-(2*S*)-3-[3-({*N*-[(allyloxy)carbonyl]-4,4,4-trifluorvalyl}amino)-4-chlorphenyl]-2-methyl-propanoat (*Isomerengemisch*) | LC-MS (Methode 2): Rₜ = 1.23 min; m/z = 479 (M+H)⁺. ¹H-NMR (400 MHz, DMSO-*d₆*): beide Diastereomere δ [ppm] = 1.04-1.18 (m, 9H), 2.64-2.74 (m, 2H), 2.79-2.89 (m, 1H), 2.95-3.14 (m, 1H), 4.01 (q, 2H), 4.47-4.57 (m, 2H), 4.67/4.83 (je dd, zus. 1H), 5.15-5.39 (m, 2H), 5.84-5.99 (m, 1H), 7.06 (dd, 1H), 7.35-7.48 (m, 2H), 7.82/7.97 (je d, zus. 1H), 9.76 (s, 1H). Diastereomerenverhältnis ca. 4.2:1 |
| | 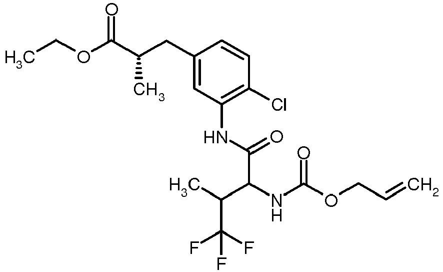 | |
| | aus N-[(Allyloxy)carbonyl]-4,4,4-trifluorvalin *(racemisches Diastereomerengemisch)* und (+)-Ethyl-(2*S*)-3-(3-amino-4-chlorphenyl)-2-methyl-propanoat | |
| **40A** | *tert*.-Butyl-(3*R*)-3-[3-({*N*-[(allyloxy)carbonyl]-4,4,4-trifluorvalyl}amino)-4-chlorphenyl]butanoat (*Isomerengemisch*) | LC-MS (Methode 2): Rₜ = 1.29 min; m/z = 507 (M+H)⁺. ¹H-NMR (400 MHz, DMSO-*d₆*): Hauptdiastereomer δ [ppm] = 1.15 (d, 3H), 1.19 (d, 3H), 1.30 (s, 9H), 2.39-2.47 (m, ca. 2H), 3.03-3.15 (m, 2H), 4.54 (d, 2H), 4.84 (dd, 1H), 5.19 (d, 1H), 5.32 (d, 1H), 5.92 (ddd, 1H), 7.14 (d, 1H), 7.42 (d, 1H), 7.51 (s, 1H), 7.82 (d, 1H), 9.76 (s, 1H). |
| | 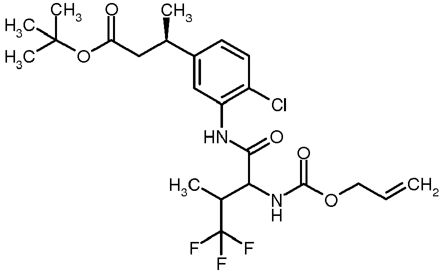 | |
| | aus N-[(Allyloxy)carbonyl]-4,4,4-trifluorvalin (*racemisches Diastereomerengemisch*) und (+)-*tert*.-Butyl-(3*R*)-3-(3-amino-4-chlorphenyl)butanoat | |
| **41A** | *tert*.-Butyl-(3*S*)-3-[3-({*N*-[(allyloxy)carbonyl]-4,4,4-trifluorvalyl}amino)-4-chlorphenyl]butanoat (*Isomerengemisch*) | LC-MS (Methode 2): Rₜ = 1.33 min; m/z = 507 (M+H)⁺. ¹H-NMR (400 MHz, DMSO-*d₆*): Hauptdiastereomer δ [ppm] = 1.15 (d, 3H), 1.19 (d, 3H), 1.30 (s, 9H), 2.39-2.48 (m, ca. 2H), 3.03-3.15 (m, 2H), 4.54 (d, 2H), 4.84 (dd, 1H), 5.19 (dd, 1H), 5.32 (dd, 1H), 5.85-5.99 (m, 1H), 7.14 (d, 1H), 7.42 (d, 1H), 7.51 (s, 1H), 7.82 (d, 1H), 9.76 (s, 1H). |
| | 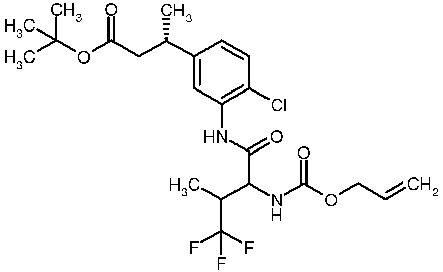 | |
| | aus *N*-[(Allyloxy)carbonyl]-4,4,4-trifluorvalin (r*acemisches Diastereomerengemisch*) und (-)-*tert*.-Butyl-(3*S*)-3-(3-amino-4-chlorphenyl)butanoat | |
| **42A** | *tert*.-Butyl-(3*S*)-3-[3-({(3*R*)-*N*-[(allyloxy)carbonyl]-4,4,4-trifluorvalyl}amino)-4-chlorphenyl]butanoat (*Diastereomerengemisch*) | LC-MS (Methode 2): Rₜ = 1.30 min; m/z = 505 (M-H)⁻. ¹H-NMR (400 MHz, DMSO-*d₆*): Hauptdiastereomer δ [ppm] = 1.14-1.21 (m, 6H), 1.30 (s, 9H), 2.39-2.48 (m, ca. 2H), 3.02-3.15 (m, 2H), 4.54 (d, 2H), 4.84 (dd, 1H), 5.19 (d, 1H), 5.28-5.36 (m, 1H), 5.83-5.99 (m, 1H), 7.14 (dd, 1H), 7.42 (d, 1H), 7.50 (s, 1H), 7.83 (d, 1H), 9.78 (s, 1H). |
| | 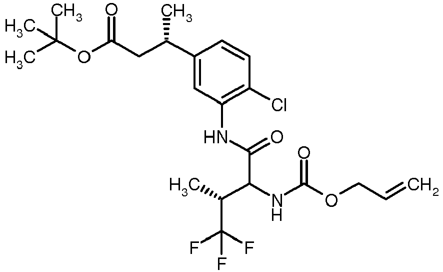 | |
| | aus (3*R*)-*N*-[(Allyloxy)carbonyl]-4,4,4-trifluorvalin (*Diastereomerengemisch*) und (-)-*tert*.-Butyl-(3*S*)-3-(3-amino-4-chlorphenyl)butanoat | |

### Beispiel 43A

### Ethyl-(2S)-3-[3-({(3R)-N-[(benzyloxy)carbonyl]-4,4,4-trifluorvalyl}amino)-4-fluorphenyl]-2-methylpropanoat (Diastereomerengemisch)

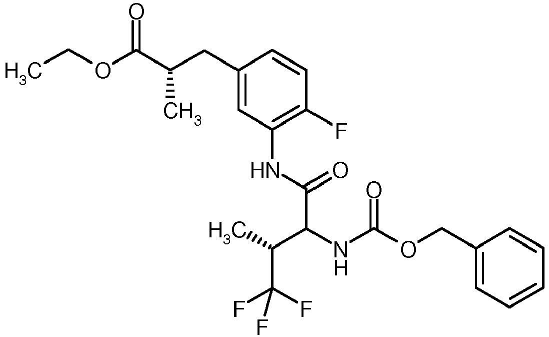

0.87 g (2.85 mmol) (3*R*)-*N*-[(Benzyloxy)carbonyl]-4,4,4-trifluorvalin (Diastereomerengemisch) und 0.77 g (3.42 mmol) (+)-Ethyl-(2*S*)-3-(3-amino-4-fluorphenyl)-2-methylpropanoat wurden in einem Gemisch aus 5 ml DMF und 2.5 ml Pyridin gelöst und bei RT mit 1.41 g (3.71 mmol) HATU versetzt. Die Reaktionsmischung wurde über Nacht bei RT gerührt und danach mit Ethylacetat verdünnt. Das Gemisch wurde nacheinander mit 1 N Salzsäure, ges. Natriumhydrogencarbonat-Lösung und ges. Natriumchlorid-Lösung gewaschen, über Magnesiumsulfat getrocknet und im Vakuum eingeengt. Das Rohprodukt wurde durch Chromatographie an Silicagel gereinigt (Laufmittel Cyclohexan/Ethylacetat 6:1). Es wurden 1.27 g des Zielprodukts erhalten (87.2% d. Th., Diastereomerenverhältnis ca. 4.8:1).

LC-MS (Methode 3): Rₜ = 1.41 min; m/z = 513 (M+H)⁺.

¹H-NMR (400 MHz, DMSO-*d*₆): beide Diastereomere δ [ppm] = 1.04-1.15 (m, 9H), 2.62-2.72 (m, ca. 2H), 2.76-2.86 (m, 1H), 2.96-3.06 (m, 1H), 3.96-4.05 (m, 2H), 4.84 (dd, 1H), 5.07/5.08 (je s, zus. 2H), 7.01 (td, 1H), 7.17 (dd, 1H), 7.27-7.41 (m, 5H), 7.50-7.59 (m, 1H), 7.82-7.88 (m, 1H), 9.99/10.13 (je s, zus. 1H).

### Beispiel 44A

### Ethyl-(2S)-3-[3-({(3R)-N-[(benzyloxy)carbonyl]-4,4,4-trifluorvalyl}amino)-4-chlorphenyl]-2-methylpropanoat (Diastereomerengemisch)

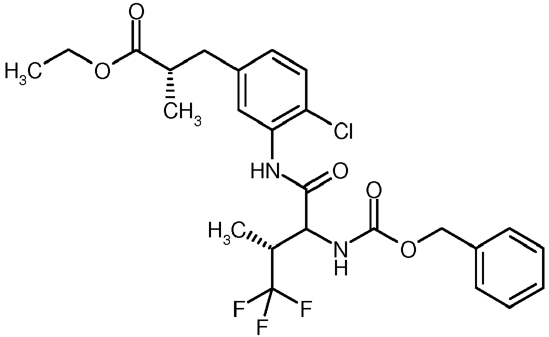

1.26 g (76% Reinheit, ca. 3.14 mmol) (3*R*)-*N*-[(Benzyloxy)carbonyl]-4,4,4-trifluorvalin (Diastereomerengemisch) und 0.91 g (3.76 mmol) (+)-Ethyl-(2*S*)-3-(3-amino-4-chlorphenyl)-2-methyl-propanoat wurden in einem Gemisch aus 10 ml DMF und 4 ml Pyridin gelöst und bei RT mit 1.55 g (4.08 mmol) HATU versetzt. Die Reaktionsmischung wurde über Nacht bei RT gerührt und dann auf Wasser gegeben. Es wurde dreimal mit Ethylacetat extrahiert, und die vereinigten organischen Phasen wurden nacheinander mit ges. Natriumhydrogencarbonat-Lösung, 1 N Salzsäure und ges. Natriumchlorid-Lösung gewaschen, über Magnesiumsulfat getrocknet und im Vakuum eingeengt. Der angefallene Feststoff wurde in einem Gemisch aus Cyclohexan und Diethylether (ca. 3:1) verrührt, abgesaugt und im Hochvakuum getrocknet. Es wurden 0.99 g des Zielprodukts erhalten (59.9% d. Th., Diastereomerenverhältnis ca. 7:1).

LC-MS (Methode 2): Rₜ = 1.32 min; m/z = 529 (M+H)⁺.

¹H-NMR (400 MHz, DMSO-*d*₆): Hauptdiastereomer δ [ppm] = 1.03-1.18 (m, 9H), 2.62-2.75 (m, 2H), 2.80-2.90 (m, 1H), 3.03-3.13 (m, 1H), 4.01 (q, 2H), 4.85 (dd, 1H), 5.04-5.16 (m, 2H), 7.06 (dd, 1H), 7.25-7.49 (m, ca. 7H), 7.89 (d, 1H), 9.75 (s, 1H).

### Beispiel 45A

### tert.-Butyl-1-[3-({(3R)-N-[(allyloxy)carbonyl]-4,4,4-trifluorvalyl}amino)-4-chlorbenzyl]cyclopropancarboxylat (Diastereomerengemisch)

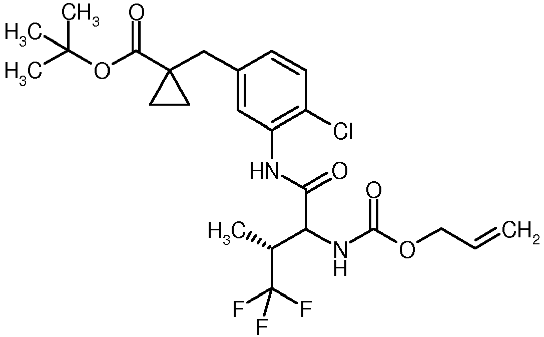

398.5 mg (1.56 mmol) (3*R*)-*N*-[(Allyloxy)carbonyl]-4,4,4-trifluorvalin (Diastereomerengemisch) und 400 mg (1.42 mmol) *tert*.-Butyl-1-(3-amino-4-chlorbenzyl)cyclopropancarboxylat wurden in einem Gemisch aus 4.7 ml DMF und 1.6 ml Pyridin gelöst und bei RT mit 647.8 mg (1.70 mmol) HATU versetzt. Die Reaktionsmischung wurde über Nacht bei RT gerührt. Es wurden weitere 400 mg (1.42 mmol) *tert*.-Butyl-1-(3-amino-4-chlorbenzyl)cyclopropancarboxylat sowie 647.8 mg (1.70 mmol) HATU zugesetzt, und die Reaktionsmischung wurde nochmals 4 h bei 50°C gerührt. Nach Verdünnen mit Ethylacetat wurde das Gemisch nacheinander mit ges. Natriumhydrogencarbonat-Lösung, 1 N Salzsäure und ges. Natriumchlorid-Lösung gewaschen, über Magnesiumsulfat getrocknet und im Vakuum eingeengt. Das Rohprodukt wurde durch präparative RP-HPLC gereinigt (Eluent Acetonitril/Wasser). Es wurden 453 mg des Zielprodukts erhalten (55.9% d. Th., Diastereomerenverhältnis >8:1).

LC-MS (Methode 2): Rₜ = 1.31 min; m/z = 519 (M+H)⁺.

¹H-NMR (400 MHz, DMSO-*d*₆): δ [ppm] = 0.84 (q, 2H), 1.04-1.09 (m, 2H), 1.15 (d, 3H), 1.30 (s, 9H), 2.83 (s, 2H), 3.02-3.11 (m, 1H), 4.54 (d, 2H), 4.79-4.86 (m, 1H), 5.19 (d, 1H), 5.28-5.35 (m, 1H), 5.84-5.99 (m, 1H), 7.12 (dd, 1H), 7.40-7.45 (m, 1H), 7.49-7.56 (m, 1H), 7.84 (d, 1H), 9.78 (s, 1H).

### Beispiel 46A und Beispiel 47A

### (+/-)-tert.-Butyl-1-{4-fluor-3-[(4,4,4-trifluorvalyl)amino]benzyl)cyclopropancarboxylat (Diastereomer 1 und 2)

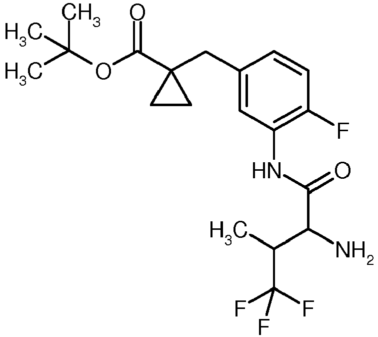

5.31 g (9.61 mmol) *tert*.-Butyl-1-[3-({N-[(benzyloxy)carbonyl]-4,4,4-trifluorvalyl}amino)-4-fluor-benzyl]cyclopropancarboxylat (Beispiel 32A, racemisches Diastereomerengemisch) wurden in einer Mischung aus 100 ml Ethanol und 20 ml THF gelöst. Die Lösung wurde mit Argon inertisiert und mit 511 mg Palladium auf Kohle (10%-ig) versetzt. Die Reaktionsmischung wurde über Nacht bei Normaldruck unter einer Wasserstoffatmosphäre kräftig gerührt. Nach Filtration über Kieselgur und Nachwaschen mit Ethanol/THF wurde das Filtrat im Vakuum eingeengt. Der Rückstand wurde durch Chromatographie an Silicagel (Laufmittel Cyclohexan/Ethylacetat 50:1 → 4:1) gereinigt und die Diastereomeren aufgetrennt:

### Beispiel 46A (Diastereomer 1):

Ausbeute: 3.03 g (75.4% d. Th.)

LC-MS (Methode 2): Rₜ = 1.05 min; m/z = 419 (M+H)⁺.

¹H-NMR (400 MHz, DMSO-*d*₆): δ [ppm] = 0.80-0.86 (m, 2H), 1.00-1.10 (m, 5H), 1.30 (s, 9H), 2.75-2.87 (m, 2H), 2.92-3.04 (m, 1H), 3.80 (br. s, 1H), 6.95-7.07 (m, 1H), 7.18 (dd, 1H), 7.99 (dd, 1H), 10.01 (br. s, ca. 1H).

### Beispiel 47A (Diastereomer 2):

Ausbeute: 0.88 g (21.9% d. Th.)

LC-MS (Methode 2): Rₜ = 0.96 min; m/z = 419 (M+H)⁺.

¹H-NMR (400 MHz, DMSO-*d*₆): δ [ppm] = 0.79-0.85 (m, 2H), 1.04-1.10 (m, 2H), 1.18 (d, 3H), 1.30 (s, 9H), 2.81 (s, 2H), 2.86-2.97 (m, 1H), 3.61 (br. s, 1H), 6.93-7.04 (m, 1H), 7.17 (dd, 1H), 7.93 (dd, 1H), 9.89 (br. s, ca. 1H).

### Beispiel 48A und Beispiel 49A

### (+/-)-tert.-Butyl-3-{4-fluor-3-[(4,4,4-trifluorvalyl)amino]phenyl}propanoat (Diastereomer 1 und 2)

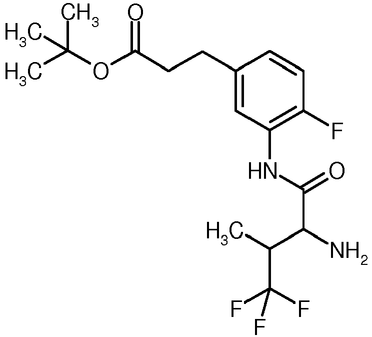

12.29 g (23.3 mmol) *tert*.-Butyl-3-[3-({*N*-[(benzyloxy)carbonyl]-4,4,4-trifluorvalyl}amino)-4-fluorphenyl]propanoat (Beispiel 33A, racemisches Diastereomerengemisch) wurden in einer Mischung aus 80 ml Ethanol und 20 ml THF gelöst. Die Lösung wurde mit Argon inertisiert und mit 745 mg Palladium auf Kohle (10%-ig) versetzt. Die Reaktionsmischung wurde über Nacht bei Normaldruck unter einer Wasserstoffatmosphäre kräftig gerührt. Nach Filtration über Kieselgur und Nachwaschen mit Ethanol/THF wurde das Filtrat im Vakuum eingeengt. Der Rückstand wurde durch Chromatographie an Silicagel (Laufmittel Cyclohexan/Ethylacetat 20:1 → 4:1) gereinigt und die Diastereomeren aufgetrennt:

### Beispiel 48A (Diastereomer 1):

Ausbeute: 5.72 g (62.5% d. Th.)

LC-MS (Methode 2): Rₜ = 0.92 min; m/z = 393 (M+H)⁺.

¹H-NMR (400 MHz, DMSO-*d*₆): δ [ppm] = 1.04 (d, 3H), 1.35 (s, 9H), 2.49 (t, ca. 2H, verdeckt), 2.78 (t, 2H), 2.92-3.04 (m, 1H), 3.80 (br. s, 1H), 6.85-7.04 (m, 1H), 7.18 (dd, 1H), 7.92 (dd, 1H).

### Beispiel 49A (Diastereomer 2):

Ausbeute: 2.61 g (28.5% d. Th.)

LC-MS (Methode 2): Rₜ = 0.87 min; m/z = 393 (M+H)⁺.

¹H-NMR (400 MHz, DMSO-*d*₆): δ [ppm] = 1.18 (d, 3H), 1.35 (s, 9H), 2.49 (t, ca. 2H, verdeckt), 2.78 (t, 2H), 2.84-2.97 (m, 1H), 3.55-3.66 (m, 1H), 7.00 (td, 1H), 7.17 (dd, 1H), 7.86 (dd, 1H).

### Beispiel 50A

### (+)-tert.-Butyl-3-(4-fluor-3-{[(3R)-4,4,4-trifluor-D-valyl]amino}phenyl)propanoat

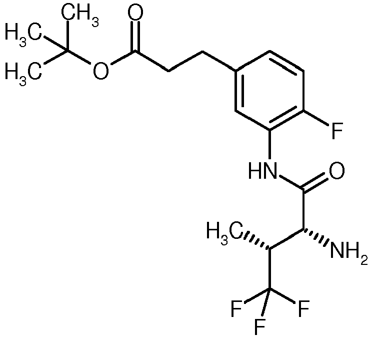

Das oben erhaltene racemische Diastereomer 1 von *tert*.-Butyl-3-{4-fluor-3-[(4,4,4-trifluorvalyl)-amino]phenyl}propanoat (Beispiel 48A) wurde durch präparative HPLC an chiraler Phase in die Enantiomere aufgetrennt [Säule: Daicel Chiralpak AD-H, 5 µm, 250 mm x 20 mm; Injektionsvolumen: 0.25 ml; Temperatur: 45°C; Eluent: 80% Isohexan / 20% Ethanol (+ 0.2% Diethylamin); Fluss: 15 ml/min; Detektion: 220 nm]. Ausgehend von 5.70 g Racemat wurden 2.59 g der Titelverbindung (als Enantiomer 2) isoliert.

LC-MS (Methode 2): Rₜ = 0.93 min; m/z = 393 (M+H)⁺.

¹H-NMR (400 MHz, DMSO-*d*₆): δ [ppm] = 1.04 (d, 3H), 1.35 (s, 9H), 2.49 (t, ca. 2H, verdeckt), 2.78 (t, 2H), 2.92-3.04 (m, 1H), 3.80 (br. s, 1H), 6.95-7.03 (m, 1H), 7.19 (dd, 1H), 7.92 (dd, 1H).

[α]_{D}²⁰ = +24.2°, c = 0.500, Chloroform.

### Beispiel 51A und Beispiel 52A

### (+/-)-tert.-Butyl-3-{4-chlor-3-[(4,4,4-trifluorvalyl)amino]phenyl}propanoat (Diastereomer 1 und 2)

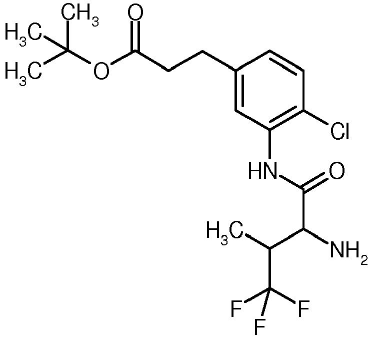

3.19 g (5.87 mmol) *tert*.-Butyl-3-[3-({*N*-[(benzyloxy)carbonyl]-4,4,4-trifluorvalyl}amino)-4-chlorphenyl]propanoat (Beispiel 34A, racemisches Diastereomerengemisch) wurden in 50 ml Ethylacetat gelöst. Die Lösung wurde mit Argon inertisiert und mit 125 mg Palladium auf Kohle (5%-ig) versetzt. Die Reaktionsmischung wurde 3 h bei Normaldruck unter einer Wasserstoffatmosphäre kräftig gerührt. Nach Filtration über Kieselgur wurde das Filtrat im Vakuum eingeengt. Der Rückstand wurde durch Chromatographie an Silicagel (Laufmittel Cyclohexan/Ethylacetat 50:1 → 3:1) gereinigt und die Diastereomeren aufgetrennt:

### Beispiel 51A (Diastereomer 1):

Ausbeute: 1.73 g (72.1 % d. Th.)

LC-MS (Methode 2): Rₜ = 1.09 min; m/z = 409 (M+H)⁺.

¹H-NMR (400 MHz, DMSO-*d*₆): δ [ppm] = 1.04 (d, 3H), 1.35 (s, 9H), 2.48-2.53 (m, ca. 2H, verdeckt), 2.74-2.83 (m, 2H), 2.96-3.17 (m, 1H), 3.82 (br. s, 1H), 7.02 (dd, 1H), 7.42 (d, 1H), 8.00-8.16 (m, 1H).

### Beispiel 52A (Diastereomer 2):

Ausbeute: 0.193 g (8.0% d. Th.)

LC-MS (Methode 2): Rₜ = 0.99 min; m/z = 409 (M+H)⁺.

¹H-NMR (400 MHz, DMSO-*d*₆): δ [ppm] = 1.22 (d, 3H), 1.35 (s, 9H), 2.48-2.52 (m, ca. 2H, verdeckt), 2.76-2.85 (m, 2H), 2.95-3.12 (m, 1H), 3.61 (br. s, 1H), 7.01 (dd, 1H), 7.41 (d, 1H), 8.05 (d, 1H).

### Beispiel 53A

### (+)-tert. -Butyl-3-(4-chlor-3-{[(3R)-4,4,4-trifluor-D-valyl]amino}phenyl)propanoat

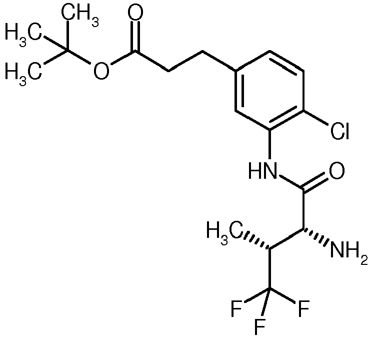

1.17 g (2.16 mmol) *tert*.-Butyl-3-[3-({(3*R*)-*N*-[(benzyloxy)carbonyl]-4,4,4-trifluorvalyl}amino)-4-chlorphenyl]propanoat (Beispiel 35A, Diastereomerengemisch) wurden in 20 ml Ethylacetat gelöst. Die Lösung wurde mit Argon inertisiert und mit 229 mg Palladium auf Kohle (5%-ig) versetzt. Die Reaktionsmischung wurde 8 h bei Normaldruck unter einer Wasserstoffatmosphäre kräftig gerührt. Nach Filtration über Kieselgur wurde das Filtrat im Vakuum eingeengt. Der erhaltene Rückstand wurde wieder in 50 ml Ethylacetat gelöst. Nach Inertisierung der Lösung mit Argon wurden erneut 230 mg Palladium auf Kohle (5%-ig) zugesetzt, und die Reaktionsmischung wurde weitere 4 h unter Wasserstoffnormaldruck kräftig gerührt. Nach Filtration über Kieselgur wurde das Filtrat im Vakuum eingeengt und der Rückstand erneut in 20 ml THF gelöst. Nach Inertisierung der Lösung mit Argon wurden nochmals 230 mg Palladium auf Kohle (5%-ig) zugesetzt, und die Reaktionsmischung wurde weitere 3 h unter Wasserstoffnormaldruck kräftig gerührt. Nach Filtration über Kieselgur wurde das Filtrat im Vakuum eingeengt, und der erhaltene Rückstand wurde durch Chromatographie an Silicagel (Laufmittel Cyclohexan/Ethylacetat 40:1 → 5:1) gereinigt und in die Diastereomeren aufgetrennt. Es wurden so 197 mg der Titelverbindung erhalten (22.4% d. Th.).

LC-MS (Methode 2): Rₜ = 1.08 min; m/z = 409 (M+H)⁺.

¹H-NMR (400 MHz, DMSO-*d*₆): δ [ppm] = 1.04 (d, 3H), 1.35 (s, 9H), 2.48-2.52 (m, ca. 2H, verdeckt), 2.76-2.84 (m, 2H), 3.02-3.12 (m, 1H), 3.80-3.86 (m, 1H), 7.02 (dd, 1H), 7.42 (d, 1H), 8.10-8.14 (m, 1H).

[α]_{D}²⁰ = +15.6°, c = 0.625, Chloroform.

### Beispiel 54A

### Ethyl-(2S)-3-{4-fluor-3-[(4,4,4-trifluorvalyl)amino]phenyl}-2-methylpropanoat (Diastereomerengemisch 1)

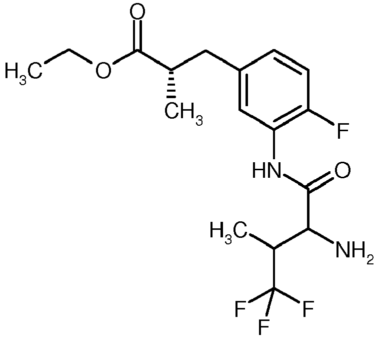

2.60 g (5.07 mmol) Ethyl-(2*S*)-3-[3-({*N*-[(benzyloxy)carbonyl]-4,4,4-trifluorvalyl}amino)-4-fluor-phenyl]-2-methylpropanoat (Beispiel 36A, Gemisch aus 4 Isomeren) wurden in einer Mischung aus 10.7 ml Ethanol und 10.7 ml THF gelöst. Die Lösung wurde mit Argon inertisiert und mit 322 mg Palladium auf Kohle (10%-ig) versetzt. Die Reaktionsmischung wurde über Nacht bei Normaldruck unter einer Wasserstoffatmosphäre kräftig gerührt. Nach Filtration über Kieselgur und Nachwaschen mit Dichlormethan/Methanol wurde das Filtrat im Vakuum eingeengt. Der Rückstand wurde durch Chromatographie an Silicagel (Laufmittel Cyclohexan/Ethylacetat 10:1 → 4:1) gereinigt und aufgetrennt. Es wurden 1.39 g der Titelverbindung als Diastereomerengemisch 1 erhalten (72.4% d. Th.).

LC-MS (Methode 5): Rₜ = 1.60 min; m/z = 379 (M+H)⁺.

¹H-NMR (400 MHz, DMSO-*d*₆): δ [ppm] = 1.01-1.15 (m, 9H), 2.62-2.72 (m, 2H), 2.78-2.88 (m, 1H), 2.90-3.06 (m, 1H), 3.81 (br. s, 1H), 4.01 (q, 2H), 6.90-7.00 (m, 1H), 7.18 (dd, 1H), 7.88 (d, 1H).

### Beispiel 55A

### (+)-Ethyl-(2S)-3-(4-fluor-3-{[(3R)-4,4,4-trifluor-D-valyl]amino}phenyl)-2-methylpropanoat

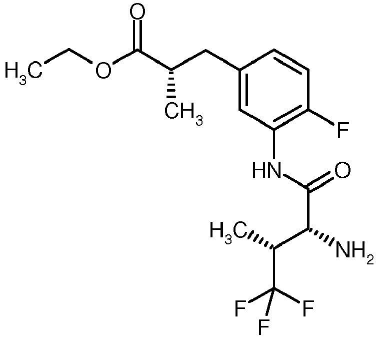

### Verfahren A:

Das oben erhaltene Diastereomerengemisch 1 von Ethyl-(2*S*)-3-{4-fluor-3-[(4,4,4-trifluorvalyl)-amino]phenyl}-2-methylpropanoat (Beispiel 54A) wurde durch präparative HPLC an chiraler Phase weiter aufgetrennt [Säule: Daicel Chiralpak AD-H, 5 µm, 250 mm x 20 mm; Injektionsvolumen: 0.25 ml; Temperatur: 45°C; Eluent: 30% Isohexan / 70% Ethanol (+ 0.2% Diethylamin); Fluss: 15 ml/min; Detektion: 220 nm]. Ausgehend von 1.39 g Diastereomerengemisch wurden 0.626 g der Titelverbindung (als Enantiomer 2) isoliert.

LC-MS (Methode 5): Rₜ = 1.58 min; m/z = 379 (M+H)⁺.

¹H-NMR (400 MHz, DMSO-*d*₆): δ [ppm] = 1.02-1.15 (m, 9H), 2.62-2.72 (m, 2H), 2.76-2.88 (m, 1H), 2.91-3.06 (m, 1H), 3.81 (br. s, 1H), 3.97-4.04 (m, 2H), 6.88-7.00 (m, 1H), 7.18 (dd, 1H), 7.88 (dd, 1H).

[α]_{D}²⁰ = +54.1°, c = 0.530, Chloroform.

### Verfahren B:

1.274 g (2.49 mmol) Ethyl-(2*S*)-3-[3-({(3*R*)-*N*-[(benzyloxy)carbonyl]-4,4,4-trifluorvalyl}amino)-4-fluorphenyl]-2-methylpropanoat (Beispiel 43A, Diastereomerengemisch) wurden in einer Mischung aus 10 ml Ethanol und 10 ml THF gelöst. Die Lösung wurde mit Argon inertisiert und mit 132 mg Palladium auf Kohle (10%-ig) versetzt. Die Reaktionsmischung wurde über Nacht bei Normaldruck unter einer Wasserstoffatmosphäre kräftig gerührt. Nach Filtration über Kieselgur und Nachwaschen mit Dichlormethan/Methanol wurde das Filtrat im Vakuum eingeengt. Der Rückstand wurde durch Chromatographie an Silicagel (Laufmittel Cyclohexan/Ethylacetat 5:1 → 2:1) gereinigt und aufgetrennt. Es wurden 713 mg der Titelverbindung erhalten (75.9% d. Th.).

LC-MS (Methode 3): Rₜ = 0.93 min; m/z = 379 (M+H)⁺.

¹H-NMR (400 MHz, DMSO-*d*₆): δ [ppm] = 1.02-1.14 (m, 9H), 2.63-2.72 (m, 2H), 2.78-2.88 (m, 1H), 2.91-3.07 (m, 1H), 3.81 (br. s, 1H), 3.94-4.05 (m, 2H), 6.91-6.99 (m, 1H), 7.18 (dd, 1H), 7.89 (dd, 1H).

[α]_{D}²⁰ = +48.1°, c = 0.525, Chloroform.

### Beispiel 56A

### Ethyl-(2R)-3-{4-fluor-3-[(4,4,4-trifluorvalyl)amino]phenyl}-2-methylpropanoat (Diastereomerengemisch 1)

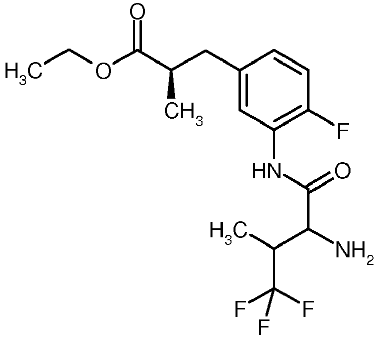

2.36 g (4.61 mmol) Ethyl-(2*R*)-3-[3-({*N*-[(benzyloxy)carbonyl]-4,4,4-trifluorvalyl}amino)-4-fluor-phenyl]-2-methylpropanoat (Beispiel 37A, Gemisch aus 4 Isomeren) wurden in einer Mischung aus 9.8 ml Ethanol und 9.8 ml THF gelöst. Die Lösung wurde mit Argon inertisiert und mit 293 mg Palladium auf Kohle (10%-ig) versetzt. Die Reaktionsmischung wurde über Nacht bei Normaldruck unter einer Wasserstoffatmosphäre kräftig gerührt. Nach Filtration über Kieselgur und Nachwaschen mit Dichlormethan/Methanol wurde das Filtrat im Vakuum eingeengt und der Rückstand durch Chromatographie an Silicagel (Laufmittel Cyclohexan/Ethylacetat 10:1 → 4:1) gereinigt und aufgetrennt. Es wurden 1.35 g der Zielverbindung als Diastereomerengemisch 1 erhalten (77.2% d. Th.).

LC-MS (Methode 5): Rₜ = 1.54 min; m/z = 379 (M+H)⁺.

¹H-NMR (400 MHz, DMSO-*d*₆): δ [ppm] = 1.05 (d, 3H), 1.07 (d, 3H), 1.11 (t, 3H), 2.61-2.72 (m, 2H), 2.77-2.88 (m, 1H), 2.91-3.06 (m, 1H), 3.81 (br. s, 1H), 4.01 (q, 2H), 6.92-7.00 (m, 1H), 7.18 (dd, 1H), 7.88 (d, 1H).

### Beispiel 57A

### (+)-Ethyl-(2R)-3-(4-fluor-3-{[(3R)-4,4,4-trifluor-D-valyl]amino}phenyl)-2-methylpropanoat

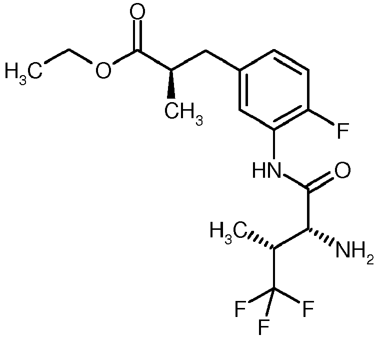

Das oben erhaltene Diastereomerengemisch 1 von Ethyl-(2*R*)-3-{4-fluor-3-[(4,4,4-trifluorvalyl)-amino]phenyl}-2-methylpropanoat (Beispiel 56A) wurde durch präparative HPLC an chiraler Phase weiter aufgetrennt [Säule: Daicel Chiralpak AD-H, 5 µm, 250 mm x 20 mm; Injektionsvolumen: 0.4 ml; Temperatur: 45°C; Eluent: 80% Isohexan / 20% Ethanol (+ 0.2% Diethylamin); Fluss: 15 ml/min; Detektion: 220 nm]. Ausgehend von 1.35 g Diastereomerengemisch wurden 0.635 g der Titelverbindung (als Diastereomer 2) isoliert.

LC-MS (Methode 2): Rₜ = 0.85 min; m/z = 379 (M+H)⁺.

¹H-NMR (400 MHz, DMSO-*d*₆): δ [ppm] = 1.05 (d, 3H), 1.07 (d, 3H), 1.11 (t, 3H), 2.64-2.70 (m, 2H), 2.77-2.88 (m, 1H), 2.91-3.04 (m, 1H), 3.81 (br. s, 1H), 4.01 (q, 2H), 6.89-6.99 (m, 1H), 7.18 (dd, 1H), 7.88 (dd, 1H).

[α]_{D}²⁰ = +2.0°, c = 0.640, Chloroform.

### Beispiel 58A

### Ethyl-(2R)-3-{4-chlor-3-[(4,4,4-trifluorvalyl)amino]phenyl}-2-methylpropanoat (Diastereomerengemisch 1)

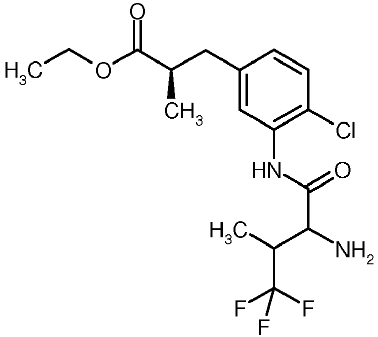

1.40 g (2.92 mmol) Ethyl-(2*R*)-3-[3-({*N*-[(allyloxy)carbonyl]-4,4,4-trifluorvalyl}amino)-4-chlor-phenyl]-2-methylpropanoat (Beispiel 38A, Gemisch aus 4 Isomeren) und 3.28 g (23.4 mmol) Dimedon wurden in 10 ml abs. THF vorgelegt. Die Lösung wurde bei RT durch 30-minütiges Durchleiten von Argon deoxygeniert. Dann wurden 67.6 mg (0.058 mmol) Tetrakis(triphenyl-phosphin)palladium(0) hinzugefügt, und die Mischung wurde über Nacht bei RT gerührt. Nach Verdünnen mit Ethylacetat wurde das Gemisch zweimal mit ges. Natriumhydrogencarbonat-Lösung und einmal mit ges. Natriumchlorid-Lösung gewaschen. Die organische Phase wurde über Magnesiumsulfat getrocknet und im Vakuum eingeengt. Der Rückstand wurde durch Chromatographie an Silicagel (Laufmittel Cyclohexan/Ethylacetat 40:1 → 20:1) gereinigt und aufgetrennt. Es wurden 844 mg der Zielverbindung als Diastereomerengemisch 1 erhalten (73.1 % d. Th.).

LC-MS (Methode 2): Rₜ = 0.99 min; m/z = 395 (M+H)⁺.

¹H-NMR (400 MHz, DMSO-*d*₆): δ [ppm] = 1.00-1.15 (m, 9H), 2.62-2.75 (m, 2H), 2.79-2.90 (m, 1H), 2.99-3.13 (m, 1H), 3.83 (br. s, 1H), 4.01 (q, 2H), 6.98 (dd, 1H), 7.42 (d, 1H), 8.07 (t, 1H).

### Beispiel 59A

### Ethyl-(2R)-3-(4-chlor-3-{[(3R)-4,4,4-trifluor-D-valyl]amino}phenyl)-2-methylpropanoat

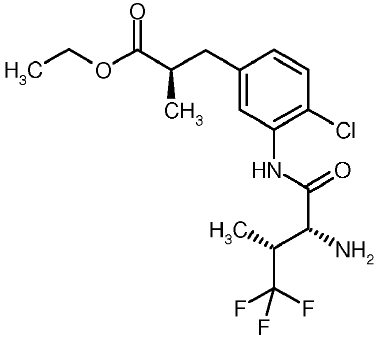

Das oben erhaltene Diastereomerengemisch 1 von Ethyl-(2R)-3-{4-chlor-3-[(4,4,4-trifluorvalyl)-amino]phenyl}-2-methylpropanoat (Beispiel 58A) wurde durch präparative HPLC an chiraler Phase weiter aufgetrennt [Säule: Daicel Chiralpak AD-H, 5 µm, 250 mm x 20 mm; Injektionsvolumen: 0.40 ml; Temperatur: 45°C; Eluent: 80% Isohexan / 20% Ethanol (+ 2% Diethylamin); Fluss: 15 ml/min; Detektion: 220 nm]. Ausgehend von 840 mg Diastereomerengemisch wurden 382 mg der Titelverbindung (als Diastereomer 2) isoliert.

LC-MS (Methode 3): Rₜ = 1.05 min; m/z = 395 (M+H)⁺.

¹H-NMR (400 MHz, DMSO-*d*₆): δ [ppm] = 1.05 (d, 3H), 1.07 (d, 3H), 1.11 (t, 3H), 2.62-2.75 (m, 2H), 2.80-2.90 (m, 1H), 3.01-3.13 (m, 1H), 3.83 (br. s, 1H), 4.01 (q, 2H), 6.98 (dd, 1H), 7.42 (d, 1H), 8.07 (d, 1H).

[α]_{D}²⁰ = -5.9°, c = 0.500, Chloroform.

### Beispiel 60A

### Ethyl-(2S)-3-{4-chlor-3-[(4,4,4-trifluorvalyl)amino]phenyl}-2-methylpropanoat (Diastereomerengemisch 1)

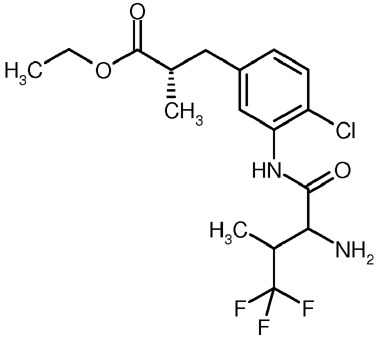

2.24 g (4.68 mmol) Ethyl-(2*S*)-3-[3-({*N*-[(allyloxy)carbonyl]-4,4,4-trifluorvalyl}amino)-4-chlorphenyl]-2-methylpropanoat (Beispiel 39A, Gemisch aus 4 Isomeren) und 5.25 g (37.4 mmol) Dimedon wurden in 25 ml abs. THF vorgelegt. Die Lösung wurde bei RT durch 30-minütiges Durchleiten von Argon deoxygeniert. Dann wurden 108.1 mg (0.094 mmol) Tetrakis(triphenylphosphin)palladium(0) hinzugefügt, und die Mischung wurde über Nacht bei RT gerührt. Nach Verdünnen mit Ethylacetat wurde das Gemisch zweimal mit ges. Natriumhydrogencarbonat-Lösung und einmal mit ges. Natriumchlorid-Lösung gewaschen. Die organische Phase wurde über Magnesiumsulfat getrocknet und im Vakuum eingeengt. Der Rückstand wurde durch Chromatographie an Silicagel (Laufmittel Cyclohexan/Ethylacetat 40:1 → 20:1) gereinigt und aufgetrennt. Es wurden 1.65 g der Zielverbindung als Diastereomerengemisch 1 erhalten (89.4% d. Th.).

LC-MS (Methode 2): Rₜ = 0.99 min; m/z = 395 (M+H)⁺.

¹H-NMR (400 MHz, DMSO-*d*₆): δ [ppm] = 1.05 (d, 3H), 1.07 (d, 3H), 1.11 (t, 3H), 2.64-2.75 (m, 2H), 2.80-2.90 (m, 1H), 3.02-3.12 (m, 1H), 3.83 (br. s, 1H), 4.01 (q, 2H), 6.98 (dd, 1H), 7.42 (d, 1H), 8.07 (d, 1H).

### Beispiel 61A

### (+)-Ethyl-(2S)-3-(4-chlor-3-1[(3R)-4,4,4-trifluor-D-valyl]amino}phenyl)-2-methylpropanoat

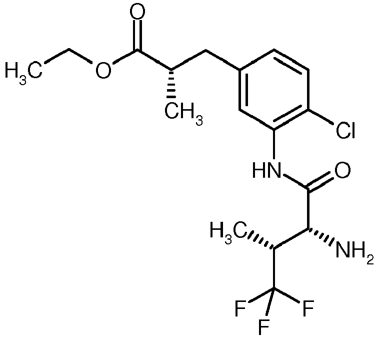

### Verfahren A:

Das oben erhaltene Diastereomerengemisch 1 von Ethyl-(2*S*)-3-{4-chlor-3-[(4,4,4-trifluorvalyl)-amino]phenyl}-2-methylpropanoat (Beispiel 60A) wurde durch präparative HPLC an chiraler Phase weiter aufgetrennt [Säule: Daicel Chiralpak AD-H, 5 µm, 250 mm x 20 mm; Injektionsvolumen: 0.25 ml; Temperatur: 45°C; Eluent: 80% Isohexan / 20% Ethanol (+ 0.2% Diethylamin); Fluss: 15 ml/min; Detektion: 220 nm]. Ausgehend von 1.65 g Diastereomerengemisch wurden 0.78 g der Zielverbindung isoliert.

LC-MS (Methode 2): Rₜ = 0.99 min; m/z = 395 (M+H)⁺.

¹H-NMR (400 MHz, DMSO-*d*₆): δ [ppm] = 1.04 (d, 3H), 1.08 (d, 3H), 1.10 (t, 3H), 2.64-2.74 (m, 2H), 2.80-2.88 (m, 1H), 3.01-3.12 (m, 1H), 3.83 (br. s, 1H), 3.97-4.05 (m, 2H), 6.98 (dd, 1H), 7.42 (d, 1H), 8.07 (d, 1H).

[α]_{D}²⁰ = +40.4°, c = 0.565, Chloroform.

### Verfahren B:

0.99 g (1.87 mmol) Ethyl-(2*S*)-3-[3-({(3*R*)-*N*-[(benzyloxy)carbonyl]-4,4,4-trifluorvalyl}amino)-4-chlorphenyl]-2-methylpropanoat (Beispiel 44A, Diastereomerengemisch) wurden in 50 ml Ethylacetat gelöst. Die Lösung wurde mit Argon inertisiert und mit 40 mg Palladium auf Kohle (5%-ig) versetzt. Die Reaktionsmischung wurde 4 h bei Normaldruck unter einer Wasserstoffatmosphäre kräftig gerührt. Nach Filtration über Kieselgur wurde das Filtrat im Vakuum eingeengt und der Rückstand durch Chromatographie an Silicagel (Laufmittel Cyclohexan/Ethylacetat 20:1 → 5:1) gereinigt und aufgetrennt. Es wurden 444 mg der Titelverbindung erhalten (60.1 % d. Th.).

LC-MS (Methode 2): Rₜ = 0.95 min; m/z = 395 (M+H)⁺.

¹H-NMR (400 MHz, DMSO-*d*₆): δ [ppm] = 1.04 (d, 3H), 1.08 (d, 3H), 1.10 (t, 3H), 2.62-2.74 (m, 2H), 2.79-2.89 (m, 1H), 3.03-3.12 (m, 1H), 3.83 (br. s, 1H), 3.97-4.08 (m, 2H), 6.98 (dd, 1H), 7.42 (d, 1H), 8.08 (d, 1H).

[α]_{D}²⁰ = +34.1°, c = 0.525, Chloroform.

### Beispiel 62A

### tert.-Butyl-(3R)-3-{4-chlor-3-[(4,4,4-trifluorvalyl)amino]phenyl}butanoat (Diastereomerengemisch 1)

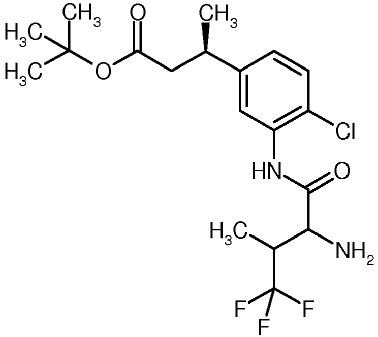

630 mg (1.24 mmol) *tert*.-Butyl-(3*R*)-3-[3-({*N*-[(allyloxy)carbonyl]-4,4,4-trifluorvalyl}amino)-4-chlorphenyl]butanoat (Beispiel 40A, Gemisch aus 4 Isomeren) und 1.394 g (9.94 mmol) Dimedon wurden in 10 ml abs. THF vorgelegt. Die Lösung wurde bei RT durch 30-minütiges Durchleiten von Argon deoxygeniert. Dann wurden 28.7 mg (0.025 mmol) Tetrakis(triphenylphosphin)palladium(0) hinzugefügt, und die Mischung wurde über Nacht bei RT gerührt. Nach Verdünnen mit Ethylacetat wurde das Gemisch zweimal mit ges. Natriumhydrogencarbonat-Lösung und einmal mit ges. Natriumchlorid-Lösung gewaschen. Die organische Phase wurde über Magnesiumsulfat getrocknet und im Vakuum eingeengt. Der Rückstand wurde durch Chromatographie an Silicagel (Laufmittel Cyclohexan/Ethylacetat 40:1 → 20:1) gereinigt und aufgetrennt. Es wurden 420 mg der Zielverbindung als Diastereomerengemisch 1 erhalten (79.9% d. Th.).

LC-MS (Methode 2): Rₜ = 1.14 min; m/z = 423 (M+H)⁺.

¹H-NMR (400 MHz, DMSO-*d*₆): beide Diastereomere δ [ppm] = 1.04/1.05 (je d, zus. 3H), 1.19/ 1.20 (je d, zus. 3H), 1.29 (s, 9H), 2.35-2.48 (m, 2H), 2.97-3.16 (m, 2H), 3.83 (br. s, 1H), 7.06 (dt, 1H), 7.43 (dd, 1H), 8.16 (d, 1H).

### Beispiel 63A

### tert.-Butyl-(3S)-3-{4-chlor-3-[(4,4,4-trifluorvalyl)amino]phenyl}butanoat (Diastereomerengemisch 1)

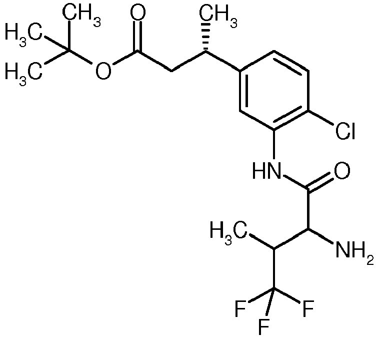

1.39 g (2.74 mmol) *tert*.-Butyl-(3*S*)-3-[3-({*N*-[(allyloxy)carbonyl]-4,4,4-trifluorvalyl}amino)-4-chlorphenyl]butanoat (Beispiel 41A, Gemisch aus 4 Isomeren) und 3.08 g (21.9 mmol) Dimedon wurden in 9.4 ml abs. THF vorgelegt. Die Lösung wurde bei RT durch 30-minütiges Durchleiten von Argon deoxygeniert. Dann wurden 63.4 mg (0.055 mmol) Tetrakis(triphenylphosphin)palladium(0) hinzugefügt, und die Mischung wurde über Nacht bei RT gerührt. Nach Verdünnen mit Ethylacetat wurde das Gemisch zweimal mit ges. Natriumhydrogencarbonat-Lösung und einmal mit ges. Natriumchlorid-Lösung gewaschen. Die organische Phase wurde über Magnesiumsulfat getrocknet und im Vakuum eingeengt. Der Rückstand wurde durch Chromatographie an Silicagel (Laufmittel Cyclohexan/Ethylacetat 40:1 → 20:1) gereinigt und aufgetrennt. Es wurden 950 mg der Zielverbindung als Diastereomerengemisch 1 erhalten (81.9% d. Th.).

LC-MS (Methode 2): Rₜ = 1.15 min; m/z = 423 (M+H)⁺.

¹H-NMR (400 MHz, DMSO-*d*₆): beide Diastereomere δ [ppm] = 1.04/1.05 (je d, zus. 3H), 1.19/ 1.20 (je d, zus. 3H), 1.29 (s, 9H), 2.29-2.48 (m, 2H), 2.99-3.16 (m, 2H), 3.83 (br. s, 1H), 7.06 (dt, 1H), 7.43 (dd, 1H), 8.16 (d, 1H).

### Beispiel 64A

### tert.-Butyl-(3S)-3-(4-chlor-3-{[(3R)-4,4,4-trifluor-D-valyl]amino}phenyl)butanoat

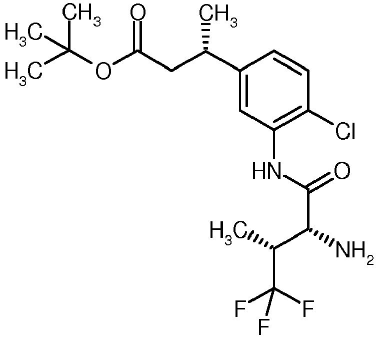

1.30 g (2.56 mmol) *tert*.-Butyl-(3*S*)-3-[3-({(3*R*)-*N*-[(allyloxy)carbonyl]-4,4,4-trifluorvalyl}amino)-4-chlorphenyl]butanoat (Beispiel 42A, Diastereomerengemisch) und 2.88 g (20.5 mmol) Dimedon wurden in 8.7 ml abs. THF vorgelegt. Die Lösung wurde bei RT durch 30-minütiges Durchleiten von Argon deoxygeniert. Dann wurden 59.3 mg (0.051 mmol) Tetrakis(triphenylphosphin)palladium(0) hinzugefügt, und die Mischung wurde über Nacht bei RT gerührt. Nach Verdünnen mit Ethylacetat wurde das Gemisch zweimal mit ges. Natriumhydrogencarbonat-Lösung und einmal mit ges. Natriumchlorid-Lösung gewaschen. Die organische Phase wurde über Magnesiumsulfat getrocknet und im Vakuum eingeengt. Der Rückstand wurde durch Chromatographie an Silicagel (Laufmittel Cyclohexan/Ethylacetat 10:1) gereinigt und aufgetrennt. Es wurden 591 mg der Zielverbindung erhalten (54.5% d. Th.).

LC-MS (Methode 2): Rₜ = 1.14 min; m/z = 423 (M+H)⁺.

¹H-NMR (400 MHz, DMSO-*d*₆): δ [ppm] = 1.05 (d, 3H), 1.19 (d, 3H), 1.29 (s, 9H), 2.38-2.48 (m, 2H), 3.02-3.16 (m, 2H), 3.83 (d, 1H), 7.06 (dd, 1H), 7.43 (d, 1H), 8.16 (d, 1H).

### Beispiel 65A

### (+)-tert.-Butyl-1-(4-chlor-3-{[(3R)-4,4,4-trifluor-D-valyl]amino}benzyl)cyclopropancarboxylat

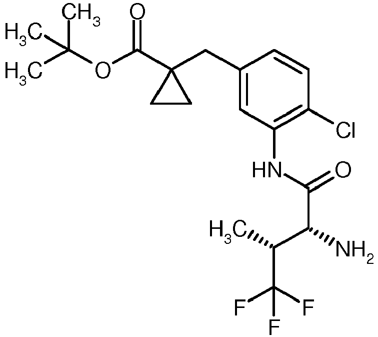

450 mg (0.867 mmol) *tert*.-Butyl-1-[3-({(3*R*)-*N*-[(allyloxy)carbonyl]-4,4,4-trifluorvalyl}amino)-4-chlorbenzyl]cyclopropancarboxylat (Beispiel 45A, Diastereomerengemisch) und 972.4 mg (6.94 mmol) Dimedon wurden in 3 ml abs. THF vorgelegt. Die Lösung wurde bei RT durch 30-minütiges Durchleiten von Argon deoxygeniert. Dann wurden 20.0 mg (0.017 mmol) Tetrakis-(triphenylphosphin)palladium(0) hinzugefügt, und die Mischung wurde über Nacht bei RT gerührt. Nach Verdünnen mit Ethylacetat wurde das Gemisch zweimal mit ges. Natriumhydrogencarbonat-Lösung und einmal mit ges. Natriumchlorid-Lösung gewaschen. Die organische Phase wurde über Magnesiumsulfat getrocknet und im Vakuum eingeengt. Der Rückstand wurde durch Chromatographie an Silicagel (Laufmittel Cyclohexan/Ethylacetat 20:1) gereinigt und aufgetrennt. Es wurden 221.4 mg der Zielverbindung erhalten (58.7% d. Th.).

LC-MS (Methode 2): Rₜ = 1.15 min; m/z = 435 (M+H)⁺.

¹H-NMR (400 MHz, DMSO-*d*₆): δ [ppm] = 0.82-0.87 (m, 2H), 1.04 (d, 3H), 1.06-1.10 (m, 2H), 1.29 (s, 9H), 2.77-2.90 (m, 2H), 3.02-3.12 (m, 1H), 3.83 (br. s, 1H), 7.04 (dd, 1H), 7.42 (d, 1H), 8.12-8.23 (m, 1H).

[α]_{D}²⁰ = +16.1°, c = 0.425, Chloroform.

### Beispiel 66A

### 3-(Trifluormethyl)pentan-1,5-diol

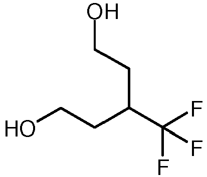

12.0 g (59.97 mmol) 3-(Trifluormethyl)pentandisäure wurden in 70 ml abs. THF gelöst und nach Abkühlen auf -10°C tropfenweise mit 120 ml (120 mmol) einer 1 M Lösung von Lithiumaluminiumhydrid in THF versetzt. Nach Ende der Zugabe wurde die Reaktionsmischung über Nacht auf RT erwärmt. Nach Abkühlen auf 0°C wurde vorsichtig und tropfenweise zunächst mit ca. 10 ml Isopropanol und dann mit 20 ml Isopropanol/Wasser-Gemisch (3:1) versetzt. Die resultierende Suspension wurde durch Zugabe von 1 N Salzsäure sauer gestellt und mit Celite vermischt. Es wurde filtriert, und das erhaltene Filtrat wurde im Vakuum eingeengt. Um Reste an Wasser zu entfernen, wurde das erhaltene Öl mit Acetonitril versetzt und erneut im Vakuum eingeengt. Das Rohprodukt wurde durch Chromatographie an Silicagel gereinigt (Laufmittel Cyclohexan/Ethylacetat 4:1 → 1:1). Es wurden 4.5 g des Zielprodukts erhalten (43.6% d. Th.).

GC-MS (Methode 1): Rₜ = 2.76 min; m/z = 153 (M-H₃O)⁺.

¹H-NMR (400 MHz, DMSO-*d*₆): δ [ppm] = 1.49-1.57 (m, 2H), 1.67-1.75 (m, 2H), 2.30-2.48 (m, 1H), 3.47 (q, 4H), 4.61 (t, 2H).

### Beispiel 67A

### 3-Phenylpentan-1,5-diol

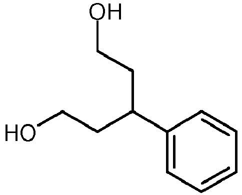

3.0 g (14.4 mmol) 3-Phenylglutarsäure wurden in 20 ml abs. THF gelöst, auf -10°C gekühlt und tropfenweise mit 31.7 ml (31.7 mmol) einer 1 M Lösung von Lithiumaluminiumhydrid in THF versetzt. Während der Zugabe wurden weitere 50 ml abs. THF hinzugefügt. Die resultierende Suspension wurde langsam auf RT erwärmt und über Nacht bei RT gerührt. Nach Abkühlen auf 0°C wurde vorsichtig und tropfenweise zunächst mit Isopropanol und dann mit einem Gemisch aus Isopropanol und Wasser (3:1) versetzt. Die resultierende Suspension wurde durch Zugabe von 1 N Salzsäure sauer gestellt und mit Celite vermischt. Es wurde über Celite filtriert, mit Isopropanol nachgewaschen und das erhaltene Filtrat im Vakuum eingeengt. Das Rohprodukt wurde durch Chromatographie an Silicagel gereinigt (Laufmittel Cyclohexan/Ethylacetat 2:1 → 1:1). Es wurden 1.9 g des Zielprodukts erhalten (73% d. Th.).

GC-MS (Methode 1): Rₜ = 5.53 min; m/z = 163 (M-OH)⁺.

¹H-NMR (400 MHz, DMSO-*d₆*): δ [ppm] = 1.56-1.71 (m, 2H), 1.72-1.83 (m, 2H), 2.68-2.86 (m, 1H), 3.12-3.29 (m, 4H), 4.32 (t, 2H), 7.10-7.21 (m, 3H), 7.23-7.32 (m, 2H).

Weitere nicht kommerziell erhältliche, 3-substituierte Pentan-1,5-diole lassen sich z.B. analog zur folgenden dreistufigen Sequenz 68A - 69A - 70A herstellen:

### Beispiel 68A

### Ethyl-3-cyclopropylacrylat

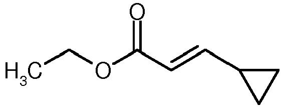

Zu einer Suspension von 2.59 g (64.85 mmol) Natriumhydrid (60%-ig in Mineralöl) in 110 ml abs. THF wurden bei 0°C 13.0 ml (64.85 mmol) Diethylphosphonoessigsäureethylester getropft. Nach 30 min wurde die Mischung auf RT erwärmt und nach erneutem Abkühlen auf 0°C tropfenweise mit 5.3 ml (71.34 mmol) Cyclopropancarboxaldehyd versetzt. Nach Ende der Zugabe wurde die Reaktionsmischung auf RT erwärmt und mit 35 ml DMF verdünnt. Die Mischung wurde über Nacht bei RT gerührt und dann auf Wasser gegeben. Es wurde dreimal mit Ethylacetat extrahiert. Die vereinigten organischen Phasen wurden im Vakuum eingeengt und das Rohprodukt durch Filtration an Silicagel gereinigt (Laufmittel Cyclohexan/Ethylacetat 10:1 → 6:1). Es wurden 8.53 g des Zielprodukts erhalten (93.8% d. Th.).

GC-MS (Methode 1): Rₜ = 2.83 min; m/z = 112 (M-C₂H₄)⁺.

¹H-NMR (400 MHz, DMSO-*d₆*): δ [ppm] = 0.59-0.70 (m, 2H), 0.88-0.95 (m, 2H), 1.19 (t, 3H), 1.54-1.72 (m, 1H), 4.08 (q, 2H), 5.93 (d, 1H), 6.39 (dd, 1H).

### Beispiel 69A

### Diethyl-3-cyclopropylpentandioat

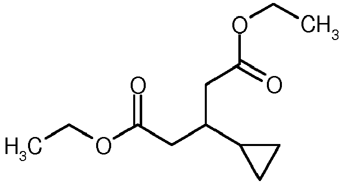

Zu einer auf -40°C gekühlten Lösung von 10.2 ml (72.8 mmol) Diisopropylamin in 35 ml abs. THF wurden 29.1 ml (72.8 mmol) einer 2.5 M Lösung von n-Butyllithium in Hexan getropft. Nach Ende der Zugabe wurde 30 min bei -40°C nachgerührt, dann auf -78°C abgekühlt und tropfenweise mit einer Lösung von 7.1 ml (72.8 mmol) Ethylacetat in 10 ml abs. THF versetzt. Die Reaktionsmischung wurde 30 min bei -78°C nachgerührt, bevor eine Lösung von 8.50 g (60.54 mmol) Ethyl-3-cyclopropylacrylat in wenig THF zugetropft wurde. Nach 1 h bei -78°C wurde die Mischung langsam auf -40°C, dann auf 0°C erwärmt und schließlich mit gesättigter wässriger Ammoniumchlorid-Lösung versetzt. Nach Extraktion mit Ethylacetat wurde die organische Phase mit 1 N Salzsäure und ges. Natriumchlorid-Lösung gewaschen, über Natriumsulfat getrocknet und im Vakuum eingeengt. Das Rohprodukt wurde durch Chromatographie an Silicagel gereinigt (Laufmittel Cyclohexan/Ethylacetat 30:1 → 6:1). Es wurden 3.28 g des Zielprodukts erhalten (23.7% d. Th.).

GC-MS (Methode 1): Rₜ = 4.56 min; m/z = 183 (M-C₂H₅O)⁺.

¹H-NMR (400 MHz, DMSO-d₆): δ [ppm] = 0.07-0.13 (m, 2H), 0.32-0.41 (m, 2H), 0.62-0.75 (m, 1H), 1.15-1.22 (m, 6H), 1.41-1.52 (m, 1H), 2.33-2.46 (m, 4H), 4.00-4.10 (m, 4H).

### Beispiel 70A

### 3-Cyclopropylpentan-1,5-diol

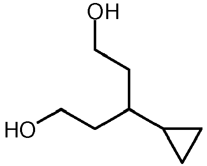

3.2 g (14.02 mmol) Diethyl-3-cyclopropylpentandioat wurden in 24.7 ml abs. THF gelöst und nach Abkühlen auf -10°C tropfenweise mit 25.2 ml (25.2 mmol) einer 1 M Lösung von Lithiumaluminiumhydrid in THF versetzt. Die Reaktionsmischung wurde über Nacht bei RT gerührt. Nach Abkühlen auf 0°C wurden vorsichtig 100 ml Isopropanol zugetropft. Die Mischung wurde durch Zugabe von 1 N Salzsäure sauer gestellt und mit Celite vermengt. Nach Filtration über Celite und Nachwaschen mit Ethylacetat und Wasser wurde das Filtrat mit Natriumchlorid gesättigt und dreimal mit Ethylacetat extrahiert. Die vereinigten organischen Phasen wurden über Magnesiumsulfat getrocknet und im Vakuum eingeengt. Das Rohprodukt wurde durch Chromatographie an Silicagel gereinigt (Laufmittel Cyclohexan/Ethylacetat 10:1 → 2:1). Es wurden 1.35 g des Zielprodukts erhalten (66.8% d. Th.).

GC-MS (Methode 1): Rₜ = 3.98 min; m/z = 115 (M-C₂H₅)⁺.

¹H-NMR (400 MHz, DMSO-*d₆*): δ [ppm] = 0.01-0.07 (m, 2H), 0.32-0.40 (m, 2H), 0.41-0.52 (m, 1H), 0.69-0.82 (m, 1H), 1.49 (q, 4H), 3.41-3.54 (m, 4H), 4.24 (t, 2H).

### Beispiel 71A

### 3-Ethylpentan-1,5-diol

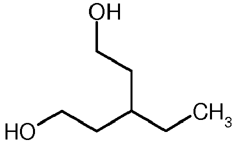

Die Titelverbindung kann in Analogie zum oben beschriebenen, dreistufigen Prozess 68A - 69A - 70A erhalten werden.

GC-MS (Methode 1): Rₜ = 3.41 min; m/z = 114 (M-H₂O)⁺.

¹H-NMR (400 MHz, DMSO-*d₆*): δ [ppm] = 0.81 (t, 3H), 1.16-1.31 (m, 2H), 1.31-1.52 (m, 5H), 3.34-3.48 (m, 4H), 4.29 (t, 2H).

### Allgemeine Vorschrift 2: Oxidation von 3-substituierten Pentan-1,5-diolen zu Dialdehyden (bzw. ihren cyclischen Acetalen)

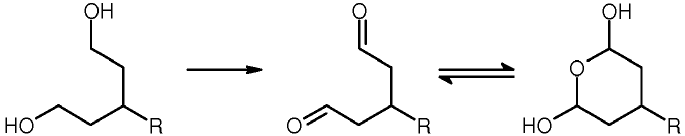

2.6 eq. Oxalylchlorid werden in abs. Dichlormethan (1.0 bis 2.5 mol/l) gelöst, auf ca. -50°C gekühlt und tropfenweise mit einer Lösung von 5 eq. DMSO in abs. Dichlormethan (ca. 5 mol/l) versetzt. Nach 10 min bei ca. -50°C wird eine Lösung des betreffenden Diols (1.0 eq.) in abs. Dichlormethan (0.5 bis 1.0 mol/l) zugetropft. Nach weiteren 10 min wird die Reaktionsmischung auf -78°C abgekühlt und eine Mischung aus Triethylamin (ca. 10.5 eq.) und abs. Dichlormethan (Volumenverhältnis 1:1 bis 1:2) zugetropft. Nach Ende der Zugabe wird 30 min bei ca. -78°C nachgerührt und dann langsam erwärmt (bis auf -20°C, 0°C oder RT), bevor die Reaktionsmischung mit Dichlormethan verdünnt, mit ges. Natriumhydrogencarbonat-Lösung gewaschen, über Natriumsulfat getrocknet und im Vakuum eingeengt wird. Das anfallende Rohprodukt wird nicht weiter gereinigt, sondern kann direkt in Folgereaktionen eingesetzt werden.

Die folgenden Beispiele wurden gemäß der Allgemeinen Vorschrift 2 hergestellt und ohne weitere Reinigung eingesetzt (die Dialdehyde konnten in der Regel als Rohprodukt bei -20°C unter Argon gelagert werden):

| **Beispiel** | **Name / Struktur / Edukt** | **Analytische Daten** |
|---|---|---|
| **72A** | 3-Methylpentandial | GC-MS (Methode 1): Rₜ = 2.22 min; m/z = 95 (M-H₃O)⁺, 96 (M-H₂O)⁺. |
| | 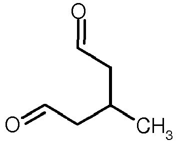 | |
| | aus 3-Methylpentan-1,5-diol | |
| **73A** | 3-Ethylpentandial | GC-MS (Methode 1): Rₜ = 2.68 min; m/z = 110 (M-H₂O)⁺. |
| | 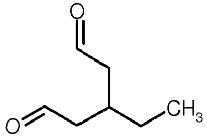 | |
| | aus 3-Ethylpentan-1,5-diol | |
| **74A** | 3 -(Trifluormethyl)pentandial | GC-MS (Methode 1): Rₜ = 1.82 min; m/z = 148 (M-HF)⁺. |
| | 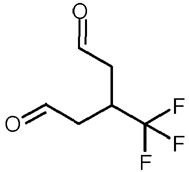 | |
| | aus 3-(Trifluormethyl)pentan-1,5-diol | |
| **75A** | 3-Cyclopropylpentandial | GC-MS (Methode 1): Rₜ = 3.26 min; m/z = 122 (M-H₂O)⁺. |
| | 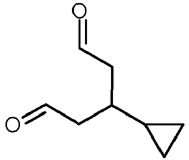 | |
| | aus 3-Cyclopropylpentan-1,5-diol | |

| **Beispiel** | **Name / Struktur / Edukt** | **Analytische Daten** |
|---|---|---|
| **76A** | 3-Phenylpentandial | GC-MS (Methode 1): Rₜ = 5.04 min; m/z = 176 (M)⁺, 158 (M-H₂O)⁺. |
| | 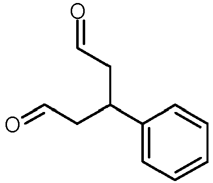 | |
| | aus 3-Phenylpentan-1,5-diol | |

### Beispiel 77A

### Methyl-cyclopropylidenacetat

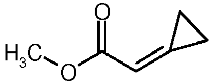

Eine Suspension von 240 g (1.38 mol) [(1-Ethoxycyclopropyl)oxy](trimethyl)silan, 598 g (1.79 mol) (Methoxycarbonylmethylen)triphenylphosphoran und 21.9 g (0.179 mol) Benzoesäure in 3.94 Liter Toluol wurde über Nacht bei 85°C gerührt. Nach dem Abkühlen wurde die Reaktionsmischung direkt durch Chromatographie an 4.8 kg Silicagel aufgereinigt (Laufmittel: Petrolether/ Dichlormethan-Gemische von 1:1 bis zu reinem Dichlormethan). Die Produktfraktionen wurden vereinigt und im leichten Vakuum konzentriert. Es wurden 159 g des Zielprodukts als klares, farbloses Öl erhalten (Rohprodukt, noch Lösungsmittelreste enthaltend).

GC-MS (Methode 1): Rₜ = 1.84 min; m/z = 112 (M+H)⁺.

¹H-NMR (400 MHz, DMSO-*d₆*): δ [ppm] = 1.24-1.31 (m, 2H), 1.38-1.46 (m, 2H), 3.66 (s, 3H), 6.23-6.36 (m, ca. 1H).

### Beispiel 78A

### Ethyl-methyl-2,2'-cyclopropan-1,1-diyldiacetat

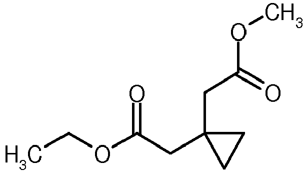

240 ml (1.70 mol) Diisopropylamin wurden in 700 ml abs. THF gelöst, auf -40°C gekühlt und langsam mit 681 ml (1.7 mol) einer 2.5 M Lösung von n-Butyllithium in Hexan versetzt. Die Lösung wurde 30 min bei -40°C nachgerührt, dann auf -78°C abgekühlt und mit einer Lösung von 166.6 ml (1.7 mol) p.a. Ethylacetat in 200 ml abs. THF versetzt. Nach Ende der Zugabe wurde weitere 30 min bei -78°C nachgerührt. Anschließend wurden 159 g (Rohprodukt, ca. 1.42 mol) Methyl-cyclopropylidenacetat, gelöst in wenig abs. THF, zugetropft. Die Reaktionsmischung wurde 1 h bei -78°C gerührt, dann langsam über -40°C auf ca. 0°C erwärmt und schließlich mit gesättigter wässriger Ammoniumchlorid-Lösung versetzt. Es wurde mit Ethylacetat extrahiert, und die organische Phase wurde mit 1 N Salzsäure und ges. Natriumchlorid-Lösung gewaschen, über Natriumsulfat getrocknet und im Vakuum eingeengt. Der Rückstand wurde über Silicagel chromatographisch gereinigt (Laufmittel Cyclohexan/Ethylacetat 9:1). Es wurden 109.5 g der Zielverbindung, verunreinigt durch Diethylesterderivat, erhalten, die nicht weiter aufgereinigt wurden.

GC-MS (Methode 1): Rₜ = 3.82 min; m/z = 168 (M-CH₄O)⁺.

### Beispiel 79A

### 2,2'-Cyclopropan-1,1-diyldiethanol

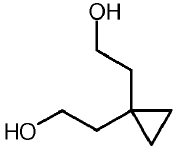

109.5 g (Rohprodukt, ca. 547 mmol) Ethyl-methyl-2,2'-cyclopropan-1,1-diyldiacetat wurden in 750 ml p.a. THF gelöst und bei -20°C tropfenweise mit 984 ml (984 mmol) einer 1 M Lösung von Lithiumaluminiumhydrid in THF versetzt. Die Reaktionsmischung wurde über Nacht auf RT erwärmt und nach erneutem Abkühlen auf -10°C vorsichtig mit 500 ml Isopropanol versetzt. Die Mischung wurde mit 1 N Salzsäure sauer gestellt und über Celite abgesaugt. Der Rückstand wurde mit Ethylacetat und Wasser nachgewaschen. Das Filtrat wurde mit Natriumchlorid gesättigt und mehrfach mit Ethylacetat extrahiert. Die vereinigten organischen Phasen wurden über Natriumsulfat getrocknet und im Vakuum eingeengt. Der Rückstand wurde aus Acetonitril umkristallisiert. Es wurden 50.4 g des Zielprodukts erhalten (70.8% d. Th.).

GC-MS (Methode 1): Rₜ = 3.48 min; m/z = 112 (M-H₂O)⁺.

¹H-NMR (400 MHz, DMSO-*d₆*): δ [ppm] = 0.21 (s, 4H), 1.37 (t, 4H), 3.46 (q, 4H), 4.28 (t, 2H).

### Beispiel 80A

### 2,2'-Cyclopropan-1,1-diyldiacetaldehyd

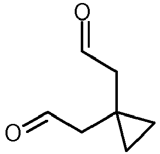

7.0 ml (79.9 mmol) Oxalylchlorid wurden in 45 ml abs. Dichlormethan gelöst, auf -50°C gekühlt und tropfenweise mit einer Lösung von 10.9 ml (163.6 mmol) DMSO in 45 ml abs. Dichlormethan versetzt. Nach Ende der Zugabe wurde noch 10 min bei -50°C gerührt und dann langsam 4.0 g (30.7 mmol) 2,2'-Cyclopropan-1,1-diyldiethanol, gelöst in 45 ml abs. Dichlormethan, hinzugegeben. Die resultierende Suspension wurde 10 min bei -50°C gerührt, danach auf -78°C abgekühlt und tropfenweise mit einer Lösung von 44.5 ml (320 mmol) Triethylamin in 75 ml abs. Dichlormethan versetzt. Nach Ende der Zugabe wurde die Reaktionsmischung 30 min bei -78°C gerührt, anschließend langsam auf RT erwärmt und dann mit gesättigter wässriger Natriumhydrogensulfat-Lösung gewaschen. Die organische Phase wurde über Natriumsulfat getrocknet und im Vakuum eingeengt. Es wurden 5.10 g des Zielprodukts als oranges Öl erhalten (ca. 70%-iges Rohprodukt, ca. 92% d. Th.).

GC-MS (Methode 1): Rₜ = 2.72 min; m/z = 108 (M-H₂O)⁺, 107 (M-H₃O)⁺.

### Beispiel 81A

### tert.-Butyl-3-(4-chlor-3-{[4,4,4-trifluor-3-methyl-2-(4-methylpiperidin-1-yl)butanoyl]amino}-phenyl)propanoat (racemisches Diastereomerengemisch)

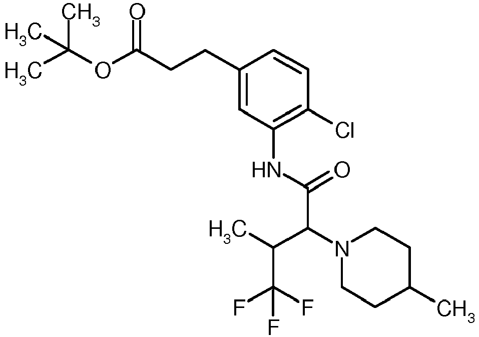

### Stufe 1:

300 mg (1.45 mmol) (+/-)-D,L-4,4,4-Trifluorvalin-Hydrochlorid in 6 ml Ethanol wurden mit 705 mg (2.89 mmol) 1,5-Dibrom-3-methylpentan und 7.2 ml 1 N Natronlauge versetzt und über Nacht unter Rückfluss gerührt. Nach Zugabe von weiteren 1.0 eq. 1,5-Dibrom-3-methylpentan und 5.0 eq. Natriumhydroxid wurde die Mischung erneut 8 h unter Rückfluss gerührt und dann nach Abkühlen im Vakuum eingeengt. Der Rückstand wurde in wenig Wasser aufgenommen und mit 1 N Salzsäure neutral gestellt. Die wässrige Phase wurde mehrfach mit Ethylacetat extrahiert. Die vereinigten organischen Phasen wurden über Natriumsulfat getrocknet und im Vakuum eingeengt. Es wurden als Rohprodukt 270 mg 4,4,4-Trifluor-3-methyl-2-(4-methylpiperidin-l-yl)butansäure (racemisches Diastereomerengemisch) erhalten.

### Stufe 2:

270 mg 4,4,4-Trifluor-3-methyl-2-(4-methylpiperidin-1-yl)butansäure (Rohprodukt, racemisches Diastereomerengemisch) wurden in 2.5 ml Pyridin und 4.9 ml DMF gelöst und bei RT mit 286 mg (1.12 mmol) *tert*.-Butyl-3-(3-amino-4-chlorphenyl)propanoat und 527 mg (1.37 mmol) HATU versetzt. Die Mischung wurde über Nacht bei 40°C gerührt. Es wurden weitere 0.5 eq. HATU sowie 2 ml *N*,*N*-Diisopropylethylamin zugesetzt, und die Reaktionsmischung wurde erneut für 5 h bei 50°C gerührt und dann auf Wasser gegeben. Es wurde dreimal mit Ethylacetat extrahiert, und die vereinigten organischen Phasen wurden mit wenig 1 N Salzsäure gewaschen, über Magnesiumsulfat getrocknet und im Vakuum eingeengt. Das Rohprodukt wurde durch präparative RP-HPLC gereinigt (Eluent Acetonitril/Wasser). Es wurden 50 mg des Zielprodukts (9.6% d. Th.) als Diastereomerengemisch (ca. 3:2) erhalten.

LC-MS (Methode 2): Rₜ = 1.53 min und 1.57 min; jeweils m/z = 491 (M+H)⁺.

¹H-NMR (400 MHz, DMSO-*d₆*): beide Diastereomere δ [ppm] = 0.86/0.87 (je d, zus. 3H), 0.96-1.05 (m, 1H), 1.07/1.10 (je d, zus. 3H), 1.12-1.24 (m, 1H), 1.24-1.34 (m, 1H), 1.35/1.36 (je s, zus. 9H), 1.51-1.65 (m, 2H), 2.00-2.17 (m, 1H), 2.43-2.48 (m, 1H), 2.64 (d, 1H), 2.76-2.83 (m, 2H), 2.84-3.10 (m, 3H), 3.52/3.59 (je d, zus. 1H), 7.04-7.12 (m, 1H), 7.41 (dd, 1H), 7.47/7.55 (je d, zus. 1H), 9.58/9.68 (je s, zus. 1H).

### Allgemeine Vorschrift 3: Herstellung substituierter Piperidinoacetanilide aus Aminoacetaniliden gemäß nachfolgendem Reaktionsschema:

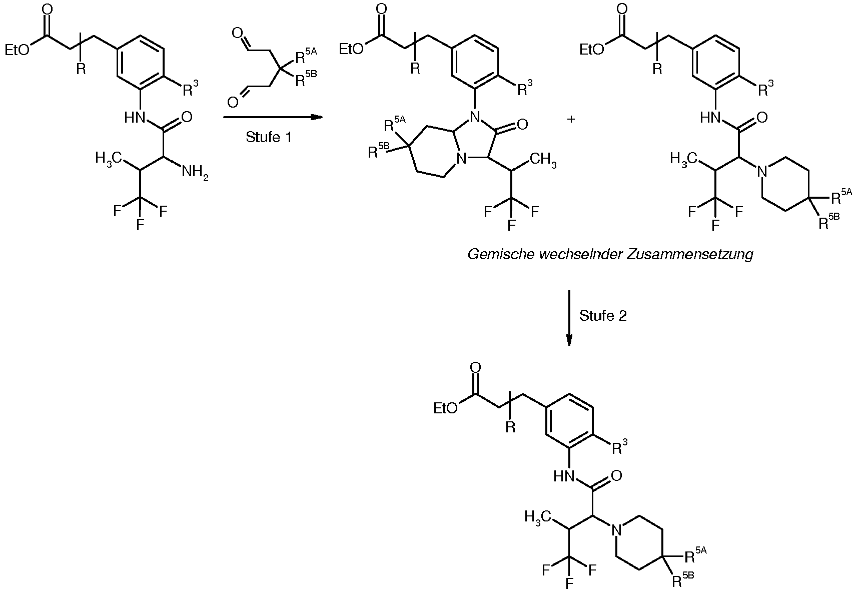

### Stufe 1:

Zu einer Lösung der betreffenden Aminoacetanilid-Komponente (1.0 eq.) in Methanol (ca. 0.05 mol/l bis 1 bis mol/l) werden bei RT Triethylamin (1.5 bis 2.0 eq.), 1 N Salzsäure (ca. 0.25 bis 0.5 eq.), der betreffende Dialdehyd (bzw. dessen cyclische Acetalform, als Rohgemisch, ca. 2.0 bis 4.0 eq.) und nach ca. 5 bis 15 min Natriumcyanoborhydrid (2.0 bis 4.0 eq.) gegeben. Die Mischung wird ca. 12 bis zu 72 Stunden bei RT gerührt, wobei gegebenenfalls zwischenzeitlich weitere Mengen an Triethylamin, 1 N Salzsäure, Dialdehyd und/oder Natriumcyanoborhydrid zugesetzt werden. Nach beendeter Umsetzung wird die Reaktionsmischung auf Wasser gegeben. Die wässrige Phase wird dreimal mit Ethylacetat extrahiert, und die vereinigten organischen Phasen werden im Vakuum eingeengt. Das erhaltene Rohproduktgemisch kann direkt in der Folgestufe eingesetzt werden oder (bevorzugt) wird zuvor durch präparative RP-HPLC aufgereinigt (Eluent Acetonitril/ Wasser-Gradient).

### Stufe 2:

Das betreffende Zwischenproduktgemisch aus Stufe 1 (roh oder aufgereinigt) wird bei 0°C oder RT in Trifluoressigsäure gelöst (Konzentration ca. 0.1 bis 2.0 mol/l) und anschließend bei 0°C bis RT tropfenweise mit einem großen Überschuss Triethylsilan (6 bis 15 eq.) versetzt. Die Mischung wird 4 bis 24 h bei RT gerührt; gegebenenfalls wird die Mischung bis auf 40°C erwärmt, bis vollständiger Umsatz erreicht ist. Danach wird das Reaktionsgemisch im Vakuum eingeengt. Der Rückstand wird in Ethylacetat aufgenommen und mit Wasser und Natriumhydrogencarbonat-Lösung annähernd neutral (pH 6-7) gewaschen. Nach Einengen im Vakuum kann das Rohprodukt durch präparative RP-HPLC (Eluent Acetonitril/Wasser- oder Methanol/Wasser-Gradient), durch Chromatographie an Silicagel (Eluent Cyclohexan/Ethylacetat- oder Dichlormethan/Methanol-Gemische) oder durch eine Kombination dieser Methoden gereinigt werden, oder es wird ohne Reinigung direkt weiter umgesetzt.

Die folgenden Beispiele wurden gemäß der Allgemeinen Vorschrift 3 hergestellt:

| **Beispiel** | **Name / Struktur / Edukte** | **Analytische Daten** |
|---|---|---|
| **82A** | Ethyl-(2S)-3-(3-{[2-(6-azaspiro[2.5]oct-6-yl)-4,4,4-trifluor-3-methylbutanoyl]amino}-4-chlorphenyl)-2-methylpropanoat (*Diastereomerengemisch 1*) | LC-MS (Methode 5): Rₜ = 3.02 min; m/z = 489 (M+H)⁺. ¹H-NMR (400 MHz, DMSO-*d₆*): δ [ppm] = 0.21 (s, 4H), 1.06-1.15 (m, 9H), 1.32 (br. s, 4H), 2.60-2.76 (m, 4H), 2.53-2.58 (m, ca. 2H), 2.78-2.88 (m, 1H), 3.07 (dd, 1H), 3.56 (d, 1H), 4.01 (q, 2H), 7.04 (dt, 1H), 7.41 (d, 1H), 7.44 (t, 1H), 9.60 (s, 1H). |
| | 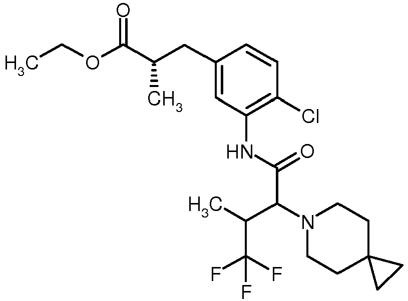 | |
| | aus Ethyl-(2S)-3-{4-chlor-3-[(4,4,4-trifluorvalyl)-amino]phenyl}-2-methylpropanoat (*Diastereomerengemisch 1*) und 2,2'-Cyclopropan-1,1-diyldiacetaldehyd | |
| **83A** | Ethyl-(2*S*)-3-(4-chlor-3-{[(2*R*,3*R*)-4,4,4-trifluor-3-methyl-2-(4-methylpiperidin-1-yl)butanoyl]-amino}phenyl)-2-methylpropanoat | LC-MS (Methode 2): Rₜ = 1.48 min; m/z = 477 (M+H)⁺. ¹H-NMR (400 MHz, DMSO-*d₆*): δ [ppm] = 0.87-0.95 (m, ca. 3H), 1.01-1.31 (m, ca. 13H), 1.53-1.63 (m, 2H), 2.05 (t, 1H), 2.59-2.76 (m, 3H), 2.76-2.96 (m, 2H), 2.99-3.07 (m, 1H), 3.51 (d, 1H), 3.95-4.05 (m, 2H), 7.03 (dd, 1H), 7.37-7.44 (m, 2H), 9.60 (s, 1H). |
| | 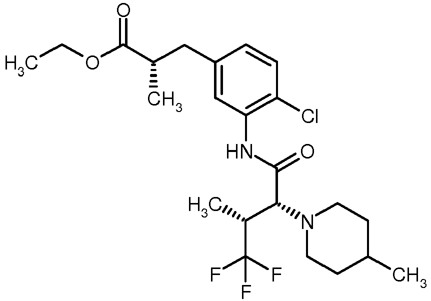 | |
| | aus (+)-Ethyl-(2*S*)-3-(4-chlor-3-{[(3*R*)-4,4,4-trifluor-D-valyl]amino}phenyl)-2-methylpropanoat und 3-Methylpentandial | |
| **84A** | Ethyl-(2*S*)-3-(3-{[(2*R*,3*R*)-2-(6-azaspiro[2.5]oct-6-yl)-4,4,4-trifluor-3-methylbutanoyl]amino}-4-fluorphenyl)-2-methylpropanoat | LC-MS (Methode 2): Rₜ = 1.42 min; m/z = 473 (M+H)⁺. ¹H-NMR (400 MHz, DMSO-*d₆*): δ [ppm] = 0.16-0.23 (m, 4H), 1.05-1.16 (m, 9H), 1.29 (br. s, 4H), 2.52-2.61 (m, ca. 4H), 2.61-2.74 (m, 2H), 2.75-2.86 (m, 1H), 3.05 (dd, 1H), 3.55 (d, 1H), 3.95-4.04 (m, 2H), 6.94-7.02 (m, 1H), 7.16 (dd, 1H), 7.63 (dd, 1H), 9.73 (s, 1H). |
| | 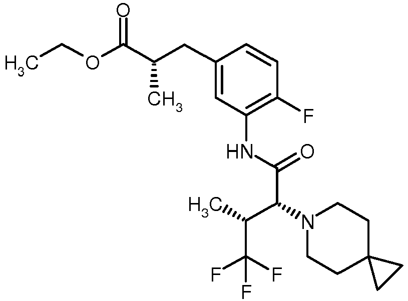 | |
| | aus (+)-Ethyl-(2*S*)-3-(4-fluor-3-{[(3*R*)-4,4,4-trifluor-D-valyl] amino }phenyl)-2-methylpropanoat und 2,2'-Cyclopropan-1,1-diyldiacetaldehyd | |
| **85A** | Ethyl-(2*R*)-3-(4-fluor-3-{[(2*R*,3*R*)-4,4,4-trifluor-3-methyl-2-(4-methylpiperidin-1-yl)butanoyl]-amino}phenyl)-2-methylpropanoat | LC-MS (Methode 5): Rₜ = 2.76 min; m/z = 461 (M+H)⁺. ¹H-NMR (400 MHz, DMSO-*d₆*): δ [ppm] = 0.86 (d, 3H), 0.96-1.05 (m, 1H), 1.05-1.13 (m, 9H), 1.13-1.36 (m, 2H), 1.57 (t, 2H), 1.96-2.07 (m, 1H), 2.35-2.45 (m, 1H), 2.59-2.73 (m, 3H), 2.76-2.83 (m, 1H), 2.89 (d, 1H), 2.97-3.08 (m, 1H), 3.49 (d, 1H), 3.96-4.04 (m, 2H), 6.88-7.03 (m, 1H), 7.15 (dd, 1H), 7.57 (dd, 1H), 9.71 (s, 1H). |
| | 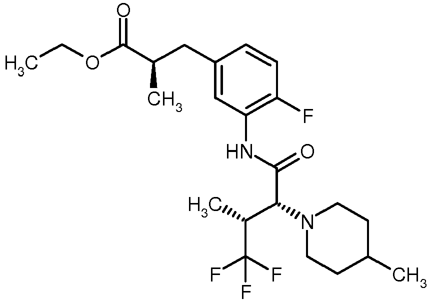 | |
| | aus (+)-Ethyl-(2*R*)-3-(4-fluor-3-{[(3*R*)-4,4,4-trifluor-D-valyl]amino}phenyl)-2-methylpropanoatund 3-Methylpentandial | |
| **86A** | Ethyl-(2*R*)-3-(3-({[(2*R*,3*R*)-2-(6-azaspiro[2.5]oct-6-yl)-4,4,4-trifluor-3-methylbutanoyl]amino}-4-chlorphenyl)-2-methylpropanoat | LC-MS (Methode 2): Rₜ = 1.47 min; m/z = 489 (M+H)⁺. ¹H-NMR (400 MHz, DMSO-*d₆*): δ [ppm] = 0.21 (br. s, 4H), 1.06-1.16 (m, 9H), 1.23-1.42 (m, 4H), 2.50-2.58 (m, ca. 2H, verdeckt), 2.61-2.75 (m, 4H), 2.78-2.88 (m, 1H), 3.02-3.12 (m, 1H), 3.56 (d, 1H), 4.01 (q, 2H), 7.04 (dd, 1H), 7.35-7.47 (m, 2H), 9.61 (s, 1H). |
| | 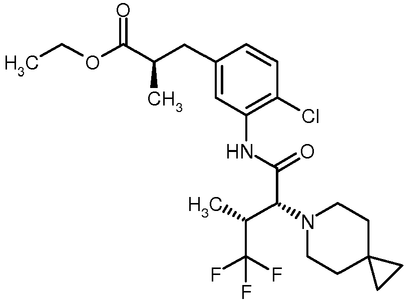 | |
| | aus (-)-Ethyl-(2*R*)-3-(4-chlor-3-{[(3*R*)-4,4,4-trifluor-D-valyl] amino }phenyl)-2-methylpropanoat und 2,2'-Cyclopropan-1,1-diyldiacetaldehyd | |
| **87A** | Ethyl-(2*S*)-3-(4-fluor-3-{[(2*R*,3*R*)-4,4,4-trifluor-3-methyl-2-(4-methylpiperidin-1-yl)butanoyl]-amino }phenyl)-2-methylpropanoat | LC-MS (Methode 2): Rₜ = 1.39 min; m/z = 461 (M+H)⁺. ¹H-NMR (400 MHz, DMSO-*d₆*): δ [ppm] = 0.87 (d, 3H), 0.97-1.05 (m, 1H), 1.05-1.13 (m, 9H), 1.13-1.35 (m, 2H), 1.57 (t, 2H), 2.02 (t, 1H), 2.39 (t, 1H), 2.59-2.73 (m, 3H), 2.75-2.93 (m, 2H), 2.95-3.10 (m, 1H), 3.49 (d, 1H), 3.93-4.06 (m, 2H), 6.98 (td, 1H), 7.15 (dd, 1H), 7.58 (dd, 1H), 9.70 (s, 1H). |
| | 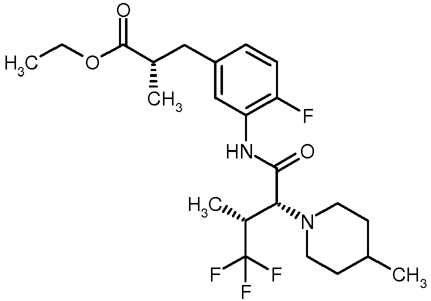 | |
| | aus (+)-Ethyl-(2*S*)-3-(4-fluor-3-{[(3*R*)-4,4,4-trifluor-D-valyl] amino }phenyl)-2-methylpropanoat und 3-Methylpentandial | |
| **88A** | Ethyl-(2*R*)-3-(4-chlor-3-{[(2*R*,3*R*)-4,4,4-trifluor-3-methyl-2-(4-methylpiperidin-1-yl)butanoyl]amino}-phenyl)-2-methylpropanoat | LC-MS (Methode 2): Rₜ = 1.48 min; m/z = 477 (M+H)⁺. ¹H-NMR (400 MHz, DMSO-*d₆*): δ [ppm] = 0.87 (d, 3H), 0.97-1.05 (m, 1H), 1.08-1.13 (m, 9H), 1.14-1.35 (m, 2H), 1.51-1.68 (m, 2H), 2.05 (t, 1H), 2.50-2.57 (m, ca. 1H, verdeckt), 2.60-2.74 (m, 3H), 2.77-2.96 (m, 2H), 2.98-3.07 (m, 1H), 3.51 (d, 1H), 4.01 (q, 2H), 7.03 (dd, 1H>, 7.34-7.52 (m, 2H), 9.60 (s, 1H). |
| | 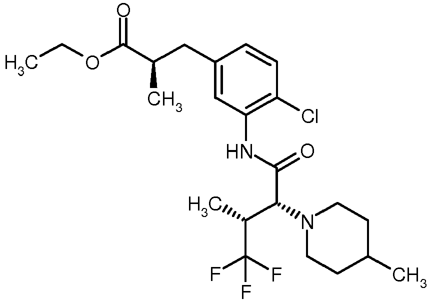 | |
| | aus (-)-Ethyl-(2*R*)-3-(4-chlor-3-{[(3*R*)-4,4,4-trifluor-D-valyl] amino }phenyl)-2-methylpropanoat und 3-Methylpentandial | |
| **89A** | Ethyl-(2*R)*-3-(3-{[(2*R*,3*R*)-2-(6-azaspiro[2.5]oct-6-yl)-4,4,4-trifluor-3-methylbutanoyl]amino}-4-fluorphenyl)-2-methylpropanoat | LC-MS (Methode 2): Rₜ = 1.40 min; m/z = 473 (M+H)⁺. ¹H-NMR (400 MHz, DMSO-*d₆*): δ [ppm] = 0.20 (br. s, 4H), 1.03-1.17 (m, 9H), 1.30 (br. s, 4H), 2.52-2.62 (m, ca. 4H, verdeckt), 2.63-2.74 (m, 2H), 2.77-2.87 (m, 1H), 3.00-3.10 (m, 1H), 3.54 (d, 1H), 4.01 (q, 2H), 6.99 (td, 1H>, 7.16 (dd, 1H), 7.61 (dd, 1H), 9.72 (s, 1H). [α]_{D}²⁰ = +5.0', c = 0.450, Chloroform. |
| | 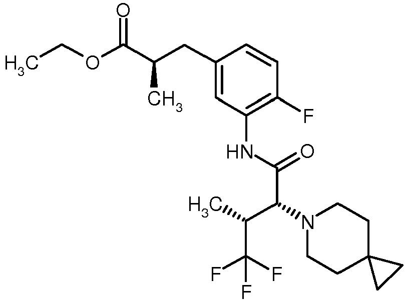 | |
| | aus (+)-Ethyl-(2*R*)-3-(4-fluor-3-{[(3*R*)-4,4,4-trifluo-D-valyl]amino}phenyl)-2-methylpropanoat und 2,2'-Cyclopropan-1,1-diyldiacetaldehyd | |
| **90A** | Ethyl-(2*S*)-3-[4-chlor-3-(1(2*R*,3*R*)-4,4,4-trifluor-3-methyl-2-[4-(trifluormethyl)piperidin-1-yl]-butanoyl}amino)phenyl]-2-methylpropanoat | LC-MS (Methode 3): Rₜ = 1.62 min; m/z = 531 (M+H)⁺. ¹H-NMR (400 MHz, DMSO-*d₆*): δ [ppm] = 1.04-1.15 (m, 9H), 1.30-1.40 (m, 1H), 1.44-1.58 (m, 1H), 1.72-1.87 (m, 2H), 2.11 (t, 1H), 2.20-2.30 (m, 1H), 2.55-2.64 (m, 1H), 2.65-2.74 (m, 2H), 2.75-2.89 (m, 2H), 3.06 (d, 2H), 3.57 (d, 1H), 4.01 (q, 2H), 7.04 (dd, 1H), 7.37-7.46 (m, 2H), 9.67 (s, 1H). |
| | 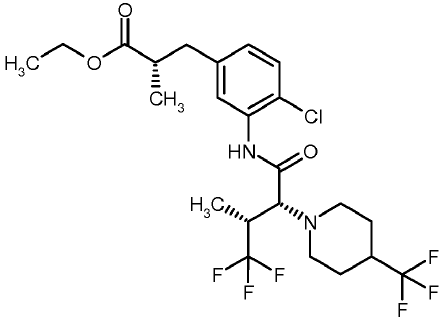 | |
| | aus (+)-Ethyl-(2*S*)-3-(4-chlor-3-{[(3*R*)-4,4,4-trifluor-D-valyl]amino}phenyl)-2-methylpropanoat und 3-(Trifluormethyl)pentandial | |

### Beispiel 91A

### rac-Cyclopent-1-en-1-yl(4-methoxypiperidin-1-yl)essigsäure

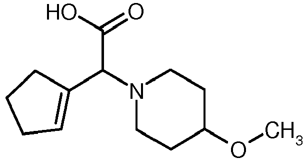

50 mg (0.45 mmol) Cyclopent-1-en-1-ylboronsäure wurden mit 51 mg (0.45 mmol) 4-Methoxypiperidin und 41 mg (0.45 mmol) Glyoxalsäure-Monohydrat in 2 ml Dichlormethan vorgelegt und mit 78 µl (57 mg, 0.45 mmol) *N*,*N*-Diisopropylethylamin versetzt. Der Ansatz wurde 2 Tage bei RT gerührt und dann im Vakuum eingeengt. Der Rückstand wurde über präparative HPLC aufgereinigt (RP18-Säule; Acetonitril/Wasser-Gradient unter Zusatz von 0.1% TFA, 10:90 → 95:5; Laufzeit 38 min). Es wurden 31 mg (29% d. Th.) der Titelverbindung erhalten.

LC-MS (Methode 5): Rₜ = 0.95 min; m/z = 240 (M+H)⁺.

### Beispiel 92A

### rac-Cyclopent-1-en-1-yl(3,4-dihydroisochiriolin-2(1H)-yl)essigsäure

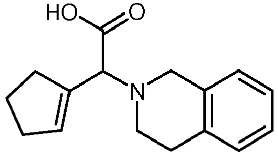

50 mg (0.45 mmol) Cyclopent-1-en-1-ylboronsäure wurden mit 59.5 mg (0.45 mmol) 1,2,3,4-Tetrahydroisochinolin und 41 mg (0.45 mmol) Glyoxalsäure-Monohydrat in 2 ml Dichlormethan vorgelegt und mit 78 µl (57 mg, 0.45 mmol) *N*,*N*-Diisopropylethylamin versetzt. Der Ansatz wurde über Nacht bei RT gerührt und dann im Vakuum eingeengt. Der Rückstand wurde über präparative HPLC aufgereinigt (RP18-Säule; Acetonitril/Wasser-Gradient unter Zusatz von 0.1% TFA, 10:90 → 95:5; Laufzeit 38 min). Es wurden 101 mg (91% Reinheit, 80% d. Th.) der Titelverbindung erhalten.

LC-MS (Methode 5): Rₜ = 1.22 min; m/z = 258 (M+H)⁺.

### Beispiel 93A

### rac-tert.-Butyl-1-(3-{[cyclopent-1-en-1-yl(4-methoxypiperidin-1-yl)acetyl]amino}-4-fluorbenzyl)-cyclopropancarboxylat

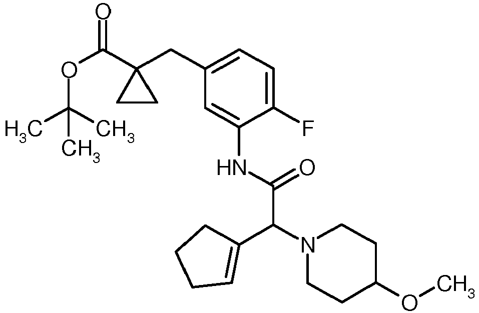

30.9 mg (0.13 mmol) *rac*-Cyclopent-1-en-1-yl(4-methoxypiperidin-1-yl)essigsäure wurden in 0.87 ml DMF/Pyridin (3:1) vorgelegt, mit 63.8 mg (0.17 mmol) HATU versetzt und 30 min bei RT gerührt. Anschließend wurden 34.3 mg (0.13 mmol) *tert*.-Butyl-1-(3-amino-4-fluorbenzyl)cyclopropancarboxylat hinzugefügt, und das Reaktionsgemisch wurde über Nacht bei RT gerührt. Danach wurde der Ansatz mit Ethylacetat verdünnt, mehrmals mit gesättigter Natriumchlorid-Lösung gewaschen, über Magnesiumsulfat getrocknet und im Vakuum eingeengt. Der Rückstand wurde über präparative HPLC gereinigt (RP18-Säule; Acetonitril/Wasser-Gradient unter Zusatz von 0.1% TFA, 10:90 → 95:5; Laufzeit 38 min). Es wurden 20.7 mg (33% d. Th.) der Titelverbindung erhalten.

LC-MS (Methode 5): Rt = 1.85 min; m/z = 487.4 (M+H)⁺.

### Beispiel 94A

### rac-tert.-Butyl-1-(3-1{[cyclopent-1-en-1-yl(3,4-dihydroisochinolin-2(1H-yl)acetyl]amino}-4-fluorbenzyl)cyclopropancarboxylat

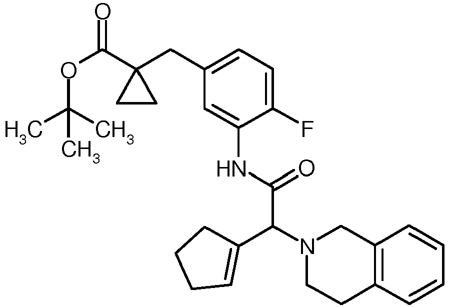

100 mg (0.39 mmol) *rac*-Cyclopent-1-en-1-yl(3,4-dihydroisochinolin-2(1*H*)-yl)essigsäure wurden in 2.6 ml DMF/Pyridin (3:1) vorgelegt, mit 192 mg (0.51 mmol) HATU versetzt und 30 min bei RT gerührt. Anschließend wurden 103 mg (0.39 mmol) *tert*.-Butyl-1-(3-amino-4-fluorbenzyl)-cyclopropancarboxylat hinzugefügt, und das Reaktionsgemisch wurde über Nacht bei RT gerührt. Danach wurde der Ansatz direkt über präparative HPLC aufgereinigt (RP18-Säule; Acetonitril/ Wasser-Gradient unter Zusatz von 0.1% TFA, 10:90 → 95:5; Laufzeit 38 min). Das so erhaltene Produkt wurde in Ethylacetat aufgenommen und mehrmals mit 1 N Salzsäure gewaschen. Die organische Phase wurde über Magnesiumsulfat getrocknet und im Vakuum eingeengt. Es wurden 103 mg (72% Reinheit, 37% d. Th.) der Titelverbindung erhalten, welche ohne weitere Reinigung umgesetzt wurden.

LC-MS (Methode 5): Rₜ = 2.26 min; m/z = 505.3 (M+H)⁺.

### Beispiel 95A

### rac-tert.-Butyl-1-(3-{[cyclopentyl(4-methoxypiperidin-1-yl)acetyl]amino}-4-fluorbenzyl)cyclopropancarboxylat

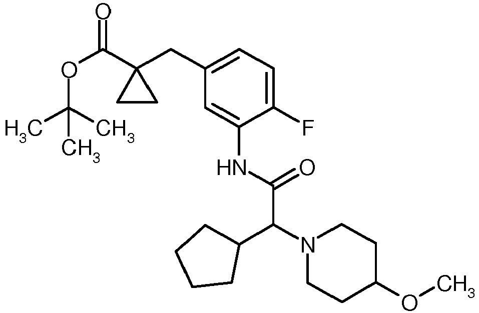

20.7 mg (0.04 mmol) *rac*-*tert*.-Butyl-1-(3-{[cyclopent-1-en-1-yl(4-methoxypiperidin-1-yl)acetyl]-amino}-4-fluorbenzyl)cyclopropancarboxylat wurden in 3 ml Methanol vorgelegt, mit 3 mg (0.01 mmol) Platin(IV)oxid versetzt und 5 h bei RT und Normaldruck hydriert. Danach wurde der Ansatz über Kieselgur filtriert, der Rückstand gut mit Methanol nachgewaschen und das Filtrat im Vakuum eingeengt. Es wurden 19.5 mg (100% Reinheit, 94% d. Th.) der Titelverbindung erhalten.

LC-MS (Methode 5): Rₜ = 1.92 min; m/z = 489.4 (M+H)⁺.

### Beispiel 96A

### rac-tert.-Butyl-1-(3-{[cyclopentyl(3,4-dihydroisochinolin-2(1H)-yl)acetyl]amino}-4-fluorbenzyl)-cyclopropancarboxylat

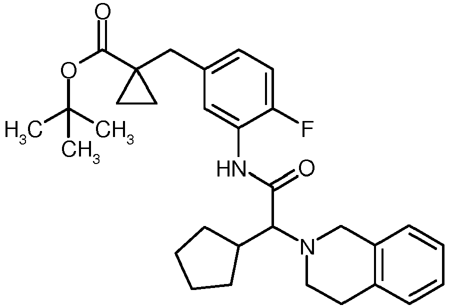

102 mg (0.2 mmol, 72% Reinheit) *rac*-*tert*.-Butyl-1-(3-{[cyclopent-1-en-1-yl(3,4-dihydroisochinolin-2(1*H*)-yl)acetyl]amino}-4-fluorbenzyl)cyclopropancarboxylat wurden in 14.4 ml Methanol vorgelegt, mit 14.4 mg (0.06 mmol) Platin(IV)oxid versetzt und 5 h bei RT und Normaldruck hydriert. Danach wurde der Ansatz über Kieselgur filtriert, der Rückstand gut mit Methanol nachgewaschen und das Filtrat im Vakuum eingeengt. Es wurden 102 mg (36% Reinheit) der Titelverbindung erhalten, welche ohne Reinigung weiter umgesetzt wurden.

LC-MS (Methode 5): Rₜ = 1.34 min; m/z = 507.2 (M+H)⁺.

### Ausführungsbeispiele:

### Beispiel 1

### 3-(4-Chlor-3-{[4,4,4-trifluor-3-methyl-2-(4-methylpiperidin-1-yl)butanoyl]amino}phenyl)-propansäure (racemisches Diastereomerengemisch)

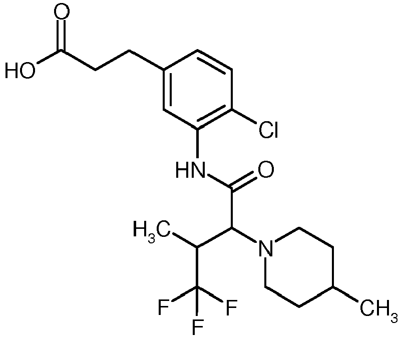

45.0 mg (0.092 mmol) *tert*.-Butyl-3-(4-chlor-3-{[4,4,4-trifluor-3-methyl-2-(4-methylpiperidin-1-yl)butanoyl]amino}phenyl)propanoat (racemisches Diastereomerengemisch) wurden in 0.1 ml Dichlormethan gelöst und bei RT mit 0.35 ml Trifluoressigsäure versetzt. Die Reaktionsmischung wurde über Nacht bei RT gerührt und dann im Vakuum eingeengt. Das Rohprodukt wurde durch präparative RP-HPLC gereinigt (Eluent Acetonitril/Wasser). Es wurden 39.0 mg des Zielprodukts als Diastereomerengemisch erhalten (97.9% d. Th.).

LC-MS (Methode 2): Rₜ = 1.10 min und 1.21 min; jeweils m/z = 435 (M+H)⁺.

¹H-NMR (400 MHz, DMSO-*d₆*): beide Diastereomere δ [ppm] = 0.86/0.87 (je dd, zus. 3H), 0.94-1.05 (m, 1H), 1.07/1.10 (je dd, zus. 3H), 1.13-1.35 (m, 2H), 1.49-1.65 (m, 2H), 2.00-2.16 (m, 1H), 2.56-2.68 (m, ca. 1H), 2.77-2.99 (m, ca. 5H), 3.51/3.59 (je d, zus. 1H), 7.05-7.15 (m, 1H), 7.40/ 7.42 (je d, zus. 1H), 7.45/7.54 (je d, zus. 1H), 9.61/9.71 (je s, zus. 1H), 12.16 (s, 1H).

### Allgemeine Vorschrift 4: Herstellung substituierter Piperidinoacetanilide aus Aminoacetaniliden gemäß nachfolgendem Reaktionsschema:

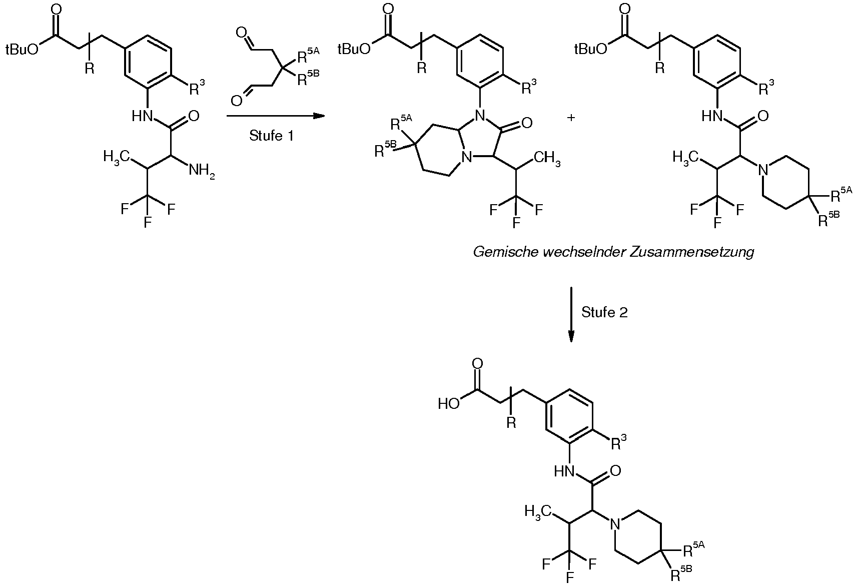

### Stufe 1:

Zu einer Lösung der betreffenden Aminoacetanilid-Komponente (1.0 eq.) in Methanol (ca. 0.05 mol/l bis 1 bis mol/l) werden bei RT Triethylamin (1.5 bis 2.0 eq.), 1 N Salzsäure (ca. 0.25 bis 0.5 eq.), der betreffende Dialdehyd (bzw. dessen cyclische Acetalform, als Rohgemisch, ca. 2.0 bis 4.0 eq.) und nach ca. 5 bis 15 min Natriumcyanoborhydrid (2.0 bis 4.0 eq.) gegeben. Die Mischung wird ca. 12 bis zu 72 Stunden bei RT gerührt, wobei gegebenenfalls zwischenzeitlich weitere Mengen an Triethylamin, 1 N Salzsäure, Dialdehyd und/oder Natriumcyanoborhydrid zugesetzt werden. Nach beendeter Umsetzung wird die Reaktionsmischung auf Wasser gegeben. Die wässrige Phase wird dreimal mit Ethylacetat extrahiert, und die vereinigten organischen Phasen werden im Vakuum eingeengt. Das erhaltene Rohproduktgemisch kann direkt in der Folgestufe eingesetzt werden oder (bevorzugt) wird zuvor durch präparative RP-HPLC aufgereinigt (Eluent Acetonitril/ Wasser-Gradient).

### Stufe 2:

Das betreffende Zwischenproduktgemisch aus Stufe 1 (roh oder aufgereinigt) wird bei 0°C oder RT in Trifluoressigsäure gelöst (Konzentration ca. 0.1 bis 2.0 mol/l) und anschließend bei 0°C bis RT tropfenweise mit einem großen Überschuss Triethylsilan (10 bis 20 eq.) versetzt. Die Mischung wird 4 bis 24 h bei RT gerührt; gegebenenfalls wird die Mischung bis auf 40°C erwärmt, bis vollständiger Umsatz erreicht ist. Danach wird das Reaktionsgemisch im Vakuum eingeengt. Der Rückstand wird in Ethylacetat aufgenommen und mit Wasser (das durch Zusatz von wenig ges. Natriumhydrogencarbonat-Lösung annähernd neutral (pH 6-7) gestellt ist) gewaschen. Nach Einengen im Vakuum kann das Rohprodukt durch präparative RP-HPLC (Eluent Acetonitril/Wasser- oder Methanol/Wasser-Gradient), durch Kristallisation aus Acetonitril oder Wasser/ Acetonitril-Gemischen oder durch eine Kombination dieser Methoden gereinigt werden.

Die folgenden Beispiele wurden gemäß der Allgemeinen Vorschrift 4 hergestellt:

| **Beispiel** | **Name / Struktur / Edukte** | **Analytische Daten** |
|---|---|---|
| 2 | (+/-)-1-(4-Fluor-3-{[4,4,4-trifluor-3-methyl-2-(piperidin-1-yl)butanoyl]amino}benzyl)-cyclopropancarbonsäure (*Diastereomer 1*) | LC-MS (Methode 2): Rₜ = 1.07 min; m/z = 431 (M+H)⁺. ¹H-NMR (400 MHz, DMSO-*d₆*): δ [ppm] = 0.77-0.85 (m, 2H), 1.06-1.15 (m, 5H), 1.37 (d, 2H), 1.41-1.59 (m, 4H), 2.40-2.55 (m, ca. 4H, verdeckt), 2.84 (s, 2H), 2.98-3.08 (m, 1H), 3.45 (d, 1H), 7.00-7.09 (m, 1H), 7.10-7.20 (m, 1H), 7.63 (dd, 1H), 9.70 (s, 1H), 12.16 (br. s, 1H). |
| | 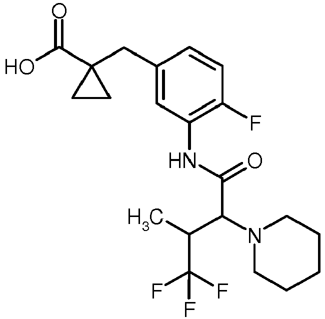 | |
| | aus (+/-)-*tert*.-Butyl-1-{4-fluor-3-[(4,4,4-trifluorvalyl)amino]benzyl}cyclopropancarboxylat (*Diastereomer 1*) und Glutardialdehyd (als 50%-ige Lösung in Wasser) | |
| 3 | (+/-)-1-(4-Fluor-3-{[4,4,4-trifluor-3-methyl-2-(piperidin-1-yl)butanoyl]amino}benzyl)-cyclopropancarbonsäure (*Diastereomer 2*) | LC-MS (Methode 2): Rₜ = 0.95 min; m/z = 431 (M+H)⁺. ¹H-NMR (400 MHz, DMSO-*d₆*): δ [ppm] = 0.79-0.86 (m, 2H), 1.02 (d, 3H), 1.09-1.14 (m, 2H), 1.31-1.52 (m, 6H), 2.40-2.48 (m, 2H), 2.56-2.66 (m, 2H), 2.85 (s, 2H), 2.95 (dd, 1H), 3.55 (d, 1H), 7.05-7.21 (m, 2H), 7.64 (d, 1H), 9.76 (s, 1H), 12.16 (s, 1H). |
| | 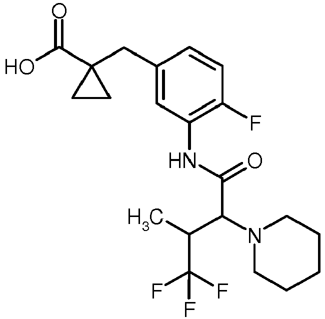 | |
| | aus (+/-)-*tert*.-Butyl-1-{4-fluor-3-[(4,4,4-trifluorvalyl)amino]benzyl}cyclopropancarboxylat (*Diastereomer 2*) und Glutardialdehyd (als 50%-ige Lösung in Wasser) | |
| 4 | (+/-)-1-(4-Fluor-3-{[4,4,4-trifluor-3-methyl-2-(4-methylpiperidin-1-yl)butanoyl]amino}benzyl)-cyclopropancarbonsäure (*Diastereomer 1*) | LC-MS (Methode 2): Rₜ = 1.18 min; m/z = 445 (M+H)⁺. ¹H-NMR (400 MHz, DMSO-*d₆*): δ [ppm] = 0.78-0.84 (m, 2H), 0.87 (d, 3H), 0.95-1.05 (m, 1H), 1.06-1.13 (m, 5H), 1.13-1.22 (m, 1H), 1.22-1.33 (m, 1H), 1.58 (t, 2H), 2.02 (t, 1H), 2.36-2.46 (m, 1H), 2.59-2.67 (m, 1H), 2.84 (s, 2H), 2.85-2.92 (m, 1H), 2.98-3.08 (m, 1H), 3.47 (d, 1H), 7.01-7.09 (m, 1H), 7.10-7.19 (m, 1H), 7.58-7.68 (m, 1H), 9.70 (s, 1H), 12.15 (br. s, 1H). |
| | 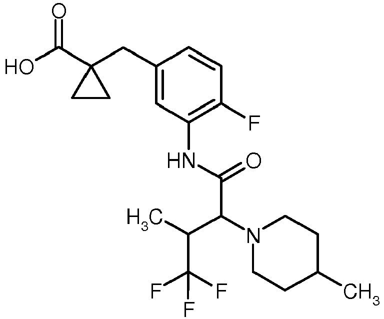 | |
| | aus (+/-)-*tert*.-Butyl-1-{4-fluor-3-[(4,4,4-trifluorvalyl)amino]benzyl}cyclopropancarboxylat (*Diastereomer 1*) und 3-Methylpentandial | |
| **5** | (+/-)-3-(4-Fluor-3-{[4,4,4-trifluor-3-methyl-2-(4-methylpiperidin-1-yl)butanoyl]amino}phenyl)-propansäure (*Diastereomer 1*) | LC-MS (Methode 2): Rₜ = 1.11 min; m/z = 419 (M+H)⁺. ¹H-NMR (400 MHz, DMSO-*d₆*): δ [ppm] = 0.87 (d, 3H), 0.95-1.06 (m, 1H), 1.10 (d, 3H), 1.12-1.22 (m, 1H), 1.22-1.33 (m, 1H), 1.57 (t, 2H), 2.03 (t, 1H), 2.37-2.46 (m, 1H), 2.49-2.53 (m, ca. 2H, verdeckt), 2.63 (d, 1H), 2.79 (t, 2H), 2.89 (d, 1H), 2.97-3.08 (m, 1H), 3.49 (d, 1H), 6.98-7.08 (m, 1H), 7.15 (dd, 1H), 7.60 (dd, 1H), 9.70 (s, 1H), 12.12 (s, 1H). |
| | 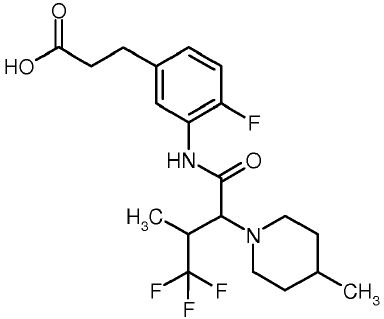 | |
| | aus (+/-)-*tert*.-Butyl-3-{4-fluor-3-[(4,4,4-trifluorvalyl)amino]phenyl}propanoat *(Diastereomer 1*) und 3-Methylpentandial | |
| **6** | (+/-)-3-(4-Fluor-3-{[4,4,4-trifluor-3-methyl-2-(4-methylpiperidin-1-yl)butanoyl]amino}phenyl)-propansäure (*Diastereomer 2*) | LC-MS (Methode 2): Rₜ = 0.97 min; m/z = 419 (M+H)⁺. ¹H-NMR (400 MHz, DMSO-*d₆*): δ [ppm] = 0.86 (d, 3H), 0.94-1.02 (m, 1H), 1.03 (d, 3H), 1.05-1.18 (m, 1H), 1.20-1.34 (m, 1H), 1.56 (t, 2H), 2.04 (t, 1H), 2.30-2.41 (m, 1H), 2.79 (t, 2H), 2.87 (d, 2H), 2.89-2.99 (m, 1H), 3.58 (d, 1H), 7.01-7.09 (m, 1H), 7.17 (dd, 1H), 7.61 (dd, 1H), 9.77 (s, 1H), 12.14 (s, 1H). |
| | 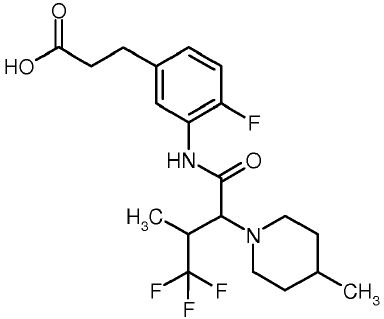 | |
| | aus (+/-)-*tert*.-Butyl-3-{4-fluor-3-[(4,4,4-trifluorvalyl)amino]phenyl}propanoat (*Diastereomer 2*) und 3-Methylpentandial | |
| 7 | (+)-3-(4-Fluor-3-{[(2*R*,3*R*)-4,4,4-trifluor-3-methyl-2-(4-methylpiperidin-1-yl)butanoyl]amino}phenyl)-propansäure | LC-MS (Methode 2): Rₜ = 1.11 min; m/z = 419 (M+H)⁺. ¹H-NMR (400 MHz, DMSO-*d₆*): δ [ppm] = 0.86 (d, 3H), 0.95-1.07 (m, 1H), 1.09 (d, 3H), 1.11-1.21 (m, 1H), 1.21-1.36 (m, 1H), 1.57 (t, 2H), 1.95-2.07 (m, 1H), 2.44 (t, ca. 2H), 2.63 (d, 1H), 2.77 (t, 2H), 2.88 (d, 1H), 2.97-3.08 (m, 2H), 3.49 (d, 2H), 6.96-7.07 (m, 1H), 7.13 (dd, 1H), 7.58 (dd, 1H), 9.70 (s, 1H). [α]_{D}²⁰ = +27.5°, c = 0.430, Chloroform. |
| | 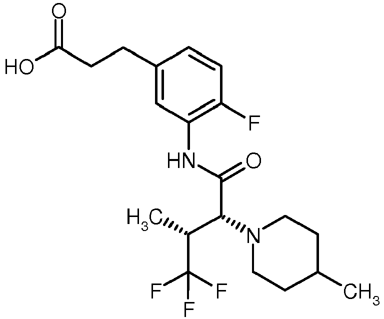 | |
| | aus (+)-*tert*.-Butyl-3-(4-fluor-3-{[(3*R*)-4,4,4-trifluor-D-valyl] amino }phenyl)propanoat und 3-Methylpentandial | |
| 8 | (+/-)-1-(3-{[2-(4-Ethylpiperidin-1-yl)-4,4,4-trifluor-3-methylbutanoyl]amino}-4-fluorbenzyl)-cyclopropancarbonsäure (*Diastereomer 1*) | LC-MS (Methode 2): Rₜ = 1.26 min; m/z = 459 (M+H)⁺. ¹H-NMR (400 MHz, DMSO-*d₆*): δ [ppm] = 0.79-0.86 (m, 5H), 0.94-1.24 (m, 10H), 1.63 (t, 2H), 2.01 (t, 1H), 2.36-2.45 (m, 1H), 2.65 (d, 1H), 2.84 (s, 2H), 2.91 (d, 1H), 2.98-3.09 (m, 1H), 3.47 (d, 1H), 7.01-7.09 (m, 1H), 7.10-7.20 (m, 1H), 7.61 (dd, 1H), 9.68 (s, 1H), 12.13 (br. s, 1H). |
| | 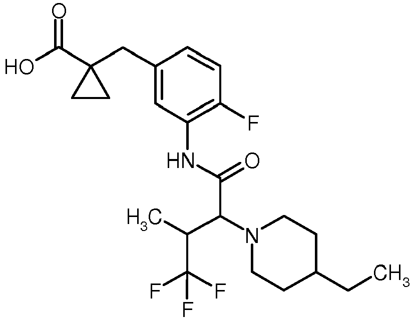 | |
| | aus (+/-)-*tert*.-Butyl-1-{4-fluor-3-[(4,4,4-trifluor-valyl)amino]benzyl }cyclopropancarboxylat (*Diastereomer 1*) und 3-Ethylpentandial | |
| **9** | (+/-)-3-(3-{[2-(4-Ethylpiperidin-1-yl)-4,4,4-trifluor-3-methylbutanoyl]amino}-4-fluorphenyl)-propansäure (*Diastereomer 1*) | LC-MS (Methode 2): Rₜ = 1.15 min; m/z = 433 (M+H)+. ¹H-NMR (400 MHz, DMSO-*d₆*): δ [ppm] = 0.83 (t, 3H), 0.94-1.24 (m, 8H), 1.63 (t, 2H), 1.96-2.06 (m, 1H), 2.35-2.46 (m, 1H), 2.52-2.56 (m, ca. 2H, verdeckt), 2.65 (d, 1H), 2.79 (t, 2H), 2.91 (d, 1H), 2.97-3.08 (m, 1H), 3.49 (d, 1H), 6.98-7.07 (m, 1H), 7.15 (dd, 1H), 7.59 (dd, 1H), 9.70 (s, 1H), 12.13 (br. s, 1H). |
| | 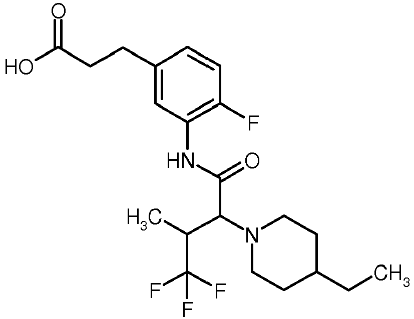 | |
| | aus (+/-)-*tert*.-Butyl-3-{4-fluor-3-[(4,4,4-trifluorvalyl)amino]phenyl}propanoat (*Diastereomer 1*) und 3-Ethylpentandial | |
| **10** | (+/-)-3-(3-{[2-(4-Ethylpiperidin-1-yl)-4,4,4-trifluor-3-methylbutanoyl]amino}-4-fluorphenyl)-propansäure (*Diastereomer 2*) | LC-MS (Methode 2): Rₜ = 1.01 min; m/z = 433 (M+H)⁺. ¹H-NMR (400 MHz, DMSO-*d₆*): δ [ppm] = 0.83 (t, 3H), 0.94-1.23 (m, 8H), 1.53-1.69 (m, 2H), 2.04 (t, 1H), 2.36 (t, 1H), 2.80 (t, 2H), 2.84-3.04 (m, 3H), 3.58 (d, 1H), 6.99-7.10 (m, 1H), 7.17 (dd, 1H), 7.58-7.71 (m, 1H), 9.76 (s, 1H), 12.15 (br. s, ca. 1H). |
| | 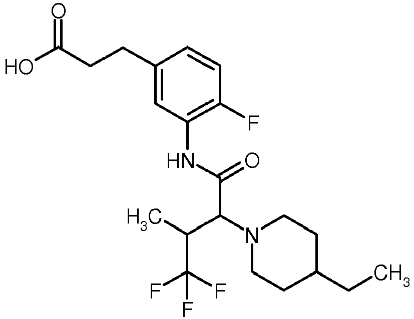 | |
| | aus (+/-)-*tert*.-Butyl-3-{4-fluor-3-[(4,4,4-trifluorvalyl)amino]phenyl}propanoat (*Diastereomer 2*) und 3-Ethylpentandial | |
| **11** | (+/-)-3-(4-Chlor-3-{[2-(4-ethylpiperidin-1-yl)-4,4,4-trifluor-3-methylbutanoyl]amino}phenyl)-propansäure (*Diastereomer 1*) | LC-MS (Methode 2): Rₜ = 1.29 min; m/z = 449 (M+H)⁺. ¹H-NMR (400 MHz, DMSO-*d₆*): δ [ppm] = 0.83 (t, 3H), 0.95-1.05 (m, 1H), 1.07-1.28 (m, 7H), 1.64 (dd, 2H), 2.00-2.14 (m, 1H), 2.39 (t, 2H), 2.53-2.57 (m, ca. 1H, verdeckt), 2.66 (d, 1H), 2.76 (t, 2H), 2.94 (d, 1H), 2.99-3.09 (m, 1H), 3.51 (d, 1H), 7.07 (dd, 1H), 7.37 (d, 1H), 7.40-7.52 (m, 1H), 9.60 (s, 1H). |
| | 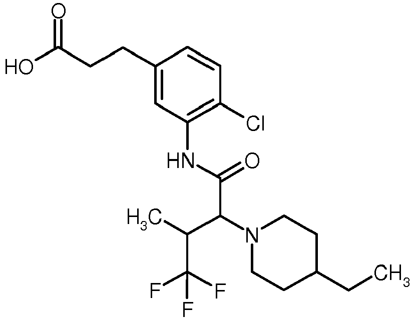 | |
| | aus (+/-)-*tert*.-Butyl-3-{4-chlor-3-[(4,4,4-trifluorvalyl)amino]phenyl}propanoat (*Diastereomer 1*) und 3-Ethylpentandial | |
| **12** | (+/-)-3-(4-Chlor-3-{[2-(4-ethylpiperidin-1-yl)-4,4,4-trifluor-3-methylbutanoyl]amino}phenyl)-propansäure (*Diastereomer 2*) | LC-MS (Methode 2): Rₜ = 1.20 min; m/z = 449 (M+H)⁺. ¹H-NMR (400 MHz, DMSO-*d₆*): δ [ppm] = 0.83 (t, 3H), 0.94-1.24 (m, 8H), 1.58-1.66 (m, 2H), 2.09 (t, 1H), 2.39-2.48 (m, 1H), 2.81 (t, 2H), 2.84-3.03 (m, 3H), 3.59 (d, 1H), 7.10 (dd, 1H), 7.41 (d, 1H), 7.51-7.67 (m, 1H), 9.70 (s, 1H), 12.20 (br. s, ca. 1H). |
| | 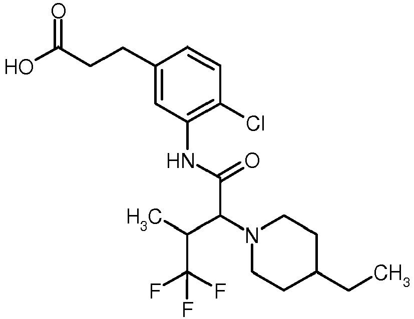 | |
| | aus (+/-)-*tert*.-Butyl-3-{4-chlor-3-[(4,4,4-trifluorvalyl)amino]phenyl}propanoat (*Diastereomer 2*) und 3-Ethylpentandial | |
| **13** | (+/-)-3-(4-Chlor-3-{[4,4,4-trifluor-3-methyl-2-(4-methylpiperidin-1-yl)butanoyl]amino}phenyl)-propansäure (*Diastereomer 1*) | LC-MS (Methode 2): Rₜ = 1.21 min; m/z = 435 (M+H)⁺. ¹H-NMR (400 MHz, DMSO-*d₆*): δ [ppm] = 0.87 (d, 3H), 0.96-1.08 (m, 1H), 1.10 (d, 3H), 1.12-1.23 (m, 1H), 1.23-1.38 (m, 1H), 1.49-1.66 (m, 2H), 2.02-2.12 (m, 1H), 2.23 (t, 2H), 2.54-2.68 (m, ca. 2H), 2.73 (t, 2H), 2.91 (d, 1H), 2.99-3.09 (m, 1H), 3.50 (d, 1H), 7.06 (dd, 1H), 7.34 (d, 1H), 7.38-7.50 (m, 1H), 9.61 (s, 1H). |
| | 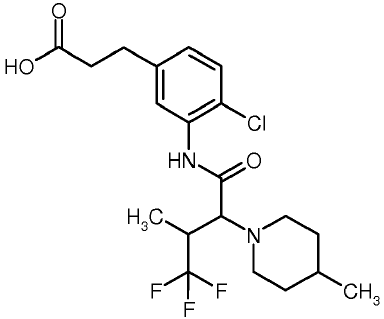 | |
| | aus (+/-)-*tert*.-Butyl-3-{4-chlor-3-[(4,4,4-trifluorvalyl)amino]phenyl}propanoat (*Diastereomer 1*) und 3-Methylpentandial | |
| **14** | (+/-)-3-[4-Chlor-3-({4,4,4-trifluor-3-methyl-2-[4-(trifluormethyl)piperidin-1-yl]butanoyl}amino)-phenyl]propansäure (*Diastereomer 1*) | LC-MS (Methode 2): Rₜ = 1.23 min; m/z = 489 (M+H)⁺. ¹H-NMR (400 MHz, DMSO-*d₆*): δ [ppm] = 1.11 (d, 3H), 1.30-1.40 (m, 1H), 1.46-1.55 (m, 1H), 1.69-1.88 (m, 2H), 2.05-2.18 (m, 1H), 2.20-2.35 (m, 1H), 2.56-2.65 (m, 1H), 2.74-2.85 (m, 3H), 3.05 (d, 2H), 3.56 (d, 1H), 7.09 (dd, 1H), 7.41 (d, 1H), 7.47 (d, 1H), 9.67 (s, 1H), 12.16 (br. s, 1H). |
| | 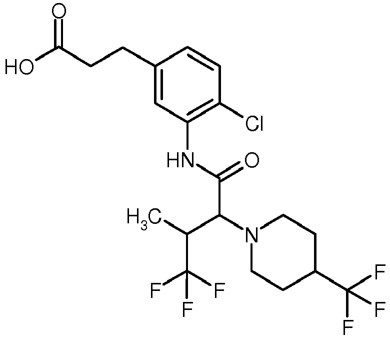 | |
| | aus (+/-)-*tert*.-Butyl-3-{4-chlor-3-[(4,4,4-trifluorvalyl)amino]phenyl}propanoat (*Diastereomer 1*) und 3-(Trifluormethyl)pentandial | |
| **15** | (+/-)-3-(3-{[2-(6-Azaspiro[2.5]oct-6-yl)-4,4,4-trifluor-3-methylbutanoyl]amino}-4-fluorphenyl)-propansäure (*Diastereomer 1*) | LC-MS (Methode 3): Rₜ = 1.26 min; m/z = 431 (M+H)⁺. ¹H-NMR (400 MHz, DMSO-*d₆*): δ [ppm] = 0.20 (s, 4H), 1.14 (d, 3H), 1.30 (br. s, 4H), 2.50-2.63 (m, ca. 6H, verdeckt), 2.78 (t, 2H), 3.02-3.13 (m, 1H), 3.55 (d, 1H), 7.02-7.08 (m, 1H), 7.18 (t, 1H), 7.65 (d, 1H), 9.75 (s, 1H), 12.14 (br.s, 1H). |
| | 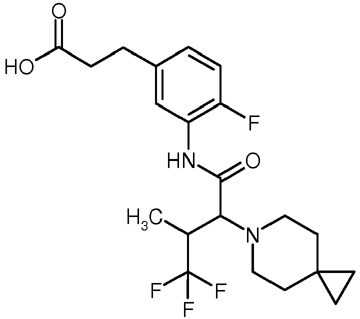 | |
| | aus (+/-)-*tert*.-Butyl-3-{4-fluor-3-[(4,4,4-trifluorvalyl)amino]phenyl}propanoat (*Diastereomer 1*) und 2,2'-Cyclopropan-1,1-diyldiacetaldehyd | |
| **16** | (+)-3-(3-{[(2*R*,3*R*)-2-(6-Azaspiro[2.5]oct-6-yl)-4,4,4-trifluor-3-methylbutanoyl]amino}-4-fluorphenyl)propansäure | LC-MS (Methode 2): Rₜ = 1.14 min; m/z = 431 (M+H)⁺. ¹H-NMR (400 MHz, DMSO-*d₆*): δ [ppm] = 0.20 (s, 4H), 1.13 (d, 3H), 1.21-1.41 (m, 4H), 2.31 (t, 2H), 2.52-2.63 (m, ca. 4H, verdeckt), 2.74 (t, 2H), 3.00-3.10 (m, 1H), 3.53 (d, 1H), 6.95-7.05 (m, 1H), 7.07-7.16 (m, 1H), 7.59 (d, 1H), 9.72 (s, 1H). [α]_{D}²⁰ = +23.2°, c = 0.435, Chloroform. |
| | 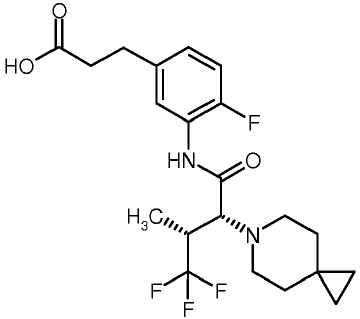 | |
| | aus (+)-*tert*.-Butyl-3-(4-fluor-3-{[(3*R*)-4,4,4-trifluor-D-valyl] amino }phenyl)propanoat und 2,2'-Cyclopropan-1,1-diyldiacetaldehyd | |
| **17** | (-)-3-[4-Chlor-3-({(2*R*,3*R*)-4,4,4-trifluor-3-methyl-2-[4-(trifluormethyl)piperidin-1-yl]butanoyl}amino)-phenyl]propansäure | LC-MS (Methode 3): Rₜ = 1.38 min; m/z = 489 (M+H)⁺. ¹H-NMR (400 MHz, DMSO-*d₆*): δ [ppm] = 1.11 (d, 3H), 1.28-1.41 (m, 1H), 1.43-1.56 (m, 1H), 1.69-1.87 (m, 2H), 2.07-2.18 (m, 1H), 2.21-2.35 (m, 1H), 2.55-2.65 (m, 1H), 2.74-2.86 (m, 3H), 3.03-3.10 (m, 2H), 3.56 (d, 1H), 7.09 (dd, 1H), 7.41 (d, 1H), 7.47 (d, 1H), 9.67 (s, 1H), 12.16 (br. s, 1H). [α]_{D}²⁰ = -5.1°, c = 0.370, Chloroform. |
| | 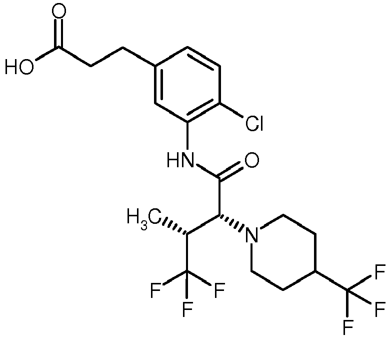 | |
| | aus (+)-*tert*.-Butyl-3-(4-chlor-3-{[(3*R*)-4,4,4-trifluor-D-valyl] amino}phenyl)propanoat und 3-(Trifluormethyl)pentandial | |
| **18** | (+)-3-(3-{[(2*R*,3*R*)-2-(4-Cyclopropylpiperidin-1-yl)-4,4,4-trifluor-3-methylbutanoyl]amino}-4-fluorphenyl)propansäure | LC-MS (Methode 5): Rₜ = 2.39 min; m/z = 445 (M+H)⁺. ¹H-NMR (400 MHz, DMSO-*d₆*): δ [ppm] = 0.04 (d, 2H), 0.33 (d, 2H), 0.51 (br. s, 2H), 1.13 (d, 3H), 1.15-1.42 (m, ca. 3H), 1.68 (br. t, 2H), 2.32-2.46 (m, 1H), 2.64-2.85 (m, 3H), 2.96 (br. s, 1H), 3.07 (br. s, 1H), 6.97-7.08 (m, 1H), 7.15 (dd, 1H), 7.59 (dd, 1H), 9.76 (br. s, 1H). [α]_{D}²⁰ = +32.9°, c = 0.385, Methanol. |
| | 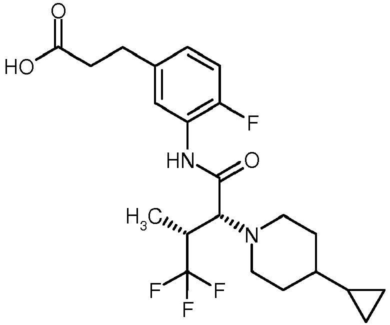 | |
| | aus (+)-*tert*.-Butyl-3-(4-fluor-3-{[(3*R*)-4,4,4-trifluor-D-valyl] amino }phenyl)propanoat und 3 -Cyclopropylpentandial | |
| **19** | 3-(3-{[(2*R*,3*R*)-2-(6-Azaspiro[2.5]oct-6-yl)-4,4,4-trifluor-3-methylbutanoyl]amino}-4-chlorphenyl)-propansäure | LC-MS (Methode 3): Rₜ = 1.36 min; m/z = 447 (M+H)⁺. ¹H-NMR (400 MHz, DMSO-*d₆*): δ [ppm] = 0.16-0.25 (m, 4H), 1.14 (d, 3H), 1.26-1.38 (m, 4H), 2.52-2.57 (m, ca. 4H), 2.60-2.72 (m, 2H), 2.81 (t, 2H), 3.01-3.14 (m, 1H), 3.56 (d, 1H), 7.09 (dd, 1H), 7.41 (d, 1H), 7.48 (d, 1H), 9.62 (s, 1H), 12.16 (br. s, 1H). [α]_{D}²⁰ = -7.8°, c = 0.370, Chloroform. |
| | 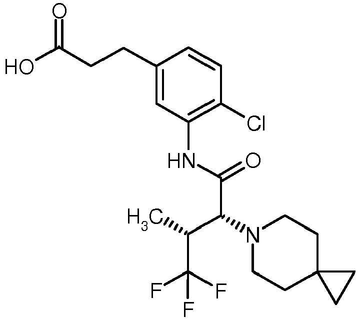 | |
| | aus (+)-*tert*.-Butyl-3-(4-chlor-3-{[(*3R*)-4,4,4-trifluor-D-valyl]amino}phenyl)propanoat und 2,2'-Cyclopropan-1,1-diyldiacetaldehyd | |
| **20** | (3S)-3-(4-Chlor-3-{[4,4,4-trifluor-3-methyl-2-(4-methylpiperidin-1-yl)butanoyl]amino}phenyl)-butansäure *(Diastereomerengemisch 1*) | LC-MS (Methode 2): Rₜ = 1.25 min; m/z = 449 (M+H)⁺. |
| | 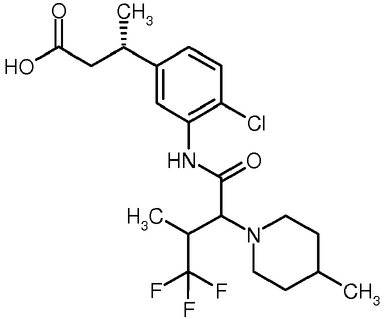 | |
| | aus *tert*.-Butyl-(3*S*)-3-{4-chlor-3-[(4,4,4-trifluorvalyl)amino]phenyl}butanoat (*Diastereomerengemisch 1*) und 3-Methylpentandial | |
| **21** | (3*S*)-3-(3-{[2-(6-Azaspiro[2.5]oct-6-yl)-4,4,4-trifluor-3-methylbutanoyl]amino}-4-chlorphenyl)-butansäure (*Diastereomerengemisch 1*) | LC-MS (Methode 2): Rₜ = 1.28 min; m/z = 479 (M+H)⁺. ¹H-NMR (400 MHz, DMSO-*d₆*): δ [ppm] = 0.20 (s, 4H), 1.14 (d, 3H), 1.20 (d, 3H), 1.28-1.40 (m, 4H), 2.52-2.72 (m, 4H), 3.04-3.15 (m, 2H), 3.57 (d, 1H), 6.12 (d, 1H), 7.42 (d, 1H), 7.48 (s, 1H), 9.67 (s, 1H), 12.20 (br. s, 1H). |
| | 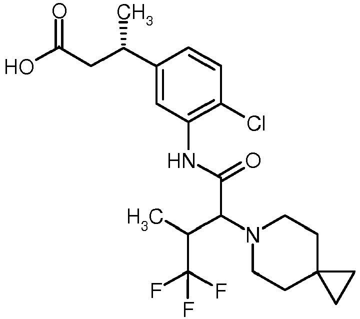 | |
| | aus *tert*.-Butyl-(3*S*)-3-{4-chlor-3-[(4,4,4-trifluorvalyl)amino]phenyl}butanoat (*Diastereomerengemisch 1*) und 2,2'-Cyclopropan-1,1-diyl-diacetaldehyd | |
| **22** | (+)-3-(4-Fluor-3-{[(2*R*,3*R*)-4,4,4-trifluor-3-methyl-2-(4-phenylpiperidin-1-yl)butanoyl]amino}phenyl)-propansäure | LC-MS (Methode 2): Rₜ = 1.22 min; m/z = 481 (M+H)⁺. ¹H-NMR (400 MHz, DMSO-*d₆*): δ [ppm] = 1.16 (d, 3H), 1.50-1.60 (m, 1H), 1.62-1.85 (m, 3H), 2.19 (t, 1H), 2.40-2.49 (m, 1H), 2.55-2.68 (m, ca. 3H), 2.75-2.83 (m, 3H), 2.98-3.15 (m, 2H), 3.57 (d, 1H), 6.99-7.09 (m, 1H), 7.12-7.34 (m, 6H), 7.66 (dd, 1H), 9.78 (s, 1H), 12.18 (br. s, ca. 1H). [α]_{D}²⁰ = +49.3°, c = 0.535, Chloroform. |
| | 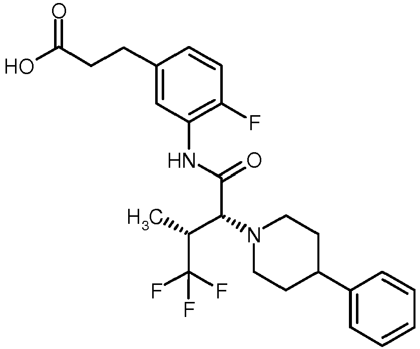 | |
| | aus (+)-*tert*.-Butyl-3-(4-fluor-3-{[(3*R*)-4,4,4-trifluor-D-valyl]amino}phenyl)propanoat und 3-Phenylpentandial | |
| **23** | (-)-(3*S*)-3-[4-Chlor-3-({(2*R*,3*R*)-4,4,4-trifluor-3-methyl-2-[4-(trifluormethyl)piperidin-1-yl]-butanoyl}amino)phenyl]butansäure | LC-MS (Methode 3): Rₜ = 1.42 min; m/z = 503 (M+H)⁺. ¹H-NMR (400 MHz, DMSO-*d₆*): δ [ppm] = 1.11 (d, 3H), 1.19 (d, 3H), 1.28-1.41 (m, 1H), 1.44-1.58 (m, 1H), 1.71-1.89 (m, 2H), 2.09-2.19 (m, 1H), 2.21-2.38 (m, 2H), 2.56-2.68 (m, 1H), 2.79 (d, 1H), 3.00-3.21 (m, 4H), 3.57 (d, 1H), 7.13 (dd, 1H), 7.42 (d, 1H), 7.48 (d, 1H), 9.68 (s, 1H). [α]_{D}²⁰ = -14.5°, c = 0.370, Chloroform. |
| | 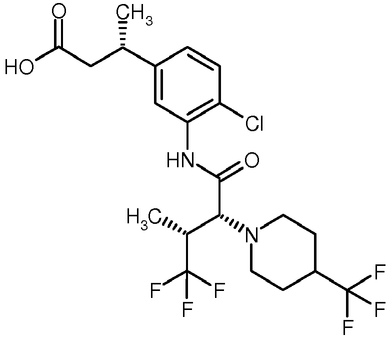 | |
| | aus *tert*.-Butyl-(3*S*)-3-(4-chlor-3-{[(3*R*)-4,4,4-trifluor-D-valyl] amino }phenyl)butanoat und 3 -(Trifluormethyl)pentandial | |
| **24** | (+)-3-[4-Fluor-3-({(2*R*,3*R*)-4,4,4-trifluor-3-methyl-2-[4-(trifluormethyl)piperidin-1-yl]butanoyl}-amino)phenyl]propansäure | LC-MS (Methode 2): Rₜ = 1.17 min; m/z = 473 (M+H)⁺. ¹H-NMR (400 MHz, DMSO-*d₆*): δ [ppm] = 1.10 (d, 3H), 1.34 (qd, 1H), 1.48 (qd, 1H), 1.79 (t, 2H), 2.03-2.13 (m, 1H), 2.19-2.34 (m, 1H), 2.42-2.48 (m, 1H), 2.74-2.83 (m, 3H), 2.98-3.11 (m, 2H), 3.55 (d, 1H), 6.97-7.07 (m, 1H), 7.16 (dd, 1H), 7.64 (dd, 1H), 9.80 (s, 1H). |
| | 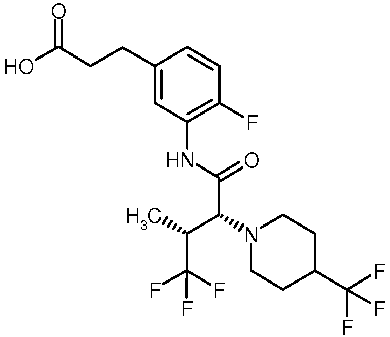 | |
| | aus (+)-*tert*.-Butyl-3-(4-fluor-3-{[(3*R*)-4,4,4-trifluor-D-valyl] amino }phenyl)propanoat und 3 -(Trifluormethyl)pentandial | [α]_{D}²⁰ = +27.8°, c = 0.500, Chloroform. |

### Beispiel 25

### 1-(3-{[(2R,3R)-2-(6-Azaspiro[2.5]oct-6-yl)-4,4,4-trifluor-3-methylbutanoyl]amino}-4-chlorbenzyl)cyclopropancarbonsäure

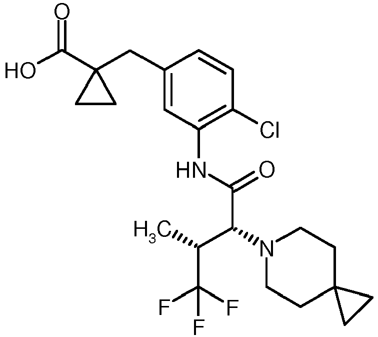

Die Titelverbindung wurde gemäß Allgemeiner Vorschrift 4 ausgehend von (+)-*tert*.-Butyl-1-(4-chlor-3-{[(3*R*)-4,4,4-trifluor-D-valyl]amino}benzyl)cyclopropancarboxylat und 2,2'-Cyclopropan-1,1-diyldiacetaldehyd erhalten. Ausbeute: 111.7 mg (63.8% d. Th.).

LC-MS (Methode 2): Rₜ = 1.25 min; m/z = 473 (M+H)⁺.

¹H-NMR (400 MHz, DMSO-*d₆*): δ [ppm] = 0.21 (s, 4H), 0.82 (q, 2H), 1.12-1.17 (m, 5H), 1.31 (br. s, 4H), 2.52-2.59 (m, 2H), 2.61-2.70 (m, 2H), 2.80-2.94 (m, 2H), 2.98-3.15 (m, 1H), 3.53 (d, 1H), 7.11 (dd, 1H), 7.39 (d, 1H), 7.50 (d, 1H), 9.61 (s, 1H).

### Beispiel 26

### 1-(4-Chlor-3-{[(2R,3R)-2-(4-ethylpiperidin-1-yl)-4,4,4-trifluor-3-methylbutanoyl]amino}benzyl)-cyclopropancarbonsäure

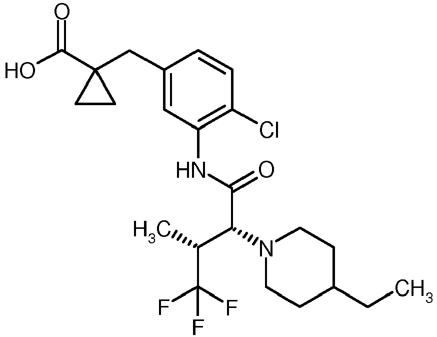

Die Titelverbindung wurde gemäß Allgemeiner Vorschrift 4 ausgehend von (+)-*tert*.-Butyl-1-(4-chlor-3-{[(3*R*)-4,4,4-trifluor-D-valyl]amino}benzyl)cyclopropancarboxylat und 3-Ethylpentandial erhalten. Ausbeute: 33 mg (39.7% d. Th.).

LC-MS (Methode 2): Rₜ = 1.31 min; m/z = 475 (M+H)⁺.

¹H-NMR (400 MHz, DMSO-*d₆*): δ [ppm] = 0.76-0.88 (m, 5H), 0.95-1.26 (m, ca. 10H), 1.64 (t, 2H), 2.06 (t, 1H), 2.52-2.60 (m, ca. 1H), 2.63-2.72 (m, 1H), 2.86 (s, 2H), 2.90-2.97 (m, 1H), 2.99-3.10 (m, 1H), 3.50 (d, 1H), 7.11 (dd, 1H), 7.39 (d, 1H), 7.43-7.54 (m, 1H), 9.60 (s, 1H).

### Allgemeine Vorschrift 5: Spaltung von Ethylestern zu den entsprechenden Carbonsäuren mit einem Gemisch von Salzsäure oder Schwefelsäure mit Essigsäure

Eine Lösung des betreffenden Ethylesters in einem Gemisch (ca. 1:1 bis 3:1) aus Essigsäure und konzentrierter Salzsäure oder aus Essigsäure und 10%-iger, 20%-iger oder halbkonzentrierter Schwefelsäure wird bei Temperaturen von 80°C bis 140°C (gegebenenfalls unter Rückfluss) für 30 min bis zu 12 h gerührt. Nach dem Abkühlen wird die Reaktionsmischung entweder direkt im Vakuum eingeengt oder auf Wasser gegeben. Die wässrige Phase wird dann mit Natronlauge annähernd neutralisiert und mit Ethylacetat oder Dichlormethan extrahiert, und die vereinigten organischen Phasen werden im Vakuum eingeengt. Der Rückstand wird in Ethylacetat aufgenommen und mit Wasser (das durch Zusatz von wenig ges. Natriumhydrogencarbonat-Lösung annähernd neutral (pH 6-7.5) gestellt ist) gewaschen. Nach Einengen im Vakuum kann das Rohprodukt durch präparative RP-HPLC (Eluent Acetonitril/Wasser- oder Methanol/Wasser-Gradient), durch Kristallisation aus Acetonitril oder Wasser/Acetonitril-Gemischen oder durch eine Kombination dieser Methoden gereinigt werden.

Die folgenden Beispiele wurden gemäß der Allgemeinen Vorschrift 5 hergestellt:

| **Beispiel** | **Name / Struktur / Edukt** | **Analytische Daten** |
|---|---|---|
| **27** | (+)-(2*S*)-3-(4-Chlor-3-{[(2*R*,3*R*)-4,4,4-trifluor-3-methyl-2-(4-methylpiperidin-1-yl)butanoyl]amino} - phenyl)-2-methylpropansäure | LC-MS (Methode 2): Rₜ = 1.25 min; m/z = 449 (M+H)⁺. ¹H-NMR (400 MHz, DMSO-*d₆*): δ [ppm] = 0.87 (d, 3H), 0.97-1.07 (m, 4H), 1.11 (d, 3H), 1.15-1.36 (m, 2H), 1.54-1.63 (m, 2H), 1.97-2.13 (m, 1H), 2.54-2.69 (m, 4H), 2.82-2.95 (m, 2H), 2.99-3.09 (m, 1H), 3.51 (d, 1H), 7.04 (dd, 1H), 7.40 (d, 1H), 7.43 (d, 1H), 9.60 (s, 1H), 12.17 (br. s, 1H). |
| | 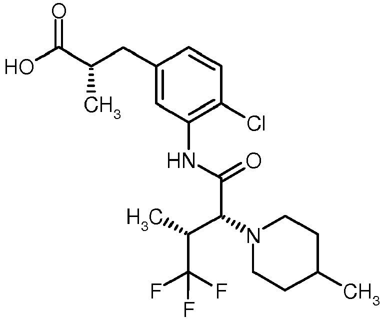 | |
| | aus Ethyl-(2S)-3-(4-chlor-3-{[(2*R*,3*R*)-4,4,4-trifluor-3-methyl-2-(4-methylpiperidin-1-yl)butanoyl]-amino }phenyl)-2-methylpropanoat | [α]_{D}²⁰ = +11.9°, c = 0.340, Chloroform. |
| **28** | (2*S*)-3-(3-{[(2*R*,3*R*)-2-(6-Azaspiro[2.5]oct-6-yl)-4,4,4-trifluor-3-methylbutanoyl]amino}-4-fluorphenyl)-2-methylpropansäure | LC-MS (Methode 3): Rₜ = 1.32 min; m/z = 445 (M+H)⁺. ¹H-NMR (400 MHz, DMSO-*d₆*): δ [ppm] = 0.16-0.26 (m, 4H), 1.05 (d, 3H), 1.13 (d, 3H), 1.30 (br. s, 4H), 2.53-2.64 (m, ca. 6H, verdeckt), 2.80-2.91 (m, 1H), 3.00-3.10 (m, 1H), 3.54 (d, 1H), 7.00 (td, 1H), 7.17 (dd, 1H), 7.61 (dd, 1H), 9.74 (s, 1H), 12.18 (br. s, 1H). |
| | 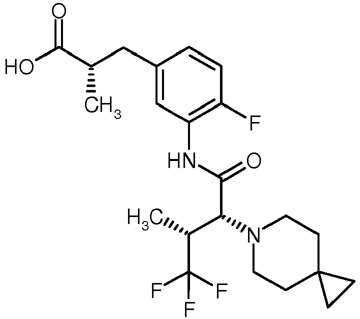 | |
| | aus Ethyl-(2*S*)-3-(3-{[(2*R*,3*R*)-2-(6-azaspiro[2.5]oct-6-yl)-4,4,4-trifluor-3-methylbutanoyl]amino}-4-fluorphenyl)-2-methylpropanoat | |
| **29** | (-)-(2*R*)-3-(3-{[(2*R*,3*R*)-2-(6-Azaspiro[2.5]oct-6-yl)-4,4,4-trifluor-3-methylbutanoyl]amino}-4-chlor-phenyl)-2-methylpropansäure | LC-MS (Methode 2): Rₜ = 1.26 min; m/z = 461 (M+H)⁺. ¹H-NMR (400 MHz, DMSO-*d₆*): δ [ppm] = 0.21 (br. s, 4H), 1.05 (d, 3H), 1.14 (d, 3H), 1.32 (br. s, 4H), 2.55-2.70 (m, ca. 6H), 2.80-2.94 (m, 1H), 3.02-3.12 (m, 1H), 3.55 (d, 1H), 7.05 (dd, 1H), 7.29-7.49 (m, 2H), 9.63 (s, 1H). [α]_{D}²⁰ = -21.6°, c = 0.405, Chloroform. |
| | 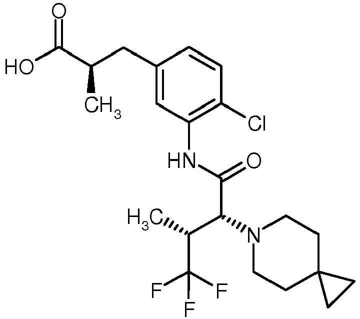 | |
| | aus Ethyl-(2*R*)-3-(3-{[(2*R*,3*R*)-2-(6-azaspiro[2.5]oct-6-yl)-4,4,4-trifluor-3-methylbutanoyl]amino}-4-chlorphenyl)-2-methylpropanoat | |
| **30** | (+)-(2*R*)-3-(4-Fluor-3-{[(2*R*,3*R*)-4,4,4-trifluor-3-methyl-2-(4-methylpiperidin-1-yl)butanoyl]amino}-phenyl)-2-methylpropansäure | LC-MS (Methode 2): Rₜ = 1.17 min; m/z = 433 (M+H)⁺. ¹H-NMR (400 MHz, DMSO-*d₆*): δ [ppm] = 0.86 (d, 3H), 0.96-1.07 (m, 4H), 1.09 (d, 3H), 1.12-1.21 (m, 1H), 1.23-1.33 (m, 1H), 1.57 (t, 2H), 2.02 (t, 1H), 2.37-2.45 (m, 1H), 2.55-2.69 (m, ca. 3H), 2.80-2.93 (m, 2H), 2.97-3.08 (m, 1H), 3.49 (d, 1H), 6.93-7.03 (m, 1H), 7.15 (dd, 1H), 7.57 (dd, 1H), 9.71 (s, 1H). [α]_{D}²⁰ = +9.2°, c = 0.450, Chloroform. |
| | 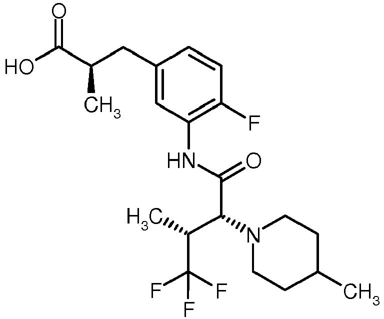 | |
| | aus Ethyl-(2*R*)-3-(4-fluor-3-{[(2*R*,3*R*)-4,4,4-trifluor-3-methyl-2-(4-methylpiperidin-1-yl)butanoyl]-amino }phenyl)-2-methylpropanoat | |
| **31** | (-)-(2*R*)-3-(4-Chlor-3-{[(2*R*,3*R*)-4,4,4-trifluor-3-methyl-2-(4-methylpiperidin-1-yl)butanoyl]amino} - phenyl)-2-methylpropansäure | LC-MS (Methode 4): Rₜ = 2.54 min; m/z = 449 (M+H)⁺. ¹H-NMR (400 MHz, DMSO-*d₆*): δ [ppm] = 0.87 (d, 3H), 0.95-1.07 (m, 4H), 1.10 (d, 3H), 1.13-1.36 (m, 2H), 1.51-1.65 (m, 2H), 2.06 (t, 1H), 2.56-2.68 (m, 4H), 2.81-2.95 (m, 2H), 2.99-3.09 (m, 1H), 3.51 (d, 1H), 7.04 (d, 1H), 7.35-7.52 (m, 2H), 9.61 (s, 1H), 12.19 (br. s, ca. 1H). [α]_{D}²⁰ = -23.6°, c = 0.425, Chloroform. |
| | 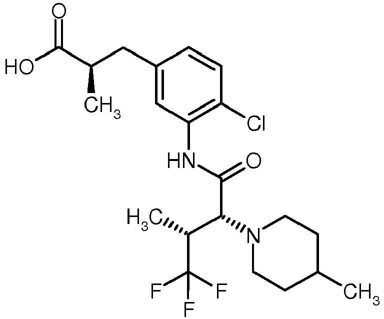 | |
| | aus Ethyl-(2*R*)-3-(4-chlor-3-{[(2*R*,3*R*)-4,4,4-trifluor-3-methyl-2-(4-methylpiperidin-1-yl)butanoyl]-amino}phenyl)-2-methylpropanoat | |
| **32** | (+)-(2*S*)-3-(4-Fluor-3-{[(2*R*,3*R*)-4,4,4-trifluor-3-methyl-2-(4-methylpiperidin-1-yl)butanoyl]amino} - phenyl)-2-methylpropansäure | LC-MS (Methode 2): Rₜ = 1.13 min; m/z = 433 (M+H)⁺. ¹H-NMR (400 MHz, DMSO-*d₆*): δ [ppm] = 0.87 (d, 3H), 0.97-1.02 (m, 1H), 1.04 (d, 3H), 1.09 (d, 3H), 1.12-1.21 (m, 1H), 1.21-1.35 (m, 1H), 1.58 (t, 2H), 1.98-2.08 (m, 1H), 2.35-2.47 (m, 1H), 2.55-2.70 (m, 3H), 2.80-2.93 (m, 2H), 2.95-3.11 (m, 1H), 3.49 (d, 1H), 6.95-7.04 (m, 1H), 7.15 (dd, 1H), 7.57 (dd, 1H), 9.72 (s, 1H), 12.17 (br. s, 1H). [α]_{D}²⁰ = +42.6°, c = 0.430, Chloroform. |
| | 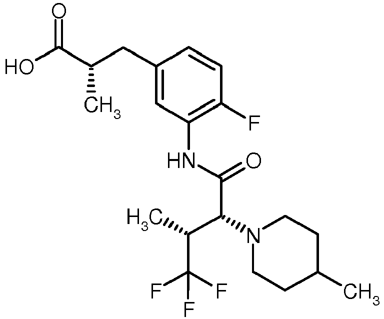 | |
| | aus Ethyl-(2*S*)-3-(4-fluor-3-{[(2*R*,3*R*)-4,4,4-trifluor-3-methyl-2-(4-methylpiperidin-1-yl)butanoyl]-amino }phenyl)-2-methylpropanoat | |
| **33** | (+)-(2*R*)-3-(3-{[(2*R*,3*R*)-2-(6-Azaspiro[2.5]oct-6-yl)-4,4,4-trifluor-3-methylbutanoyl]amino}-4-fluor-phenyl)-2-methylpropansäure | LC-MS (Methode 2): Rₜ = 1.20 min; m/z = 445 (M+H)⁺. ¹H-NMR (400 MHz, DMSO-*d₆*): δ [ppm] = 0.21 (s, 4H), 1.05 (d, 3H), 1.13 (d, 3H), 1.30 (br. s, 4H), 2.50-2.62 (m, ca. 6H, verdeckt), 2.80-2.92 (m, 1H), 3.00-3.10 (m, 1H), 3.54 (d, 1H), 6.94-7.06 (m, 1H), 7.17 (dd, 1H), 7.61 (dd, 1H), 9.74 (s, 1H), 12.17 (br. s, 1H). [α]_{D}²⁰ = +5.0°, c = 0.450, Chloroform. |
| | 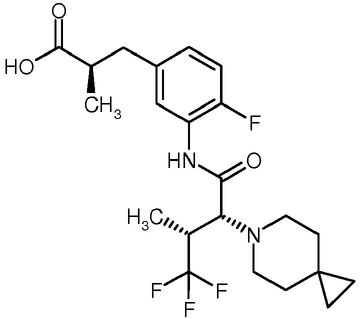 | |
| | aus Ethyl-(2*R*)-3-(3-{[(2*R*,3*R*)-2-(6-azaspiro[2.5]oct-6-yl)-4,4,4-trifluor-3-methylbutanoyl]amino}-4-fluorphenyl)-2-methylpropanoat | |
| **34** | (+)-(2*S*)-3-[4-Chlor-3-({(2*R*,3*R*)-4,4,4-trifluor-3-methyl-2-[4-(trifluormethyl)piperidin-1-yl]-butanoyl}amino)phenyl]-2-methylpropansäure | LC-MS (Methode 3): Rₜ = 1.43 min; m/z = 503 (M+H)⁺. ¹H-NMR (400 MHz, DMSO-*d₆*): δ [ppm] = 1.04 (d, 3H), 1.11 (d, 3H), 1.24-1.41 (m, 1H), 1.42-1.61 (m, 1H), 1.70-1.86 (m, 2H), 2.05-2.18 (m, 1H), 2.22-2.35 (m, 1H), 2.52-2.74 (m, ca. 3H), 2.75-2.92 (m, 2H), 3.00-3.11 (m, 2H), 3.56 (d, 1H), 7.05 (dd, 1H), 7.36-7.53 (m, 2H), 9.68 (s, 1H), 12.26 (br. s, ca. 1H). [α]_{D}²⁰ = + 12.7°, c = 0.350, Chloroform. |
| | 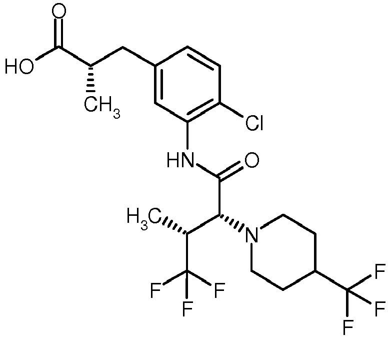 | |
| | aus Ethyl-(2*S*)-3-[4-chlor-3-({(2*R*,3*R*)-4,4,4-trifluor-3-methyl-2-[4-(trifluormethyl)piperidin-1-yl]-butanoyl}amino)phenyl]-2-methylpropanoat | |

### Beispiel 35

### (+)-1-(4-Fluor-3-{[4,4,4-trifluor-3-methyl-2-(piperidin-1-yl)butanoyl]amino}benzyl)cyclopropancarbonsäure (Enantiomer 2)

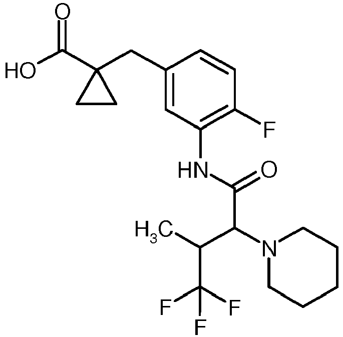

Das oben erhaltene racemische Gemisch der 1-(4-Fluor-3-{[4,4,4-trifluor-3-methyl-2-(piperidin-1-yl)butanoyl]amino}benzyl)cyclopropancarbonsäure (Beispiel 2, racemisches Diastereomer 1) wurde durch präparative HPLC an chiraler Phase weiter aufgetrennt [Säule: Daicel Chiralpak AD-H, 5 µm, 250 mm x 20 mm; Injektionsvolumen: 0.50 ml; Temperatur: 40°C; Eluent: 75% Isohexan / 25% Isopropanol (+ 0.2% TFA + 1% H₂O); Fluss: 15 ml/min; Detektion: 220 nm]. Ausgehend von 290 mg Gemisch wurden 127 mg der Titelverbindung als Enantiomer 2 isoliert.

LC-MS (Methode 2): Rₜ = 1.09 min; m/z = 431 (M+H)⁺.

¹H-NMR (400 MHz, DMSO-*d₆*): δ [ppm] = 0.76-0.85 (m, 2H), 1.07-1.14 (m, 5H), 1.33-1.56 (m, 6H), 2.38-2.48 (m, 2H), 2.84 (s, 2H), 2.97-3.13 (m, 1H), 3.45 (d, 1H), 7.02-7.09 (m, 1H), 7.14 (dd, 1H), 7.63 (dd, 1H), 9.70 (s, 1H), 12.14 (br. s, 1H).

[α]_{D}²⁰ = +21.2°, c = 0.550, Chloroform.

### Beispiel 36

### (+)-1-(4-Fluor-3-{[4,4,4-trifluor-3-methyl-2-(4-methylpiperidin-1-yl)butanoyl]amino}benzyl)-cyclopropancarbonsäure (Enantiomer 2)

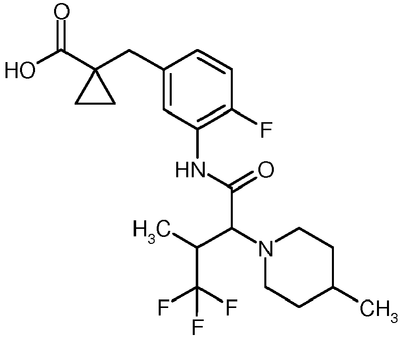

Das oben erhaltene racemische Gemisch der 1-(4-Fluor-3-{[4,4,4-trifluor-3-methyl-2-(4-methyl-piperidin-1-yl)butanoyl]amino}benzyl)cyclopropancarbonsäure (Beispiel 4, racemisches Diastereomer 1) wurde durch präparative HPLC an chiraler Phase weiter aufgetrennt [Säule: Daicel Chiralpak AD-H, 5 µm, 250 mm x 20 mm; Injektionsvolumen: 0.30 ml; Temperatur: 40°C; Eluent: 75% Isohexan / 25% Isopropanol (+ 0.2% TFA + 1% H₂O); Fluss: 15 ml/min; Detektion: 220 nm]. Ausgehend von 400 mg Gemisch wurden 271 mg der Titelverbindung (Enantiomer 2) als Trifluoressigsäure-Salz isoliert. Dieses Salz wurde in wenig Wasser/Acetonitril suspendiert, unter Rühren mit 1 N Natronlauge neutralisiert und dann nochmals durch präparative RP-HPLC gereinigt (Eluent: Acetonitril/Wasser-Gradient). Es wurden so 60 mg der Titelverbindung erhalten.

LC-MS (Methode 2): Rₜ = 1.18 min; m/z = 445 (M+H)⁺.

¹H-NMR (400 MHz, DMSO-*d₆*): δ [ppm] = 0.74-0.83 (m, 2H), 0.87 (d, 3H), 0.96-1.36 (m, 7H), 1.57 (t, 2H), 2.02 (t, 1H), 2.35-2.47 (m, 1H), 2.63 (d, 1H), 2.84 (s, 2H), 2.99 (d, 1H), 2.98-3.08 (m, 1H), 3.47 (d, 1H), 7.01-7.09 (m, 1H), 7.09-7.19 (m, 1H), 7.61 (dd, 1H), 9.70 (s, 1H).

[α]_{D}²⁰ = +22.7°, c = 0.460, Chloroform.

### Beispiel 37

### (+)-3-(4-Fluor-3-{[4,4,4-trifluor-3-methyl-2-(4-methylpiperidin-1-yl)butanoyl]amino}phenyl)-propansäure-Trifluoracetat (Enantiomer 2)

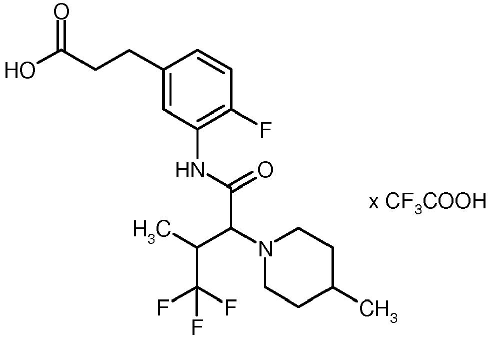

Das oben erhaltene racemische Gemisch der 3-(4-Fluor-3-{[4,4,4-trifluor-3-methyl-2-(4-methyl-piperidin-1-yl)butanoyl]amino}phenyl)propansäure (Beispiel 6, racemisches Diastereomer 2) wurde durch präparative HPLC an chiraler Phase weiter aufgetrennt [Säule: Daicel Chiralpak AD-H, 5 µm, 250 mm x 20 mm; Injektionsvolumen: 0.40 ml; Temperatur: 25°C; Eluent: 80% Isohexan / 20% Ethanol (+ 0.2% TFA + 1% H₂O); Fluss: 15 ml/min; Detektion: 220 nm]. Ausgehend von 58 mg Gemisch wurden 38 mg der Titelverbindung als Enantiomer 2 isoliert.

LC-MS (Methode 2): Rₜ = 0.95 min; m/z = 419 (M+H)⁺.

[α]_{D}²⁰ = +15.8°, c = 0.490, Chloroform.

### Beispiel 38 und Beispiel 39

### 1-(3- {[2-(4-Ethylpiperidin-1-yl)-4,4,4-trifluor-3-methylbutanoyl]amino}-4-fluorbenzyl)cyclopropancarbonsäure (Enantiomer 1 und 2)

Das oben erhaltene racemische Gemisch der 1-(3-{[2-(4-Ethylpiperidin-1-yl)-4,4,4-trifluor-3-methylbutanoyl]amino}-4-fluorbenzyl)cyclopropancarbonsäure (Beispiel 8, racemisches Diastereomer 1) wurde durch präparative HPLC an chiraler Phase weiter aufgetrennt [Säule: Daicel Chiralpak AD-H, 5 µm, 250 mm x 20 mm; Injektionsvolumen: 1.5 ml; Temperatur: RT; Eluent: 50% Isohexan / 50% Isopropanol; Fluss: 18 ml/min; Detektion: 230 nm]. Ausgehend von 275 mg Gemisch wurden 125 mg von Enantiomer 1 (*Beispiel 38*) und 122 mg von Enantiomer 2 (*Beispiel* 39) isoliert:

### Beispiel 38

### (-)-1-(3-{[(2S,3S)-2-(4-Ethylpiperidin-1-yl)-4,4,4-trifluor-3-methylbutanoyl]amino}-4-fluorbenzyl)cyclopropancarbonsäure (Enantiomer 1)

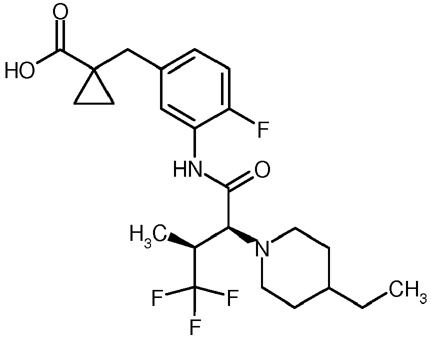

LC-MS (Methode 2): Rₜ = 1.25 min; m/z = 459 (M+H)⁺.

¹H-NMR (400 MHz, DMSO-*d₆*): δ [ppm] = 0.77-0.88 (m, ca. 5H), 0.97-1.26 (m, ca. 10H), 1.63 (t, 2H), 1.97-2.05 (m, 1H), 2.36-2.45 (m, 1H), 2.65 (d, 1H), 2.84 (s, 2H), 2.88-2.96 (m, 1H), 2.96-3.10 (m, 1H), 3.47 (d, 1H), 7.00-7.10 (m, 1H), 7.10-7.22 (m, 1H), 7.61 (dd, 1H), 9.68 (s, 1H), 12.12 (br. s, 1H).

[α]_{D}²⁰ = -19.5°, c = 0.545, Chloroform.

### Beispiel 39

### (+)-1-(3-{[(2R,3R)-2-(4-Ethylpiperidin-1-yl)-4,4,4-trifluor-3-methylbutanoyl]amino}-4-fluorbenzyl)cyclopropancarbonsäure (Enantiomer 2)

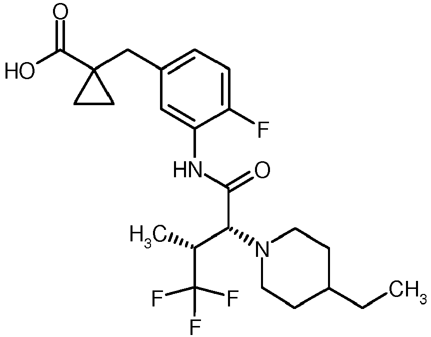

LC-MS (Methode 2): Rₜ = 1.25 min; m/z = 459 (M+H)⁺.

¹H-NMR (400 MHz, DMSO-*d₆*): δ [ppm] = 0.80-0.88 (m, ca. 5H), 0.97-1.26 (m, ca. 10H), 1.63 (t, 2H), 2.01 (t, 1H), 2.36-2.45 (m, 1H), 2.65 (d, 1H), 2.84 (s, 2H), 2.91 (d, 1H), 2.98-3.08 (m, 1H), 3.47 (d, 1H), 7.00-7.09 (m, 1H), 7.10-7.19 (m, 1H), 7.61 (dd, 1H), 9.68 (s, 1H), 12.12 (br. s, 1H).

[α]_{D}²⁰ = +20.3°, c = 0.555, Chloroform.

### Beispiel 40 und Beispiel 41

### 3-(3-{[2-(4-Ethylpiperidin-1-yl)-4,4,4-trifluor-3-methylbutanoyl]amino}-4-fluorphenyl)propansäure (Enantiomer 1 und 2)

Das oben erhaltene racemische Gemisch der 3-(3-{[2-(4-Ethylpiperidin-1-yl)-4,4,4-trifluor-3-methylbutanoyl]amino}-4-fluorphenyl)propansäure (Beispiel 9, racemisches Diastereomer 1) wurde durch präparative HPLC an chiraler Phase weiter aufgetrennt [Säule: Daicel Chiralpak AD-H, 5 µm, 250 mm x 20 mm; Injektionsvolumen: 1.5 ml; Temperatur: RT; Eluent: 60% Isohexan / 40% Isopropanol; Fluss: 18 ml/min; Detektion: 230 nm]. Ausgehend von 260 mg Gemisch wurden 101 mg von Enantiomer 1 (*Beispiel 40*) und 92 mg von Enantiomer 2 (*Beispiel 41*) isoliert:

### Beispiel 40

### (-)-3-(3-{[(2S,3S)-2-(4-Ethylpiperidin-1-yl)-4,4,4-trifluor-3-methylbutanoyl]amino}-4-fluorphenyl)propansäure (Enantiomere 1)

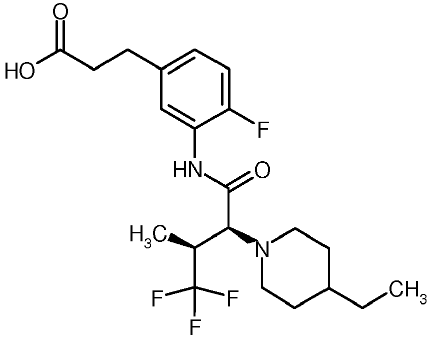

LC-MS (Methode 2): Rₜ = 1.18 min; m/z = 433 (M+H)⁺.

¹H-NMR (400 MHz, DMSO-*d₆*): δ [ppm] = 0.83 (t, 3H), 0.96-1.25 (m, ca. 8H), 1.63 (t, 2H), 1.96-2.09 (m, 1H), 2.35-2.46 (m, 1H), 2.65 (d, 1H), 2.79 (t, 2H), 2.87-2.94 (m, 1H), 2.97-3.08 (m, 1H), 3.49 (d, 1H), 6.95-7.08 (m, 1H), 7.15 (dd, 1H), 7.59 (dd, 1H), 9.70 (s, 1H).

[α]_{D}²⁰ = -20.8°, c = 0.460, Chloroform.

### Beispiel 41

### (+)-3-(3-{[(2R,3R)-2-(4-Ethylpiperidin-1-yl)-4,4,4-trifluor-3-methylbutanoyl]amino}-4-fluorphenyl)propansäure (Enantiomer 2)

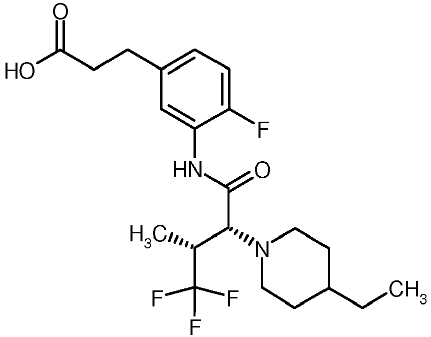

LC-MS (Methode 2): Rₜ = 1.18 min; m/z = 433 (M+H)⁺.

¹H-NMR (400 MHz, DMSO-*d₆*): δ [ppm] = 0.83 (t, 3H), 0.96-1.26 (m, ca. 8H), 1.63 (t, 2H), 1.97-2.06 (m, 1H), 2.36-2.45 (m, 1H), 2.65 (d, 1H), 2.75-2.83 (m, 2H), 2.88-2.95 (m, 1H), 2.97-3.08 (m, 1H), 3.49 (d, 1H), 6.98-7.07 (m, 1H), 7.15 (dd, 1H), 7.59 (dd, 1H), 9.70 (s, 1H), 12.05 (br. s, ca. 1H).

[α]_{D}²⁰ = +22.4°, c = 0.575, Chloroform.

### Beispiel 42 und Beispiel 43

### 3-(4-Chlor-3-{[4,4,4-trifluor-3-methyl-2-(4-methylpiperidin-1-yl)butanoyl]amino}phenyl)propansäure (Enantiomer 1 und 2)

Das oben erhaltene racemische Gemisch der 3-(4-Chlor-3-{[4,4,4-trifluor-3-methyl-2-(4-methyl-piperidin-1-yl)butanoyl]amino}phenyl)propansäure (Beispiel 13, racemisches Diastereomer 1) wurde durch präparative HPLC an chiraler Phase weiter aufgetrennt [Säule: Daicel Chiralpak AD-H, 5 µm, 250 mm x 20 mm; Injektionsvolumen: 1.0 ml; Temperatur: 30°C; Eluent: 70% Isohexan / 30% Isopropanol; Fluss: 15 ml/min; Detektion: 220 nm]. Ausgehend von 180 mg Gemisch wurden 77 mg von Enantiomer 1 (*Beispiel 42*) und 85 mg von Enantiomer 2 (*Beispiel 43*) isoliert:

### Beispiel 42

### 3-(4-Chlor-3-{[(2S,3S)-4,4,4-trifluor-3-methyl-2-(4-methylpiperidin-1-yl)butanoyl]amino}phenyl)-propansäure (Enantiomer 1)

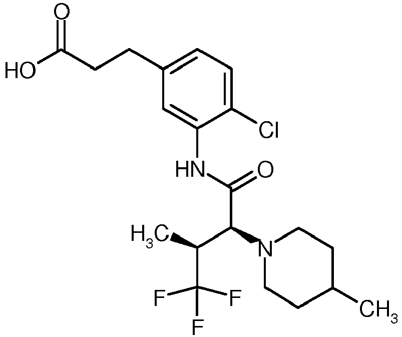

LC-MS (Methode 3): Rₜ = 1.33 min; m/z = 435 (M+H)⁺.

¹H-NMR (400 MHz, DMSO-*d₆*): δ [ppm] = 0.87 (d, 3H), 0.96-1.08 (m, 1H), 1.10 (d, 3H), 1.12-1.23 (m, 1H), 1.23-1.37 (m, 1H), 1.51-1.68 (m, 2H), 2.07 (t, 1H), 2.26 (t, 2H), 2.55-2.60 (m, ca. 1H), 2.64 (d, 1H), 2.73 (t, 2H), 2.90 (d, 1H), 2.97-3.12 (m, 1H), 3.50 (d, 1H), 7.06 (dd, 1H), 7.35 (d, 1H), 7.38-7.44 (m, 1H), 9.62 (s, 1H).

### Beispiel 43

### (+)-3-(4-Chlor-3-{[(2R,3R)-4,4,4-trifluor-3-methyl-2-(4-methylpiperidin-1-yl)butanoyl]amino}-phenyl)propansäure (Enantiomer 2)

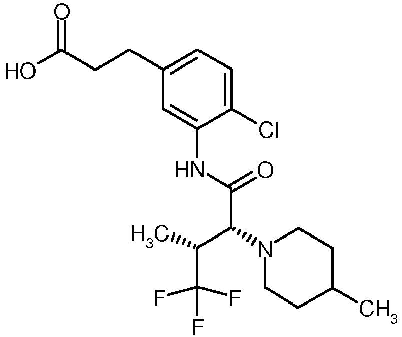

LC-MS (Methode 3): Rₜ = 1.33 min; m/z = 435 (M+H)⁺.

¹H-NMR (400 MHz, DMSO-*d₆*): δ [ppm] = 0.87 (d, 3H), 0.96-1.08 (m, 1H), 1.10 (d, 3H), 1.12-1.23 (m, 1H), 1.23-1.37 (m, 1H), 1.51-1.66 (m, 2H), 2.01-2.16 (m, 1H), 2.28 (t, 2H), 2.52-2.60 (m, ca. 1H), 2.64 (d, 1H), 2.74 (t, 2H), 2.91 (d, 1H), 2.99-3.09 (m, 1H), 3.50 (d, 1H), 7.06 (dd, 1H), 7.36 (d, 1H), 7.39-7.46 (m, 1H), 9.61 (s, 1H).

[α]_{D}²⁰ = +6.5°, c = 0.400, Chloroform.

### Beispiel 44 und Beispiel 45

### 3-(4-Chlor-3-{[2-(4-ethylpiperidin-1-yl)-4,4,4-trifluor-3-methylbutanoyl]amino}phenyl)propansäure (Enantiomer 1 und 2)

Das oben erhaltene racemische Gemisch der 3-(4-Chlor-3-{[2-(4-ethylpiperidin-1-yl)-4,4,4-trifluor-3-methylbutanoyl]amino}phenyl)propansäure (Beispiel 11, racemisches Diastereomer 1) wurde durch präparative HPLC an chiraler Phase weiter aufgetrennt [Säule: Daicel Chiralpak AD-H, 5 µm, 250 mm x 20 mm; Injektionsvolumen: 1.0 ml; Temperatur: 30°C; Eluent: 70% Isohexan / 30% Isopropanol; Fluss: 15 ml/min; Detektion: 220 nm]. Ausgehend von 151 mg Gemisch wurden 52.2 mg von Enantiomer 1 (*Beispiel 44*) und 72.2 mg von Enantiomer 2 (*Beispiel 45*) isoliert:

### Beispiel 44

### (+)-3-(4-Chlor-3-{[(2S,3S)-2-(4-ethylpiperidin-1-yl)-4,4,4-trifluor-3-methylbutanoyl]amino}-phenyl)propansäure (Enantiomere 1)

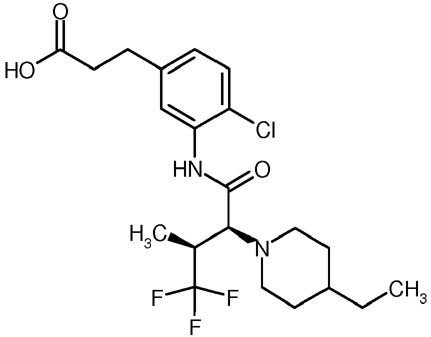

LC-MS (Methode 2): Rₜ = 1.26 min; m/z = 449 (M+H)⁺.

¹H-NMR (400 MHz, DMSO-*d₆*): δ [ppm] = 0.84 (t, 3H), 0.94-1.26 (m, 8H), 1.56-1.71 (m, 2H), 2.05 (t, 1H), 2.39-2.48 (m, 2H), 2.52-2.58 (m, ca. 1H), 2.66 (d, 1H), 2.78 (t, 2H), 2.94 (d, 1H), 2.98-3.10 (m, 1H), 3.51 (d, 1H), 7.08 (dd, 1H), 7.39 (d, 1H), 7.41-7.49 (m, 1H), 9.61 (s, 1H).

[α]_{D}²⁰ = +1.6°, c = 0.350, Chloroform.

### Beispiel 45

### (-)-3-(4-Chlor-3-{[(2R,3R)-2-(4-ethylpiperidin-1-yl)-4,4,4-trifluor-3-methylbutanoyl]amino}-phenyl)propansäure (Enantiomer 2)

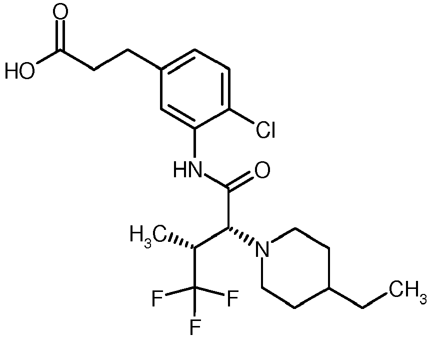

LC-MS (Methode 2): Rₜ = 1.26 min; m/z = 449 (M+H)⁺.

¹H-NMR (400 MHz, DMSO-*d₆*): δ [ppm] = 0.84 (t, 3H), 0.95-1.25 (m, 8H), 1.64 (dd, 2H), 2.00-2.11 (m, 1H), 2.42-2.48 (m, 2H), 2.52-2.58 (m, ca. 1H), 2.66 (d, 1H), 2.78 (t, 2H), 2.94 (d, 1H), 2.99-3.09 (m, 1H), 3.51 (d, 1H), 7.08 (dd, 1H), 7.39 (d, 1H), 7.44 (d, 1H), 9.61 (s, 1H).

[α]_{D}²⁰ = -0.7°, c = 0.340, Chloroform.

### Beispiel 46

### (+)-(2S)-3-(3-{[(2R,3R)-2-(6-Azaspiro[2.5]oct-6-yl)-4,4,4-trifluor-3-methylbutanoyl]amino}-4-chlorphenyl)-2-methylpropansäure (Diastereomer 2)

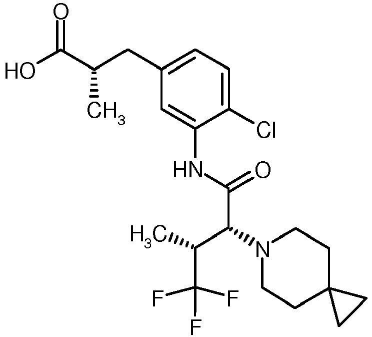

Ausgehend von Ethyl-(2*S*)-3-(3-{[2-(6-azaspiro[2.5]oct-6-yl)-4,4,4-trifluor-3-methylbutanoyl]-amino}-4-chlorphenyl)-2-methylpropanoat (Beispiel 82A) wurde nach Allgemeiner Vorschrift 5 die entsprechende Carbonsäure als Diastereomerengemisch hergestellt. Dieses Diastereomerengemisch wurde dann durch präparative HPLC an chiraler Phase weiter aufgetrennt [Säule: Daicel Chiralpak AD-H, 5 µm, 250 mm x 20 mm; Injektionsvolumen: 1.0 ml; Temperatur: RT; Eluent: 60% Isohexan / 40% Isopropanol; Fluss: 18 ml/min; Detektion: 230 nm]. Ausgehend von 348 mg Diastereomerengemisch wurden 160 mg von Diastereomer 2 isoliert, das mittels präparativer RP-HPLC (Eluent: Acetonitril/Wasser-Gradient) weiter aufgereinigt wurde. Es wurden so 113 mg der reinen Titelverbindung erhalten.

LC-MS (Methode 2): Rₜ = 1.27 min; m/z = 461 (M+H)⁺.

¹H-NMR (400 MHz, DMSO-*d₆*): δ [ppm] = 0.21 (s, 4H), 1.05 (d, 3H), 1.14 (d, 3H), 1.32 (br. s, 4H), 2.55-2.71 (m, ca. 6H), 2.83-2.93 (m, 1H), 3.01-3.14 (m, 1H), 3.55 (d, 1H), 7.05 (dd, 1H), 7.36-7.48 (m, 2H), 9.62 (s, 1H), 12.19 (br. s, 1H).

[α]_{D}²⁰ = +14.0°, c = 0.405, Chloroform.

### Beispiel 47

### (+)-(3R)-3-(3-{[(2R,3R)-2-(6-Azaspiro[2.5]oct-6-yl)-4,4,4-trifluor-3-methylbutanoyl]amino}-4-chlorphenyl)butansäure (Diastereomer 2)

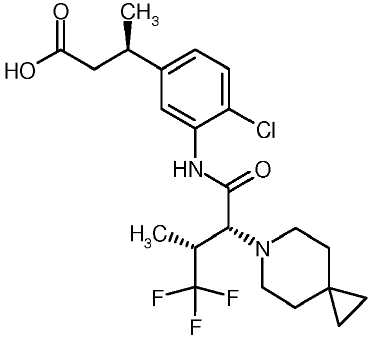

Das gemäß Allgemeiner Arbeitsvorschrift 4 aus *tert*.-Butyl-(3*R*)-3-{4-chlor-3-[(4,4,4-trifluorvalyl)-amino]phenyl}butanoat (Beispiel 62A) und 2,2'-Cyclopropan-1,1-diyldiacetaldehyd hergestellte Diastereomerengemisch von (3*R*)-3-(3-{[(2*R*,3*R*)-2-(6-Azaspiro[2.5]oct-6-yl)-4,4,4-trifluor-3-methylbutanoyl]amino}-4-chlorphenyl)butansäure und (3*R*)-3-(3-{[(2*S*,3*S*)-2-(6-Azaspiro[2.5]oct-6-yl)-4,4,4-trifluor-3-methylbutanoyl]amino}-4-chlorphenyl)butansäure wurde durch präparative HPLC an chiraler Phase aufgetrennt [Säule: Daicel Chiralpak AD-H, 5 µm, 250 mm x 20 mm; Injektionsvolumen: 0.75 ml; Temperatur: RT; Eluent: 70% Isohexan / 30% Isopropanol; Fluss: 18 ml/min; Detektion: 230 nm]. Ausgehend von 209 mg Diastereomerengemisch wurden 76 mg der Titelverbindung als Diastereomer 2 isoliert.

LC-MS (Methode 2): Rₜ = 1.25 min; m/z = 461 (M+H)⁺.

¹H-NMR (400 MHz, DMSO-*d₆*): δ [ppm] = 0.21 (s, 4H), 1.14 (d, 3H), 1.20 (d, 3H), 1.24-1.40 (m, 4H), 2.52-2.60 (m, ca. 4H, verdeckt), 2.62-2.70 (m, 2H), 3.03-3.19 (m, 2H), 3.56 (d, 1H), 7.12 (dd, 1H), 7.42 (d, 1H), 7.49 (d, 1H), 9.63 (s, 1H).

[α]_{D}²⁰ = +5.7°, c = 0.363, Chloroform.

### Beispiel 48

### (+)-(3R)-3-(4-Chlor-3-{[(2R,3R)-4,4,4-trifluor-3-methyl-2-(4-methylpiperidin-1-yl)butanoyl]-amino}phenyl)butansäure (Diastereomere 2)

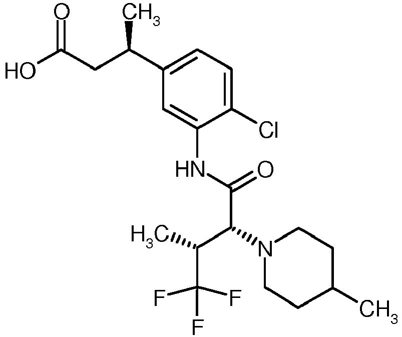

Das gemäß Allgemeiner Arbeitsvorschrift 4 aus *tert*.-Butyl-(3*R*)-3-{4-chlor-3-[(4,4,4-trifluorvalyl)-amino]phenyl}butanoat (Beispiel 62A) und 3-Methylpentandial hergestellte Diastereomerengemisch von (3*R*)-3-(4-Chlor-3-{[(2*R*,3*R*)-4,4,4-trifluor-3-methyl-2-(4-methylpiperidin-1-yl)-butanoyl]amino}phenyl)butansäure und (3*R*)-3-(4-Chlor-3-{[(2*S*,3*S*)-4,4,4-trifluor-3-methyl-2-(4-methylpiperidin-1-yl)butanoyl]amino}phenyl)butansäure wurde durch präparative HPLC an chiraler Phase aufgetrennt [Säule: Daicel Chiralpak AD-H, 5 µm, 250 mm x 20 mm; Injektionsvolumen: 0.75 ml; Temperatur: RT; Eluent: 70% Isohexan / 30% Isopropanol; Fluss: 20 ml/min; Detektion: 230 nm]. Ausgehend von 195 mg Diastereomerengemisch wurden 92 mg der Titelverbindung als Diastereomer 2 isoliert.

LC-MS (Methode 5): Rₜ = 2.47 min; m/z = 449 (M+H)⁺.

¹H-NMR (400 MHz, DMSO-*d₆*): δ [ppm] = 0.87 (d, 3H), 0.96-1.08 (m, 1H), 1.11 (d, 3H), 1.15-1.25 (m, 1H), 1.19 (d, 3H), 1.25-1.38 (m, 1H), 1.59 (t, 2H), 2.00-2.13 (m, 1H), 2.53-2.60 (m, ca. 1H), 2.65 (d, 1H), 2.87-2.95 (m, 1H), 2.99-3.18 (m, 2H), 3.52 (d, 1H), 7.12 (dd, 1H), 7.41 (d, 1H), 7.47 (d, 1H), 9.60 (s, 1H).

[α]_{D}²⁰ =+7.8°, c = 0.380, Chloroform.

### Beispiel 49

### (-)-(3S)-3-(4-Chlor-3-{[(2R,3R)-4,4,4-trifluor-3-methyl-2-(4-methylpiperidin-1-yl)butanoyl]-amino}phenyl)butansäure (Diastereomer 1)

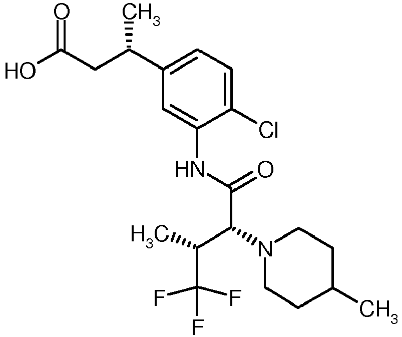

Das oben erhaltene Gemisch von (3*S*)-3-(4-Chlor-3-{[(2*R*,3*R*)-4,4,4-trifluor-3-methyl-2-(4-methyl-piperidin-1-yl)butanoyl]amino}phenyl)butansäure und (3*S*)-3-(4-Chlor-3-{[(2*S*,3*S*)-4,4,4-trifluor-3-methyl-2-(4-methylpiperidin-1-yl)butanoyl]amino}phenyl)butansäure (Beispiel 20, Diastereomerengemisch 1) wurde durch präparative HPLC an chiraler Phase aufgetrennt [Säule: Daicel Chiralpak AS-H, 5 µm, 250 mm x 20 mm; Injektionsvolumen: 1.2 ml; Temperatur: RT; Eluent: 70% Isohexan / 30% Isopropanol; Fluss: 20 ml/min; Detektion: 230 nm]. Ausgehend von 182 mg Diastereomerengemisch wurden 77 mg der Titelverbindung als Diastereomer 1 isoliert.

LC-MS (Methode 4): Rₜ = 2.53 min; m/z = 449 (M+H)⁺.

¹H-NMR (400 MHz, DMSO-*d₆*): δ [ppm] = 0.87 (d, 3H), 1.03-1.24 (m, 8H), 1.24-1.35 (m, 1H), 1.58 (t, 2H), 2.01-2.11 (m, 1H), 2.54-2.60 (m, ca. 1H), 2.65 (d, 1H), 2.86-2.96 (m, 1H), 2.98-3.18 (m, 2H), 3.52 (d, 1H), 7.12 (dd, 1H), 7.42 (d, 1H), 7.46 (d, 1H), 9.62 (s, 1H), 12.10 (br. s, 1H).

[α]_{D}²⁰ = -22.1°, c = 0.360, Chloroform.

### Beispiel 50

### (-)-(3S)-3-(3-{[(2R,3R)-2-(6-Azaspiro[2.5]oct-6-yl)-4,4,4-trifluor-3-methylbutanoyl]amino}-4-chlorphenyl)butansäure (Diastereomer 1)

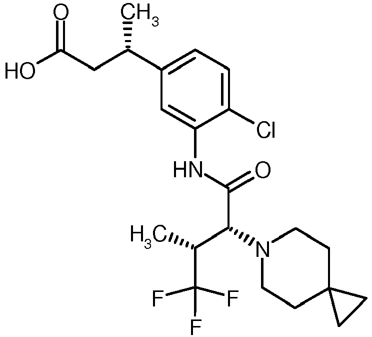

Das oben erhaltene Gemisch von (3*S*)-3-(3-{[(2*R*,3*R*)-2-(6-Azaspiro[2.5]oct-6-yl)-4,4,4-trifluor-3-methylbutanoyl]amino}-4-chlorphenyl)butansäure und (3*S*)-3-(3-{[(2*S*,3*S*)-2-(6-Azaspiro[2.5]oct-6-yl)-4,4,4-trifluor-3-methylbutanoyl]amino}-4-chlorphenyl)butansäure (Beispiel 21, Diastereomerengemisch 1) wurde durch präparative HPLC an chiraler Phase aufgetrennt [Säule: Daicel Chiralpak AS-H, 5 µm, 250 mm x 20 mm; Injektionsvolumen: 1.0 ml; Temperatur: RT; Eluent: 10% Isohexan / 90% Isopropanol; Fluss: 20 ml/min; Detektion: 230 nm]. Ausgehend von 252 mg Diastereomerengemisch wurden 104 mg der Titelverbindung als Diastereomer 1 isoliert.

LC-MS (Methode 4): Rₜ = 2.61 min; m/z = 461 (M+H)⁺.

¹H-NMR (400 MHz, DMSO-*d₆*): δ [ppm] = 0.21 (s, 4H), 1.14 (d, 3H), 1.20 (d, 3H), 1.22-1.40 (m, 4H), 2.50-2.58 (m, ca. 4H, verdeckt), 2.61-2.72 (m, 2H), 2.99-3.19 (m, 2H), 3.56 (d, 1H), 7.13 (dd, 1H), 7.43 (d, 1H), 7.48 (d, 1H), 9.64 (s, 1H), 12.10 (br. s, 1H).

[α]_{D}²⁰ = -17.7°, c = 0.440, Chloroform.

### Beispiel 51

### 1-(4-Chlor-3-{[(2R,3R)-4,4,4-trifluor-3-methyl-2-(4-methylpiperidin-1-yl)butanoyl]amino}benzyl)-cyclopropancarbonsäure

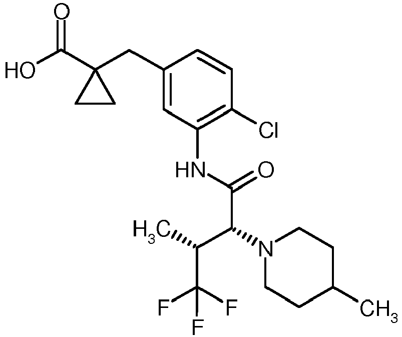

Die Titelverbindung wurde gemäß Allgemeiner Vorschrift 4 ausgehend von (+)-*tert*.-Butyl-1-(4-chlor-3-{[(3*R*)-4,4,4-trifluor-D-valyl]amino}benzyl)cyclopropancarboxylat (Beispiel 65A) und 3-Methylpentandial erhalten.

LC-MS (Methode 2): Rₜ = 1.24 min; m/z = 461 (M+H)⁺.

¹H-NMR (400 MHz, DMSO-*d₆*): δ [ppm] = 0.77-0.83 (m, 2H), 0.87 (d, 3H), 0.96-1.34 (m, 6H), 1.10 (d, 3H), 1.59 (t, 2H), 2.06 (t, 1H), 2.60-2.68 (m, 1H), 2.85 (d, 2H), 2.87-2.94 (m, 1H), 2.98-3.11 (m, 1H), 3.49 (d, 1H), 7.11 (dd, 1H), 7.39 (d, 1H), 7.48 (d, 1H), 9.62 (s, 1H), 11.85-12.54 (br. s, 1H).

[α]_{D}²⁰ = -4.7°, c = 0.325, Chloroform.

### Beispiel 52

### rac-1-(3-{{[Cyclopentyl(4-methoxypiperidin-1-yl)acetyl]amino}-4-fluorbenzyl)cyclopropan-carbonsäure-Trifluoracetat

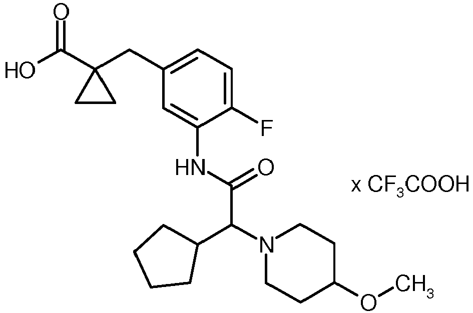

20.1 mg (0.04 mmol) *rac-tert*.-Butyl-1-(3-{[cyclopentyl(4-methoxypiperidin-1-yl)acetyl]amino}-4-fluorbenzyl)cyclopropancarboxylat (Beispiel 95A) wurden in 2.4 ml 20%-iger Trifluoressigsäure in Dichlormethan gelöst und 5 h bei RT gerührt. Danach wurde der Ansatz am Rotationsverdampfer eingeengt und der Rückstand über präparative HPLC gereinigt (RP18-Säule; Eluent: Acetonitril/Wasser-Gradient unter Zusatz von 0.1 % TFA, 10:90 → 95:5; Laufzeit 38 min). Es wurden 6.8 mg (30% d. Th.) der Titelverbindung erhalten.

LC-MS (Methode 5): Rₜ = 0.86 min; m/z = 433.1 (M+H)⁺.

¹H-NMR (400 MHz, DMSO-*d₆*): δ [ppm] = 0.80-0.86 (m, 2H), 1.10-1.16 (m, 2H), 1.35-1.73 (m, 8H), 1.79-2.07 (m, 4H), 2.08-2.21 (m, 1H), 2.86 (br. s, 2H), 3.26 (s, 3H), 3.29-3.35 (m, 2H), 4.06-4.15 (m, 1H), 7.12-7.27 (m, 2H), 7.67 (t, 1H), 9.61 (br. s, 1H), 10.43-10.51 (m, 1H) [weitere Signale unter Lösungsmittel-Peaks verborgen].

### Beispiel 53

### rac-1-(3-{[Cyclopentyl(3,4-dihydroisochinolin-2(1H)-yl)acetyl]amino}-4-fluorbenzyl)cyclopropancarbonsäure-Trifluoracetat

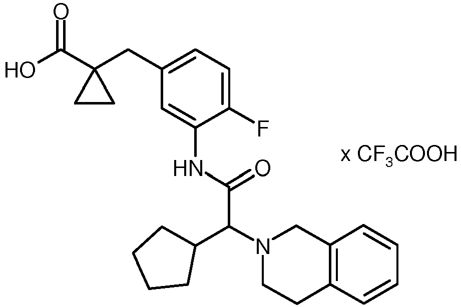

101 mg (0.2 mmol) *rac*-*tert*.-Butyl-1-(3-{[cyclopentyl(3,4-dihydroisochinolin-2(1*H*)-yl)acetyl]-amino}-4-fluorbenzyl)cyclopropancarboxylat (Beispiel 96A) wurden in 11.5 ml 20%-iger Trifluoressigsäure in Dichlormethan gelöst und 5 h bei RT gerührt. Danach wurde der Ansatz am Rotationsverdampfer eingeengt und der Rückstand über präparative HPLC gereinigt (RP18-Säule; Eluent: Acetonitril/Wasser-Gradient unter Zusatz von 0.1% TFA, 10:90 → 95:5; Laufzeit 38 min). Es wurden 14.4 mg (13% d. Th.) der Titelverbindung erhalten.

LC-MS (Methode 2): Rₜ = 0.91 min; m/z = 451.3 (M+H)⁺.

¹H-NMR (400 MHz, DMSO-*d₆*): δ [ppm] = 0.79-0.87 (m, 2H), 1.07-1.14 (m, 2H), 1.38-1.50 (m, 1H), 1.51-1.69 (m, 5H), 1.70-1.81 (m, 1H), 1.90-2.04 (m, 1H), 2.86 (s, 2H), 3.05-3.21 (m, 2H), 4.20-4.36 (m, 1H), 4.50-4.64 (m, 1H), 7.02-7.37 (m, 6H), 7.63-7.73 (m, 1H) [weitere Signale unter Lösungsmittel-Peaks verborgen].

### B. Bewertung der pharmakologischen Wirksamkeit

Die pharmakologische Wirkung der erfindungsgemäßen Verbindungen kann in folgenden Assays gezeigt werden:

### B-1. Stimulation der rekombinanten löslichen Guanylatcyclase (sGC) in vitro

Die Untersuchungen zur Stimulation der rekombinanten löslichen Guanylatcyclase (sGC) durch die erfindungsgemäßen Verbindungen mit und ohne Natriumnitroprussid sowie mit und ohne den Häm-abhängigen sGC-Inhibitor 1*H*-1,2,4-Oxadiazolo[4,3-a]chinoxalin-1-on (ODQ) werden nach der in folgender Literaturstelle im Detail beschriebenen Methode durchgeführt: M. Hoenicka, E. M. Becker, H. Apeler, T. Sirichoke, H. Schroeder, R. Gerzer und J.-P. Stasch, "Purified soluble guanylyl cyclase expressed in a baculovirus/Sf9 system: Stimulation by YC-1, nitric oxide, and carbon oxide", J. Mol. Med. 77 (1999), 14-23. Die Häm-freie Guanylatcyclase wird durch Zugabe von Tween 20 zum Probenpuffer (0.5% in der Endkonzentration) erhalten.

Die Aktivierung der sGC durch eine Prüfsubstanz wird als x-fache Stimulation der Basalaktivität angegeben. Das Ergebnis für Beispiel 25 ist in Tabelle 1 gezeigt:

**Tabelle 1: Stimulation (x-fach) der rekombinanten löslichen Guanylatcyclase (sGC) in vitro durch Beispiel 25**

| **Konzentration Beispiel 25 [µM]** | **Häm-haltige sGC** | | | **Häm-freie sGC** |
|---|---|---|---|---|
| | **Basal (n=2)** | **+0.01 µM DEA/NO** | **+ 10 µM ODQ** | **Basal (n=2)** |
| 0 | 1.0 ± 0.0 | 11.2 ± 5.0 | 3.6 ± 1.2 | 1.0 ± 0.0 |
| 0.01 | 0.9 ± 0.2 | 11.4 ± 3.9 | 2.3 ± 0.1 | 1.9 ± 0.8 |
| 0.1 | 0.9 ± 0.0 | 11.9 ± 4.2 | 2.7 ± 0.2 | 2.4 ± 0.8 |
| 1.0 | 2.8 ± 0.4 | 12.0 ± 3.9 | 10.5 ± 2.4 | 7.3 ± 3.6 |
| 10 | 8.6 ± 0.2 | 15.1 ± 2.9 | 32.5 ± 5.4 | 26.6 ± 7.3 |

[DEA/NO = 2-(*N*,*N*-Diethylamino)diazenolat-2-oxid; ODQ = 1*H*-1,2,4-Oxadiazolo[4,3-a]chinoxalin-1-on].

Aus der Tabelle 1 ist ersichtlich, dass eine Stimulation sowohl des Häm-haltigen als auch des Häm-freien Enzyms erreicht wird. Weiterhin zeigt die Kombination von Beispiel 25 mit 2-(*N*,*N-*Diethylamino)diazenolat-2-oxid (DEA/NO), einem NO-Donor, keinen synergistischen Effekt, d.h. die Wirkung von DEA/NO wird nicht potenziert, wie dies bei einem über einen Häm-abhängigen Mechanismus wirkenden sGC-Aktivator zu erwarten wäre. Darüber hinaus wird die Wirkung des erfindungsgemäßen sGC-Aktivators durch 1*H*-1,2,4-Oxadiazolo[4,3-a]chinoxalin-1-on (ODQ), einen Häm-abhängigen Inhibitor der löslichen Guanylatcyclase, nicht blockiert, sondern sogar gesteigert. Die Ergebnisse in Tabelle 1 belegen somit den Wirkmechanismus der erfindungsgemäßen Verbindungen als Aktivatoren der löslichen Guanylatcyclase.

### B-2. Wirkung an rekombinanter Guanylatcyclase-Reporterzelllinie

Die zelluläre Wirkung der erfindungsgemäßen Verbindungen wird an einer rekombinanten Guanylatcyclase-Reporterzelllinie, wie in F. Wunder et al., Anal. Biochem. 339, 104-112 (2005) beschrieben, bestimmt.

Repräsentative Ergebnisse zu den erfindungsgemäßen Verbindungen sind in Tabelle 2 aufgeführt:

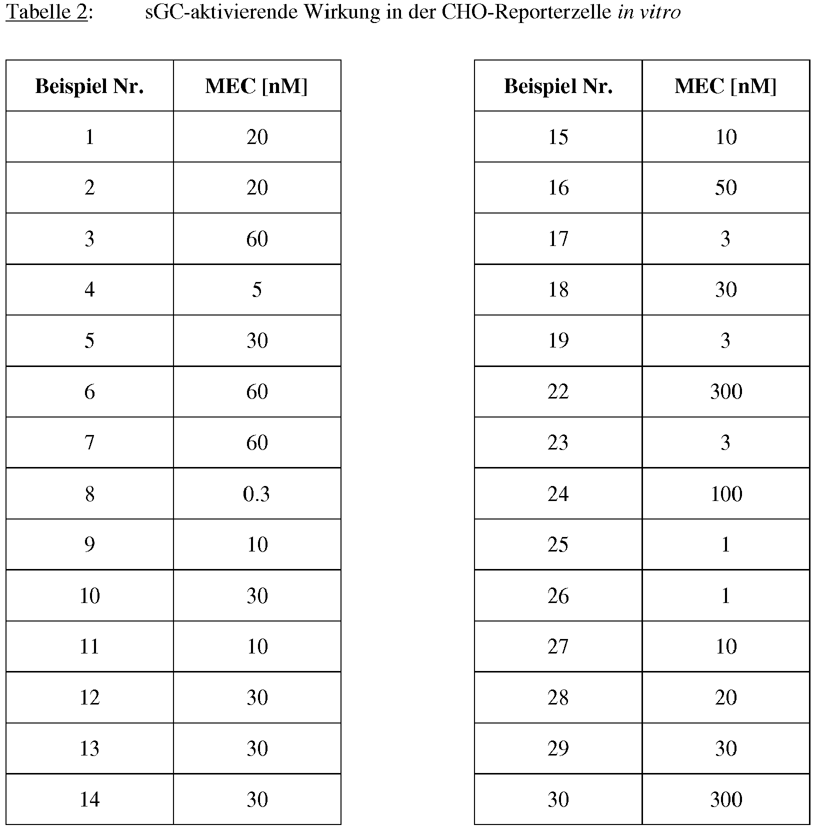

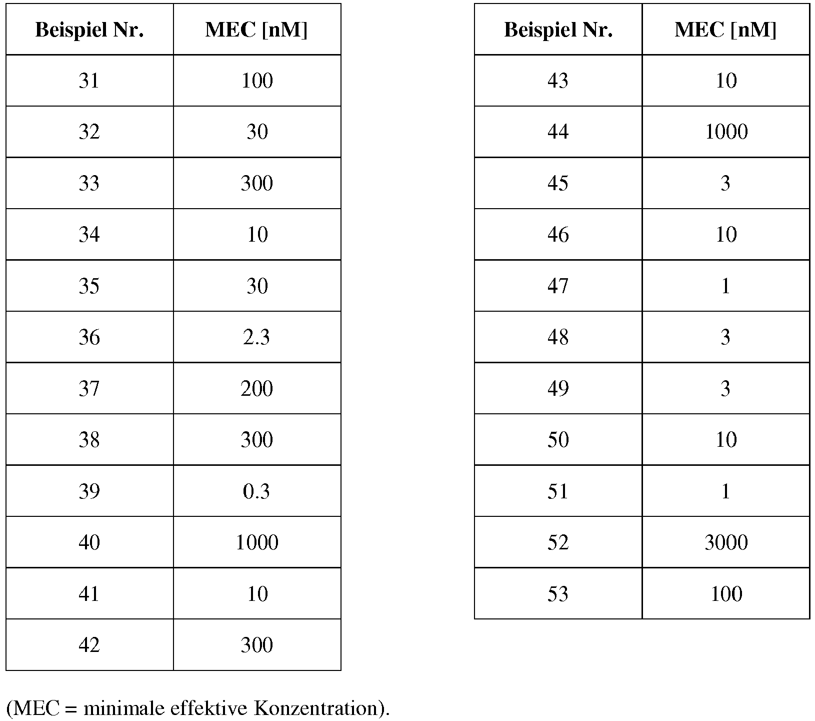

### B-3. Stimulation der sGC-Enzymaktivität

Lösliche Guanylatcyclase (sGC) setzt unter Stimulation GTP zu cGMP und Pyrophosphat (PPi) um. PPi wird mit Hilfe des nachfolgend beschriebenen Tests nachgewiesen. Das im Test entstehende Signal nimmt mit fortschreitender Umsetzung zu und dient als Maß für die sGC-Enzymaktivität unter der gegebenen Stimulation.

Zur Durchführung des Tests werden 29 µl Enzymlösung [0-10 nM lösliche Guanylatcyclase (hergestellt nach Hönicka et al., J. Mol. Med. 77, 14-23 (1999)) in 50 mM TEA, 2 mM MgCl₂, 0.1% BSA (Fraktion V), 0.005% Brij^{®}, pH 7.5] in eine Mikroplatte vorgelegt und 1 µl der zu testenden Substanz (als seriell verdünnte Lösung in DMSO) hinzugegeben. Der Ansatz wird 10 min bei Raumtemperatur inkubiert. Anschließend werden 20 µl Detektionsmix [1.2 nM Firefly-Luciferase (*Photinus* pyralis-Luciferase, Fa. Promega), 29 µM Dehydro-Luziferin (hergestellt nach Bitler & McElroy, Arch. Biochem. Biophys. 72, 358 (1957)), 122 µM Luziferin (Fa. Promega), 153 µM ATP (Fa. Sigma) und 0.4 mM DTT (Fa. Sigma) in 50 mM TEA, 2 mM MgCl₂, 0.1% BSA (Fraktion V), 0.005% Brij^{®}, pH 7.5] zugegeben. Die Enzymreaktion wird durch Zugabe von 20 µl Substratlösung [1.25 mM Guanosin-5'-triphosphat (Fa. Sigma) in 50 mM TEA, 2 mM MgCl₂, 0.1% BSA (Fraktion V), 0.005% Brij^{®}, pH 7.5] gestartet und kontinuierlich luminometrisch vermessen. Das Maß der Stimulation durch die zu testende Substanz kann relativ zum Signal der nicht stimulierten Reaktion bestimmt werden.

Durch Zugabe von 25 µM 1*H*-1,2,4-Oxadiazolo[4,3-a]chinoxalin-1-on (ODQ) zur Enzymlösung und anschließende 30-minütige Inkubation wird die Aktivierung der Häm-freien Guanylatcyclase untersucht und mit der Stimulation des nativen Enzyms verglichen.

Repräsentative Ergebnisse zu den erfindungsgemäßen Verbindungen sind in Tabelle 3 aufgeführt:

**Tabelle 3: Aktivierende Wirkung am sGC-Enzym in vitro**

| **Beispiel Nr.** | **MEC [nM]** | **EC₅₀ [nM]** |
|---|---|---|
| 8 | 1.2 | 21 |
| 11 | 17 | 390 |
| 18 | 6.9 | 540 |
| 25 | 2.2 | 15 |
| 26 | 0.8 | 10 |
| 27 | 10.5 | 210 |
| 34 | 10.5 | 90 |
| 36 | 3 | 25 |
| 39 | 1.1 | 7.9 |
| 46 | 17 | 210 |
| 47 | 3.9 | 81 |
| 48 | 1.1 | 33 |
| 51 | 1.2 | *n.d.* |
| 53 | 160 | 840 |

| | | |
|---|---|---|
| (MEC = minimale effektive Konzentration; EC₅₀ = Konzentration bei 50% der maximalen Wirksamkeit; *n.d.* = nicht bestimmt). | | |

### B-4. Gefäßrelaxierende Wirkung in vitro

Kaninchen werden durch intravenöse Injektion von Thiopental-Natrium narkotisiert bzw. getötet (ca. 50 mg/kg) und entblutet. Die *Arteria Saphena* wird entnommen und in 3 mm breite Ringe geteilt. Die Ringe werden einzeln auf je einem triangelförmigen, am Ende offenen Häkchenpaar aus 0.3 mm starkem Spezialdraht (Remanium^{®}) montiert. Jeder Ring wird unter Vorspannung in 5 ml-Organbäder mit 37°C warmer, carbogenbegaster Krebs-Henseleit-Lösung folgender Zusammensetzung gebracht: NaCl 119 mM; KCl 4.8 mM; CaCl₂ x 2 H₂O 1 mM; MgSO₄ x 7 H₂O 1.4 mM; KH₂PO₄ 1.2 mM; NaHCO₃ 25 mM; Glucose 10 mM; Rinderserumalbumin 0.001%. Die Kontraktionskraft wird mit Statham UC2-Zellen erfasst, verstärkt und über A/D-Wandler (DAS-1802 HC, Keithley Instruments, München) digitalisiert sowie parallel auf Linienschreibern registriert. Kontraktionen werden durch Zugabe von Phenylephrin induziert.

Nach mehreren (allgemein 4) Kontrollzyklen wird die zu untersuchende Substanz in jedem weiteren Durchgang in steigender Dosierung zugesetzt und die Höhe der unter dem Einfluss der Testsubstanz erzielten Kontraktion mit der Höhe der im letzten Vordurchgang erreichten Kontraktion verglichen. Daraus wird die Konzentration errechnet, die erforderlich ist, um die in der Vorkontrolle erreichte Kontraktion auf 50% zu reduzieren (IC₅₀-Wert). Das Standard-Applikationsvolumen beträgt 5 µl. Der DMSO-Anteil in der Badlösung entspricht 0.1%.

### B-5. Radiotelemetrische Messung von Blutdruck und Herzfrequenz an wachen SH-Ratten

Für die im Folgenden beschriebenen Messungen an wachen SH-Ratten wird ein im Handel erhältliches Telemetriesystem der Firma Data Sciences International DSI, USA, eingesetzt.

Das System besteht aus 3 Hauptkomponenten: (*1*) Implantierbare Sender, (*2*) Empfänger, die über einen Multiplexer mit einem (*3*) Datenakquisitionscomputer verbunden sind. Die Telemetrieanlage ermöglicht eine kontinuierliche Erfassung von Blutdruck und Herzfrequenz an wachen Tieren in ihrem gewohnten Lebensraum.

Die Untersuchungen werden an ausgewachsenen weiblichen, spontan-hypertensiven Ratten (SH-Ratten) mit einem Körpergewicht von >200 g durchgeführt. Die Versuchstiere werden nach Senderimplantation einzeln in Makrolon-Käfigen Typ 3 gehalten. Sie haben freien Zugang zu Standardfutter und Wasser. Der Tag/Nacht-Rhythmus im Versuchslabor wird per Raumbeleuchtung um 6:00 Uhr morgens und um 19:00 Uhr abends gewechselt.

Die eingesetzten Telemetriesender (TAM PA-C40, DSI) werden den Versuchstieren mindestens 14 Tage vor dem ersten Versuchseinsatz unter aseptischen Bedingungen chirurgisch implantiert. Die so instrumentierten Tiere sind nach Abheilen der Wunde und Einwachsen des Implantats wiederholt einsetzbar.

Zur Implantation werden die nüchternen Tiere mit Pentobarbital (Nembutal, Sanofi, 50 mg/kg i.p.) narkotisiert und an der Bauchseite weiträumig rasiert und desinfiziert. Nach Eröffnung des Bauchraumes entlang der Linea alba wird der flüssigkeitsgefüllte Messkatheter des Systems oberhalb der Bifurcation nach cranial in die Aorta descendens eingesetzt und mit Gewebekleber (VetBonD™, 3M) befestigt. Das Sendergehäuse wird intraperitoneal an der Bauchwandmuskulatur fixiert und die Wunde schichtweise verschlossen. Postoperativ wird zur Infektionsprophylaxe ein Antibiotikum verabreicht (Tardomyocel COMP, Bayer AG, 1 ml/kg s.c.).

### Versuchsablauf:

Die zu untersuchenden Substanzen werden jeweils einer Gruppe von Tieren (n = 6) per Schlundsonde oral verabreicht. Entsprechend einem Applikationsvolumen von 5 ml/kg Körpergewicht werden die Testsubstanzen in geeigneten Lösungsmittelgemischen gelöst oder in 0.5%-iger Tylose suspendiert. Eine Lösungsmittel-behandelte Gruppe von Tieren wird als Kontrolle eingesetzt.

Die Telemetrie-Messeinrichtung ist für 24 Tiere konfiguriert. Jeder Versuch wird unter einer Versuchsnummer registriert.

Den in der Anlage lebenden instrumentierten Ratten ist jeweils eine eigene Empfangsantenne zugeordnet (1010 Receiver, DSI). Die implantierten Sender sind über einen eingebauten Magnetschalter von außen aktivierbar und werden bei Versuchsvorlauf auf Sendung geschaltet. Die ausgestrahlten Signale können durch ein Datenakquisitionssystem (Dataquest™ A.R.T. for Windows, DSI) online erfasst und entsprechend aufgearbeitet werden. Die Ablage der Daten erfolgt jeweils in einem hierfür eröffneten Ordner, der die Versuchsnummer trägt.

Im Standardablauf werden über je 10 Sekunden Dauer gemessen: (*1*) systolischer Blutdruck (SBP), (2) diastolischer Blutdruck (DBP), (*3*) arterieller Mitteldruck (MAP) und (*4*) Herzfrequenz (HR).

Die Messwerterfassung wird rechnergesteuert in 5 Minuten-Abständen wiederholt. Die als Absolutwert erhobenen Quelldaten werden im Diagramm mit dem aktuell gemessenen Barometerdruck korrigiert und in Einzeldaten abgelegt. Weitere technische Details sind in der Dokumentation der Herstellerfirma (DSI) aufgeführt.

Die Verabreichung der Prüfsubstanzen erfolgt am Versuchstag um 9:00 Uhr. Im Anschluss an die Applikation werden die oben beschriebenen Parameter über 24 Stunden gemessen. Nach Versuchsende werden die erhobenen Einzeldaten mit der Analysis-Software (Dataquest™ A.R.T. Analysis) sortiert. Als Leerwert wird der Zeitpunkt 2 Stunden vor Substanz-Applikation angenommen, so dass der selektierte Datensatz den Zeitraum von 7:00 Uhr am Versuchstag bis 9:00 Uhr am Folgetag umfasst.

Die Daten werden über eine voreinstellbare Zeit durch Mittelwertbestimmung geglättet (15 Minuten-Mittelwert, 30 Minuten-Mittelwert) und als Textdatei auf einen Datenträger übertragen. Die so vorsortierten und komprimierten Messwerte werden in Excel-Vorlagen übertragen und tabellarisch dargestellt.

### C. Ausführungsbeispiele für pharmazeutische Zusammensetzungen

Die erfindungsgemäßen Verbindungen können folgendermaßen in pharmazeutische Zubereitungen überführt werden:

### Tablette:

### Zusammensetzung:

100 mg der erfindungsgemäßen Verbindung, 50 mg Lactose (Monohydrat), 50 mg Maisstärke (nativ), 10 mg Polyvinylpyrrolidon (PVP 25) (Fa. BASF, Ludwigshafen, Deutschland) und 2 mg Magnesiumstearat.

Tablettengewicht 212 mg. Durchmesser 8 mm, Wölbungsradius 12 mm.

### Herstellung:

Die Mischung aus erfindungsgemäßer Verbindung, Lactose und Stärke wird mit einer 5%-igen Lösung (m/m) des PVPs in Wasser granuliert. Das Granulat wird nach dem Trocknen mit dem Magnesiumstearat 5 Minuten gemischt. Diese Mischung wird mit einer üblichen Tablettenpresse verpresst (Format der Tablette siehe oben). Als Richtwert für die Verpressung wird eine Presskraft von 15 kN verwendet.

### Oral applizierbare Suspension:

### Zusammensetzung:

1000 mg der erfindungsgemäßen Verbindung, 1000 mg Ethanol (96%), 400 mg Rhodigel^{®} (Xanthan gum der Firma FMC, Pennsylvania, USA) und 99 g Wasser.

Einer Einzeldosis von 100 mg der erfindungsgemäßen Verbindung entsprechen 10 ml orale Suspension.

### Herstellung:

Das Rhodigel wird in Ethanol suspendiert, die erfindungsgemäße Verbindung wird der Suspension zugefügt. Unter Rühren erfolgt die Zugabe des Wassers. Bis zum Abschluß der Quellung des Rhodigels wird ca. 6 h gerührt.

### Oral applizierbare Lösung:

### Zusammensetzung:

500 mg der erfindungsgemäßen Verbindung, 2.5 g Polysorbat und 97 g Polyethylenglycol 400. Einer Einzeldosis von 100 mg der erfindungsgemäßen Verbindung entsprechen 20 g orale Lösung.

### Herstellung:

Die erfindungsgemäße Verbindung wird in der Mischung aus Polyethylenglycol und Polysorbat unter Rühren suspendiert. Der Rührvorgang wird bis zur vollständigen Auflösung der erfindungsgemäßen Verbindung fortgesetzt.

### i.v.-Lösung:

Die erfindungsgemäße Verbindung wird in einer Konzentration unterhalb der Sättigungslöslichkeit in einem physiologisch verträglichen Lösungsmittel (z.B. isotonische Kochsalzlösung, Glucoselösung 5% und/oder PEG 400-Lösung 30%) gelöst. Die Lösung wird steril filtriert und in sterile und pyrogenfreie Injektionsbehältnisse abgefüllt.

## Patentansprüche

1. Verbindung der Formel (I) 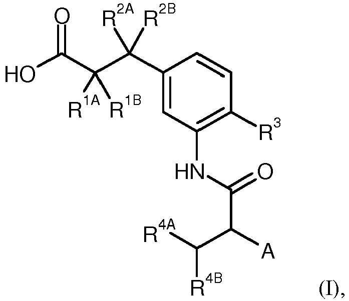 in welcher
R^{1A} und R^{1B} unabhängig voneinander für Wasserstoff, Methyl, Ethyl oder *n*-Propyl stehen
oder
miteinander verknüpft sind und gemeinsam mit dem Kohlenstoffatom, an das sie gebunden sind, einen Cyclopropyl- oder Cyclobutyl-Ring bilden,
R^{2A} für Wasserstoff, Methyl, Ethyl, *n*-Propyl, Isopropyl, Cyclopropyl, Methoxy, Ethoxy oder Cyclopropyloxy steht,
R^{2B} für Wasserstoff oder Methyl steht,
oder
R^{2A} und R^{2B} miteinander verknüpft sind und gemeinsam mit dem Kohlenstoffatom, an das sie gebunden sind, einen Cyclopropyl- oder Cyclobutyl-Ring bilden,
R³ für Wasserstoff, Fluor, Chlor, Methyl, Trifluormethyl oder Ethyl steht,
R^{4A} und R^{4B} unabhängig voneinander für Methyl, Trifluormethyl oder Ethyl stehen
oder
miteinander verknüpft sind und gemeinsam mit dem Kohlenstoffatom, an das sie gebunden sind, einen Cyclopropyl-, Cyclobutyl- oder Cyclopentyl-Ring bilden, der bis zu zweifach mit Fluor substituiert sein kann,
und
A für einen optional substituierten oder anellierten Piperidin-Ring der Formel 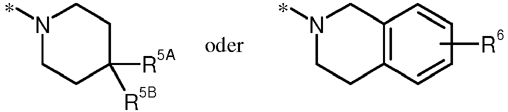 steht, worin
* die Verknüpfungsstelle mit dem Rest des Moleküls bezeichnet,
R^{5A} Wasserstoff, (C₁-C₄)-Alkyl, Cyclopropyl, Cyclobutyl, Methoxy, Ethoxy oder Phenyl bedeutet, wobei (C₁-C₄)-Alkyl seinerseits bis zu dreifach mit Fluor substituiert sein kann,
R^{5B} Wasserstoff oder Methyl bedeutet,
oder
R^{5A} und R^{5B} miteinander verknüpft sind und gemeinsam mit dem Kohlenstoffatom, an das sie gebunden sind, einen Cyclopropyl- oder Cyclobutyl-Ring bilden,
und
R⁶ Wasserstoff, Fluor, Chlor, Methyl oder Trifluormethyl bedeutet,
sowie ihre Salze, Solvate und Solvate der Salze.

2. Verbindung der Formel (I) nach Anspruch 1, in welcher
R^{1A} für Wasserstoff oder Methyl steht,
R^{1B} für Wasserstoff steht,
oder
R^{1A} und R^{1B} miteinander verknüpft sind und gemeinsam mit dem Kohlenstoffatom, an das sie gebunden sind, einen Cyclopropyl- oder Cyclobutyl-Ring bilden,
R^{2A} für Wasserstoff, Methyl, Ethyl, Isopropyl oder Cyclopropyl steht,
R^{2B} für Wasserstoff steht,
oder
R^{2A} und R^{2B} miteinander verknüpft sind und gemeinsam mit dem Kohlenstoffatom, an das sie gebunden sind, einen Cyclopropyl-Ring bilden,
R³ für Fluor, Chlor oder Methyl steht,
R^{4A} für Methyl steht,
R^{4B} für Trifluormethyl steht,
oder
R^{4A} und R^{4B} miteinander verknüpft sind und gemeinsam mit dem Kohlenstoffatom, an das sie gebunden sind, einen Cyclopentyl-Ring bilden, der bis zu zweifach mit Fluor substituiert sein kann,
und
A für einen optional substituierten oder anellierten Piperidin-Ring der Formel 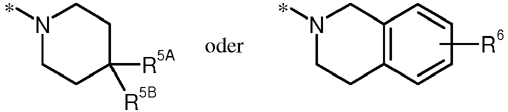 steht, worin
* die Verknüpfungsstelle mit dem Rest des Moleküls bezeichnet,
R^{5A} Wasserstoff, Methyl, Trifluormethyl, Ethyl, Isopropyl oder Cyclopropyl bedeutet,
R^{5B} Wasserstoff bedeutet,
oder
R^{5A} und R^{5B} miteinander verknüpft sind und gemeinsam mit dem Kohlenstoffatom, an das sie gebunden sind, einen Cyclopropyl-Ring bilden,
und
R⁶ Wasserstoff oder Fluor bedeutet,
sowie ihre Salze, Solvate und Solvate der Salze.

3. Verbindung der Formel (I) nach Anspruch 1 oder 2, in welcher
R^{1A} für Wasserstoff oder Methyl steht,
R^{1B} für Wasserstoff steht,
oder
R^{1A} und R^{1B} miteinander verknüpft sind und gemeinsam mit dem Kohlenstoffatom, an das sie gebunden sind, einen Cyclopropyl-Ring bilden,
R^{2A} für Wasserstoff oder Methyl steht,
R^{2B} für Wasserstoff steht,
R³ für Fluor oder Chlor steht,
R^{4A} für Methyl steht,
R^{4B} für Trifluormethyl steht,
und
A für einen optional substituierten Piperidin-Ring der Formel 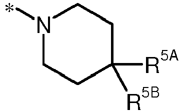 steht, worin
* die Verknüpfungsstelle mit dem Rest des Moleküls bezeichnet,
R^{5A} Wasserstoff, Methyl, Trifluormethyl oder Ethyl bedeutet,
R^{5B} Wasserstoff bedeutet,
oder
R^{5A} und R^{5B} miteinander verknüpft sind und gemeinsam mit dem Kohlenstoffatom, an das sie gebunden sind, einen Cyclopropyl-Ring bilden,
sowie ihre Salze, Solvate und Solvate der Salze.

4. Verfahren zur Herstellung einer Verbindung der Formel (I), wie in den Ansprüchen 1 bis 3 definiert, **dadurch gekennzeichnet, dass** man eine geschützte α-Aminocarbonsäure der Formel (II) 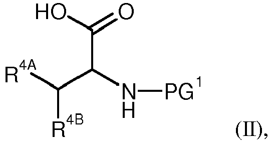 in welcher R^{4A} und R^{4B} die in den Ansprüchen 1 bis 3 angegebenen Bedeutungen haben
und
PG¹ für eine geeignete Amino-Schutzgruppe wie beispielsweise Allyloxycarbonyl (Alloc), Benzyloxycarbonyl (Z), *tert*.-Butoxycarbonyl (Boc) oder 9-Fluorenylmethoxycarbonyl (Fmoc) steht,
in einem inerten Lösungsmittel mit Hilfe eines Kondensationsmittels in Gegenwart einer Base mit einem Amin der Formel (III) 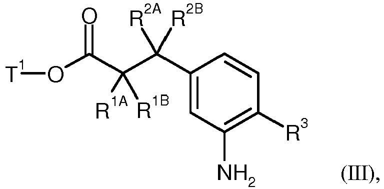 in welcher R^{1A}, R^{1B} , R^{2A}, R^{2B} und R³ die in den Ansprüchen 1 bis 3 angegebenen Bedeutungen haben
und
T¹ für (C₁-C₄)-Alkyl oder Benzyl steht,
zu einem Carbonsäureamid der Formel (IV) 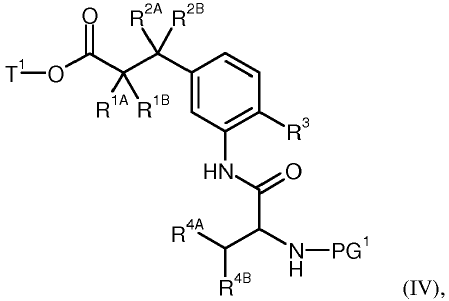 in welcher PG¹, R^{1A}, R^{1B}, R^{2A}, R^{2B}, R³, R^{4A}, R^{4B} und T¹ die oben angegebenen Bedeutungen haben,
kuppelt, anschließend durch Abspaltung der Schutzgruppe PG¹ die Amin-Verbindung (V) 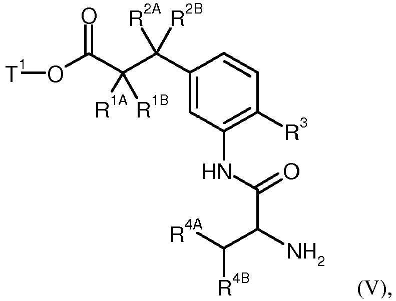 in welcher R^{1A}, R^{1B}, R^{2A}, R^{2B}, R³, R^{4A}, R^{4B} und T¹ die oben angegebenen Bedeutungen haben,
freisetzt, diese dann in Gegenwart eines geeigneten Reduktionsmittels mit einem Dialdehyd der Formel (VI) 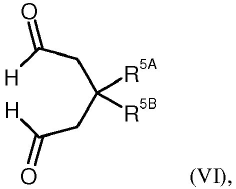 in welcher R^{5A} und R^{5B} die in den Ansprüchen 1 bis 3 angegebenen Bedeutungen haben,
zu einem Gemisch (mit variierenden Anteilen) der beiden Cyclisierungsprodukte (VII) und (VIII) 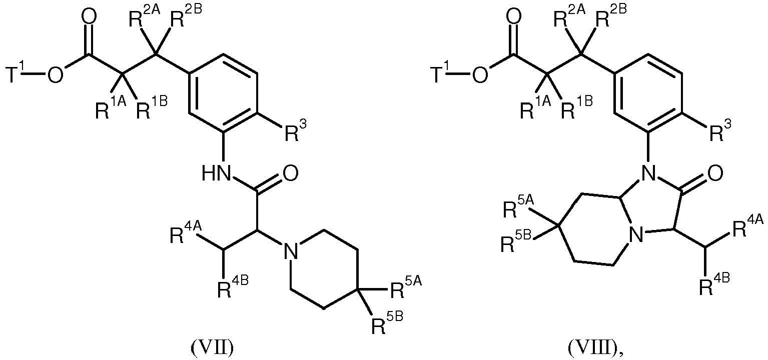 in welchen R^{1A}, R^{1B}, R^{2A}, R^{2B}, R³, R^{4A}, R^{4B}, R^{5A}, R^{5B} und T¹ die oben angegebenen Bedeutungen haben,
umsetzt, dieses Gemisch danach mit einem Überschuss an Triethylsilan in Trifluoressigsäure behandelt, um so auch das "falsche" Cyclisierungsprodukt (VIII) in die gewünschte Zielverbindung (VII) umzuwandeln, und schließlich den Ester-Rest T¹ durch basische oder saure Solvolyse oder im Fall, dass T¹ für Benzyl steht, auch durch Hydrogenolyse unter Erhalt der Carbonsäure der Formel (I-A) 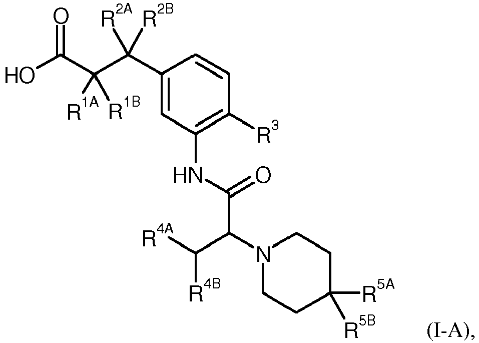 in welcher R^{1A}, R^{1B}, R^{2A}, R^{2B}, R³, R^{4A}, R^{4B}, R^{5A} und R^{5B} die oben angegebenen Bedeutungen haben,
abspaltet
und gegebenenfalls die so hergestellten erfindungsgemäßen Verbindungen der Formel (I-A) in ihre Enantiomere und/oder Diastereomere trennt und/oder mit den entsprechenden (*i*) Lösungsmitteln und/oder (*ii*) Säuren oder Basen in ihre Solvate, Salze und/oder Solvate der Salze überführt.

5. Verbindung, wie in einem der Ansprüche 1 bis 3 definiert, zur Behandlung und/oder Prävention von Krankheiten.

6. Verwendung einer Verbindung, wie in einem der Ansprüche 1 bis 3 definiert, zur Herstellung eines Arzneimittels zur Behandlung und/oder Prävention von Herzinsuffizienz, Angina pectoris, Hypertonie, pulmonaler Hypertonie, thromboembolischen Erkrankungen, Ischämien, Gefäßerkrankungen, Mikrozirkulationsstörungen, Niereninsuffizienz, fibrotischen Erkrankungen und Arteriosklerose.

7. Arzneimittel enthaltend eine Verbindung, wie in einem der Ansprüche 1 bis 3 definiert, in Kombination mit einem oder mehreren inerten, nicht-toxischen, pharmazeutisch geeigneten Hilfsstoffen.

8. Arzneimittel enthaltend eine Verbindung, wie in einem der Ansprüche 1 bis 3 definiert, in Kombination mit einem oder mehreren weiteren Wirkstoffen ausgewählt aus der Gruppe bestehend aus organischen Nitraten, NO-Donatoren, cGMP-PDE-Inhibitoren, Stimulatoren der Guanylatcyclase, antithrombotisch wirkenden Mitteln, den Blutdruck senkenden Mitteln sowie den Fettstoffwechsel verändernden Mitteln.

9. Arzneimittel nach Anspruch 7 oder 8 zur Behandlung und/oder Prävention von Herzinsuffizienz, Angina pectoris, Hypertonie, pulmonaler Hypertonie, thromboembolischen Erkrankungen, Ischämien, Gefäßerkrankungen, Mikrozirkulationsstörungen, Niereninsuffizienz, fibrotischen Erkrankungen und Arteriosklerose.

## Claims

1. Compound of the formula (I) 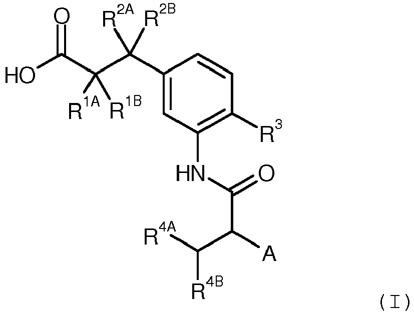 in which
R^{1A} and R^{1B} independently of one another represent hydrogen, methyl, ethyl or *n*-propyl or are attached to one another and together with the carbon atom to which they are attached form a cyclopropyl or cyclobutyl ring,
R^{2A} represents hydrogen, methyl, ethyl, *n*-propyl, isopropyl, cyclopropyl, methoxy, ethoxy or cyclopropyloxy,
R^{2B} represents hydrogen or methyl,
or
R^{2A} and R^{2B} are attached to one another and together with the carbon atom to which they are attached form a cyclopropyl or cyclobutyl ring,
R³ represents hydrogen, fluorine, chlorine, methyl, trifluoromethyl or ethyl,
R^{4A} and R^{4B} independently of one another represent methyl, trifluoromethyl or ethyl
or
are attached to one another and together with the carbon atom to which they are attached form a cyclopropyl, cyclobutyl or cyclopentyl ring which may be substituted up to two times by fluorine
and
A represents an optionally substituted or fused piperidine ring of the formula 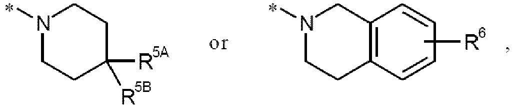 wherein * denotes the point of attachment to the remainder of the molecule,
R^{5A} represents hydrogen, (C₁-C₄)-alkyl, cyclopropyl, cyclobutyl, methoxy, ethoxy or phenyl, where (C₁-C₄)-alkyl for its part may be substituted up to three times by fluorine,
R^{5B} represents hydrogen or methyl,
or
R^{5A} and R^{5B} are attached to one another and together with the carbon atom to which they are attached form a cyclopropyl or cyclobutyl ring,
and
R⁶ represents hydrogen, fluorine, chlorine, methyl or trifluoromethyl,
and their salts, solvates and solvates of the salts.

2. Compound of the formula (I) according to Claim 1 in which
R^{1A} represents hydrogen or methyl,
R^{1B} represents hydrogen,
or
R^{1A} and R^{1B} are attached to one another and together with the carbon atom to which they are attached form a cyclopropyl or cyclobutyl ring,
R^{2A} represents hydrogen, methyl, ethyl, isopropyl or cyclopropyl,
R^{2B} represents hydrogen,
or
R^{2A} and R^{2B} are attached to one another and together with the carbon atom to which they are attached form a cyclopropyl ring,
R³ represents fluorine, chlorine or methyl,
R^{4A} represents methyl,
R^{4B} represents trifluoromethyl,
or
R^{4A} and R^{4B} are attached to one another and together with the carbon atom to which they are attached form a cyclopentyl ring which may be substituted up to two times by fluorine,
and
A represents an optionally substituted or fused piperidine ring of the formula 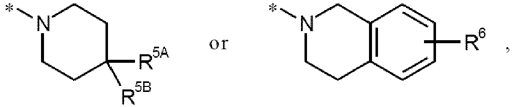 wherein * denotes the point of attachment to the remainder of the molecule,
R^{5A} represents hydrogen, methyl, trifluoromethyl, ethyl, isopropyl or cyclopropyl,
R^{5B} represents hydrogen
or
R^{5A} and R^{5B} are attached to one another and together with the carbon atom to which they are attached form a cyclopropyl ring,
and
R⁶ represents hydrogen or fluorine,
and their salts, solvates and solvates of the salts.

3. Compound of the formula (I) according to Claim 1 or 2 in which
R^{1A} represents hydrogen or methyl,
R^{1B} is hydrogen,
or
R^{1A} and R^{1B} are attached to one another and together with the carbon atom to which they are attached form a cyclopropyl ring,
R^{2A} represents hydrogen or methyl,
R^{2B} represents hydrogen,
R³ represents fluorine or chlorine,
R^{4A} represents methyl,
R^{4B} represents trifluoromethyl,
and
A represents an optionally substituted piperidine ring of the formula 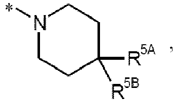 wherein * denotes the point of attachment to the remainder of the molecule,
R^{5A} represents hydrogen, methyl, trifluoromethyl or ethyl,
R^{5B} represents hydrogen
or
R^{5A} and R^{5B} are attached to one another and together with the carbon atom to which they are attached form a cyclopropyl ring,
and their salts, solvates and solvates of the salts.

4. Process for preparing a compound of the formula (I) as defined in Claims 1 to 3, **characterized in that** a protected α-aminocarboxylic acid of the formula (II) 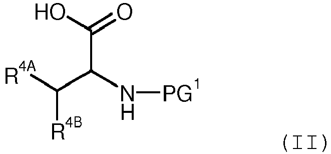 in which R^{4A} and R^{4B} have the meanings given in Claims 1 to 3
and
PG¹ represents a suitable amino protective group such as, for example, allyloxycarbonyl (Alloc), benzyloxycarbonyl (Z), *tert-*butoxycarbonyl (Boc) or 9-fluorenylmethoxycarbonyl (Fmoc),
is coupled in an inert solvent with the aid of a condensing agent in the presence of a base with an amine of the formula (III) 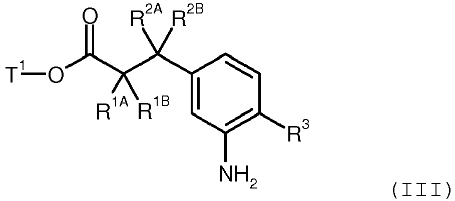 in which R^{1A}, R^{1B}, R^{2A}, R^{2B} and R³ have the meanings given in Claims 1 to 3
and
T¹ represents (C₁-C₄)-alkyl or benzyl,
to give a carboxamide of the formula (IV) 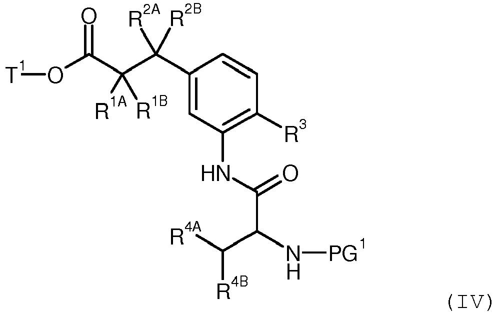 in which PG¹, R^{1A}, R^{1B}, R^{2A}, R^{2B}, R³, R^{4A}, R^{4B} and T¹ have the meanings given above,
then, by removal of the protective group PG¹, the amine compound (V) 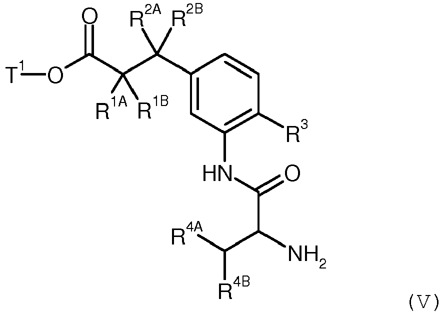 in which R^{1A}, R^{1B}, R^{2A}, R^{2B}, R³, R^{4A}, R^{4B} and T¹ have the meanings given above
is released, this is then reacted in the presence of a suitable reducing agent with a dialdehyde of the formula (VI) 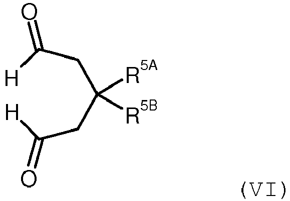 in which R^{5A} and R^{5B} have the meanings given in Claims 1 to 3,
to give a mixture (with varying proportions) of the two cyclization products (VII) and (VIII) 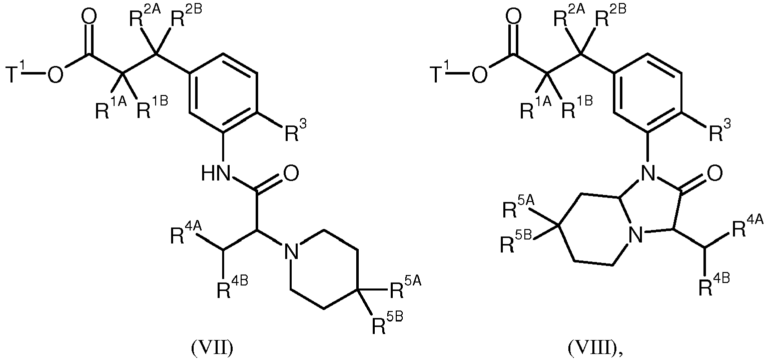 in which R^{1A}, R^{1B}, R^{2A}, R^{2B}, R³, R^{4A}, R^{4B}, R^{5A}, R^{5B} and T¹ have the meanings given above,
this mixture is then treated with an excess of triethylsilane in trifluoroacetic acid so that the "wrong" cyclization product (VIII) is also converted into the desired target compound (VII), and the ester radical T¹ is finally removed by basic or acidic solvolysis or, in the case that T¹ represents benzyl, also by hydrogenolysis, giving the carboxylic acid of the formula (I-A) 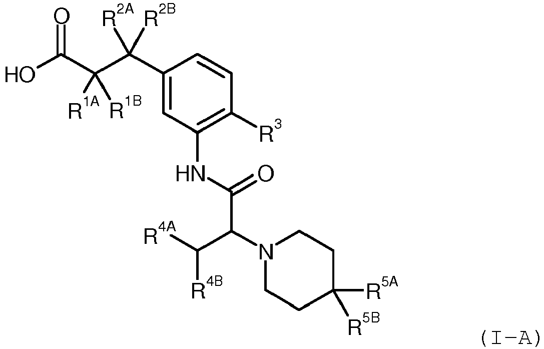 in which R^{1A}, R^{1B}, R^{2A}, R^{2B}, R³, R^{4A}, R^{4B}, R^{5A} and R^{5B} have the meanings given above
and the resulting compounds of the formula (I-A) according to the invention are optionally separated into their enantiomers and/or diastereomers and/or converted using the appropriate (i) solvents and/or (ii) bases or acids into the solvates, salts and/or solvates of the salts thereof.

5. Compound as defined in any of Claims 1 to 3 for treatment and/or prevention of diseases.

6. Use of a compound as defined in any of Claims 1 to 3 for production of a medicament for treatment and/or prevention of heart failure, angina pectoris, hypertension, pulmonary hypertension, thromboembolic disorders, ischaemias, vascular disorders, impaired microcirculation, renal insufficiency, fibrotic disorders and arteriosclerosis.

7. Medicament comprising a compound as defined in any of Claims 1 to 3 in combination with one or more inert, nontoxic, pharmaceutically suitable excipients.

8. Medicament comprising a compound as defined in any of Claims 1 to 3 in combination with one or more further active compounds selected from the group consisting of organic nitrates, NO donors, cGMP-PDE inhibitors, stimulators of guanylate cyclase, antithrombotic agents, hypotensive agents and lipid metabolism modifiers.

9. Medicament according to Claim 7 or 8 for the treatment and/or prevention of heart failure, angina pectoris, hypertension, pulmonary hypertension, thromboembolic disorders, ischaemias, vascular disorders, impaired microcirculation, renal insufficiency, fibrotic disorders and arteriosclerosis.

## Revendications

1. Composé de formule (I) 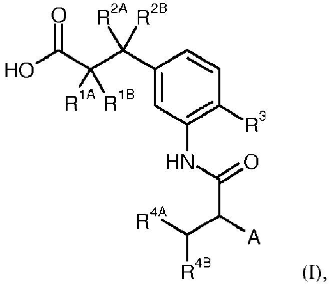 dans laquelle
R^{1A} et R^{1B} représentent, indépendamment l'un de l'autre, hydrogène, méthyle, éthyle ou n-propyle ou sont liés l'un à l'autre et forment, ensemble avec l'atome de carbone auquel ils sont liés, un cycle cyclopropyle ou cyclobutyle,
R^{2A} représente hydrogène, méthyle, éthyle, n-propyle, isopropyle, cyclopropyle, méthoxy, éthoxy ou cyclopropyloxy,
R^{2B} représente hydrogène ou méthyle, ou
R^{2A} et R^{2B} sont liés l'un à l'autre et forment, ensemble avec l'atome de carbone auquel ils sont liés, un cycle cyclopropyle ou cyclobutyle,
R³ représente hydrogène, fluor, chlore, méthyle, trifluorométhyle ou éthyle,
R^{4A} et R^{4B} représentent, indépendamment l'un de l'autre, méthyle, trifluorométhyle ou éthyle ou
sont liés l'un à l'autre et forment, ensemble avec l'atome de carbone auquel ils sont liés, un cycle cyclopropyle, cyclobutyle ou cyclopentyle, qui peut être jusqu'à disubstitué par fluor, et
A représente un cycle pipéridine éventuellement substitué ou annelé de formule 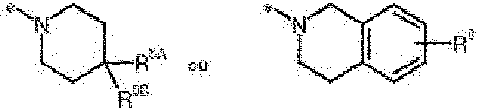 dans lesquelles
* désigne le site de liaison avec le reste de la molécule,
R^{5A} signifie hydrogène, (C₁-C₄)-alkyle, cyclopropyle, cyclobutyle, méthoxy, éthoxy ou phényle, (C₁-C₄)-alkyle pouvant à son tour être jusqu'à trisubstitué par fluor,
R^{5B} signifie hydrogène ou méthyle ou
R^{5A} et R^{5B} sont liés l'un à l'autre et forment, ensemble avec l'atome de carbone auquel ils sont liés, un cycle cyclopropyle ou cyclobutyle, et
R⁶ signifie hydrogène, fluor, chlore, méthyle ou trifluorométhyle,
ainsi que ses sels, solvates et solvates des sels.

2. Composé de formule (I) selon la revendication 1, dans laquelle
R^{1A} représente hydrogène ou méthyle,
R^{1B} représente hydrogène, ou
R^{1A} et R^{1B} sont liés l'un à l'autre et forment, ensemble avec l'atome de carbone auquel ils sont liés, un cycle cyclopropyle ou cyclobutyle,
R^{2A} représente hydrogène, méthyle, éthyle, isopropyle ou cyclopropyle,
R^{2B} représente hydrogène, ou
R^{2A} et R^{2B} sont liés l'un à l'autre et forment, ensemble avec l'atome de carbone auquel ils sont liés, un cycle cyclopropyle,
R³ représente fluor, chlore ou méthyle,
R^{4A} représente méthyle,
R^{4B} représente trifluorométhyle, ou
R^{4A} et R^{4B} sont liés l'un à l'autre et forment, ensemble avec l'atome de carbone auquel ils sont liés, un cycle cyclopentyle, qui peut être jusqu'à disubstitué par fluor, et
A représente un cycle pipéridine éventuellement substitué ou annelé des formules 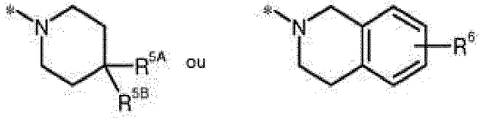 dans lesquelles
* désigne le site de liaison avec le reste de la molécule,
R^{5A} signifie hydrogène, méthyle, trifluorométhyle, éthyle, isopropyle ou cyclopropyle,
R^{5B} signifie hydrogène, ou
R^{5A} et R^{5B} sont liés l'un à l'autre et forment, ensemble avec l'atome de carbone auquel ils sont liés, un cycle cyclopropyle, et
R⁶ signifie hydrogène ou fluor,
ainsi que ses sels, solvates et solvates des sels.

3. Composé de formule (I) selon la revendication 1 ou 2, dans laquelle
R^{1A} représente hydrogène ou méthyle,
R^{1B} représente hydrogène, ou
R^{1A} et R^{1B} sont liés l'un à l'autre et forment, ensemble avec l'atome de carbone auquel ils sont liés, un cycle cyclopropyle,
R^{2A} représente hydrogène ou méthyle,
R^{2B} représente hydrogène,
R³ représente fluor ou chlore,
R^{4A} représente méthyle,
R^{4B} représente trifluorométhyle et
A représente un cycle pipéridine éventuellement substitué de formule 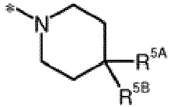 dans laquelle
* désigne le site de liaison avec le reste de la molécule,
R^{5A} signifie hydrogène, méthyle, trifluorométhyle ou éthyle,
R^{5B} signifie hydrogène, ou
R^{5A} et R^{5B} sont liés l'un à l'autre et forment, ensemble avec l'atome de carbone auquel ils sont liés, un cycle cyclopropyle,
ainsi que ses sels, solvates et solvates des sels.

4. Procédé pour la préparation d'un composé de formule (I) tel que défini dans les revendications 1 à 3, **caractérisé en ce qu'**on couple un acide α-aminocarboxylique protégé de formule (II) 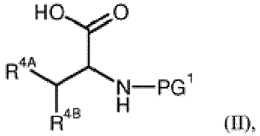 dans laquelle R^{4A} et R^{4B} présentent les significations indiquées dans les revendications 1 à 3 et
PG¹ représente un groupe de protection approprié de la fonction amino, tel que par exemple allyloxycarbonyle (Alloc), benzyloxycarbonyle (Z), tert-butoxycarbonyle (Boc) ou 9-fluorénylméthoxycarbonyle (Fmoc),
dans un solvant inerte à l'aide d'un agent de condensation en présence d'une base avec une amine de formule (III) 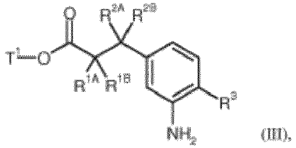 dans laquelle R^{1A}, R^{1B}, R^{2A}, R^{2B} et R³ présentent les significations indiquées dans les revendications 1 à 3 et
T¹ représente (C₁-C₄)-alkyle ou benzyle,
en un amide d'acide carboxylique de formule (IV) 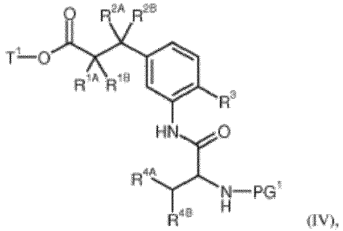 dans laquelle PG¹, R^{1A}, R^{1B}, R^{2A}, R^{2B}, R³, R^{4A}, R^{4B} et T¹ présentent les significations indiquées ci-dessus,
puis on libère le composé amine (V) par dissociation du groupe de protection PG¹ 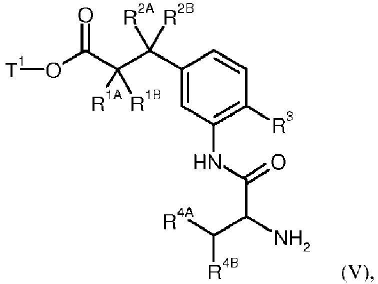 dans laquelle R^{1A}, R^{1B}, R^{2A}, R^{2B}, R³, R^{4A}, R^{4B} et T¹ présentent les significations indiquées ci-dessus,
on transforme ensuite celui-ci en présence d'un agent de réduction approprié avec un dialdéhyde de formule (VI) 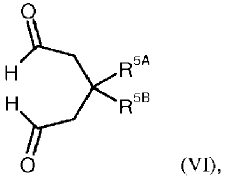 dans laquelle R^{5A} et R^{5B} présentent les significations indiquées dans les revendications 1 à 3,
en un mélange (avec des proportions variables) des deux produits de cyclisation (VII) et (VIII) 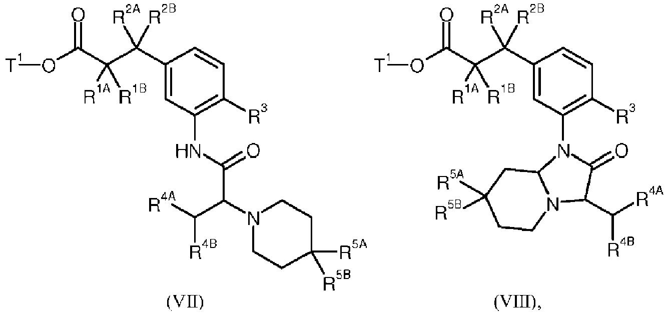 dans lesquelles R^{1A}, R^{1B}, R^{2A}, R^{2B}, R³, R^{4A}, R^{4B}, R^{5A}, R^{5B} et T¹ présentent les significations indiquées ci-dessus,
on traite ensuite ce mélange avec un excès de triéthylsilane dans de l'acide trifluoroacétique pour ainsi également transformer le "faux" produit de cyclisation (VIII) en composé cible souhaité (VII), et on dissocie enfin le radical ester T¹ par solvolyse basique ou acide ou, dans le cas où T¹ représente benzyle, également par hydrogénolyse avec obtention de l'acide carboxylique de formule (I-A) 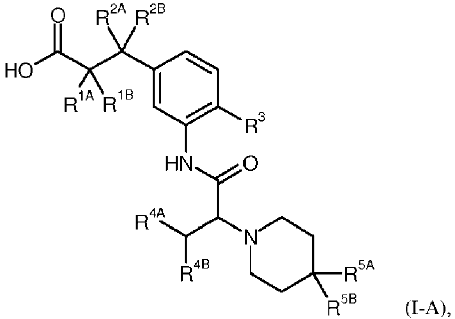 dans laquelle R^{1A}, R^{1B}, R^{2A}, R^{2B}, R³, R^{4A}, R^{4B}, R^{5A} et R^{5B} présentent les significations indiquées ci-dessus, et et on sépare le cas échéant les composés de formule (I-A) selon l'invention ainsi préparés en leurs énantiomères et/ou diastéréo-isomères et/ou on les transforme avec (i) des solvants et/ou (ii) des bases ou acides correspondants en leurs solvates, sels et/ou solvates des sels.

5. Composé, tel que défini dans l'une quelconque des revendications 1 à 3, pour le traitement et/ou la prévention de maladies.

6. Utilisation d'un composé, tel que défini dans l'une quelconque des revendications 1 à 3, pour la préparation d'un médicament pour le traitement et/ou la prévention de l'insuffisance cardiaque, de l'angine de poitrine, de l'hypertonie, de l'hypertonie pulmonaire, des maladies thromboemboliques, des ischémies, des maladies vasculaires, des troubles microcirculatoires, de l'insuffisance rénale, des maladies fibrotiques et de l'artériosclérose.

7. Médicament contenant un composé, tel que défini dans l'une quelconque des revendications 1 à 3, en combinaison avec un ou plusieurs adjuvants inertes, non toxiques, pharmaceutiquement appropriés.

8. Médicament contenant un composé tel que défini dans l'une quelconque des revendications 1 à 3, en combinaison avec une ou plusieurs autres substances actives choisies dans le groupe constitué par les nitrates organiques, les donneurs de NO, les inhibiteurs de cGMP-PDE, les stimulateurs de la guanylate-cyclase, les agents à effet antithrombotique, les agents hypotenseurs ainsi que les agents modifiant le métabolisme des lipides.

9. Médicament selon la revendication 7 ou 8 pour le traitement et/ou la prévention de l'insuffisance cardiaque, de l'angine de poitrine, de l'hypertonie, de l'hypertonie pulmonaire, des maladies thromboemboliques, des ischémies, des maladies vasculaires, des troubles microcirculatoires, de l'insuffisance rénale, des maladies fibrotiques et de l'artériosclérose.
